# EUROPEAN PATENT APPLICATION

(11) **EP 2 199 407 A1**
(43) Date of publication of application: **23.06.2010**
(21) Application number: 09154209.2
(22) Date of filing: 03.03.2009
(51) Int. Cl.: C12Q 1/68, C12N 15/11

(54) **In vitro methods and compositions for the diagnosis and/or treatment of adenocarcinoma**

(30) Priority: 10.12.2008 EP 08171210
(71) Applicant: Vereniging voor Christelijk Hoger Onderwijs, Wetenschappelijk Onderzoek en Patiëntenzorg, 1081 HV Amsterdam (NL)
(72) Inventor: The designation of the inventor has not yet been filed
(74) Representative: Jungen, Carmen

(57) **Abstract**

The present invention relates to *in vitro* methods and compositions for diagnosing, preventing and/or treating an adenocarcinoma associated with a chromosomal aberration on chromosome 20q and/or the predisposition for developing such an adenocarcinoma by determining the expression levels of a set of particular marker genes, wherein an elevated expression level of said marker genes in target cells as compared to control cells is indicative of such an adenocarcinoma and by specifically modifying the gene expression of said marker genes in such way that the elevated expression level(s) identified in the target cells are down-regulated.

SEQUENCE LISTING

<110>  Philips

<120>  IN VITRO METHODS AND COMPOSITIONS FOR THE
DIAGNOSIS AND/OR
       TREATMENT OF ADENOCARCINOMA

<130>  PH012910 EP1

<160>  62

<170>  PatentIn version 3.3

<210>  1
<211>  2401
<212>  DNA
<213>  Homo sapiens

<400>  1
gcagcgccgg tcgggagcgc agcgcggcgc agctcggcgc
gcacggcggg agcggcgcgc     60

gagtggtcgg gcctggcggc tggacgggcg cccctcgctg
ccccgcgcgc tccccgccgc    120

cccccatgag cgcagccccg cgcggcccgg gtccgtaggc
ggcggggcgc cccccatgct    180

gctgcagccc gcgccgtgcg ccccgagcgc gggcttcccg
cggcccctgg ccgcccccgg    240

cgccatgcac ggctcgcaga aggacaccac gttcaccaag
atcttcgtgg gcggcctgcc    300

gtaccacact accgacgcct cgctcaggaa gtacttcgag
ggcttcggcg acatcgagga    360

ggccgtggtc atcaccgacc gccagacggg caagtcccgc
ggctacggct tcgtgaccat    420

ggccgaccgg gcggcagctg agagggcttg caaagacccg
aaccccatca tcgacggccg    480

caaggccaac gtgaacctgg catatctggg cgccaagccg
cggagcctcc agacgggctt    540

tgccattggg gtgcagcagc tgcaccccac cttgatccag
cggacttacg ggctgacccc    600

gcactacatc tacccaccag ccatcgtgca gcccagcgtg
gtgatcccag ccgcccctgt    660

cccgtcgctg tcctcgccct acattgagta cacgccggcc
agcccggcct acgcccagta    720

cccaccggcc acctatgacc agtacccata cgccgcctcg
cctgccacgg ctgccagctt    780

cgtgggctac agctaccctg ccgccgtgcc ccaggccctc
tcagccgcag cacccgcggg    840

caccactttc gtgcagtacc aggcgccgca gctgcagcct
gacaggatgc agtgaggggc    900

gttcctgccc cgaggactgt ggcattgtca ccttcacagc
agacagagct gccaggccat    960

gatgggctgg cgacagcccg gctgagcttc agtgaggtgc
caccagcacc cgtgcctccg   1020

aagaccgctc gggcattccg cctgcgccct gggacagcgg
agagacggct tctctttaat   1080

ctaggtccca ttgtgtcttg agggaggact ttaagaatga
ctgagaacta tttaaagacg   1140

caatcccagg ttccttgcac accatggcag cctctccttg
caccttctcc tgcctctcca   1200

cactccaggt tccctcaggc ttgtgtcccc actgctgcat
cgtggcgggg tgtcacagac   1260

cctctgcagc ccctggctgc cctggactgt gcagagatgc
ctgactccag ggaaacctga   1320

aagcaagaag ttaatggact gtttattgta acttgatcct
cccgagctgt gagcgcagtc   1380

tgaggtgtga ggacacggcc tcctgttgga gtcccatttt
ctccatcagg gcacgtgggc   1440

ggcttcctca agcccggagg agctcccagg cgcacagggg
ccgccggtaa caggggccgc   1500

cggccaaagg cccctttcca gtcatagcac tgaagttgca
actttttttct tgtaattgtt   1560

ttgctactaa gataatttca gaagttcagt ctattttttc
agcggatact gccgccacca   1620

agaatccaaa acctattttt gacttggaga gacttgcttt
tgttggttcc gcccgtggag   1680

acgacgatag tgtttctgta taataaagtg tctgccggct
cgcgggccag gatcctctcg   1740

gtgggatggg caccacagac aggaggcccc tcaggcccgt
gcgggccact gtctgctgcc   1800

gcctgccggg gtggcagagt gagttgtctc aggaccccgt
cactgcgacg ttgacactcc   1860

tctcccttcc cttcctcccc aactccccaa acactgtgga
aggggagaag gaagtgatcc   1920

acagcattca ggccacttgg ggtctagacc atggtggtgc
cagcctgggg ggggcagtgg   1980

ccctcagctc tgcccgctgg agcggttgag tgcagaaggg
tgcgcctctt ccctctaccc   2040

ccgcaccacc tgctgtgtgc cagcctgaga cggttcctgc
ctgtcttggg ggttggtgga   2100

gggtggaggc agttctgcca gccgtggcag ggctgctatg
gggcatccag ggctgtgggg   2160

gtctggagga ggggacatga ggtgagaggt atcctggccg
agggcggggg gcagcggggg   2220

gtctccctcc ggacctacct cagggagctg agcgtgcagg
cgctccaggg caggcctggg   2280

acagagtcaa ggctcagaga ataaaggtag ctaatctcat
cataatattt ttattagaat   2340

gttctgatga taaaaataaa acttgttttc tttaaagaaa

aaaaaaaaaa aaaaaaaaaa   2400

a
                  2401


<210>  2
<211>  2474
<212>  DNA
<213>  Homo sapiens

<400>  2
cgggccgggg gtcgcctccc gcctcccgcc tcccgcctcc
cgccgcccgc cgcccgcgtc     60

gccctcgccg ccgttgggcc gcgccgcgcc gccatgtcgg
gccccggacc gcgggagccg    120

ccgccggagg caggcgcggc aggcggcgag gcggccgtcg
agggcgcggg cggcggggac    180

gcggcgctgg gcgagccggg gctgagcttc acgaccaccg
acctgagcct ggtggagatg    240

acggaggtgg agtacacgca gctgcagcac atcctctgct
cgcacatgga ggcggcggct    300

gacggcgagc tcgagacgcg cctcaactcg gcgctgctgg
cggcggcggg cccgggcgca    360

ggcgcgggcg gcttcgcggc gggcggtcag gggggcgcgg
cgcccgtgta ccccgtgctg    420

tgcccgtccg cgctggcggc cgacgcgccc tgcctgggcc
acatcgactt ccaggagctg    480

cgcatgatgc tgctaagcga ggcgggcgcg gcggagaaga
cgtcgggcgg cggggacgga    540

gcgagggccc gggccgacgg cgccgccaag gagggcgcgg
gcgcggctgc ggctgcggct    600

ggacccgacg gcgcgcccga ggcccgggcc aagccggccg
tgcgcgtccg cctggaggac    660

cgcttcaaca gcatccccgc cgagccgccg cccgcgccgc

gcggccccga gccccccgag    720

ccgggcgggg cgctcaacaa tttggtaact ctcattcgac
atccatctga actaatgaat    780

gttcctcttc agcaacaaaa caaatgtaca gcattagtga
aaaataaaac tgcggctaca    840

actactgctt tgcaatttac atacccactg tttactacaa
atgcttgctc tactagtgga    900

aattctaatc tttcacagac acagagttct agtaactcat
gttctgtact tgaagctgcc    960

aagcaccagg atattggatt gcctagagca ttttctttct
gttatcagca agaaattgaa   1020

tccactaaac agacgttagg tagtagaaac aaagttttgc
ctgagcaagt ttggattaaa   1080

gtgggagaag cagcgctatg caaacaagca ctgaagagga
atcggagtag aatgcgtcag   1140

ttggacacaa atgtagagcg aagagccctt ggagagattc
agaatgtggg cgaaggtgcc   1200

accgccacac aaggcgcttg gcagtcctcg gagtcctcac
aggcaaacct gggggagcag   1260

gcccagagtg ggccccaggg aggaaggtct caacgtaggg
agaggcataa ccgaatggaa   1320

agagatagaa ggcgcagaat ccgcatttgc tgtgatgagt
tgaatctctt agtgccgttc   1380

tgcaatgccg agactgacaa ggccacaact ctgcagtgga
ccacagcatt cctgaaatac   1440

atccaggaaa gacatggaga ttctcttaaa aaggaatttg
agagcgtatt ttgcggtaaa   1500

actggccgaa ggctaaagct gaccagaccg gactccttgg
tgacctgtcc tgcacagggg   1560

agtttacaga gcagcccctc gatggagatc aagtgatcgg
actgaacagg aatcctcggg   1620

gggtgaacag ccattccttc gtgacctgtg cacgccttct
gcaaccctgg agctctgctc   1680

ggctagtctg actcgaaaag ggcgtgactc aagctgacgg
gactccagta gggactttga   1740

gagcacattt tgtaaaaata tttatctaga cgcaaatgct
tatccatgaa tgtcctctta   1800

gaccatttgg ggatgaagcc atcttaataa ttagtaataa
ttaattagta ataattagta   1860

agcattttct caatgctctg attccatcat gttttcttaa
catgataact taaaaaattg   1920

acatcctttg tactttcttt aatcttaaaa agtacacggc
tttttactta tttacctttt   1980

aaatatgccc ctttagcaat tggaacaagt taaattgtta
actaaaaaca gtttggaaat   2040

tttatttcat tcgttatatc acaccccctt gtcatgactc
tgagtcacgt gctgctgtat   2100

tgcaacgtgc aggaccattt taaacctgtg tgctaaaaat
tttccagata cttgctttaa   2160

agctactttt gtccacaaat gaaatactgt cacagtagac
gcttaaatgc cacgttttca   2220

taccaagagt cattcattac ttcatgtgtc acaaactgtg
gtgtttggaa ttgggttttt   2280

caatgagtgg ctttacttat caatcacaac aggtaatagc
aatagacgtt agtgcaatac   2340

aaagtcaccc tcaataaata ctgttaattg gagatgtgag
tttgtacaca aaacatcaga   2400

ctagaccttt gtatgggaga gaatttactg tacattaaat
tctttatttt ttgttaaaaa   2460

aaaaaaaaaa aaaa
          2474

<210>  3
<211>  1091
<212>  DNA
<213>  Homo sapiens

<400>  3
cgcggcgcct gctctgtaga gccggcggaa ccgggtagct
tggccaggtt gtgaggaacc      60

gcagcgcgcc gcaggaccgg gccgctgagc ctgcagccgc
cccgcgccgt gacctgcgac     120

cctagacccc gactcccttt ggctcagccc gcgcgcccca
ggcccggccc gggcggcgcg     180

acgggaggat gagcggcggg cggcggaagg aggagccgcc
tcagccgcag ctggccaacg     240

gggccctcaa agtctccgtc tggagtaagg tgctgcggag
cgacgcggcc tgggaggata     300

aggatgaatt tttagatgtg atctactggt tccgacagat
cattgctgtg gtcctgggtg     360

tcatttgggg agttttgcca ttacgagggt tcttgggaat
agcaggattc tgcctgatca     420

atgcaggagt cctgtacctc tacttcagca attacctaca
gattgatgag gaagaatatg     480

gtggcacgtg ggagctcacg aaggaagggt ttatgacctc
ttttgccttg ttcatggtca     540

tttggatcat cttttacact gccatccatt atgactgatg
gtgtacagct cccaagtgct     600

ccctatccag tccaaaggac cctcttgatt acagcacagg
aacttgatcg ttggggaacc     660

ccagcccctt ggaacttgga agacccgtgt ttcctggacc
gcgaatcagt gtgttgggca     720

tcagtgtttt ctgcaagggt tgtgacctga aactttttaa
aaaccaccca cctttgggga     780

agcatttctg aatttatcca tcaccaacca tttcttcttg
gataccatca agtaacagct    840

attatttgcc aagtggagct gtcatttaat ttgatgcacc
tctggattca gatgaaacat    900

taaattgtct tcctcgattc tccatcgggt gtagagtttt
taaactatca atggcatttc    960

aagtcttctg aaacagcatg gctgtatgtg cgtggtccat
agcacagtac atgcagcatc   1020

taataagagt ttccattgta gaatgttttc acatacttga
ataaatcaaa tctttaattg   1080

agaaaaaaaa a
                1091

<210>  4
<211>  2346
<212>  DNA
<213>  Homo sapiens

<400>  4
acaaggcagc ctcgctcgag cgcaggccaa tcggctttct
agctagaggg tttaactcct     60

atttaaaaag aagaaccttt gaattctaac ggctgagctc
ttggaagact tgggtccttg    120

ggtcgcaggt gggagccgac gggtgggtag accgtggggg
atatctcagt ggcggacgag    180

gacggcgggg acaaggggcg gctggtcgga gtggcggagc
gtcaagtccc ctgtcggttc    240

ctccgtccct gagtgtcctt ggcgctgcct tgtgcccgcc
cagcgccttt gcatccgctc    300

ctgggcaccg aggcgccctg taggatactg cttgttactt
attacagcta gaggcatcat    360

ggaccgatct aaagaaaact gcatttcagg acctgttaag
gctacagctc cagttggagg    420

tccaaaacgt gttctcgtga ctcagcaatt tccttgtcag
aatccattac ctgtaaatag    480

tggccaggct cagcgggtct tgtgtccttc aaattcttcc
cagcgcattc ctttgcaagc    540

acaaaagctt gtctccagtc acaagccggt tcagaatcag
aagcagaagc aattgcaggc    600

aaccagtgta cctcatcctg tctccaggcc actgaataac
acccaaaaga gcaagcagcc    660

cctgccatcg gcacctgaaa ataatcctga ggaggaactg
gcatcaaaac agaaaaatga    720

agaatcaaaa aagaggcagt gggctttgga agactttgaa
attggtcgcc ctctgggtaa    780

aggaaagttt ggtaatgttt atttggcaag agaaaagcaa
agcaagttta ttctggctct    840

taaagtgtta tttaaagctc agctggagaa agccggagtg
gagcatcagc tcagaagaga    900

agtagaaata cagtcccacc ttcggcatcc taatattctt
agactgtatg gttatttcca    960

tgatgctacc agagtctacc taattctgga atatgcacca
cttggaacag tttatagaga   1020

acttcagaaa ctttcaaagt ttgatgagca gagaactgct
acttatataa cagaattggc   1080

aaatgccctg tcttactgtc attcgaagag agttattcat
agagacatta agccagagaa   1140

cttacttctt ggatcagctg gagagcttaa aattgcagat
tttgggtggt cagtacatgc   1200

tccatcttcc aggaggacca ctctctgtgg caccctggac
tacctgcccc ctgaaatgat   1260

tgaaggtcgg atgcatgatg agaaggtgga tctctggagc
cttggagttc tttgctatga   1320

atttttagtt gggaagcctc cttttgaggc aaacacatac

caagagacct acaaaagaat 1380

atcacgggtt gaattcacat tccctgactt tgtaacagag
ggagccaggg acctcatttc 1440

aagactgttg aagcataatc ccagccagag gccaatgctc
agagaagtac ttgaacaccc 1500

ctggatcaca gcaaattcat caaaaccatc aaattgccaa
aacaaagaat cagctagcaa 1560

acagtcttag gaatcgtgca gggggagaaa tccttgagcc
agggctgcca tataacctga 1620

caggaacatg ctactgaagt ttattttacc attgactgct
gccctcaatc tagaacgcta 1680

cacaagaaat atttgtttta ctcagcaggt gtgccttaac
ctccctattc agaaagctcc 1740

acatcaataa acatgacact ctgaagtgaa agtagccacg
agaattgtgc tacttatact 1800

ggttcataat ctggaggcaa ggttcgactg cagccgcccc
gtcagcctgt gctaggcatg 1860

gtgtcttcac aggaggcaaa tccagagcct ggctgtgggg
aaagtgacca ctctgccctg 1920

accccgatca gttaaggagc tgtgcaataa ccttcctagt
acctgagtga gtgtgtaact 1980

tattgggttg gcgaagcctg gtaaagctgt tggaatgagt
atgtgattct ttttaagtat 2040

gaaaataaag atatatgtac agacttgtat tttttctctg
gtggcattcc tttaggaatg 2100

ctgtgtgtct gtccggcacc ccggtaggcc tgattgggtt
tctagtcctc cttaaccact 2160

tatctcccat atgagagtgt gaaaaatagg aacacgtgct
ctacctccat ttagggattt 2220

gcttgggata cagaagaggc catgtgtctc agagctgtta
agggcttatt tttttaaaac 2280

attggagtca tagcatgtgt gtaaacttta aatatgcaaa
taaataagta tctatgtcta   2340

aaaaaa
                2346

<210>  5
<211>  1659
<212>  DNA
<213>  Homo sapiens

<400>  5
agtgcgcctg cgcggagctc gtggccgcgc ctgctcccgc
cgggggctcc ttgctcggcc     60

gggccgcggc catgggagag gccgaggtgg gcggcggggg
cgccgcaggc gacaagggcc    120

cgggggaggc ggccaccagc ccggcggagg agacagtggt
gtggagcccc gaggtggagg    180

tgtgcctctt ccacgccatg ctgggccaca agcccgtcgg
tgtgaaccga cacttccaca    240

tgatttgtat tcgggacaag ttcagccaga acatcgggcg
gcaggtccca tccaaggtca    300

tctgggacca tctgagcacc atgtacgaca tgcaggcgct
gcatgagtct gagattcttc    360

cattcccgaa tccagagagg aacttcgtcc ttccagaaga
gatcattcag gaggtccgag    420

aaggaaaagt gatgatagaa gaggagatga aagaggagat
gaaggaagac gtggaccccc    480

acaatggggc tgacgatgtt ttttcatctt cagggagttt
ggggaaagca tcagaaaaat    540

ccagcaaaga caaagagaag aactcctcag acttggggtg
caaagaaggc gcagacaagc    600

ggaagcgcag ccgggtcacc gacaaagtcc tgaccgcaaa
cagcaaccct tccagtccca    660

gtgctgccaa gcggcgccgc acgtagaccc tcagccctgg
tggcggcaga gaagcgggcg    720

aggcactgtg gtcgctgagg gggttggctg ggtctgagtg
ccacccccca ggccacagtg    780

ataccatccc agtgccatga gcccacactg cccgccctca
ggctctcagg tgaacgtggc    840

cgtcagcggg gaaacgtgtg tgtcagttgg accatgtggg
accctgatgg acctgaaaga    900

ccaggatcgg tccagctcag atattgaggg ctctgaagcc
tagttctgtc ttctctggag    960

cagctgtggc ttccccgtgg ctgcttggtg acatggatta
gcgctacgtg ggctgcagca   1020

tttgggatcc aggctaccta gaggggcatc gggccaggga
aaacctcgga ttagcaagca   1080

ataaaaacat gacctcactc ttcctcaaag gagcccctgg
tcttccctgt gtgactcagt   1140

tctttccatc tgtttgtccc gctgcaagcc tctttctgcg
ctgactgtga cattggaacg   1200

tggccttcct gtcaccccct ccgtgccacg cactgaaggc
cacccccacc cacctgggaa   1260

actaagaact ggatattttg cctcattcac ttgtactgta
acaatgtata taatttggtt   1320

ggtatttcac tatttaattt ttaagaagcc tattttacta
gtgttttata tgaacaaagt   1380

actgcagaag ttaaacctgt gttgtatttt ttctgagatg
ttttgcttta agagatactt   1440

tttgctcagt ttttatatgc cagatacaga gaatttgtag
cggttatttt tgtatgatct   1500

agtaacttgc aaacagacca aatggatgag aggcggggac
cgtgcagctg tcggctgatg   1560

aggaggcggc cgccccagtg ctgatggaga tgccactttc
gtgtgactgc gaacattaaa   1620

gcacaaaaaa atccaaaaaa aaaaaaaaaa aaaaaaaaa
                1659

<210>  6
<211>  1410
<212>  DNA
<213>  Homo sapiens

<400>  6
tccggcgggg cccggcaggc gccgaggagg aagagcgagc
ccggacggcg cctctcgaac     60

gagtgtgggc gcgaggcagg atgacgacct caggcgcgct
ctttccaagc ctggtgccag    120

gctctcgggg cgcctccaac aagtacttgg tggagtttcg
ggcgggaaag atgtccctga    180

aggggaccac cgtgactccg gataagcgga aagggctggt
gtacattcag cagacggacg    240

actcgcttat tcacttctgc tggaaggaca ggacgtccgg
gaacgtggaa gacgacttga    300

tcatcttccc tgacgactgt gagttcaagc gggtgccgca
gtgccccagc gggagggtct    360

acgtgctgaa gttcaaggca gggtccaagc ggcttttctt
ctggatgcag gaacccaaga    420

cagaccagga tgaggagcat tgccggaaag tcaacgagta
tctgaacaac cccccgatgc    480

ctggggcgct gggggccagc ggaagcagcg gccacgaact
ctctgcgcta ggcggtgagg    540

gtggcctgca gagcctgctg ggaaacatga gccacagcca
gctcatgcag ctcatcggac    600

cagccggcct cggaggactg ggtgggctgg gggccctgac
tggacctggc ctggccagct    660

tactggggag cagtgggcct ccagggagca gctcctcctc
cagctcccgg agccagtcgg    720

cagcggtcac cccgtcatcc accacctctt ccacccgtgc
caccccagcc ccttctgctc    780

cagcagctgc ctcagcaact agcccgagcc ccgcgcccag
ttccgggaat ggagccagca    840

cagcagccag cccgacccag cccatccagc tgagcgacct
ccagagcatc ctggccacga    900

tgaacgtacc agccgggcca gcaggcggcc agcaagtgga
cctggccagt gtgctgacgc    960

cggagataat ggctcccatc ctcgccaacg cggatgtcca
ggagcgcctg cttccctact   1020

tgccatctgg ggagtcgctg ccgcagaccg cggatgagat
ccagaatacc ctgacctcgc   1080

cccagttcca gcaggccctg ggcatgttca gcgcagcctt
ggcctcgggg cagctgggcc   1140

ccctcatgtg ccagttcggt ctgcctgcag aggctgtgga
ggccgccaac aagggcgatg   1200

tggaagcgtt tgccaaagcc atgcagaaca acgccaagcc
cgagcagaaa gagggcgaca   1260

cgaaggacaa gaaggacgaa gaggaggaca tgagcctgga
ctgagccacg cgccgtcctc   1320

cgaggaactg ggcgcttgca gtgcgttgca caccctcacc
tcccacccac tgattattaa   1380

taaagtcttt tcttttacct gccaaaaaaa
                1410


<210>  7
<211>  2162
<212>  DNA
<213>  Homo sapiens

<400>  7

atgcgccgcg ctcgctcgcg ggagggcatg gcgggggccg
tgccgggcgc catcatggac     60

gaggactact acgggagcgc ggccgagtgg ggcgacgagg
ctgacggcgg ccagcaggag    120

gatgattctg gagaaggaga ggatgatgcg gaggttcagc
aagaatgcct gcataaattt    180

tccacccggg attatatcat ggaaccctcc atcttcaaca
ctctgaagag gtattttcag    240

gcaggagggt ctccagagaa tgttatccag ctcttatctg
aaaactacac cgctgtggcc    300

cagactgtga acctgctggc cgagtggctc attcagacag
gtgttgagcc agtgcaggtt    360

caggaaactg tggaaaatca cttgaagagt ttgctgatca
aacattttga cccccgcaaa    420

gcagattcta tttttactga agaaggagag accccagcgt
ggctggaaca gatgattgca    480

cataccacgt ggcgggacct tttttataaa ctggctgaag
cccatccaga ctgtttgatg    540

ctgaacttca ccgttaagct tatttctgac gcagggtacc
agggggagat caccagtgtg    600

tccacagcat gccagcagct agaagtgttc tcgagagtgc
tccggacctc tctagctaca    660

attttagatg gaggagaaga aaaccttgaa aaaaatctcc
ctgagtttgc caagatggtg    720

tgccacgggg agcacacgta cctgtttgcc caggccatga
tgtccgtgct ggcccaggag    780

gagcaggggg gctccgctgt gcgcaggatc gcccaggaag
tgcagcgctt tgcccaggag    840

aaaggtcatg acgccagtca gatcacacta gccttgggca
cagctgcctc ctaccccagg    900

gcctgccagg ctctcggggc catgctgtcc aaaggagccc

tgaaccctgc tgacatcacc 960

gtcctgttca agatgttcac aagcatggac cctcctccgg
ttgaacttat ccgcgttcca 1020

gccttcctgg acctgttcat gcagtcactc tttaaaccag
gggctcggat caaccaggac 1080

cacaagcaca aatacatcca catcttggcg tacgcagcaa
gcgtggttga gacctggaag 1140

aagaacaagc gagtgagcat caataaagat gagctgaagt
caacgtcaaa agctgtcgaa 1200

accgttcaca atttgtgttg caacgagaac aaaggggcct
ctgaactagt ggcagaattg 1260

agcacacttt atcagtgtat taggtttcca gtggtagcaa
tgggtgtgct gaagtgggtg 1320

gattggactg tatcagaacc aaggtacttt cagctgcaga
ctgaccatac ccctgtccac 1380

ctggcgttgc tggatgagat cagcacctgc caccagctcc
tgcacccccca ggtcctgcag 1440

ctgcttgtta agctttttga gactgagcac tcccagctgg
acgtgatgga gcagcttgag 1500

ttgaagaaga cactgctgga caggatggtt cacctgctga
gtcgaggtta tgtacttcct 1560

gttgtcagtt acatccgaaa gtgtctggag aagctggaca
ctgacatttc actcattcgc 1620

tattttgtca ctgaggtgct ggacgtcatt gctcctcctt
atacctctga cttcgtgcaa 1680

cttttcctcc ccatcctgga gaatgacagc atcgcaggta
ccatcaaaac ggaaggcgag 1740

catgaccctg tgacggagtt tatagctcac tgcaaatcta
acttcatcat ggtgaactaa 1800

tttagagcat cctccagagc tgaagcagaa cattccagaa
cccgttgtgg aaaaaccctt 1860

tcaagaagct gttttaagag gctcgggcag cgtcttgaaa
atgggcaccg ctgggaggag   1920

gtggatgact tctttacaaa ggaaaatggc aggcgctggg
ctcccacgac ccctcaggac   1980

agatctggcc gtcagccgcg ggccgctggg aactccactc
ggggaactcc tttccaagct   2040

gacctcagtt ttctcacaag aacccagtta gctgatgttt
tattgtaatt gtcttaattt   2100

gctaagaaca agtaataagt aaatttttaa aaagcctttc
tgctgggttg gattaaaaaa   2160

aa
                    2162

<210>  8
<211>  3406
<212>  DNA
<213>  Homo sapiens

<400>  8
gtgctttcca gccgcgagct gtcaggccga gtgtcaggcc
gggcaggtac gcggcgcgcg     60

cccccggcgc cccccgctcc ccgccgggac gccccgcgcc
gagccggagg ccgcgtggac    120

ccgaaagccg ctgggaaaag tttacccaag gtccagccta
gcccctaggc accatgtcgg    180

acagtgatct aggtgaggac gaaggcctcc tctccctggc
gggcaaaagg aagcgcaggg    240

ggaacctgcc caaggagtcg gtgaagatcc tccgggactg
gctgtacttg caccgctaca    300

acgcctaccc ctcagagcag gagaagctga gcctttctgg
acagaccaac ctgtcagtgc    360

tgcaaatatg taactggttc atcaatgccc ggcggcggct
tctcccagac atgcttcgga    420

aggatggcaa agaccctaat cagtttacca tttcccgccg
cgggggtaag gcctcagatg    480

tggccctccc ccgtggcagc agcccctcag tgctggctgt
gtctgtccca gcccccacca    540

atgtgctctc cctgtctgtg tgctccatgc cgcttcactc
aggccagggg gaaaagccag    600

cagccccttt cccacgtggg gagctggagt ctcccaagcc
cctggtgacc cctggtagca    660

cacttactct gctgaccagg gctgaggctg gaagccccac
aggtggactc ttcaacacgc    720

caccacccac acccccagag caggacaaag aggacttcag
cagcttccag ctgctggtgg    780

aggtggcgct acagagggct gctgagatgg agcttcagaa
gcagcaggac ccatcactcc    840

cattactgca cactcccatc cctttagtct ctgaaaatcc
ccagtaggca tctgccaaga    900

agggtgctga aggctccagc cagctgtcct gggtttccgt
tttggttccc tttcatacag    960

agggtttctt atggatcact gccaaacatt gggatcatct
cctctgtcca gaggtcttca   1020

acaggaagat gccagctggc accactgcac tgtgatgggg
gccctctcct ctgctgactc   1080

tgccgtttct ccaggcctcc gctcagtgat gagaccaaga
gatcggagac aagcatggtg   1140

ctgctgcttc tgctgcttct ccagaaaatc cctgggacac
ctttgttcca gcctggtttc   1200

ctgggctggg ctcaggaaag ctgccaaatt cagtcctatg
ttgggtccaa gctgcccctg   1260

tgctgtttct gtcaagccag gtgtggacat tccaagttca
tatgcgtgaa caaaagaaaa   1320

gaggaaccca gtggatgtaa cagaaccgac tccagttgaa
tgtttagatt tttgctaaac   1380

tgttttcttt ttcccttttt tgctgtggtt tgcattcacg
gcagtagtta gcccaggtgt   1440

ggggaacgag agtgcactgc atgatagcgt tctggtgagc
tgggaaggac ccaccactgc   1500

cactgaggat tgttttggaa gaaaggaata tttttatctt
ggggaccagc taagtctctg   1560

cagtagtgtg aaattccaaa tggttgtttt atcattggtt
tggtttacca aaaaaaaggc   1620

agggaaaaaa aaaaaaaaca accgtatgag cgcattggct
tgtctgccgc aggcacagaa   1680

gggtagaaag ccacagcagg gggcagtcca gcagactctg
actcaacttt ctaggcacct   1740

agcagagaaa gataagatca aaaggtgttt ggtttttctt
ttaattttta ttgtagtttt   1800

tttgggtggg tgggggaagt aaactagact gaagcgatgg
attttttttt tctttttttt   1860

ctttagtgtt tttccctttg ttcttgaaca cttttgccct
gcagcctcag ttttgaattc   1920

ttttagcaac ttggattaga ggggcccata tgtcagaagc
tcccagcacc tcctacttgg   1980

gagaaaagtg agccatctgc tggtcaggaa gtcctccaga
gaggcagctt ttcccacaat   2040

ggtggcagga aactttgggg aaagcaggaa tggtgtccac
tgctgcggag gaactgcctt   2100

cagagaaggt ggggctggaa aagggttaga agcctcctag
ctgggattgt ctttgtttca   2160

cctttcttta aattagaatt acagaagccc ctgcccagtg
aacagataac gattggtctt   2220

atgctcctcc ctttccccca tttttctttt tgctgttttg

tttttgttt tttgtttgtt 2280

tgtttgtttt tttgagacag agtcatgctc tgtcacccgg
gctggagtgc agtggtgcga 2340

tctcagctca ctgtaacctc cgcctcccgg gttcaagcaa
ttatttgcct cagcctcccg 2400

agtagctggg attataggca cccgccacca tgtctggctt
ttagtagaga cggggtttca 2460

ccatcttggc caggctggtc ttggaactcc tgacctcgtg
agccaccacg cccagcctct 2520

tttgctgttt cattgctgac agtgttcaac aatatgcccc
atctttatat atcctaagaa 2580

acactaatcc taggttattg ctagccaaaa tatttttgtc
ctgagtagtg tcactgggcc 2640

aaaagataga tcaggacgac agcctttagt tttcctgaaa
tcaccaggtc aggcacaagg 2700

agaaaaggtt cctggatact gactaacttg ggtgggtgga
tctagccagg agaaagacag 2760

taacatgtgt tctgtacttt ctgggaagat ccctgaagcc
atcacagagg ctccccaact 2820

tctgagtcgc ccatctgttg ctgtgggagt gtgaacggat
cgctgaagga gagggagctt 2880

tgctctctct aggtgggcaa gtttcctggg ctctctgtgt
tgcctccctc tggcttcttc 2940

ctcccgtgcc ctctccccgt gtgccccagg gggatcaggg
atcctcaccc tcctgaggcc 3000

cagtggggaa gaatgaacat ggcttcatcc aggttaactg
atgctgccat ttgcccagcc 3060

tcttccatcc cagccctgtc agtgagccca ggtctggtgc
aactgctgca ggatgcctgt 3120

agtagggaac tctggaagtg tattgggctg aggtgggatt
ttccctcccc acagtgcact 3180

gagcaatgga gggtggtgag ggagccatgc tgctgaattc
tggttggcat ttccccatta   3240

tgtaaaatgg ggtgttgggt agggcagact ctgcttgggt
ttggttgtaa gataaacctg   3300

gaggagaagc acagttgtcc cattgaatta tttgagcaaa
aactactgta aataactttt   3360

ttgtcttttg tcaaataaaa tttttttttg tttttttaag cagaaa
                3406

<210>  9
<211>  1047
<212>  DNA
<213>  Homo sapiens

<400>  9
atggcctcct tggaagtcag tcgtagtcct cgcaggtctc
ggcgggagct ggaagtgcgc     60

agtccacgac agaacaaata ttcggtgctt ttacctacct
acaacgagcg cgagaacctg    120

ccgctcatcg tgtggctgct ggtgaaaagc ttctccgaga
gtggaatcaa ctatgaaatt    180

ataatcatag atgatggaag cccagatgga acaagggatg
ttgctgaaca gttggagaag    240

atctatgggt cagacagaat tcttctaaga ccacgagaga
aaaagttggg actaggaact    300

gcatatattc atggaatgaa acatgccaca ggaaactaca
tcattattat ggatgctgat    360

ctctcacacc atccaaaatt tattcctgaa tttattagga
agcaaaagga gggtaatttt    420

gatattgtct ctggaactcg ctacaaagga aatggaggtg
tatatggctg ggatttgaaa    480

agaaaaataa tcagccgtgg ggccaatttt ttaactcaga
tcttgctgag accaggagca    540

tctgatttaa caggaagttt cagattatac cgaaaagaag
ttctagagaa attaatagaa    600

aaatgtgttt ctaaaggcta cgtcttccag atggagatga
ttgttcgggc aagacagttg    660

aattatacta ttggcgaggt tccaatatca tttgtggatc
gtgtttatgg tgaatccaag    720

ttgggaggaa atgaaatagt atctttcttg aaaggattat
tgactctttt tgctactaca    780

taaaagaaag atactcattt atagttacgt tcatttcagg
ttaaacatga aagaagcctg    840

gttactgatt tgtataaaat gtactcttaa agtataaaat
ataaggtaag gtaaatttca    900

tgcatctttt tatgaagacc acctatttta tatttcaaat
taaataattt taaagttgct    960

ggcctaatga gcaatgttct caattttcgt tttcattttg
ctgtattgag acctataaat   1020

aaatgtatat ttttttttgc ataaagt
                    1047

<210>  10
<211>  3734
<212>  DNA
<213>  Homo sapiens

<400>  10
caaatgcgaa cttaggctgt tacacaactg ctggggtctg
ttctcgccgc ccgcccggca     60

gtcaggcagc gtcgccgccg tggtagcagc ctcagccgtt
tctggagtct cgggcccaca    120

gtcaccgccg cttacctgcg cctcctcgag cctccggagt
ccccgtccgc ccgcacaggc    180

cggttcgccg tctgcgtctc ccccacgccg cctcgcctgc
cgccgcgctc gtccctccgg    240

gccgacatga gtggggacca cctccacaac gattcccaga
tcgaagcgga tttccgattg    300

aatgattctc ataaacacaa agataaacac aaagatcgag
aacaccggca caaagaacac    360

aagaaggaga aggaccggga aaagtccaag catagcaaca
gtgaacataa agattctgaa    420

aagaaacaca aagagaagga gaagaccaaa cacaaagatg
gaagctcaga aaagcataaa    480

gacaaacata aagacagaga caaggaaaaa cgaaaagagg
aaaaggttcg agcctctggg    540

gatgcaaaaa taaagaagga gaaggaaaat ggcttctcta
gtccaccaca aattaaagat    600

gaacctgaag atgatggcta ttttgttcct cctaaagagg
atataaagcc attaaagaga    660

cctcgagatg aggatgatgc tgattataaa cctaagaaaa
ttaaaacaga agataccaag    720

aaggagaaga aaagaaaact agaagaagaa gaggatggta
aattgaaaaa acccaagaat    780

aaagataaag ataaaaaagt tcctgagcca gataacaaga
aaaagaagcc gaagaaagaa    840

gaggaacaga agtggaaatg gtgggaagaa gagcgctatc
ctgaaggcat caagtggaaa    900

ttcctagaac ataaaggtcc agtatttgcc ccaccatatg
agcctcttcc agagaatgtc    960

aagttttatt atgatggtaa agtcatgaag ctgagcccca
aagcagagga agtagctacg   1020

ttctttgcaa aaatgctcga ccatgaatat actaccaagg
aaatatttag gaaaaatttc   1080

tttaaagact ggagaaagga aatgactaat gaagagaaga
atattatcac caacctaagc   1140

aaatgtgatt ttacccagat gagccagtat ttcaaagccc
agacggaagc tcggaaacag    1200

atgagcaagg aagagaaact gaaaatcaaa gaggagaatg
aaaaattact gaaagaatat    1260

ggattctgta ttatggataa ccacaaagag aggattgcta
acttcaagat agagcctcct    1320

ggacttttcc gtggccgcgg caaccacccc aagatgggca
tgctgaagag acgaatcatg    1380

cccgaggata taatcatcaa ctgtagcaaa gatgccaagg
ttccttctcc tcctccagga    1440

cataagtgga aagaagtccg gcatgataac aaggttactt
ggctggtttc ctggacagag    1500

aacatccaag gttccattaa atacatcatg cttaacccta
gttcacgaat caagggtgag    1560

aaggactggc agaaatacga gactgctcgg cggctgaaaa
aatgtgtgga caagatccgg    1620

aaccagtatc gagaagactg gaagtccaaa gagatgaaag
tccggcagag agctgtagcc    1680

ctgtacttca tcgacaagct tgctctgaga gcaggcaatg
aaaaggagga aggagaaaca    1740

gcggacactg tgggctgctg ctcacttcgt gtggagcaca
tcaatctaca cccagagttg    1800

gatggtcagg aatatgtggt agagtttgac ttcctcggga
aggactccat cagatactat    1860

aacaaggtcc ctgttgagaa acgagttttt aagaacctac
aactatttat ggagaacaag    1920

cagcccgagg atgatctttt tgatagactc aatactggta
ttctgaataa gcatcttcag    1980

gatctcatgg agggcttgac agccaaggta ttccgtacat
acaatgcctc catcacgcta    2040

cagcagcagc taaaagaact gacagccccg gatgagaaca

tcccagcgaa gatcctttct    2100

tataaccgtg ccaatcgagc tgttgcaatt ctttgtaacc
atcagagggc accaccaaaa    2160

acttttgaga agtctatgat gaacttgcaa actaagattg
atgccaagaa ggaacagcta    2220

gcagatgccc ggagagacct gaaaagtgct aaggctgatg
ccaaggtcat gaaggatgca    2280

aagacgaaga aggtagtaga gtcaaagaag aaggctgttc
agagactgga ggaacagttg    2340

atgaagctgg aagttcaagc cacagaccga gaggaaaata
aacagattgc cctgggaacc    2400

tccaaactca attatctgga ccctaggatc acagtggctt
ggtgcaagaa gtggggtgtc    2460

ccaattgaga agatttacaa caaaacccag cgggagaagt
ttgcctgggc cattgacatg    2520

gctgatgaag actatgagtt ttagccagtc tcaagaggca
gagttctgtg aagaggaaca    2580

gtgtggtttg ggaaagatgg ataaactgag cctcacttgc
cctcgtgcct gggggagaga    2640

ggcagcaagt cttaacaaac caacatcttt gcgaaaagat
aaacctggag atattataag    2700

ggagagctga gccagttgtc ctatggacaa cttatttaaa
aatatttcag atatcaaaat    2760

tctagctgta tgatttgttt tgaattttgt tttatttttc
aagagggcaa gtggatggga    2820

atttgtcagc gttctaccag gcaaattcac tgtttcactg
aaatgtttgg attctcttag    2880

ctactgtatg caaagtccga ttatattggt gcgtttttac
agttagggtt ttgcaataac    2940

ttctatattt taatagaaat aaattcctaa actcccttcc
ctctctccca tttcaggaat    3000

ttaaaattaa gtagaacaaa aaacccagcg cacctgttag
agtcgtcact ctctattgtc   3060

atggggatca attttcatta aacttgaagc agtcgtggct
ttggcagtgt tttggttcag   3120

acacctgttc acagaaaaag catgatggga aaatatttcc
tgacttgagt gttccttttt   3180

aaatgtgaat ttttatttct ttttaattat tttaaaatat
ttaaaccttt ttcttgatct   3240

taaagatcgt gtagattggg gttggggagg gatgaagggc
gagtgaatct aaggataatg   3300

aaataatcag tgactgaaac catttcccca tcatcctttg
ttctgagcat tcgctgtacc   3360

ctttaagata tccatctttt tctttttaac cctaatcttt
cacttgaaag attttattgt   3420

ataaaaagtt tcacaggtca ataaacttag aggaaaatga
gtatttggtc caaaaaaagg   3480

aaaaataatc aagattttag ggcttttatt ttttcttttg
taattgtgta aaaaatggaa   3540

aaaaacataa aaagcagaat tttaatgtga agacattttt
tgctataatc attagtttta   3600

gaggcattgt tagtttagtg tgtgtgcaga gtccatttcc
cacatctttc ctcaagtatc   3660

ttctattttt atcatgaatt cccttttaat caactgtagg
ttatttaaaa taaattccta   3720

caacttaatg gaaa
                3734


<210>  11
<211>  4440
<212>  DNA
<213>  Homo sapiens

<400>  11
ccctcccgct ccccgccccc gcccccgccc ccgcctccgc
cgcggccccc acctctgcct     60

ccttctactc gggcgccccg gcggccgcca cctctcccca
gcccaggaga ggctgcggag    120

ccgcagccgc ccagaccgcg cagcgcggga ggcaggttcc
gcacgaaata aatcagaatg    180

agttatgcag aaaaacccga tgaaatcacg aaagatgagt
ggatggaaaa gctcaataac    240

ttgcatgtcc agagagcaga catgaaccgc ctcatcatga
actacctggt cacagagggc    300

tttaaggaag cagcggagaa gtttcgaatg gaatctggaa
tcgaacctag tgtggatctg    360

gaaacacttg atgaacgaat caagatccgg gagatgatac
tgaaaggtca gattcaggag    420

gccatcgcct tgatcaacag cctccaccca gagctcttgg
acacaaaccg gtatctttac    480

ttccatttgc agcaacagca tttgatcgag ctgatccgcc
agcgggagac agaggcggcg    540

ctggagtttg cacagactca gctggcggag cagggcgagg
agagccgaga gtgcctcaca    600

gagatggagc gtaccctggc actgctggcc tttgacagtc
ccgaggagtc gcccttcgga    660

gacctcctcc acaccatgca gaggcagaag gtgtggagtg
aagttaacca agctgtgcta    720

gattatgaaa atcgcgagtc aacacccaaa ctggcaaaat
tactgaaact actactttgg    780

gctcagaacg agctggacca gaagaaagta aaatatccca
aaatgacaga cctcagcaag    840

ggtgtgattg aggagcccaa gtagcgcctg cgcttgcgtg
gtggatccaa caccagccct    900

gcgtcgtggg acttgcctca gatcagcctg cgactgcaag
attcttactg cagtagagaa    960

ctctttttct cccttgtact tttttttgac ctggcatctt
tttataggga aaaatggcct   1020

ttgtaggcag tggaaaactt gcaaggaaag ctgccgtctc
tttggcagtc tgatgcagag   1080

cctgcactct ggcactcgct gaagaatctg gaaggttgcg
gtttgctctt ccagtgttcg   1140

ggggcctctg gctgctgaag gattcggtct accacggagg
gctgtgctgt taggctgcat   1200

cccactcaaa atacaggaaa agcacgaatc atgattctgc
tttctgttag cttaggcaga   1260

cattgggcct tcacctacaa gtttttcctt acccctgtgg
tttttgtgtt tttttttttt   1320

tctttttcca taggaaagaa tatataaatt tgtaaatcct
aattcaaaga tggctcatgt   1380

gtgagggcat tgagtttgat ttgttttccc tttggtctgg
gttgtgtggc ttttggggga   1440

tgcgtgtgag ggggctatgt gttttttaat tttttaaata
tatattttgg tgctgtgtgt   1500

ggtaagagac ttgttcctag tggatcaatg aaccatctct
tctgggcagt tttgttgaaa   1560

ataaaggttt ctctttgatt tcaagaatga ccaaaatggc
ctctaaaaga tgttaatcat   1620

ctcaaatgac cttttgtctt tggggcgttc ttcccctgt
gatagcggca gtggcttttt   1680

ctggtacctg cagctggaaa ggccacttgg ccctgtgctg
agtgagcggc cttcattaga   1740

gcgaggcagc ccttggccgg tggggacgca gagccccagc
aggtggtgca cgactgttgg   1800

cggaaggaac gcgtgttcat cctcagtgat ctgccctcca

gcatctcggc agcatctcat    1860

cctccatcgt cagctggctc tgccgatgtc ctgcttctgt
tcactcacag aactgtcccc    1920

tgctccgtgg tgggcaggag ggaagtggtg cagggctgcg
tgcattgcct gcgagtcggg    1980

acagttgatg ggcacatggc cttgtagctc tgggcacaga
tgtgtttgga ttcattgcag    2040

cggaccaccg ggcactgttg accccactga gcagtgctaa
gtgttggttt agtggatgtt    2100

cgtggaattg ctgacccatc caagggcgtc ctttggagcc
agtggagcct gccggcgcat    2160

ctgaggggca gaatgctgct agcacttgaa tctgggatct
cgccttattc tcaagtagca    2220

aggcatctcg acaagcatgg tctaggtctg gtggccagct
tgccagtacc tgagccggtc    2280

gggtcatctg cctctgaggg accgtcctca ccgagctcct
gcatcccttg agtgttgatc    2340

aggaggcgtc cacagcattg ttctcgcctc tgaatgatgc
ttctttctgt gttggagcct    2400

ggcgaagttg tgttttcaag ccctctactt ctctttccag
tgggtaggag cttttggcag    2460

tgtttacttt acctagatgg cttatataat ccagtaagag
atgcaaagat aaaattgctg    2520

cggttgttac agaagcatgg cggcctccag actgacccat
tggttgccct ttagatttg    2580

taaggatgcg gtgctgggga ggtggtgctt ccctaccccc
tagaaatgct gccttccaac    2640

taccactctc ccagatgtga cccttgcgat tatttcctct
gaggtttgag gatgaagata    2700

agttggaggg aaagagagta actaataggg gatgaaatat
agcagaagct agaagaaagc    2760

ggtgaggtga gagagatgca tctgcacgtt ttcttcaaca
gcaccaggtg attcagcata   2820

ttcctaatta cctttcacta ttcgtgtata taagatcgtt
tacttgcata atatatcatc   2880

aatttgacat attcttaaaa ctagagggtg tgagaagcac
agcaatagga agtctctcca   2940

caaactaggg gaacacaaat ggggtcattc acgtgcctgg
actgtcacta tgtggctgtc   3000

acgtgaagtg ctggtgttga tttccatttc agccagtggg
tagctgataa gccagtgcca   3060

gcatccagca tgagcagatg tcggggagac tgggaagtct
ccagcgttac tgctctcctt   3120

cccttcatga taagccagtg ccagcatcca gcgtgagcag
acgtcgggga gactgggaag   3180

tctccgatgt tactgcctgc cttcctttcg tgtgaggggc
tgcacttgct tttcttgtga   3240

tctgttagtg gacgaggtct tccaaggaag tgctttgcac
actttctttg ctccttttta   3300

cagtctttgt ctttgcagca agcaaatgaa attaagccac
tttgggataa tgaacattca   3360

gtataattct actttgtctc attttggatc tcactgttgt
ctttataaaa atggcacatt   3420

ttacaaagta gtttattctt attatacttt ctgctggaga
gtgccttgaa ataaaatgtg   3480

agagtattct ggtactctgt gttccagatg catgaaattg
ggtgaggaat aacccctagt   3540

ctggaatctt tgtgaagcat agggttattg caaggcaaat
gggaactaac acatcttgcc   3600

atttgaatca gggtctccag tttctagaaa aggcagacac
tggttgggac caaagtctcc   3660

atggcacatg actgaagact ggtggtcgtg tgtgtgcgga
gtccacggaa gcctcgggga   3720

ggtggagctg ctccttccat tccgtcagga cgtgatctga
aaacatgtag agaagatgag   3780

ttgaggacag cttttctaag gcaatgtgat gtctttgctt
tcttatttct ctttctctgc   3840

gttgttagtt ttgaagagtg gaggagctag gggctccaga
aagaatctta cacatgtgtt   3900

gaagacattg atgtcatagg gagcggggag ctgcattccc
ttctgggctg ttactgctaa   3960

atctcagtat gaacagacca ggcggaaagc ttggtggcca
agcagtctgt gtgcttcccc   4020

gctgatggag aacgttgcgt tgttcacaat agggcctcat
gggtgtagcc gcatggcaga   4080

cccatggctg gcgcagctgc ctgttgccgt ctgtcttcag
taactgctgc tctgttaact   4140

gttctattct gatactacgc gtgttgtttt ttacaacagg
tatgtttttg tttcagaaat   4200

atgtattgct tttctcatat tttttgcaaa ttgtattgtc
aacatgggtc atttaaagtc   4260

ctgtatgaac cataacctgc tgtggtacct ttgtacatgt
ttgattctgt attctttatt   4320

ccagtgtggc atatgtgccc ctctgtatct tttgagaagt
gcggaatagg ttgcttctac   4380

cacctgttct taatgtaaca gtaaaagttt tcacattttt
ctcagaaaaa aaaaaaaaaa   4440


<210>  12
<211>  3110
<212>  DNA
<213>  Homo sapiens

<400>  12

gcggaagtgg agctaccgcc accgccgccg ccgattccgg
agccggggta gtcgccgccg     60

ccgccgccgc tgcagccact gcaggcaccg ctgccgccgc
ctgagtagtg ggcttaggaa     120

ggaagaggtc atctcgctcg gagcttcgct cggaagggtc
tttgttccct gcagccctcc     180

cacggcagag tctccagaga tttgggccgc tacaaaaagt
gcattttgcc cattcggctg     240

tggatagaga agcaggaaga gcactggact tggagtcagg
gaatgacaat ggataaaagt     300

gagctggtac agaaagccaa actcgctgag caggctgagc
gatatgatga tatggctgca     360

gccatgaagg cagtcacaga acaggggcat gaactctcca
acgaagagag aaatctgctc     420

tctgttgcct acaagaatgt ggtaggcgcc cgccgctctt
cctggcgtgt catctccagc     480

attgagcaga aaacagagag gaatgagaag aagcagcaga
tgggcaaaga gtaccgtgag     540

aagatagagg cagaactgca ggacatctgc aatgatgttc
tggagctgtt ggacaaatat     600

cttattccca atgctacaca accagaaagt aaggtgttct
acttgaaaat gaaaggagat     660

tattttaggt atctttctga agtggcatct ggagacaaca
aacaaaccac tgtgtcgaac     720

tcccagcagg cttaccagga agcatttgaa attagtaaga
aagaaatgca gcctacacac     780

ccaattcgtc ttggtctggc actaaatttc tcagtctttt
actatgagat tctaaactct     840

cctgaaaagg cctgtagcct ggcaaaaacg gcatttgatg
aagcaattgc tgaattggat     900

acgctgaatg aagagtctta taaagacagc actctgatca

tgcagttact tagggacaat    960

ctcactctgt ggacatcgga aaaccaggga gacgaaggag
acgctgggga gggagagaac    1020

taatgtttct cgtgctttgt gatctgttca gtgtcactct
gtaccctcaa catatatccc    1080

ttgtgcgata aaaaaaaaaa aaaaaaaaaa aagagaatcg
tacgtcgact ttcgattttt    1140

cacagcctca gcctaggaaa aatggttcat gggataaaca
gctggtattt gtatctaaaa    1200

ctcagattgg tcacataaat gccacggcat tccgaagttt
tgattttgat taacattgac    1260

aggattactg tgtgtttaat tttttaaaaa ctgaacactg
tgattatggg gttttgtaat    1320

ttagcagaac tcttactggt agaaaaaata gacctgaatt
atgtgtaact ttttggaagg    1380

tttaatctga tatcaaaata atcattgaaa tacaattcca
ttgtaaagtt gtacagaaag    1440

ttatagagat tatattgtga tgctggaact tggagtgaga
cacacatcat ttggcatttg    1500

agttgaatgg taattcacag taatgctgcc gttgttcggg
acttaaagac acttgacctg    1560

tttgggctgt tgccacttaa aagttcatga ccacaaatgt
ccacagtgtc ttcctctgag    1620

gaaactcgaa tcctgaaatg gaaattcttt gtggcagata
actggcttat gacaccttga    1680

aaagttcaag tgctcatata acacaccaca ctgaaccccc
tttcctacag caatatgttc    1740

actatgttac caatttgcaa cttgtgcttc aatagtggaa
tctactttca ttgttaacac    1800

tgagctaaag aaaaaaagcc gtgtgtttta tgaatgacct
tatctgtttc ctggataata    1860

cctttaagaa taatgtcctg agtcaggcgt ggtggtgcgt
gcatctagtc ccaactattt   1920

gggaggctga ggcaggagga tcgcttgagc ccaggagttt
aaagctgcag tgccctgtgg   1980

ttgcacctgt gaataactgc actccagcct gggcaacata
gcgagacctc atctccaaaa   2040

aagaaaacaa aaaacaaaaa aaggaatgat gttctgtaga
gatggccttt cacttgagga   2100

gtactcagtt ttcaggttct tcctagctcg ggcttttaa
attttgaaat ctaaacattc   2160

tttcccacca tcctttttga ctgttgacct tggttttctc
ttctaagttt ctgtccctct   2220

gcttccttac tttttttcct ttttgaattc tatctttatc
tgtcttttgt tcactttta   2280

atgctatata tgggcagggg tgagagacat tactgagcac
cttggtgagc aagcctggct   2340

ttaaagattg gagaagagct tctggcacca gaaccctgtc
ttcctccagt tctcaacacg   2400

gtgttgctct tcagtcatac cggaatctga atcaaaaaag
tatttttaaa tatccatgat   2460

ttctccctgt attgaggcta gccctgatca tgctttttgt
gcctgtcacc aggtctccca   2520

agtgcactca tccaggtcag tgctcagatg tgtttaagga
gaccctatat tcagggaagt   2580

tgcgtgaaca ctgcagtggg gagaattgag aatagtcagg
cctatcagtc tcacagaatc   2640

acccctctac ctttgatatt ccacttagct gtagagtcca
tctgtttgtc catctgctga   2700

aatgagaaaa gaaaaattta tgcactgatt taaaacaaac
caaaaaaaaa gaaaaaaaca   2760

aaaaaaaaaa tccctccttt ctagctgaac aaaaatgtgc
agttaatact tggcgcttga   2820

aaatgcagta gtgaatgtgg aaccaagcct gtctgtatat
ctggtagctc ttttcttgct   2880

ttgttttttc ttaccagtat tctgcctaac gtttgcttct
gtgatggtta tattgcctag   2940

caagcacacc cgtggttgtg aaaatagtat agcaaaaaag
aaaaatcccc ggttattgat   3000

gtactagatt tgtgtatgtc ttttaaacag ttctagtttc
accttacaca gaataatcag   3060

gaaaagtgta aaaattcaaa agtgaaataa aaattttatc
agttaaaaaa              3110

<210>  13
<211>  5653
<212>  DNA
<213>  Homo sapiens

<400>  13
gacaaagaga actaatgctt tgtgctgatt catatttgaa
tcgaggcatt gggaaccctg     60

tatgccttgt ttgtggaaag aaccagtgac accatcactg
agcttcctaa aagttcgaag    120

aagttagagg actatacact ttcttttgaa cttttataat
aaatatttgc tctggttttt    180

ggaacccagg gctgttagag gggtgagtga caagtcttac
aagtggcctt attccaactc    240

cagaaattgc ccaacggaac tttgagatta tatgcaatcg
aaagtgacag gaaacatgcc    300

aactcaatcc ctcttaatgt acatggatgg gccagaagtg
attggcagct ctcttggcag    360

tccgatggag atggaggatg ccttgtcaat gaaagggacc
gctgttgttc cattccgagc    420

tacacaagaa aaaaatgtca tccaaatcga ggggtatatg
ccccttggatt gcatgttctg    480

cagccagacc ttcacacatt cagaagacct taataaacat
gtcttaatgc aacaccggcc    540

taccctctgt gaaccagcag ttcttcgggt tgaagcagag
tatctcagtc cgcttgataa    600

aagtcaagtg cgaacagaac ctcccaagga aaagaattgc
aaggaaaatg aatttagctg    660

tgaggtatgt gggcagacat ttagagtcgc ttttgatgtt
gagatccaca tgagaacaca    720

caaagattct ttcacttacg ggtgtaacat gtgcggaaga
agattcaagg agccttggtt    780

tcttaaaaat cacatgcgga cacataatgg caaatcgggg
gccagaagca aactgcagca    840

aggcttggag agtagtccag caacgatcaa cgaggtcgtc
caggtgcacg cggccgagag    900

catctcctct ccttacaaaa tctgcatggt ttgtggcttc
ctatttccaa ataaagaaag    960

tctaattgag caccgcaagg tgcacaccaa aaaaactgct
ttcggtacca gcagcgcgca   1020

gacagactct ccacaaggag gaatgccgtc ctcgagggag
gacttcctgc agttgttcaa   1080

cttgagacca aaatctcacc ctgaaacggg gaagaagcct
gtcagatgca tccctcagct   1140

cgatccgttc accaccttcc aggcttggca gctggctacc
aaaggaaaag ttgccatttg   1200

ccaagaagtg aaggaatcgg ggcaagaagg gagcaccgac
aacgacgatt cgagttccga   1260

gaaggagctt ggagaaacaa ataagggcag ttgtgcaggc
ctctcgcaag agaaagagaa   1320

gtgcaaacac tcccacggcg aagcgccctc cgtggacgcg

gatcccaagt tacccagtag   1380

caaggagaag cccactcact gctccgagtg cggcaaagct
ttcagaacct accaccagct   1440

ggtcttgcac tccagggtcc acaagaagga ccggagggcc
ggcgcggagt cgcccaccat   1500

gtctgtggac gggaggcagc cggggacgtg ttctcctgac
ctcgccgccc ctctggatga   1560

aaatggagcc gtggatcgag gggaaggtgg ttctgaagac
ggatctgagg atgggcttcc   1620

cgaaggaatc catctggata aaaatgatga tggaggaaaa
ataaaacatc ttacatcttc   1680

aagagagtgt agttattgtg gaaagttttt ccgttcaaat
tattacctca atattcatct   1740

cagaacgcat acaggtgaaa aaccatacaa atgtgaattt
tgtgaatatg ctgcagccca   1800

gaagacatct ctgaggtatc acttggagag acatcacaag
gaaaaacaaa ccgatgttgc   1860

tgctgaagtc aagaacgatg gtaaaaatca ggacactgaa
gatgcactat taaccgctga   1920

cagtgcgcaa accaaaaatt tgaaaagatt ttttgatggt
gccaaagatg ttacaggcag   1980

tccacctgca aagcagctta aggagatgcc ttctgttttt
cagaatgttc tgggcagcgc   2040

tgtcctctca ccagcacaca aagatactca ggatttccat
aaaaatgcag ctgatgacag   2100

tgctgataaa gtgaataaaa accctacccc tgcttacctg
gacctgttaa aaaagagatc   2160

agcagttgaa actcaggcaa ataacctcat ctgtagaacc
aaggcggatg ttactcctcc   2220

tccggatggc agtaccaccc ataaccttga agttagcccc
aaagagaagc aaacggagac   2280

cgcagctgac tgcagataca ggccaagtgt ggattgtcac
gaaaaacctt taaatttatc   2340

cgtgggggct cttcacaatt gcccggcaat ttctttgagt
aaaagtttga ttccaagtat   2400

cacctgtcca ttttgtacct tcaagacatt ttatccagaa
gttttaatga tgcaccagag   2460

actggagcat aaatacaatc ctgacgttca taaaaactgt
cgaaacaagt ccttgcttag   2520

aagtcgacgt accggatgcc cgccagcgtt gctgggaaaa
gatgtgcctc ccctctctag   2580

tttctgtaaa cccaagccca agtctgcttt cccggcgcag
tccaaatccc tgccatctgc   2640

gaaggggaag cagagccctc ctgggccagg caaggcccct
ctgacttcag ggatagactc   2700

tagcacttta gccccaagta acctgaagtc ccacagacca
cagcagaatg tgggggtcca   2760

aggggccgcc accaggcaac agcaatctga gatgtttcct
aaaaccagtg tttcccctgc   2820

accggataag acaaaaagac ccgagacaaa attgaaacct
cttccagtag ctccttctca   2880

gcccaccctc ggcagcagta acatcaatgg ttccatcgac
taccccgcca agaacgacag   2940

cccgtgggca cctccgggaa gagactattt ctgtaatcgg
agtgccagca atactgcagc   3000

agaatttggt gagccccttc caaaaagact gaagtccagc
gtggttgccc ttgacgttga   3060

ccagcccggg gccaattaca gaagaggcta tgaccttccc
aagtaccata tggtcagagg   3120

catcacatca ctgttaccgc aggactgtgt gtatccgtcg
caggcgctgc ctcccaaacc   3180

aaggttcctg agctccagcg aggtcgattc tccaaatgtg
ctgactgttc agaagcccta   3240

tggtggctcc gggccacttt acacttgtgt gcctgctggt
agtccagcat ccagctcgac   3300

gttagaagga aaaaggcctg tgtcatatca acacttatct
aacagcatgg cacaaaagag   3360

aaactatgag aattttattg ggaatgcaca ttatcgacca
aatgacaaaa aaacttgatt   3420

cactaattag ggggaaaaaa ggtcttggtg gatgtcagtg
cttactcccc atgaaattaa   3480

attttacttc atcctttgag aagcgaatgg tgaaagctac
tgaaataagc tgtgattgta   3540

ctgtacataa aacatatgag gaatctgcaa ggaacactac
agttgtgtaa agttgttctg   3600

ttaacttttg taccaaatag caatacaaac tagttggaac
agttggaact tacatacatg   3660

gggactggaa atctctattt tgtccctgaa taatattttt
cttagaattg accaaataag   3720

aagtggaatt tttgcatact tgagcgctgc tgaaaagaaa
tcatttgggt tgggtggggc   3780

gggatggggg aaaagtatat aatgcttgca cctcaggtaa
aaatctgtaa atatctaagt   3840

tgtaaacctg cttgttcaaa tactgtgtgt attccttttc
tctaatgcag ctcatcactt   3900

ggagcagttt ctgctattgt gctctttcat ttaaaatgta
tgtttttttt tttttaaact   3960

gtcaatgatt ttgtattatg ttgaatccac ccaaatctat
tgttgtctta aaattgttaa   4020

tggaagtatt gaccctctat gatatgtgct gcagatatcg
aggtcagcca ttcggaagct   4080

ggcagcattt tatcgcaact ttgagcatct cagatgggga

aggcaccttc ttcctcgcct 4140

ctccagattg tcctggaacc tccaggatcc ttgactgagg
gcgttgggat ggcttgtagg 4200

atttttaaga gagtgtgtct acagacaagc attttctctg
tagagcagcc acacgttgta 4260

taaacataaa ctgtatgtgc agttatttaa atttgtttct
gtcaaattaa tcatttttgt 4320

tggacgattc agtggcgggg gggttttgcc taaattagta
tataaaaaca aaaatgctaa 4380

attatatctg tgaattgcag gtattgggga acagttttaa
gggaaatttt gggggggaaca 4440

ttttaggttt gtatttggta gtcttaatgt atctggcatt
tgggtgaact gtggacatac 4500

tagagttgat tatagacaca ttgattttga ataaggaact
gctggccgag cccgctggga 4560

gtctagaaag agaaaatctg tttctagacc tcagttattt
tcccattttt ggttgttttg 4620

aagcagtaac atttttctca gtgcacatgc aatttgggtt
ttagagaaga tggccaccag 4680

ctggcttcct agatatttta aacttttgtt ctttaatatg
ctgtccatgg ctgagtttat 4740

tagtacatgg gcttagtgac cacaaaatat tttattaaga
aactgtttca aaaataaatt 4800

tgcactgttc atttttctgg cctcgctgtt ctccatagag
caagggtaat cctagaaaaa 4860

attttttttt tttaaattat gcaacgtaag atgtcctcct
tgatagaagt cttagctcct 4920

gtgttacaag ggagaactca tttgagatca gtctgttggc
attgcaatga agtgctttgt 4980

atcaggaaag tgtacactat tgaccttttt tcctgttcac
aagctgagcc atatgtacat 5040

aatctagatt ttgttttcat agttttgcac ttttatagcc
tatttttgaa gattaacaca   5100

tttgcaagat gattgactca atctttgcct aatccaatga
gtgttacaga gagcttgctg   5160

tgactagaac cataaatctt aaagggggta tgtgataata
gagggctgga atttaaacct   5220

gtatttaaaa aaaagaatca ccaaatctat ttgaaaacaa
gtcgatttgt attatgctgg   5280

aattttttgg gctttcagat ttctcttttt aaccacattt
ctgaatgtat aaaaatacca   5340

attattttcc tacagccctt tgtacttcaa aatatgtttt
tgtgtccatc agtattaact   5400

attggtatac tactggtttt atattttttt ttctttgaga
caacagtaca tataatagag   5460

gtacaattcg ttggattttt gtttatgtat ttatttcatt
ccagtttgat ttattttaat   5520

tgttgatact taagttgtcg aacagtagac attacttgtt
ttatttatga tatatttcag   5580

cttaaagtta tgttattata tgtggaagtg taaatataga
tttggtgttt tgcaaaaaaa   5640

aaaaaaaaaa aaa
                5653

<210>  14
<211>  1893
<212>  DNA
<213>  Homo sapiens

<400>  14
aagtaagtat ccccactttc agggtagcat cctctttccc
tagttatttc agtgcagaat     60

cgattcacgt gtaagcctct acctcctgca cttaccccaa
tgtagacccc cttccacagt    120

ccccctacag aagatctccc aaaacatttc caacacagaa
cttccattca attcccctcc    180

gactgcccgc ctccctcctc caggacagag cacgccccag
gtttcgagac tgaactaccc    240

ccatctacgg agtccacccc tccctctcta gaaccccctc
agtgtagacg cctcctcttt    300

tcctctttcc tccctacttt ttctagtttt tctgcagccg
acctccagcg tcggccactg    360

tagctccttc cttgctccac tttccccaga gtacacagcc
tgcctttcca ctctccgtgc    420

agacccatcc tctttcccgc tcctcgctct cggacgccac
caacgctctc ccgggctctt    480

ccgcgttacc tacgatggaa ggtcggggcg tgcgggcagc
tggaacccac ccctgtcttg    540

gagctccggg tagctctcaa actcgaggct gcgcaccccc
tttcccgtca gctgagctct    600

agagcgctgg ggctttcttt ttgatcgact tcttgaaata
aaaccaactt tatcattctt    660

tgggtaacag acccaaaagc cgatggacgg gcccgctggg
ctgttcccgc ccctatgccc    720

ttttttgggt ttccggccag aggcatgctg ggagcgtcac
atgcaaattg agcgtgcacc    780

cagcgttccg ccctttctac gctgggccgg ttatcgaccc
ggcccagtgc gcaggcgcgg    840

gaaagttgaa ctaataaagt ttgtacgagt tcagtggagg
agaccgcaag ttgagtggag    900

gaggcggcgg tggggccccg gaccaggtgc ctccatggca
ggctctgaag agctggggct    960

ccgggaagac acgctgaggg tcctagctgc cttccttagg
cgtggtgagg ctgccgggtc    1020

tcctgttcca actccaccta gaagccctgc ccaagaagag
ccaacagact tcctgagccg    1080

ccttcgaaga tgtcttccct gctccctggg gcgaggagca
gccccctctg agtcccctcg    1140

gccttgctct ctgcccatcc gcccctgcta tggtttagag
cctggcccag ctactccaga    1200

cttctatgct ttggtggccc agcggctgga acagctggtc
caagagcagc tgaaatctcc    1260

gcccagccca gaattacagg gtcccccatc gacagagaag
gaagccatac tgcggaggct    1320

ggtggccctg ctggaggagg aggcagaagt cattaaccag
aagctggcct cggaccccgc    1380

cctgcgcagc aagctggtcc gcctgtcctc cgactctttc
gcccgcctgg tggagctgtt    1440

ctgtagccgg gatgacagct ctcgcccaag ccgagcatgc
cccgggcccc cgcctccttc    1500

cccggagccc ctggcccgcc tggccctagc catggagctg
agccggcgcg tggccgggct    1560

ggggggcacc ctggccggac tcagcgtgga gcacgtgcac
agcttcacgc cctggatcca    1620

ggcccacggg ggctgggagg gcatcctggc tgtttcaccc
gtggacttga acttgccatt    1680

ggactgagct ctttctcaga agctgctaca agatgacacc
tcatgtccct gccctcttcg    1740

tgtgcttttc caagtcttcc tattccactc agggctgtgg
ggtggtggtt gccctacctg    1800

tttttgccaa aaataaattg tttaaaactt ttcttattaa
aaacgttaca aagtaaaaaa    1860

aaaaaaaaaa aaaaaaaaaa aaaaaaaaaa aaa
                    1893

<210>  15
<211>  2816
<212>  DNA
<213>  Homo sapiens

<400>  15
cacgcactac gggttggcgc cagagtcaaa aggcgtcggc
cctctggcaa gatggctgct     60

gcggaggcgt tggagcgcgg aaatctggaa ccgggatggc
gacgtctaca ctgagtcgga    120

ggcgaaggag cttactccac gggaacagcc tctagataat
ctgagttgtt gaaaatacga    180

agcctgttac tcgtgaacag tggctgacaa cagtgttgtt
gtgagcctgg ctgtctgctt    240

ggacccagag gtttcgtctg ccagggtttt tggttgtatt
taggatttca gggaaaagtg    300

tccaagcttt cagtgttgga gcaggtatgg acgacaaagg
cgacccgagc aatgaggagg    360

cacctaaggc catcaaaccc accagcaaag agttcaggaa
aacatggggt tttcgaagga    420

ccactatcgc caagcgagag ggcgcagggg acgcggaggc
tgacccactg gagccgccac    480

ccccacagca gcagctgggc ctgtccctgc ggcgcagtgg
gaggcagccc aagcgcactg    540

agcgcgtgga gcagttcctg accattgcgc ggcgccgcgg
caggaggagc atgcctgtct    600

ccctggagga ttctggtgag cccacgtcct gccccgccac
agacgccgag acagcctccg    660

agggcagcgt ggaaagcgct tctgagacca gaagcggccc
ccagtctgct tccacagctg    720

tgaaggaacg accagcctct tctgaaaagg tgaaaggagg
ggatgaccac gatgacacct    780

ccgatagtga cagcgatggc ctgaccttga aagagcttca

gaatcgcctt cgcaggaagc    840

gggaacagga gcccactgag aggcccctga aagggatcca
gagtcgcctg cggaagaagc    900

gccgggagga gggtcccgcc gagactgtgg gctccgaggc
cagtgacact gtggagggcg    960

tcctgcccag taagcaggag cccgagaacg atcagggggt
tgtgtcccag gctgggaaag   1020

atgacagaga gagtaagttg gagggaaagg cggctcagga
catcaaagat gaggagcctg   1080

gagacttggg ccgaccgaag cctgaatgtg agggttacga
ccccaacgcc ctgtattgca   1140

tttgccgcca gcctcacaac aacaggttta tgatttgctg
tgaccgctgt gaagaatggt   1200

ttcatggcga ttgtgtgggc atttctgagg ctcgagggag
gcttttggaa aggaatgggg   1260

aagactatat ctgcccaaac tgcaccattc tgcaagtgca
ggatgagact cattcagaaa   1320

cggcagatca gcaggaagct aaatggagac ctggagatgc
tgatggcacc gattgtacaa   1380

gtataggaac aatagagcag aagtctagcg aagaccaagg
gataaagggt agaattgaga   1440

aagctgcaaa tccaagtggc aagaagaaac tcaagatctt
ccagcctgtg atagaggcgc   1500

ctggtgcctc aaaatgtatt ggccccgggt gctgtcacgt
ggcgcagccc gactcggtgt   1560

actgcagtaa tgactgtatc ctcaaacacg ccgcagcgac
aatgaagttt ctaagctcag   1620

gtaaagaaca gaagccaaag cctaaagaaa agatgaagat
gaagccagag aagcccagtc   1680

ttccgaaatg cggtgctcag gcaggtatta aaatctcttc
tgtgcacaag agaccagctc   1740

cagaaaaaaa agagaccaca gtgaagaagg cagtggtggt
ccctgcgcgg agtgaagcac   1800

tcgggaagga agcagcttgt gagagcagca cgccgtcgtg
ggcgagcgat cacaattaca   1860

atgcagtaaa gccagaaaag actgctgctc cctcgccgtc
actgttgtat aaatgtatgt   1920

atcacctagg ggttggcctc ctggacccct cccgttcttt
ctggatagcc atcccctggg   1980

cctgtccagg actgggagtt gcagctttgt gttaagctga
tcacagacac cggctgcacc   2040

atcagcggga agcagagccc atgtccagga tgcctcctgc
tgccctgtgt ccatccctag   2100

tctgtcagga cttcctgtca ctgttttcca aagctgtaaa
cctcactggt gaacgttcac   2160

cttaatgatt gattctttaa tctctgtttt cactctcagg
ctctggtaag tattcgtatt   2220

ctcttcatcc cagtctgatt gcatagccac actgcccggc
acgccacatc cacccctgtc   2280

tgcacatgag ttgttctgac aacagcgctg tatacgcttc
agtttttcca cattgtccac   2340

ggccagcaca tgaaagcatc acttcttttt tatgttgtgg
gaatctttgc aagttagtgt   2400

tgcatctgat tttcaggtgt acatttattt ttgactgggc
agatagggga tttttttttt   2460

tccatgtccg attcacacgc tacacaccca catgaacaca
ttcgaacttc gaaggccaca   2520

cactcctgct tcataggccc cacggtaagt gagttcacac
ctagaacact gtcctgaccg   2580

caggacgcgt gccttggact tggtattcta catgtgactg
gctttcttgc cctcgtctct   2640

tgaatgttta gactcttaag atcatatcct gccccaaatt
tcaaattaat gaaatgaaga    2700

tatttcaaac agatctttga aacctcagat tctgtggtgc
aattttaatg ttttcttgtt    2760

tctcagtttt ctgctataaa actattttca attcagtcaa
aaaaaaaaaa aaaaaa      2816


<210>  16
<211>  3579
<212>  DNA
<213>  Homo sapiens

<400>  16
tcaggctcgc tgtcgcgcca ttttgccggg gtttgaatgt
gaggcggagc ggcggcagga      60

gcgggtagtg ccagctacgg tccgcggctg gggttccctc
ctccgtttct gtatccccac     120

gagatcctat agcaatggaa ctcagcgatg caaatctgca
aacactaaca gaatatttaa     180

agaaaacact tgatcctgat cctgccatcc gacgtccagc
tgagaaattt cttgaatctg     240

ttgaaggaaa tcagaattat ccactgttgc ttttgacatt
actggagaag tcccaggata     300

atgttatcaa agtatgtgct tcagtaacat tcaaaaacta
tattaaaagg aactggagaa     360

ttgttgaaga tgaaccaaac aaaatttgtg aagccgatcg
agtggccatt aaagccaaca     420

tagtgcactt gatgcttagc agcccagagc aaattcagaa
gcagttaagt gatgcaatta     480

gcattattgg cagagaagat tttccacaga aatggcctga
cttgctgaca gaaatggtga     540

atcgctttca gagtggagat ttccatgtta ttaatggagt
cctccgtaca gcacattcat     600

tatttaaaag ataccgtcat gaatttaagt caaacgagtt
atggactgaa attaagcttg    660

ttctggatgc ctttgctttg cctttgacta atctttttaa
ggccactatt gaactctgca    720

gtacccatgc aaatgatgcc tctgccctga ggattctgtt
ttcttccctg atcctgatct    780

caaaattgtt ctatagttta aactttcagg atctccctga
atttttgaa gataatatgg    840

aaacttggat gaataatttt catactctct taacattgga
taataagctt ttacaaactg    900

atgatgaaga ggaagccggc ttattggagc tcttaaaatc
ccagatttgt gataatgccg    960

cactctatgc acaaaagtac gatgaagaat tccagcgata
cctgcctcgt tttgttacag   1020

ccatctggaa tttactagtt acaacgggtc aagaggttaa
atatgatttg ttggtaagta   1080

atgcaattca atttctggct tcagtttgtg agagacctca
ttataagaat ctatttgagg   1140

accagaacac gctgacaagt atctgtgaaa aggttattgt
gcctaacatg gaatttagag   1200

ctgctgatga agaagcattt gaagataatt ctgaggagta
cataaggaga gatttggaag   1260

gatctgatat tgatactaga cgcagggctg cttgtgatct
ggtacgagga ttatgcaagt   1320

tttttgaggg acctgtgaca ggaatcttct ctggttatgt
taattccatg ctgcaggaat   1380

acgcaaaaaa tccatctgtc aactggaaac acaaagatgc
agccatctac ctagtgacat   1440

ctttggcatc aaaagcccaa acacagaagc atggaattac
acaagcaaat gaacttgtaa   1500

acctaactga gttctttgtg aatcacatcc tccctgattt

aaaatcagct aatgtgaatg    1560

aatttcctgt ccttaaagct gacggtatca aatatattat
gatttttaga aatcaagtgc    1620

caaaagaaca tcttttagtc tcgattcctc tcttgattaa
tcatcttcaa gctgaaagta    1680

ttgttgttca tacttacgca gctcatgctc ttgaacggct
ctttactatg cgagggccta    1740

acaatgccac tctctttaca gctgcagaaa tcgcaccgtt
tgttgagatt ctgctaacaa    1800

accttttcaa agctctcaca cttcctggct cttcagaaaa
tgaatatatt atgaaagcta    1860

tcatgagaag tttttctctc ctacaagaag ccataatccc
ctacatccct actctcatca    1920

ctcagcttac acagaagcta ttagctgtta gtaagaaccc
aagcaaacct cactttaatc    1980

actacatgtt tgaagcaata tgtttatcca taagaataac
ttgcaaagct aaccctgctg    2040

ctgttgtaaa ttttgaggag gctttgtttt tggtgtttac
tgaaatctta caaaatgatg    2100

tgcaagaatt tattccatac gtctttcaag tgatgtcttt
gcttctggaa acacacaaaa    2160

atgacatccc gtcttcctat atggccttat ttcctcatct
ccttcagcca gtgctttggg    2220

aaagaacagg aaatattcct gctctagtga ggcttcttca
agcattctta gaacgcggtt    2280

caaacacaat agcaagtgct gcagctgaca aaattcctgg
gttactaggt gtctttcaga    2340

agctgattgc atccaaagca aatgaccacc aaggttttta
tcttctaaac agtataatag    2400

agcacatgcc tcctgaatca gttgaccaat ataggaaaca
aatcttcatt ctgctattcc    2460

agagacttca gaattccaaa acaaccaagt ttatcaagag
tttttagtc tttattaatt   2520

tgtattgcat aaaatatggg gcactagcac tacaagaaat
atttgatggt atacaaccaa   2580

aaatgtttgg aatggttttg gaaaaaatta ttattcctga
aattcagaag gtatctggaa   2640

atgtagagaa aaagatctgt gcggttggca taaccaaatt
actaacagaa tgtcccccaa   2700

tgatggacac tgagtatacc aaactgtgga ctccattatt
acagtctttg attggtcttt   2760

ttgagttacc cgaagatgat accattcctg atgaggaaca
ttttattgac atagaagata   2820

caccaggata tcagactgcc ttctcacagt tggcatttgc
tgggaaaaaa gagcatgatc   2880

ctgtaggtca aatggtgaat aaccccaaaa ttcacctggc
acagtcactt cacaagttgt   2940

ctaccgcctg tccaggaagg gttccatcaa tggtgagcac
cagcctgaat gcagaagcgc   3000

tccagtatct ccaagggtac cttcaggcag ccagtgtgac
actgctttaa actgcatttt   3060

tctaatgggc taaacccaga tggtttccta ggaaatcaca
ggcttctgag cacagctgca   3120

ttaaaacaaa ggaagttctc cttttgaact tgtcacgaat
tccatcttgt aaaggatatt   3180

aaatgttgct ttaacctgaa ccttgagcaa attagttggt
ttgtgtgatc atacagttat   3240

gtgggtggct tctagtttgc aacttcaagg gacaagtatt
aatagttcag tgtatggcgt   3300

tggtttgtgt tgagcgtttg cacggtttgg ataatcttaa
attttgacgg acactgtgga   3360

gactttctgt tactaaatcc ttttgttttg aagctgttgc
tatttgtatt tctcttgtcc    3420

tttatatttt ttgtctgttt atttacgctt ttattggaaa
tgtgaataag taaagaatta    3480

cttgtgttac ttgccaagca gtgcacattt catagtttca
aatctgtaat cagcaataaa    3540

aatcctaaaa tatgtaccta aaaaaaaaaa aaaaaaaaa
                    3579


<210>  17
<211>  1384
<212>  DNA
<213>  Homo sapiens

<400>  17
accgttcttt taactgcgca ggcgcgccgg aagcacctag
agagcggcgc gtgcgcagcg      60

ggagtcgaag cggagatccc ggggtcgcgc gagagccgca
agcggagttg gtgggcgcta     120

tgctatcacc cgaggcagag cgagtgctgc ggtaccttgt
agaagtggag gagctcgccg     180

aggaggtgct ggcggacaag cggcagattg tggacctgga
cactaaaagg aatcagaatc     240

gagagggcct gagggccctg cagaaggatc tcagcctctc
tgaagatgtg atggtttgct     300

tcgggaacat gtttatcaag atgcctcacc ctgagacaaa
ggaaatgatt gaaaaagatc     360

aagatcatct ggataaagaa atagaaaaac tgcggaagca
acttaaagtg aaggtcaacc     420

gcctttttga ggcccaaggc aaaccggagc tgaagggttt
taacttgaac cccctcaacc     480

aggatgagct taaagctctc aaggtcatct tgaaaggatg
agactcaaga accaagatgg     540

gggaccagca accccccagg gtcatggagg acccaggacc
ctccaacctt gacacctgta    600

aggacaggat ctgccctgta aggggccagc cgtcaggaat
ctggccatga aaacctcttt    660

gtagtgcttg gctactctgt gatggcagga gggaaccttc
agcctgtctg gctgctggac    720

ctggacacca gggctcggtg gacacaagat ctattgacgg
gccttggtag ccaccagtgg    780

gtgtgtgggg cagtggctgt gggggtgtaa gaatgactgc
aacaggcact tcccaacaat    840

ggcctgctgt tcacatggac cctgagcaag gaaggaggga
gggaggggca gagtggagtg    900

tcattccagc attcctctca gaagggagag aggttttcag
gctggtgcca tgcgattgga    960

ataaagcagg aggctcatgg gtggttgctg aatgaagaac
agaatcttgg tgctttgtgg   1020

ctcaccacag ccatctgtgg ggcaggcaca cacacctccc
gccagctcca attttgcact   1080

ttttccctgc ttgattccaa gagtaggtgc tgcctagcag
cccttcgtgg ccactcttta   1140

ctcaggaggg ccttgcagag tcctgcacca ggcctgggtg
agtggatgcg cctcttacca   1200

tatgacacgt gtcaagatgc ccttccgccc cctctgaaag
tggggcccgg ccagcactgc   1260

tcgttactgt ctgccttcag tggtctgagg tcccagtatg
aactgccgtg aagtcaaaac   1320

tcttatgtgt tcattaaggg ctcaataaat gttagctgaa
tgaatgaata gcaaaaaaaa   1380

aaaa                    1384

<210>  18
<211>  3685
<212>  DNA
<213>  Homo sapiens

<400>  18
agtggactca cgcaggcgca ggagactaca cttcccagga
actccgggcc gcgttgttcg     60

ctggtacctc cttctgactt ccggtattgc tgcggtctgt
agggccaatc gggagcctgg    120

aattgctttc ccggcgctct gattggtgca ttcgactagg
ctgcctgggt tcaaaatttc    180

aacgatactg aatgagtccc gcggcgggtt ggctcgcgct
tcgttgtcag atctgaggcg    240

aggctaggtg agccgtggga agaaaagagg gagcagctag
ggcgcgggtc tccctcctcc    300

cggagtttgg aacggctgaa gttcaccttc cagcccctag
cgccgttcgc gccgctaggc    360

ctggcttctg aggcggttgc ggtgctcggt cgccgcctag
gcggggcagg gtgcgagcag    420

gggcttcggg ccacgcttct cttggcgaca ggattttgct
gtgaagtccg tccgggaaac    480

ggaggaaaaa aagagttgcg ggaggctgtc ggctaataac
ggttcttgat acatatttgc    540

cagacttcaa gatttcagaa aaggggtgaa agagaagatt
gcaactttga gtcagacctg    600

taggcctgat agactgatta aaccacagaa ggtgacctgc
tgagaaaagt ggtacaaata    660

ctgggaaaaa cctgctcttc tgcgttaagt gggagacaat
gtcacaagtt aaaagctctt    720

attcctatga tgccccctcg gatttcatca atttttcatc
cttggatgat gaaggagata    780

ctcaaaacat agattcatgg tttgaggaga aggccaattt

ggagaataag ttactgggga    840

agaatggaac tggagggctt tttcagggca aaactccttt
gagaaaggct aatcttcagc    900

aagctattgt cacacctttg aaaccagttg acaacactta
ctacaaagag gcagaaaaag    960

aaaatcttgt ggaacaatcc attccgtcaa atgcttgttc
ttccctggaa gttgaggcag   1020

ccatatcaag aaaaactcca gcccagcctc agagaagatc
tcttaggctt tctgctcaga   1080

aggatttgga acagaaagaa aagcatcatg taaaaatgaa
agccaagaga tgtgccactc   1140

ctgtaatcat cgatgaaatt ctaccctcta agaaaatgaa
agtttctaac aacaaaaaga   1200

agccagagga agaaggcagt gctcatcaag atactgctga
aaagaatgca tcttccccag   1260

agaaagccaa gggtagacat actgtgcctt gtatgccacc
tgcaaagcag aagtttctaa   1320

aaagtactga ggagcaagag ctggagaaga gtatgaaaat
gcagcaagag gtggtggaga   1380

tgcggaaaaa gaatgaagaa ttcaagaaac ttgctctggc
tggaataggg caacctgtga   1440

agaaatcagt gagccaggtc accaaatcag ttgacttcca
cttccgcaca gatgagcgaa   1500

tcaaacaaca tcctaagaac caggaggaat ataaggaagt
gaactttaca tctgaactac   1560

gaaagcatcc ttcatctcct gcccgagtga ctaagggatg
taccattgtt aagcctttca   1620

acctgtccca aggaaagaaa agaacatttg atgaaacagt
ttctacatat gtgccccttg   1680

cacagcaagt tgaagacttc cataaacgaa cccctaacag
atatcatttg aggagcaaga   1740

aggatgatat taacctgtta ccctccaaat cttctgtgac
caagatttgc agagacccac   1800

agactcctgt actgcaaacc aaacaccgtg cacgggctgt
gacctgcaaa agtacagcag   1860

agctggaggc tgaggagctc gagaaattgc aacaatacaa
attcaaagca cgtgaacttg   1920

atcccagaat acttgaaggt gggcccatct tgcccaagaa
accacctgtg aaaccaccca   1980

ccgagcctat tggctttgat ttggaaattg agaaaagaat
ccaggagcga gaatcaaaga   2040

agaaaacaga ggatgaacac tttgaatttc attccagacc
ttgccctact aagattttgg   2100

aagatgttgt gggtgttcct gaaaagaagg tacttccaat
caccgtcccc aagtcaccag   2160

cctttgcatt gaagaacaga attcgaatgc ccaccaaaga
agatgaggaa gaggacgaac   2220

cggtagtgat aaaagctcaa cctgtgccac attatggggt
gccttttaag ccccaaatcc   2280

cagaggcaag aactgtggaa atatgccctt tctcgtttga
ttctcgagac aaagaacgtc   2340

agttacagaa ggagaagaaa ataaaagaac tgcagaaagg
ggaggtgccc aagttcaagg   2400

cacttccctt gcctcatttt gacaccatta acctgccaga
gaagaaggta aagaatgtga   2460

cccagattga acctttctgc ttggagactg acagaagagg
tgctctgaag gcacagactt   2520

ggaagcacca gctggaagaa gaactgagac agcagaaaga
agcagcttgt ttcaaggctc   2580

gtccaaacac cgtcatctct caggagccct ttgttcccaa
gaaagagaag aaatcagttg   2640

ctgagggcct ttctggttct ctagttcagg aacctttca
gctggctact gagaagagag   2700

ccaaagagcg gcaggagctg gagaagagaa tggctgaggt
agaagcccag aaagcccagc   2760

agttggagga ggccagacta caggaggaag agcagaaaaa
agaggagctg gccaggctac   2820

ggagagaact ggtgcataag gcaaatccaa tacgcaagta
ccagggtctg gagataaagt   2880

caagtgacca gcctctgact gtgcctgtat ctcccaaatt
ctccactcga ttccactgct   2940

aaactcagct gtgagctgcg gataccgccc ggcaatggga
cctgctctta acctcaaacc   3000

taggaccgtc ttgctttgtc attgggcatg gagagaaccc
atttctccag acttttacct   3060

acccgtgcct gagaaagcat acttgacaac tgtggactcc
agttttgttg agaattgttt   3120

tcttacatta ctaaggctaa taatgagatg taactcatga
atgtctcgat tagactccat   3180

gtagttactt cctttaaacc atcagccggc cttttatatg
ggtcttcact ctgactagaa   3240

tttagtctct gtgtcagcac agtgtaatct ctattgctat
tgccccttac gactctcacc   3300

ctctccccac tttttttaaa aattttaacc agaaaataaa
gatagttaaa tcctaagata   3360

gagattaagt catggtttaa atgaggaaca atcagtaaat
cagattctgt cctcttctct   3420

gcataccgtg aatttatagt taaggatccc tttgctgtga
gggtagaaaa cctcaccaac   3480

tgcaccagtg aggaagaaga ctgcgtggat tcatggggag
cctcacagca gccacgcagc   3540

aggctctggg tggggctgcc gttaaggcac gttctttcct

tactggtgct gataacaaca   3600

gggaaccgtg cagtgtgcat tttaagacct ggcctggaat
aaatacgttt tgtctttccc   3660

tcaaaaaaaa aaaaaaaaaa aaaaa
                3685



<210>  19
<211>  2612
<212>  DNA
<213>  Homo sapiens

<400>  19
cccgccctgc cccgcctgcc cgccctggtg gccgtctggg
ggcgacaagt cctgagagaa     60

ccagacggaa gcgcgctggg actgacacgt ggacttgggc
ggtgctgccc gggtgggtca    120

gcctgggctg ggaggcagcc ccgggacaca gctgtgccca
cgccgtctga gcaccccaag    180

cccgatgcag ccacccccag acgaggcccg cagggacatg
gccggggaca cccagtggtc    240

caggcccgag tgccaggcat ggacggggac gctgctgctg
ggcacatgcc ttctgtactg    300

cgcccgctcc agcatgccca tctgcaccgt ctccatgagc
caggacttcg gctggaacaa    360

gaaggaggcc ggcatcgtgc tcagcagctt cttctggggc
tactgcctga cacaggttgt    420

gggcggccac ctcggggatc ggattggggg tgagaaggtc
atcctgctgt cagcctctgc    480

ctggggctcc atcacggccg tcaccccact gctcgcccac
ctgagcagtg cccacctggc    540

cttcatgacc ttctcacgca tcctcatggg cttgctccaa
ggggtttact tccctgccct    600

gaccagcctg ctgtcgcaga aggtgcggga gagtgagcga

gccttcacct acagcatcgt    660

gggcgccggc tcccagtttg ggacgctgct gaccggggcg
gtgggctccc tgctcctgga    720

atggtacggc tggcagagca tcttctattt ctccggcggc
ctcaccttgc tttgggtgtg    780

gtacgtgtac aggtacctgc tgagtgaaaa agatctcatc
ctggccttgg gtgtcctggc    840

ccaaagccgg ccggtgtcca ggcacagcag agtcccctgg
agacggctct tccggaagcc    900

tgctgtctgg gcagccgtcg tctcccagct ctctgcagcc
tgctccttct tcatcctcct    960

ctcctggctg cccaccttct tcgaggagac cttccccgac
gccaagggct ggatcttcaa    1020

cgtggttcct tggttggtgg cgattccggc cagtctattc
agcgggtttc tctctgatca    1080

tctcatcaat cagggttaca gagccatcac ggtgcggaag
ctcatgcagg gcatgggcct    1140

tggcctctcc agcgtctttg ctctgtgcct gggccacacc
tccagcttct gtgagtctgt    1200

ggtctttgca tcagcctcca tcggcctcca gaccttcaac
cacagtggca tttctgttaa    1260

catccaggac ttggccccgt cctgcgccgg ctttctgttt
ggtgtggcca acacagccgg    1320

ggccttggca ggtgtcgtgg gtgtgtgtct aggcggctac
ttgatggaga ccacgggctc    1380

ctggacttgc ctgttcaacc ttgtggccat catcagcaac
ctggggctgt gcaccttcct    1440

ggtgtttgga caggctcaga gggtggacct gagctctacc
catgaggacc tctagctccc    1500

aaccccacag cctctccaag gacccaggcg ccagcagccc
caggacacag ggactcagt    1560

gtgtgggact tggtcactcc atgtcagaca cacgagcaga
gaggaacaca aaccactgtg   1620

gagcctgaag ctccttaaga agagtccaca acagctggtg
ggagggtggg gtgggcctgg   1680

gtccagacca ggctcgctgc tctctgggcc tcagtttccc
cacctgccag cgggctcggc   1740

cctgtcctcc tcacaggctg gtgtggccgt cagggtgggt
ggggttattg ttagtaggcg   1800

cagcctcatt cccaccacga tctgttccgc gtggttcccg
ccaaacctcc ctcggtcgcc   1860

gtgttctccg caagcctcct gcagcgcccg cctgccaatg
tgaggctggc accaggctgc   1920

agcctcccca atcccagccc actttgctgt gtctctggcg
ggctgtcctc cttggtggga   1980

gctgtcctgc acactgtagg atgcttaaag gtatccctgg
cctccaccca cccctagcca   2040

gcagctccca gtcagacaac agccagaaat gtctccagac
tctgcccagc ctccccaggt   2100

agccaccctc gagacacgac ctcagagtct ctgtgtctcc
tagaagcctg acagagaccc   2160

ccagggcagt gggtgggtgg cgggctagag acccttgcct
gtgtccggga ccctggcgcc   2220

gctctcccct cctgtggatc cctccgcact aacagtgttc
tcagtgggca gacgcctggg   2280

caccccttgg gccctgccca gcatggccat ggcgcaggct
ctcgaacccg catggctttc   2340

ccaggcctgg tgattctgct ctccagggac ggttggcacc
ttcctcgggg gcgggcccca   2400

cgcaccccag aacacacaga cccacctttc tggcgttctt
tctacctccc ttttcgttgc   2460

ctgaggagct ggtggtttca tgagttaatg atacatcttg
caaggtgtac acatagagaa    2520

aaaaacctaa aaatgtggaa aagcacgcca aagccttatt
taaataataa ctattaaact    2580

attcaaaaag aaaaaaaaaa aaaaaaaaaa aa
                    2612

<210>  20
<211>  823
<212>  DNA
<213>  Homo sapiens

<400>  20
aaacgcgggc gggcgggccc gcagtcctgc agttgcagtc
gtgttctccg agttcctgtc      60

tctctgccaa cgccgcccgg atggcttccc aaaaccgcga
cccagccgcc actagcgtcg     120

ccgccgcccg taaaggagct gagccgagcg gggcgccgc
ccggggtccg gtgggcaaaa     180

ggctacagca ggagctgatg accctcatga tgtctggcga
taaagggatt tctgccttcc     240

ctgaatcaga caaccttttc aaatgggtag ggaccatcca
tggagcagct ggaacagtat     300

atgaagacct gaggtataag ctctcgctag agttccccag
tggctaccct tacaatgcgc     360

ccacagtgaa gttcctcacg ccctgctatc accccaacgt
ggacacccag ggtaacatat     420

gcctggacat cctgaaggaa aagtggtctg ccctgtatga
tgtcaggacc attctgctct     480

ccatccagag ccttctagga gaacccaaca ttgatagtcc
cttgaacaca catgctgccg     540

agctctggaa aaaccccaca gcttttaaga agtacctgca
agaaacctac tcaaagcagg     600

tcaccagcca ggagccctga cccaggctgc ccagcctgtc
cttgtgtcgt ctttttaatt    660

tttccttaga tggtctgtcc tttttgtgat ttctgtatag
gactctttat cttgagctgt    720

ggtatttttg ttttgttttt gtcttttaaa ttaagcctcg
gttgagccct tgtatattaa    780

ataaatgcat ttttgtcctt ttttagacaa aaaaaaaaaa aaa
                    823

<210>  21
<211>  7923
<212>  DNA
<213>  Homo sapiens

<400>  21
agcgacggcg gcggctgcgg cttagtcggt ggcggccggc
ggcggctgcg ggctgagcgg     60

cgagtttccg atttaaagct gagctgcgag gaaaatggcg
gcgggaggat caaaatactt    120

gctggatggt ggactcagag accaataaaa ataaactgct
tgaacatcct ttgactggtt    180

agccagttgc tgatgtatat tcaagatgag tggattagga
gaaaacttgg atccactggc    240

cagtgattca cgaaaacgca aattgccatg tgatactcca
ggacaaggtc ttacctgcag    300

tggtgaaaaa cggagacggg agcaggaaag taaatatatt
gaagaattgg ctgagctgat    360

atctgccaat cttagtgata ttgacaattt caatgtcaaa
ccagataaat gtgcgatttt    420

aaaggaaaca gtaagacaga tacgtcaaat aaaagagcaa
ggaaaaacta tttccaatga    480

tgatgatgtt caaaaagccg atgtatcttc tacagggcag
ggagttattg ataaagactc    540

cttaggaccg cttttacttc aggcattgga tggtttccta
tttgtggtga atcgagacgg    600

aaacattgta tttgtatcag aaaatgtcac acaatacctg
caatataagc aagaggacct    660

ggttaacaca agtgtttaca atatcttaca tgaagaagac
agaaaggatt ttcttaagaa    720

tttaccaaaa tctacagtta atggagtttc ctggacaaat
gagacccaaa gacaaaaaag    780

ccatacattt aattgccgta tgttgatgaa aacaccacat
gatattctgg aagacataaa    840

cgccagtcct gaaatgcgcc agagatatga aacaatgcag
tgctttgccc tgtctcagcc    900

acgagctatg atggaggaag ggaagatttt gcaatcttgt
atgatctgtg tggcacgccg    960

cattactaca ggagaaagaa catttccatc aaaccctgag
agctttatta ccagacatga   1020

tctttcagga aaggttgtca atatagatac aaattcactg
agatcctcca tgaggcctgg   1080

ctttgaagat ataatccgaa ggtgtattca gagatttttt
agtctaaatg atgggcagtc   1140

atggtcccag aaacgtcact atcaagaagc ttatcttaat
ggccatgcag aaaccccagt   1200

atatcgattc tcgttggctg atggaactat agtgactgca
cagacaaaaa gcaaactctt   1260

ccgaaatcct gtaacaaatg atcgacatgg ctttgtctca
acccacttcc ttcagagaga   1320

acagaatgga tatagaccaa acccaaatcc tgttggacaa
gggattagac cacctatggc   1380

tggatgcaac agttcggtag gcggcatgag tatgtcgcca
aaccaaggct tacagatgcc   1440

gagcagcagg gcctatggct tggcagaccc tagcaccaca

gggcagatga gtggagctag   1500

gtatgggggt tccagtaaca tagcttcatt gacccctggg
ccaggcatgc aatcaccatc   1560

ttcctaccag aacaacaact atgggctcaa catgagtagc
cccccacatg ggagtcctgg   1620

tcttgcccca aaccagcaga atatcatgat ttctcctcgt
aatcgtggga gtccaaagat   1680

agcctcacat cagttttctc ctgttgcagg tgtgcactct
cccatggcat cttctggcaa   1740

tactgggaac cacagctttt ccagcagctc tctcagtgcc
ctgcaagcca tcagtgaagg   1800

tgtggggact tcccttttat ctactctgtc atcaccaggc
cccaaattgg ataactctcc   1860

caatatgaat attacccaac caagtaaagt aagcaatcag
gattccaaga gtcctctggg   1920

cttttattgc gaccaaaatc cagtggagag ttcaatgtgt
cagtcaaata gcagagatca   1980

cctcagtgac aaagaaagta aggagagcag tgttgagggg
gcagagaatc aaaggggtcc   2040

tttggaaagc aaaggtcata aaaaattact gcagttactt
acctgttctt ctgatgaccg   2100

gggtcattcc tccttgacca actcccccct agattcaagt
tgtaaagaat cttctgttag   2160

tgtcaccagc ccctctggag tctcctcctc tacatctgga
ggagtatcct ctacatccaa   2220

tatgcatggg tcactgttac aagagaagca ccggattttg
cacaagttgc tgcagaatgg   2280

gaattcacca gctgaggtag ccaagattac tgcagaagcc
actgggaaag acaccagcag   2340

tataacttct tgtggggacg gaaatgttgt caagcaggag
cagctaagtc ctaagaagaa   2400

ggagaataat gcacttctta gatacctgct ggacagggat
gatcctagtg atgcactctc   2460

taaagaacta cagccccaag tggaaggagt ggataataaa
atgagtcagt gcaccagctc   2520

caccattcct agctcaagtc aagagaaaga ccctaaaatt
aagacagaga caagtgaaga   2580

gggatctgga gacttggata atctagatgc tattcttggt
gatctgacta gttctgactt   2640

ttacaataat tccatatcct caaatggtag tcatctgggg
actaagcaac aggtgtttca   2700

aggaactaat tctctgggtt tgaaaagttc acagtctgtg
cagtctattc gtcctccata   2760

taaccgagca gtgtctctgg atagccctgt ttctgttggc
tcaagtcctc cagtaaaaaa   2820

tatcagtgct ttccccatgt taccaaagca acccatgttg
ggtgggaatc caagaatgat   2880

ggatagtcag gaaaattatg gctcaagtat gggtgggcca
aaccgaaatg tgactgtgac   2940

tcagactcct tcctcaggag actggggctt accaaactca
aaggccggca gaatggaacc   3000

tatgaattca aactccatgg gaagaccagg aggagattat
aatacttctt tacccagacc   3060

tgcactgggt ggctctattc ccacattgcc tcttcggtct
aatagcatac caggtgcgag   3120

accagtattg caacagcagc agcagatgct tcaaatgagg
cctggtgaaa tccccatggg   3180

aatgggggct aatccctatg gccaagcagc agcatctaac
caactgggtt cctggcccga   3240

tggcatgttg tccatggaac aagtttctca tggcactcaa
aataggcctc ttcttaggaa   3300

ttccctggat gatcttgttg ggccaccttc caacctggaa
ggccagagtg acgaaagagc   3360

attattggac cagctgcaca ctcttctcag caacacagat
gccacaggcc tggaagaaat   3420

tgacagagct ttgggcattc ctgaacttgt caatcaggga
caggcattag agcccaaaca   3480

ggatgctttc caaggccaag aagcagcagt aatgatggat
cagaaggcag gattatatgg   3540

acagacatac ccagcacagg ggcctccaat gcaaggaggc
tttcatcttc agggacaatc   3600

accatctttt aactctatga tgaatcagat gaaccagcaa
ggcaattttc ctctccaagg   3660

aatgcaccca cgagccaaca tcatgagacc ccggacaaac
acccccaagc aacttagaat   3720

gcagcttcag cagaggctgc agggccagca gtttttgaat
cagagccgac aggcacttga   3780

attgaaaatg gaaaacccta ctgctggtgg tgctgcggtg
atgaggccta tgatgcagcc   3840

ccagcagggt tttcttaatg ctcaaatggt cgcccaacgc
agcagagagc tgctaagtca   3900

tcacttccga caacagaggg tggctatgat gatgcagcag
cagcagcagc agcaacagca   3960

gcagcagcag cagcagcagc agcaacagca acagcaacag
caacagcagc aacagcagca   4020

aacccaggcc ttcagcccac ctcctaatgt gactgcttcc
cccagcatgg atgggctttt   4080

ggcaggaccc acaatgccac aagctcctcc gcaacagttt
ccatatcaac caaattatgg   4140

aatgggacaa caaccagatc cagcctttgg tcgagtgtct
agtcctccca atgcaatgat   4200

gtcgtcaaga atgggtccct cccagaatcc catgatgcaa

cacccgcagg ctgcatccat    4260

ctatcagtcc tcagaaatga agggctggcc atcaggaaat
ttggccagga acagctcctt    4320

ttcccagcag cagtttgccc accaggggaa tcctgcagtg
tatagtatgg tgcacatgaa    4380

tggcagcagt ggtcacatgg gacagatgaa catgaacccc
atgcccatgt ctggcatgcc    4440

tatgggtcct gatcagaaat actgctgaca tctctgcacc
aggacctctt aaggaaacca    4500

ctgtacaaat gacactgcac taggattatt gggaaggaat
cattgttcca ggcatccatc    4560

ttggaagaaa ggaccagctt tgagctccat caagggtatt
ttaagtgatg tcatttgagc    4620

aggactggat tttaagccga agggcaatat ctacgtgttt
ttccccccctc cttctgctgt    4680

gtatcatggt gttcaaaaca gaaatgtttt ttggcattcc
acctcctagg gatataattc    4740

tggagacatg gagtgttact gatcataaaa cttttgtgtc
actttttttct gccttgctag    4800

ccaaaatctc ttaaatacac gtaggtgggc cagagaacat
tggaagaatc aagagagatt    4860

agaatatctg gtttctctag ttgcagtatt ggacaaagag
catagtccca gccttcaggt    4920

gtagtagttc tgtgttgacc ctttgtccag tggaattggt
gattctgaat tgtcctttac    4980

taatggtgtt gagttgctct gtccctatta tttgccctag
gctttctcct aatgaaggtt    5040

ttcatttgcc attcatgtcc tgtaatactt cacctccagg
aactgtcatg gatgtccaaa    5100

tggctttgca gaaaggaaat gagatgacag tatttaatcg
cagcagtagc aaactttca    5160

catgctaatg tgcagctgag tgcactttat ttaaaaagaa
tggataaatg caatattctt   5220

gaggtcttga gggaatagtg aaacacattc ctggtttttg
cctacactta cgtgttagac   5280

aagaactatg attttttttt ttaaagtact ggtgtcaccc
tttgcctata tggtagagca   5340

ataatgcttt ttaaaaataa acttctgaaa acccaaggcc
aggtactgca ttctgaatca   5400

gaatctcgca gtgtttctgt gaatagattt ttttgtaaat
atgaccttta agatattgta   5460

ttatgtaaaa tatgtatata cctttttttg taggtcacaa
caactcattt ttacagagtt   5520

tgtgaagcta aatatttaac attgttgatt tcagtaagct
gtgtggtgag gctaccagtg   5580

gaagagacat cccttgactt ttgtggcctg gggggagggt
agtgctccac agcttttcct   5640

tccccacccc ccagccttag atgcctcgct cttttcaatc
tcttaatcta aatgcttttt   5700

aaagagatta tttgtttaga tgtaggcatt ttaatttttt
aaaaattcct ctaccagaac   5760

taagcacttt gttaatttgg ggggaaagaa tagatatggg
gaaataaact taaaaaaaaa   5820

tcaggaattt aaaaaaacga gcaatttgaa gagaatcttt
tggattttaa gcagtccgaa   5880

ataatagcaa ttcatgggct gtgtgtgtgt gtgtatgtgt
gtgtgtgtgt gtgtatgttt   5940

aattatgtta ccttttcatc ccctttagga gcgttttcag
attttggttg ctaagacctg   6000

aatcccatat tgagatctcg agtagaatcc ttggtgtggt
ttctggtgtc tgctcagctg   6060

tcccctcatt ctactaatgt gatgctttca ttatgtccct
gtggattaga atagtgtcag   6120

ttatttctta agtaactcag tacccagaac agccagtttt
actgtgattc agagccacag   6180

tctaactgag caccttttaa acccctccct cttctgcccc
ctaccacttt tctgctgttg   6240

cctctctttg acacctgttt tagtcagttg ggaggaaggg
aaaaatcaag tttaattccc   6300

tttatctggg ttaattcatt tggttcaaat agttgacgga
attgggtttc tgaatgtctg   6360

tgaatttcag aggtctctgc tagccttggt atcattttct
agcaataact gagagccagt   6420

taattttaag aatttcacac atttagccaa tctttctaga
tgtctctgaa ggtaagatca   6480

tttaatatct ttgatatgct tacgagtaag tgaatcctga
ttatttccag acccaccacc   6540

agagtggatc ttattttcaa agcagtatag acaattatga
gtttgccctc tttcccctac   6600

caagttcaaa atatatctaa gaaagattgt aaatccgaaa
acttccattg tagtggcctg   6660

tgcttttcag atagtatact ctcctgtttg gagacagagg
aagaaccagg tcagtctgtc   6720

tctttttcag ctcaattgta tctgaccctt ctttaagtta
tgtgtgtggg gagaaataga   6780

atggtgctct tatctttctt gactttaaaa aaattattaa
aaacaaaaaa aaaataaatt   6840

tttttgcaat cctttcctca gacctggctc caggctaact
ggaaggcagc actccctttt   6900

ttatatagta gaaaaatgaa gtttattata agtttttata
ttttctactt gttcatttgg   6960

tgcaaactca agatttcttt taataggtgc agtctttgag

ataatttgtt tttacctgta   7020

ttgcccttta tcttttttag gtaattcttt gtactcctgc
tgtctacctc tcctcacacc   7080

ccagcacccc ccattttttc aaaccttggt atctgttggg
tgaacagtat aatcttttca   7140

tctgctttta gaatgtggga tatttccagt acctactttt
tttttttttt tttgctgaat   7200

ccaaagatat ataaataaaa tatatatatt ttataaagat
cagaatgata taaaggagat   7260

acatgtttct tcctttaaaa aataaacgga agttacattg
ttaatgttca tattatgatg   7320

ccacttttct aaactgcatc tggattgaaa ggtgtaaata
tcaataacag tgctacttag   7380

ttatcagtat ttaatatctg aggtgagttg ggggtatcta
tattaggggt agggtattac   7440

agaagataat tggcttgatg tcctagaagt tctttgatcc
agaggtgggt gcagctgaaa   7500

gtaaacagaa tggattgcca gttacatgta tgcctgccca
gttccctttt tatttgcaga   7560

agctgtgagt tttgttcaca attaggttcc taggagcaaa
acctcaagga ttgatttatt   7620

gttttcaact ccaaggcaca ctgttaataa acgagcaggg
tgttttctct cttcctttct   7680

aatatatgga gtttcgaaga ataaaatatg agagcaatat
ttaaattctc aggaattgac   7740

ttatactctt gagaatgaat tcagtttcaa tcaagtttac
attatgttgc ttaaaaaaat   7800

agaaattatt ctttatcttg caaagaattg aaaccacatg
aaatgactta tgggggatgg   7860

tgagctgtga ctgctttgct gaccattttg gatgtcattg
taaataaagg tttctattta   7920

aaa
                    7923

<210>  22
<211>  3120
<212>  DNA
<213>  Homo sapiens

<400>  22
gggcggggcg tgtgcggact gagcgctctg cttccggggc
gcgggtgacg cgacgacggc     60

gacactttgc tacggagtgc atcggacgtc gaagcctaga
gtctctgcgt ctttccctct    120

tccgctgcct cattcctttc cttcctagcc ttggtcgtcg
ccgccaccat gaacaagaag    180

aagaaaccgt tcctagggat gcccgcgccc ctcggctacg
tgccggggct gggccggggc    240

gccactggct tcaccacgcg gtcagacatt gggcccgccc
gtgatgcaaa tgaccctgtg    300

gatgatcgcc atgcaccccc aggcaagaga accgttgggg
accagatgaa gaaaaatcag    360

gctgctgacg atgacgacga ggatctaaat gacaccaatt
acgatgagtt taatggctat    420

gctgggagcc tcttctcaag tggaccctac gagaaagatg
atgaggaagc agatgctatc    480

tatgcagccc tggataaaag gatggatgaa agaagaaaag
aaagacggga gcaaagggag    540

aaagaagaaa tagagaaata tcgtatggaa cgccccaaaa
tccaacagca gttctcagac    600

ctcaagagga agttggcaga agtcacagaa gaagagtggc
tgagcatccc cgaggttggc    660

gatgccagaa ataaacgtca gcggaaccca cgctatgaga
agctgacccc tgttcctgac    720

agtttctttg ccaaacattt acagaccgga gagaaccata
cctcagtgga tccccgacaa    780

actcaatttg gaggtcttaa cacaccctat ccaggtggac
taaacactcc atacccaggt    840

ggaatgacgc caggactgat gacacctggc acaggtgagc
tggacatgag gaagattggc    900

caagcgagga acactctgat ggacatgagg ctgagccagg
tgtctgactc cgtgagtgga    960

cagaccgtcg ttgaccccaa aggctacctg acggatttaa
attccatgat cccgacacac   1020

ggaggagaca tcaatgatat caagaaggcg cgactgctcc
tcaagtctgt tcgggagacg   1080

aaccctcatc acccgccagc ctggattgca tcagcccgcc
tggaagaagt cactgggaag   1140

ctacaagtag ctcggaacct tatcatgaag gggacggaga
tgtgccccaa gagtgaagat   1200

gtctggctgg aagcagccag gttgcagcct ggggacacag
ccaaggccgt ggtagcccaa   1260

gctgtccgtc atctcccaca gtctgtcagg atttacatca
gagccgcaga gctggaaacg   1320

gacattcgtg caaagaagcg ggttcttcgg aaagccctcg
agcatgttcc aaactcggtt   1380

cgcttgtgga aagcagccgt tgagctggaa gaacctgaag
atgctagaat catgctgagc   1440

cgagctgtgg agtgctgccc caccagcgtg gagctctggc
ttgctctggc aaggctggag   1500

acctatgaaa atgcccgcaa ggtcttgaac aaggcgcggg
agaacattcc tacagaccga   1560

catatctgga tcacggctgc taagctggag gaagccaatg
ggaacacgca gatggtggag   1620

aagatcatcg accgagccat cacctcgctg cgggccaacg
gtgtggagat caaccgtgag   1680

cagtggatcc aggatgccga ggaatgtgac agggctggga
gtgtggccac ctgccaggcc   1740

gtcatgcgtg ccgtgattgg gattgggatt gaggaggaag
atcggaagca tacctggatg   1800

gaggatgctg acagttgtgt agcccacaat gccctggagt
gtgcacgagc catctacgcc   1860

tacgccctgc aggtgttccc cagcaagaag agtgtgtggc
tgcgcgccgc gtacttcgag   1920

aagaaccatg gcactcggga gtccctggaa gcactcctgc
agagggctgt ggcccactgc   1980

cccaaagcag aggtgctgtg gctcatgggc gccaagtcca
agtggctggc aggggatgtg   2040

cctgcagcaa ggagcatcct ggccctggcc ttccaggcca
accccaacag tgaggagatc   2100

tggctggcag ccgtgaagct ggagtccgag aatgatgagt
acgagcgggc ccggaggctg   2160

ctggccaagg cgcggagcag tgcccccacc gcccgggtgt
tcatgaagtc tgtgaagctg   2220

gagtgggtgc aagacaacat cagggcagcc caagatctgt
gcgaggaggc cctgcggcac   2280

tatgaggact tccccaagct gtggatgatg aaggggcaga
tcgaggagca gaaggagatg   2340

atggagaagg cgcgggaagc ctataaccag gggttgaaga
agtgtcccca ctccacaccc   2400

ctgtggcttt tgctctctcg gctggaggag aagattgggc
agcttactcg agcacgggcc   2460

attttggaaa agtctcgtct gaagaaccca aagaaccctg
ggctgtggtt ggagtccgtg   2520

cggctggagt accgtgcggg gctgaagaac atcgcaaata

cactcatggc caaggcgctg   2580

caggagtgcc ccaactccgg tatcctgtgg tctgaggcca
tcttcctcga ggcaaggccc   2640

cagaggagga ccaagagcgt ggatgccctg aagaagtgtg
agcatgaccc ccatgtgctc   2700

ctggccgtgg ccaagctgtt ttggagtcag cggaagatca
ccaaggccag ggagtggttc   2760

caccgcactg tgaagattga ctcggacctg gggatgcct
gggccttctt ctacaagttt   2820

gagctgcagc atggcactga ggagcagcag gaggaggtga
ggaagcgctg tgagagtgca   2880

gagcctcggc atggggagct gtggtgcgcc gtgtccaagg
acatcgccaa ctggcagaag   2940

aagatcgggg acatccttag gctggtggcc ggccgcatca
agaacacctt ctgattgagc   3000

ggttgccatg gccggtctcc gtggggcagg gttgggccgc
atgtggaagg gctctgagct   3060

gtgtcctcct tcattaaaag tttttatgtc tcgtgtcaga
aaaaaaaaaa aaaaaaaaaa   3120


<210>  23
<211>  2338
<212>  DNA
<213>  Homo sapiens

<400>  23
aaaaggagga cgtagaaaag gggacaccgg aaactcactc
ttcacccgga aatggttatt     60

gaggaacatg gcgttgctgg tgcgagtcct taggaaccag
actagcattt ctcagtgggt    120

tccagtatgc agccgattga tacctgtgtc tcctacccaa
ggacaggggg acagggctct    180

gtctcgcact tcccagtggc cccagatgag ccagtcccga

gcatgtggtg gatcagaaca    240

gattcctgga atagacatac agctgaatag gaagtatcac
accacacgta agctttctac    300

taccaaagat tccccacagc ctgttgagga gaaggttggt
gctttcacaa agataataga    360

agccatggga ttcacgggac ctttgaaata cagtaaatgg
aagattaaga ttgcggccct    420

gcgcatgtat actagctgtg tggagaaaac tgacttcgag
gaattctttc taaggtgtca    480

gatgcctgat acattcaatt catggtttct tataacccta
ctccacgtct ggatgtgtct    540

agtccgaatg aagcaggaag gccggagtgg gaagtacatg
tgtcgtatca tagttcattt    600

tatgtgggag gatgttcagc agcgcggcag agtcatgggg
gttaatccct atatcctgaa    660

gaagaacatg atcctcatga caaatcattt ctatgcagcg
atcttgggat atgatgaggg    720

gatcctttca gatgatcatg ggctggccgc tgccctctgg
agaaccttct tcaaccggaa    780

atgtgaagac cctcgacatc ttgaattgct ggtagagtat
gtgaggaaac agatacagta    840

cctggactcc atgaacgggg aggatctgct tctgacaggg
gaggtgagct ggcgccctct    900

agtggagaag aatcctcaga gcatcctgaa gccccattct
ccgacttaca acgacgaggg    960

actttgatgg gctgggccct ccgcacggcc cgccagctgg
cttcgaggaa cctccaggag    1020

agaagtgcct gttggtccag gaccctgcag aaagtggcct
gaactgacct ctgaacagca    1080

tctgtcaaat acctggcccc atttgtgttg agtttcctct
tagtgtgccc aggagtctga    1140

tctgctgggg tacagggctg ggagaacccc tagctctccc
ggggtgtcct ctcccttagg   1200

ggaagccccg agtgagagtc ccccagcaca cactccccaa
ccccctccag caactacatg   1260

tgactgatag cttttcccaa aggccaagga agggatggtg
taggttcaaa agggaaaccc   1320

cccagggcct gctgtggcct aggagcagat tgtaatgctg
ccgagtccgg tcggtgacca   1380

cgcgttgtcc ctcggctttc agccatgggg ttgagttggc
cattaaaaga aacagagact   1440

tctctctgcc atggcccttc tttattccag ggacttagaa
acttgcctga gatggtggac   1500

gcagtaatga gggcaccgcg cagctcagtt agagacggag
aaagggaaga ggctgggatg   1560

gtctctgctg ctcttgcctc tagttcatgg agatgtgtct
ctgttcaggc caagatacag   1620

ccagccaggc ctgtcgtctg ggacccagga ggcctctgat
gaccaagggc tttcacatcc   1680

taagtcattt ggaaggaggc cttgagaaca aagtcacctt
tgtcactccc agtgaactga   1740

atgaggaaca tgctgtctcc tgtcttggcc tcccctttca
tgagatactg ggagaagag   1800

aacattcctc ctggcttagt tgtagcagac ccagacctgt
gcccagcttt ggtcccctt   1860

cccaacttct gaagcacgtg ctgcagagcc accttggtct
gagcacctga ggaccagccc   1920

ctcctccctc agtgcgggtc atctcttggg ggattttctt
aaagtgaaga aaggggtgg   1980

ggaaccatat tgcccctccc tcccccatca aacttccttc
atttaacttg ctataaaatg   2040

agtcatataa agaaactcta tatgggtgag gtatatccca
cttctgtgaa aacattacaa    2100

atcaaaccgc ttctctcagt ttatttaaga tgcttttgtt
gcgagcggag ctctagagtg    2160

aagcctcctg tgtgtgtgtg agataataac accttgtaac
tcattacagc tgggcactat    2220

ttacataaac cagagctgag ccaggcagga atttgctgat
taatttattt ttaatggagt    2280

gaagtatacc atgcaccaaa ataaacttta ctgtgtgtac
ctaaaaaaaa aaaaaaaa      2338


<210>  24
<211>  2791
<212>  DNA
<213>  Homo sapiens

<400>  24
tgtgtgtgtg tatgtgtgct ggtgaatgtg agtacaggga
agcagcggcc gccatttcag      60

ggagcttgtc gacgctgtcg caggggtgga tcctgagctg
ccgaagccgc cgtcctgctc     120

tcccgcgtgg gcttctctaa ttccattgtt tttttagat
tctctcgggc ctagccgtcc     180

ttggaacccg atattcgggc tgggcggttc cgcggcctgg
gcctaggggc ttaacagtag     240

caacagaagc ggcggcggcg gcagcagcag cagcagcagc
agcaatctct tcccgaacac     300

gagcaccaca ggcgcccgaa ggccggaaca ggcgtttaga
gaaaatggca gacgatattg     360

atattgaagc aatgcttgag gctccttaca agaaggatga
gaacaagttg agcagtgcca     420

acggccatga agaacgtagc aaaaagagga aaaaaagcaa
gagcagaagt cgtagtcatg     480

aacgaaagag aagcaaaagt aaggaacgga agcgaagtag
agacagagaa aggaaaaaga    540

gcaaaagccg tgaaagaaag cgaagtagaa gcaaagagag
gcgacggagc cgctcaagaa    600

gtcgagatcg aagatttaga ggccgctaca gaagtcctta
ctccggacca aaatttaaca    660

gtgccatccg aggaaagatt gggttgcctc atagcatcaa
attaagcaga cgacgttccc    720

gaagcaaaag tccattcaga aaagacaaga gccctgtgag
agaacctatt gataatttaa    780

ctcctgagga aagagatgca aggacagtct tctgtatgca
gctggcggca agaattcgac    840

caagggattt ggaagagttt ttctctacag taggaaaggt
tcgagatgtg aggatgattt    900

ctgacagaaa ttcaagacgt tccaaaggaa ttgcttatgt
ggagttcgtc gatgttagct    960

cagtgcctct agcaatagga ttaactggcc aacgagtttt
aggcgtgcca atcatagtac   1020

aggcatcaca ggcagaaaaa aacagagctg cagcaatggc
aaacaattta caaaagggaa   1080

gtgctggacc tatgaggctt tatgtgggct cattacactt
caacataact gaagatatgc   1140

ttcgtgggat ctttgagcct tttggaagaa ttgaaagtat
ccagctgatg atggacagtg   1200

aaactggtcg atccaaggga tatggattta ttacattttc
tgactcagaa tgtgccaaaa   1260

aggctttgga acaacttaat ggatttgaac tagcaggaag
accaatgaaa gttggtcatg   1320

ttactgaacg tactgatgct tcgagtgcta gttcattttt
ggacagtgat gaactggaaa   1380

ggactggaat tgatttggga acaactggtc gtcttcagtt

aatggcaaga cttgcagagg   1440

gtacaggttt gcagattccg ccagcagcac agcaagctct
acagatgagt ggctctttgg   1500

catttggtgc tgtggcagat ttgcaaacaa gactttccca
gcagactgaa gcttcagctt   1560

tagctgcagc tgcctctgtt cagccacttg caacacaatg
tttccaactc tctaacatgt   1620

ttaaccctca aacagaagaa gaagttggat gggataccga
gattaaggat gatgtgattg   1680

aagaatgtaa taaacatgga ggagttattc atatttatgt
tgacaaaaat tcagctcagg   1740

gcaatgtgta tgtgaagtgc ccatcaattg ctgcagctat
tgctgctgtc aatgcattgc   1800

atggcaggtg gtttgctggt aaaatgataa cagcagcata
tgtacctctt ccaacttacc   1860

acaacctgtt tcctgattct atgacagcaa cacagctact
ggttccaagt agacgatgaa   1920

ggaagatata gtcccttatg tatatagctt tttttctttc
ttgagaattc atcttgagtt   1980

atcttttatt tagataaaaa taaagaggca aggatctact
gtcatttgta tgcaatttcc   2040

tgttaccttg aaaaaataaa aatgttaaca ggaatgcagt
gtgctcattc tccctaaata   2100

gtaaatccca ctgtatacaa aactgttctc ttgttctgcc
ttttaaaatg ttcatgtaga   2160

aaattaatga actataggaa tagctctagg agaacaaatg
tgctttctgt aaaaaggcag   2220

accagggatg taatgttttt aatgtttcag aagcctaact
ttttacacag tggttacatt   2280

tcacatttca ctaatgttga tatttggctg atggttgagc
agtttctgaa atacacattt   2340

agtgtatgga aatacaagac agctaaaggg ctgtttggtt
agcatctcat cttgcattct   2400

gatcaattgg caagaaaggg agatttcaaa attatatttc
ttgatggtat cttttcaatt   2460

aatgtatctg taaaagtttc tttgtaaata ctatgtgttc
tggtgtgtct taaaattcca   2520

aacaaaatga tccctgcatt tcctgaagat gtttaaacgt
gagagtctgg taggcaaagc   2580

agtctgagaa agaaatagga aatgcagaaa taggttttgt
ctggttgcat ataatctttg   2640

ctctttttaa gctctgtgag ctctgaaata tatttttggg
ttacttcagt gtgtttgaca   2700

agacagcttg atatttctat caaacaaatg actttcatat
tgcaacaatc tttgtaagaa   2760

ccactcaaat aaaagtctct taaaaaggcc a
                  2791

<210>  25
<211>  1604
<212>  DNA
<213>  Homo sapiens

<400>  25
cgtcacttcc tgttgcctta ggggaacgtg gctttccctg
cagagccggt gtctccgcct     60

gcgtccctgc tgcagcaacc ggagctggag tcggatcccg
aacgcaccct cgccatggac    120

tcggccctca gcgatccgca taacggcagt gccgaggcag
gcggccccac caacagcact    180

acgcggccgc cttccacgcc cgagggcatc gcgctggcct
acggcagcct cctgctcatg    240

gcgctgctgc ccatcttctt cggcgccctg cgctccgtac
gctgcgcccg cggcaagaat    300

gcttcagaca tgcctgaaac aatcaccagc cgggatgccg
cccgcttccc catcatcgcc    360

agctgcacac tcttggggct ctacctcttt ttcaaaatat
tctcccagga gtacatcaac    420

ctcctgctgt ccatgtattt cttcgtgctg ggaatcctgg
ccctgtccca caccatcagc    480

cccttcatga ataagttttt tccagccagc tttccaaatc
gacagtacca gctgctcttc    540

acacagggtt ctggggaaaa caaggaagag atcatcaatt
atgaatttga caccaaggac    600

ctggtgtgcc tgggcctgag cagcatcgtt ggcgtctggt
acctgctgag gaagcactgg    660

attgccaaca acctttttgg cctggccttc tcccttaatg
gagtagagct cctgcacctc    720

aacaatgtca gcactggctg catcctgctg ggcggactct
tcatctacga tgtcttctgg    780

gtatttggca ccaatgtgat ggtgacagtg gccaagtcct
tcgaggcacc aataaaattg    840

gtgtttcccc aggatctgct ggagaaaggc ctcgaagcaa
acaactttgc catgctggga    900

cttggagatg tcgtcattcc agggatcttc attgccttgc
tgctgcgctt tgacatcagc    960

ttgaagaaga atacccacac ctacttctac accagctttg
cagcctacat cttcggcctg   1020

ggccttacca tcttcatcat gcacatcttc aagcatgctc
agcctgccct cctatacctg   1080

gtccccgcct gcatcggttt tcctgtcctg gtggcgctgg
ccaagggaga agtgacagag   1140

atgttcagtt atgaggagtc aaatcctaag gatccagcgg
cagtgacaga atccaaagag   1200

ggaacagagg catcagcatc gaaggggctg gagaagaaag
agaaatgatg cagctggtgc   1260

ccgagcctct cagggccaga ccagacagat gggggctggg
cccacacagg cgtgcaccgg   1320

tagagggcac aggaggccaa gggcagctcc aggacagggc
agggggcagc aggatacctc   1380

cagccaggcc tctgtggcct ctgtttcctt ctccctttct
tggccctcct ctgctcctcc   1440

ccacaccctg caggcaaaag aaacccccag cttcccccct
ccccgggagc caggtgggaa   1500

aagtgggtgt gatttttaga ttttgtattg tggactgatt
ttgcctcaca ttaaaaactc   1560

atcccatggc cagggcgggc cactgtgctc ctggaaaaaa aaaa
                1604

<210>  26
<211>  2796
<212>  DNA
<213>  Homo sapiens

<400>  26
ctgcggcggg ggcggcccca gcggatgaat gaagcggcgc
gtggctgccg gggaggccag     60

agcgtggagc gctgcgcggc gcggcggccg ggccctcgag
acggggacgg acacaccagc    120

ccctcggata ccacttggcc actcccgctg aggccactcc
cactgcgtgg ctgaagcctc    180

gaggtcacca ggcggaggcg cggagatgcc cctgcatcag
ctgggggaca agccgctcac    240

cttccccagc cccaactcag ccatggaaaa cgggcttgac
cacaccccac ccagcaggag    300

ggcatccccg ggcacacccc tgagccccgg ctccctccgc
tccgctgccc atagccccct    360

ggacaccagc aagcagcccc tctgccagct ctgggccgag
aagcatggcg cccgggggac    420

ccatgaggtg cggtacgtct cggccgggca gagcgtggcg
tgcggctggt gggccttcgc    480

accgccgtgc ctgcaggtcc tcaacacgcc caagggcatc
ctgttcttcc tgtgtgcggc    540

cgcattcctg caggggatga ctgtgaatgg cttcatcaac
acagtcatca cctccctgga    600

gcgccgctat gacctgcaca gctaccagag cgggctcatc
gccagctcct acgacattgc    660

cgcctgcctc tgcctcacct tcgtcagcta cttcgggggc
tcagggcaca agccgcgctg    720

gctgggctgg ggcgtgctgc ttatgggcac ggggtcgctg
gtgttcgcgc tgccccactt    780

cacggctggc cgctatgagg tggagttgga cgcgggtgtc
aggacgtgcc ctgccaaccc    840

cggcgcggtg tgtgcggaca gcacctcggg cctgtcccgc
taccagctgg tcttcatgct    900

gggccagttc ctgcatggcg tgggtgccac acccctctac
acgctgggcg tcacctacct    960

ggatgagaac gtcaagtcca gctgctcgcc cgtctacatt
gccatcttct acacagcggc   1020

catcctgggc ccagctgccg gctacctgat tggaggtgcc
ctgctgaata tctacacgga   1080

aatgggccga cggacggagc tgaccaccga gagcccactg
tgggtcggcg cctggtgggt   1140

cggcttcctg ggctctgggg ccgctgcttt cttcaccgcc
gttcccatcc ttggttaccc   1200

tcggcagctg ccaggctccc agcgctacgc ggtcatgaga
gcggcggaaa tgcaccagtt   1260

gaaggacagc agccgtgggg aggcgagcaa cccggacttt

gggaaaacca tcagagacct   1320

gcctctctcc atctggctcc tgctgaagaa ccccacgttc
atcctgctct gcctggccgg   1380

ggccaccgag gccactctca tcaccggcat gtccacgttc
agccccaagt tcttggagtc   1440

ccagttcagc ctgagtgcct cagaagctgc caccttgttt
gggtacctgg tggtgccagc   1500

gggtggtggc ggcaccttcc tgggcggctt ctttgtgaac
aagctcaggc tccggggctc   1560

cgcggtcatc aagttctgcc tgttctgcac cgttgtcagc
ctgctgggca tcctcgtctt   1620

ctcactgcac tgccccagtg tgcccatggc gggcgtcaca
gccagctacg gcgggagcct   1680

cctgcccgaa ggccacctga acctaacggc tccctgcaac
gctgcctgca gctgccagcc   1740

agaacactac agccctgtgt gcggctcgga cggcctcatg
tacttctcac tgtgccacgc   1800

agggtgccct gcagccacgg agacgaatgt ggacggccag
aaggtgtacc gagactgtag   1860

ctgtatccct cagaatcttt cctctggttt tggccatgcc
actgcaggga aatgcacttc   1920

aacttgtcag agaaagcccc tccttctggt tttcatattc
gttgtaattt tctttacatt   1980

cctcagcagc attcctgcac taacggcaac tctacgatgt
gtccgtgacc ctcagagatc   2040

ctttgccctg ggaatccagt ggattgtagt tagaatacta
gggggcatcc cggggcccat   2100

cgccttcggc tgggtgatcg acaaggcctg tctgctgtgg
caggaccagt gtggccagca   2160

gggctcctgc ttggtgtacc agaattcggc catgagccgc
tacatactca tcatggggct   2220

cctgtacaag gtgctgggcg tcctcttctt tgccatagcc
tgcttcttat acaagcccct   2280

gtcggagtct tcagatggcc tggaaacttg tctgcccagc
cagtcctcag cccctgacag   2340

tgccacagat agccagctcc agagcagcgt ctgaccaccg
cccgcgccca cccggccacg   2400

gcgggcactc agcatttcct gatgacagaa cagtgccgtt
gggtgatgca atcacacggg   2460

aacttctatt tgacctgcaa ccttctactt aacctgtggt
ttaaagtcgg ctgtgacctc   2520

ctgtccccag agctgtacgg ccctgcagtg ggtgggagga
acttgcataa atatatattt   2580

atggacacac agtttgcatc agaacgtgtt tatagaatgt
gttttatacc cgatcgtgtg   2640

tggtgtgcgt gaggacaaac tccgcagggg ctgtgaatcc
cactgggagg gcggtgggcc   2700

tgcagcctga ggaaggcttg tgtgtcctca gttaaaactg
tgcatatcga aatatatttt   2760

gttatttaag cctgcgaaaa aaaaaaaaaa aaaaaa
                  2796

<210>  27
<211>  3689
<212>  DNA
<213>  Homo sapiens

<400>  27
gcgtgcgtgt gtacgactct gcgtgcgtgc gtgcgtgcgt
gcgtgccgtc agctcgccgg     60

gcaccgcggc ctcgccctcg ccctccgccc ctgcgcctgc
accgcgtaga ccgacccccc    120

cccagcgcgc ccacccggta gaggaccccc gcccgtgccc
cgaccggtcc ccgccttttt    180

gtaaaactta aagcgggcgc agcattaacg cttcccgccc
cggtgacctc tcaggggtct    240

ccccgccaaa ggtgctccgc cgctaaggaa catggcgaag
gtggagcagg tcctgagcct    300

cgagccgcag cacgagctca aattccgagg tcccttcacc
gatgttgtca ccaccaacct    360

aaagcttggc aacccgacag accgaaatgt gtgttttaag
gtgaagacta cagcaccacg    420

taggtactgt gtgaggccca acagcggaat catcgatgca
ggggcctcaa ttaatgtatc    480

tgtgatgtta cagcctttcg attatgatcc caatgagaaa
agtaaacaca agtttatggt    540

tcagtctatg tttgctccaa ctgacacttc agatatggaa
gcagtatgga aggaggcaaa    600

accggaagac cttatggatt caaaacttag atgtgtgttt
gaattgccag cagagaatga    660

taaaccacat gatgtagaaa taaataaaat tatatccaca
actgcatcaa agacagaaac    720

accaatagtg tctaagtctc tgagttcttc tttggatgac
accgaagtta agaaggttat    780

ggaagaatgt aagaggctgc aaggtgaagt tcagaggcta
cgggaggaga acaagcagtt    840

caaggaagaa gatggactgc ggatgaggaa gacagtgcag
agcaacagcc ccatttcagc    900

attagcccca actgggaagg aagaaggcct tagcacccgg
ctcttggctc tggtggtttt    960

gttctttatc gttggtgtaa ttattgggaa gattgccttg
tagaggtagc atgcacagga   1020

tggtaaattg gattggtgga tccaccatat catgggattt
aaatttatca taaccatgtg   1080

taaaaagaaa ttaatgtatg atgacatctc acaggtcttg
ccttttaaatt acccctccct 1140

gcacacacat acacagatac acacacacaa atataatgta
acgatctttt agaaagttaa 1200

aaatgtatag taactgattg agggggaaaa gaatgatctt
tattaatgac aagggaaacc 1260

atgagtaatg ccacaatggc atattgtaaa tgtcatttta
aacattggta ggccttggta 1320

catgatgctg gattacctct cttaaaatga cacccttcct
cgcctgttgg tgctggccct 1380

tggggagctg gagcccagca tgctggggag tgcggtcagc
tccacacagt agtccccacg 1440

tggcccactc ccggcccagg ctgctttccg tgtcttcagt
tctgtccaag ccatcagctc 1500

cttgggactg atgaacagag tcagaagccc aaaggaattg
cactgtggca gcatcagacg 1560

tactcgtcat aagtgagagg cgtgtgttga ctgattgacc
cagcgctttg gaaataaatg 1620

gcagtgcttt gttcacttaa agggaccaag ctaaatttgt
attggttcat gtagtgaagt 1680

caaactgtta ttcagagatg tttaatgcat atttaactta
tttaatgtat ttcatctcat 1740

gttttcttat tgtcacaaga gtacagttaa tgctgcgtgc
tgctgaactc tgttgggtga 1800

actggtattg ctgctggagg gctgtgggct cctctgtctc
tggagagtct ggtcatgtgg 1860

aggtggggtt tattgggatg ctggagaaga gctgccagga
agtgtttttt ctgggtcagt 1920

aaataacaac tgtcataggg agggaaattc tcagtagtga
cagtcaactc taggttacct 1980

tttttaatga agagtagtca gtcttctaga ttgttcttat

accaccctctc aaccattact   2040

cacacttcca gcgcccaggt ccaagtctga gcctgacctc
cccttgggga cctagcctgg   2100

agtcaggaca aatggatcgg gctgcagagg gttagaagcg
agggcaccag cagttgtggg   2160

tggggagcaa gggaagagag aaactcttca gcgaatcctt
ctagtactag ttgagagttt   2220

gactgtgaat taattttatg ccataaaaga ccaacccagt
tctgtttgac tatgtagcat   2280

cttgaaaaga aaaattataa taaagcccca aaattaagaa
ttcttttgtc attttgtcac   2340

atttgctcta tggggggaat tattatttta tcatttttat
tattttgcca ttggaaggtt   2400

aactttaaaa tgagccctat cactgagaaa tacgtgtttc
atgatttaac tctgtgtgtg   2460

tgtgtgtgtg tgtgtgtgtg tgtgtgtgta tttttttttt
ggttgtcttc agctgacagt   2520

atgaaaaatg aaactgctga aaaagctgag cacctggtca
cccttggcct tccattgctt   2580

tggccttcag taaaaagcag cctcccttct aggtcaggga
accatgccat tgagactagt   2640

aacgggcgtt ctgggcacag tcccactgtg cacaggtttg
agaggacaag ttcatcagaa   2700

ggaaggcagt ccttagaagt cacatacgtt gagccacgtt
gctcctaagc ctggctctgt   2760

caagctgggt caggggcctt gaaactggag aagtggaagt
ctatggttgg tctgagtaag   2820

taacttcctg tcttcatgaa aaaagttgac tttgaatccc
aggtactcac agaaatggtg   2880

aacagactta gttgttaccc aggcacccat ggattgtgtt
gagtgtgcag acagggaggc   2940

caccccaata ggaattcgtc tccaggattt ttcccatgtg
tcccccagta cttataaaag   3000

ggagtgaaaa gaccgagctg taaggcatgt gccttctgcc
acctgacttt ccgtgagggg   3060

actaaaattt actaattgta gttgctgcag ccagttaagt
cctgtagctt ccaggccctc   3120

atgtctttga taggagagtg cttaggtggt ccccaacagt
gcctaggggt acagtacagt   3180

cccattacac tagagcaggg ctctatttat ttttaaagga
tatggccgtg tgttttgata   3240

aaactttatt cacaaaaaca gccgggtcat gggatttggc
ttgtgagtct gtaacagttc   3300

ttaaaaagaa tatctgagaa actactctgt tttagacctt
tgaaggtgat ttagagtttt   3360

gtgtacatct aggagaaggt gttcagcttc tcagaggatg
tggacatttt ggttgcagct   3420

aaaaatcagt ctctgaagtc tctctccctt ctagaggtta
ggacttggtg aacatgtttg   3480

tgggcctttt gactgagtgg cagaaggaaa ctgctcagga
agagaaacag gtgactgatg   3540

ggaaggttga ttattttctc agtcatcctg gcagccaaaa
atgtgccagg aaaagaaaga   3600

atgtggagca cgcgtggctc ctggaggact tggagatgca
tgcacattta gggtgtttc   3660

cctagaatta cataaaaaaa aaaaaaaaa
                    3689


<210>  28
<211>  2017
<212>  DNA
<213>  Homo sapiens

<400> 28
acttccggaa gcggcggcgc tcgggaggaa gtgccgatcg
gctgctgggg cgaaaagggg 60

gcgccgggcc gctctagccg gtgaggccgg cgggctctct
gtggctgcgg ctgggaaacc 120

gcgcggagga ggtgcccggc cggggaccag ccctggtcca
gcgcctccct ctctcagcat 180

ggacgaggag agcctggagt cggccttgca gacctaccgt
gcgcagctgc agcaggtgga 240

gctggccttg ggcgccggcc tggattcgtc tgagcaggct
gacctgcgcc agctgcaggg 300

ggacctgaag gagctcatcg agctcaccga ggccagcctg
gtgtctgtca ggaagagcag 360

cttgttggcc gcgctggacg aagagcgccc gggccgccag
gaagatgctg agtaccaggc 420

tttccgggag gccatcactg aggcggtgga ggcaccagca
gcggcccgtg ggtccggatc 480

agagaccgtt cctaaagcag aggcggggcc agaatctgcg
gcaggtgggc aggaggagga 540

agagggagag gacgaggaag agctgagtgg gacaaaggtg
agcgcgccct actacagctc 600

ctggggcact ctggagtatc acaacgccat ggtggtggga
acggaagagg cggaggatgg 660

ctcggcgggt gtccgtgtgc tttacctgta ccccactcac
aagtctctga agccgtgccc 720

gttcttcctg gagggaaagt gccgctttaa ggagaactgc
aggttctccc atgggcaggt 780

ggtctctctg gatgagctgc gccccttcca ggacccagac
ctgagctccc tgcaggccgg 840

ctctgcgtgt ctggccaagc accaggatgg cctctggcac
gcagcacgca tcaccgatgt 900

ggacaacggc tactacacag tcaagtttga ctcgctgctg
ctgagggagg ccgtggtgga    960

gggggacggc atcctgcccc cactgcgcac agaggccaca
gagtccgact cagacagcga    1020

cggtacgggt gactccagct atgccagagt ggtggggtcg
gacgccgtgg actctgcaca    1080

gtcctctgcc ctctgtccgt ctcttgcagt ggtggggtca
gatgctgtgg actctgggac    1140

ctgcagctct gcctttgctg gctgggaggt gcacacgcga
ggtataggct ccagactcct    1200

caccaagatg ggctatgagt ttggcaaggg tttgggccga
cacgcggaag gccgggtgga    1260

gcccatccat gctgtggtgt tgcctcgagg gaagtcgctg
gaccagtgtg tggagaccct    1320

gcagaagcag accagggttg gcaaggctgg caccaacaag
cccccaggt gccggggaag    1380

aggggccagg cctgggggcc gcccagctcc tcggaatgtg
tttgacttcc tcaatgaaaa    1440

gctgcaaggt caggctcctg gggccctaga agccggggcg
gccccagcgg ggaggaggag    1500

caaggacatg taccatgcca gcaagagtgc caagcgggcc
ctgagcctgc ggctcttcca    1560

gactgaggag aagatcgagc gaacccagcg ggacatcagg
agcatccagg aggctctcgc    1620

ccgcaacgct ggccggcata gcgtggcgtc agcccagctg
caggagaagc tggcaggagc    1680

ccagcgccag ctggggcagc tccgggctca ggaagccggc
ctgcagcagg agcagaggaa    1740

ggcagacacc cacaagaaga tgactgagtt ctagagaccc
cacaagcact atggacgaag    1800

cgtgggaccc cagcacgggc tgccctcagg aagaccagtg

ttgcccgagg aggggccggc   1860

ctgctggcct ggggcgtgca gacactgctg agtggagaca
gagctgcggg gtcccatctg   1920

gacacttact tgcccacctg ccagtgtctt gggcatttcc
ttggcaagga cattaaagtg   1980

atttcatcac agtgtcaaaa aaaaaaaaaa aaaaaaa
                  2017

<210>  29
<211>  1833
<212>  DNA
<213>  Homo sapiens

<400>  29
acgcagcttg aggcgcccgc tttccgtcgc tccggcccgc
ctcgccgcaa ggctttctgg     60

gagccgtagt ccccacgtct ggcctctccg gcgccagcgg
cagcgcgcgc ccacccgcgg    120

aactacagag cgtggcgcac agcgcgcgag gctcctccgc
ctcgccttcc ctccccgccc    180

gcgcgcccgc cccagttatc atggcggctc ccttggtcct
ggtgctggtg gtggctgtga    240

cagtgcgggc ggccttgttc cgctccagtc tggccgagtt
catttccgag cgggtggagg    300

tggtgtcccc actgagctct tggaagagag tggttgaagg
cctttcactg ttggacttgg    360

gagtatctcc gtattctgga gcagtatttc atgaaactcc
attaataata tacctctttc    420

atttcctaat tgactatgct gaattggtgt ttatgataac
tgatgcactc actgctattg    480

ccctgtattt tgcaatccag gacttcaata aagttgtgtt
taaaaagcag aaactcctcc    540

tagaactgga ccagtatgcc ccagatgtgg ccgaactcat

ccggacccct atggaaatgc 600

gttacatccc tttgaaagtg gccctgttct atctcttaaa
tccttacacg attttgtctt 660

gtgttgccaa gtctacctgt gccatcaaca acaccctcat
tgctttcttc attttgacta 720

cgataaaagg cagtgctttc ctcagtgcta tttttcttgc
cttagcgaca taccagtctc 780

tgtacccact caccttgttt gtcccaggac tcctctatct
cctccagcgg cagtacatac 840

ctgtgaaaat gaagagcaaa gccttctgga tcttttcttg
ggagtatgcc atgatgtatg 900

tgggaagcct agtggtaatc atttgcctct ccttcttcct
tctcagctct tgggatttca 960

tccccgcagt ctatggcttt atactttctg ttccagatct
cactccaaac attggtcttt 1020

tctggtactt ctttgcagag atgtttgagc acttcagcct
cttctttgta tgtgtgtttc 1080

agatcaacgt cttcttctac accatcccct tagccataaa
gctaaaggag caccccatct 1140

tcttcatgtt tatccagatc gctgtcatcg ccatctttaa
gtcctacccg acagtggggg 1200

acgtggcgct ctacatggcc ttcttccccg tgtggaacca
tctctacaga ttcctgagaa 1260

acatctttgt cctcacctgc atcatcatcg tctgttccct
gctcttccct gtcctgtggc 1320

acctctggat ttatgcagga agtgccaact ctaatttctt
ttatgccatc acactgacct 1380

tcaacgttgg gcagatcctg ctcatctctg attacttcta
tgccttcctg cggcgggagt 1440

actacctcac acatggcctc tacttgaccg ccaaggatgg
cacagaggcc atgctcgtgc 1500

tcaagtaggc ctggctggca cagggctgca tggacctcag
ggggctgtgg ggccagaagc   1560

tgggccaagc cctccagcca gagttgccag caggcgagtg
cttgggcaga agaggttcga   1620

gtccagggtc acaagtctct ggtaccaaaa gggacccatg
gctgactgac agcaaggcct   1680

atggggaaga actgggagct ccccaacttg gacccccacc
ttgtggctct gcacaccaag   1740

gagccccctc ccagacagga aggagaagag gcaggtgagc
agggcttgtt agattgtggc   1800

tacttaataa atgttttttg ttatgaagtc taa
                  1833

<210>  30
<211>  5727
<212>  DNA
<213>  Homo sapiens

<400>  30
agaaacagcc tatagacgcc acgagtcggc ggcgctaccg
aggggctgtg ggcgcgcagc     60

tggaacctcc ggctgtcagt gcgcttacag ttcctaaccc
cgaccctgcg cgcagcccgc    120

actatggcag ccccgccgca gctaagggct ctgctcgtag
tcgtcaacgc actgctgcgc    180

aagcgccgct accacgctgc gttggccgtg cttaagggct
tccggaacgg ggctgtctat    240

ggagccaaaa tccgggcccc tcacgcgctg gtcatgacct
ttctcttccg gaatggcagc    300

ctccaggaga agctgtgggc catactgcag gccacatata
tccactcctg gaacctggca    360

cggtttgtgt tcacctacaa gggtctccgt gccctgcagt
cctacataca aggcaagacc    420

tacccagcac acgcattcct ggcggccttc ctcgggggta
tcctggtgtt tggagaaaac    480

aataacatca acagccagat caacatgtac ctgttgtcac
gcgtcctgtt tgccctgagc    540

cgcctggctg tagagaaggg ctacatccct gaacccaggt
gggacccgtt cccgctgctc    600

actgcggtgg tgtgggggct ggtgctgtgg ctctttgagt
atcaccgatc caccctgcag    660

ccctcgctgc agtcctccat gacctacctc tatgaggaca
gcaatgtatg gcacgacatc    720

tcagacttcc tcgtctataa caagagccgt ccctccaatt
aatgcagccc tgaggtgtct    780

ggctgtggct caagatttgg ccccatgcag accctcccaa
aggatactgc cttctcaaga    840

tcataggcct cagactccaa ctggtgttat cccagggttc
cgtttgctga agtaaaaaca    900

ctgattttaa aatcccagtg ggtacctttg tatggtggca
caagtggccg aatcaggctg    960

aggaatctac ggcttggttc cagctgtgca gctgacttct
gtgagactgg ggccagccac    1020

actactctct aggcctcagg ggtcaaggag ctcagaggag
ggccctgagg tctctttccg    1080

gtgggtatgt tcattcttca actgttctta tgtcacagag
ggctccttgc tggtgggcag    1140

tgggttgtaa atacttttta aaaaacacta agttccttat
ctcagatgct gttctactgg    1200

agaagttcta gattcccact gtccaataga aacacgtgag
ccatatatgt aattaaaatg    1260

tttctagtag ctgcattaca aaaaagaagc ctgggcactg
tggctcactc ctgtaatctc    1320

agaactttgg gaggctgagg caggtggatc acttgagctc
aggagcttga gaccagcctg   1380

ggcaacatgg tgaaacccag tttctacaaa aaatacaaaa
aattagccgg gttccatcac   1440

ctgtggttcc agctgaggtg ggaggatcac ttgagcctat
gaggtcgagg ctacagtgag   1500

caaagatcac gccactgcac tccagcctgg gcgacagtga
gaccctgtct caccaaagaa   1560

aaaaaaaagg tgagattaat cttcactata tattttaaca
caaatttcta aaagttattt   1620

caacatataa ttaacatcat tagatatttt gtactctttt
ttgtattaag tattcaaaat   1680

tgggtgtata ttttgccacc cacattttca ggacccagta
gccacatgtg gctagtggct   1740

gctgtgacgg gcaacacaga tatggaacac ttgtatcctc
acagattatt ctagtggaca   1800

gtgcagatct agagctagag gacatgggtt tgagtcccca
ctcccccact gtgtaacctt   1860

ggggaaggtt ctggacctct cagagcctca atttatcata
aaatgaggat aatcataatg   1920

ccgtggtcct cactggactt gtgcaactac cactgatgtg
ctactaatta ctttcattta   1980

gtgcattcat acttccactt gggtgttttc cacatattat
tattattttt tttttttaag   2040

tagcgacagg gtctcgctct gtcactaggg ctagtctcga
cctcctagcc tcaagcaatc   2100

ttcctgcctc agtctcccaa agtgccatga ttacaggcat
gagccactgc ccctagcctg   2160

cacatattat ttttaaacca tacagtggcc ttgcaaatgt
gtgttttcgt ccccatttta   2220

cagatgtaga aactgagggt caggccaggt gcagtggctc

acgcctgtaa tcccagcact   2280

ttgggaggct gaggcaggca gataacctga gatcaggagt
tcttgaccag cctggccaac   2340

atggtaaaac cccatctcta ctaaaaatac aaaaattagc
caggcatggt ggcacgtccc   2400

tgtaatccca gctactgggg aggctgaggc aggagaatcg
cttgaacctg ggaggcagag   2460

gctgcagtga gctgagatcg cacgactgca ctccagcctg
ggccacagag cgagactgtg   2520

tctaaaaaaa aaataaaaaa agaaaagaaa agaaactgag
aatcagagag gagagtaact   2580

tgcccaaggt aatacagcta gtataacagc cgaatcaaga
tttgaactca gattcgtctg   2640

atttcaaagc ccatgttttt tccagtgcct tgtttcaaat
ttagaaacct aagccatgct   2700

atgcaaatgt tagctcatta taggatatgg gccagaaagg
ggtacacatt caaatgccca   2760

taggggccta caatgacata gtgctatgcg atgaggagtg
atggggacat agggaactgg   2820

agacttgtgt accagtctga tttttctttt tttttttttt
tttgagacag agtcttgctc   2880

tctcgcccag gctggagtgc agtggcgcga tcttggctca
ctgcaagctc tgcctcccag   2940

gtttacttac accattctcc tgcctcagcc tccggagtag
ctgggcctac aggtgcccgc   3000

caccacaccc ggctaatttt ttgtattttt agtagagatg
gggtttcacc gtgttagcca   3060

ggatggtctc aatctcctga cctcatgatc cacccacctt
ggcctcccaa aatgctggga   3120

ttacaggtgt gagccaccat gcctggccaa gtctgctttt
ttcgaaacca cgatcctaga   3180

ccaggcacgg tggcttgcac ctgtaatccc agcactttgg
gaggccgagg cgggtggatc   3240

acctgaggtc aggagttcga gaccagcctg gccaacatgg
cgaaatcctg tctctactaa   3300

aaatacaaaa attagctgga catggtggca ggcacctgta
attccagcta cttgggaggc   3360

tgaggtggga gaatctcttg aacctggggg gctgctgtga
gccgagatag ttccattaca   3420

cttcagcctg ggtgacagag taagactctg tctcaaaaaa
aaaaaaaaaa aaaaaaacac   3480

aatcctggac aaatcaaaca agtctccagg aaaatctggt
ccatgggcta ccaatttgag   3540

accattggtc tggtagatac actttcatta atatacttac
tccatcactc tttctatatt   3600

ttagaagtta ctagtagaaa tgtatgcagg agtcactgga
gaccttatta aaatgcagct   3660

tctgattcag tgagctgggg tggggcctga gagtctgcat
ttctcccagg ctccctggtg   3720

ctgccagtgg tgctgctctg agtaacaagg gggtggggaa
tgatatggag ccgtccatta   3780

ttatcccatc tgacaaatga gtcacagagc ccttaggtaa
ttgaggtggg atcagtctga   3840

ttctgtaagc tgtgttttca gccacaacat ttactgcaaa
cttggagtag gtaattatga   3900

agttccttta acttgctgtg gaggttgaat gagctctaga
gtaaggggtc cgcaaccccg   3960

gtaccggtcc atggcctgtt cggaaccagg ccacgcagca
ggagttgaaa ggtgggtgag   4020

ctagcgaagc ttcatctgta tgtacagcca ctccccactg
ctcacattac cacctgagct   4080

ctgccttctg tcagatcagt ggtggcatta gatgctcgta
ggaatgcaaa ccctattgtg   4140

aactgcgcat gcaagggatc taggttgcat gctccttatg
agaatctaat gcctgatgat   4200

ctgtcactgt ctcccgtcac gccttgatgg gactgtctag
ttgctggaaa acaaactcag   4260

ggctcccact gattctacat tatggtgtgt tgtacaatta
tttcattata tattacaagg   4320

taataataat agaaataaaa tccacagtaa atgcaatgtg
cttgaatcat cctgaaacca   4380

cgtcccctcc ccagtggtcc gtggaaaaat tatcttcccc
aaaactggtc cctggtgcca   4440

aaaaggttgg ggactgctgc tctaggggtt ggggagggca
gagggcttgg gccatcacca   4500

ggcaggtttt atagcagatg gtgggggtgc cctgcctata
gctcctctaa ataacgactc   4560

tggtgcacac tagccagctt tgccagtggc cgcccctctg
tctttctgcc tgaggactct   4620

agcttgtcac tggtgcctcc tttgccagcc tctggcaggc
ctgaagggct ggagaatgaa   4680

cacatccttg gagcaacctt caaccaatga ctgataaata
cccccactcc cacagcccgc   4740

atccctcacc cctcgggtgg gataactcag aggtgcgtgc
tccacactga ctcccagggc   4800

cgcccagcag ggctgagcct caaacaccct ccccatctcc
cttcctcatg cccctaccag   4860

tgcttcctgg gaccacttcc catatgaacc acttgcactc
aaacccttgt cttagagtct   4920

gctattgggg gaacccaaac taaaactggc ctctgtacag
caggttgaga ggcaatatag   4980

catggtggtc agggacatag gctttggagc cagagtgcct

gggctcaagc cctggcccca   5040

cagtttcctt gggcaagtga ctgagccatt ctgtgcctct
atttcctcat tgataaagta   5100

ggcgttgtcg tagcacacac ctcatcgagt ggtgtggagg
gttaaagaat atgacacagt   5160

taaagtgagt ccccaataaa tgctgttatt tctaagagca
tccaaatgta gaggaagtgt   5220

gcccctgagg actgagcaca ccctgctttt agcctgtcct
gcccccagct gaaggtgaaa   5280

ccttggccgc ctgccctcct gaagtcccgc cactgctgct
gggggttggc caactccgct   5340

accgactgtc tctgctctct aagaataggt ttccctcaca
gatggcatct ctctggatgc   5400

tgctagagca tctggtgctt ccaactgcac actccaggga
ggaaggcagt gtggtctgtg   5460

ccggggagca gggctctgcc ttccagcaga tctgagagga
gtcccatttg gctccttcct   5520

agctgccctc cattctctaa gacctgtaaa acagggatgg
ccagagggct cttgttgcac   5580

agtaagtgct atgcttccta aatgctagtt cctgaaatag
aatggtttaa cctctactta   5640

ctaaacacct gctacaatcc tgttaattct tttgacatgt
aagactaaaa taaataaata   5700

aataaataaa tatatatgtg tataaaa
                    5727

<210>  31
<211>  5011
<212>  DNA
<213>  Homo sapiens

<400>  31
ggggcgcccg cgggccggag ccggggcggg ggccggggcc

taggcgcgcg gacctgcgag 60

cggacccgag aggcggcggc ggcgcagcgg aacggcagag
cgggccggag gcggccgagg 120

cgcccggcgc aggcacccgt gcctcccctc tgccaggaac
cttggggcct tgtgtgtgac 180

caggacctgg tggcccccgg gcggtggcag agcccctgtc
ccaagctgct tcctgccggc 240

acctctgatc aagtgcctag agggatgtgt gtgccagccc
tcggtccagt gcccgctcct 300

gagctgactc ctgctgggcc ccgacagctt gccgtgtttc
ctgtgcctgt agctccctgg 360

ttggatagct gccgcccggg agaggtgacc cgggcgccct
gctagggtga aggcccctgc 420

cctcggcccg ggatcatgaa aggcctcggt gacagccgcc
cccgccacct ctccgacagc 480

ctagacccac cccacgagcc cctgtttgca gggaccgacc
gcaaccccta cctgctgtcg 540

cccacggagg ccttcgcccg cgaggcccgc ttccccgggc
agaacaccct gccaggagat 600

ggcctctttc ccctcaacaa ccagctgccc ccgcccagca
gcacctttcc ccgcatccac 660

tacaactccc acttcgaggt gccagaggag agccccttcc
ccagccatgc ccaagccacc 720

aagatcaacc ggctgcccgc caacctcctg gaccagtttg
agaagcagct gcccatccac 780

cgtgatggct tcagcaccct ccaatttccc cgtggcgagg
ccaaggcccg tggtgagagc 840

cctggccgca tccgccacct ggtccactca gtccagcggc
ttttcttcac caaggcaccc 900

tcactggagg gcacagcggg caaggtcggt ggcaatggca
gcaagaaggg tggcatggag 960

gacggcaagg gccggagggc caaaagcaag gagcgggcca
aggctgggga gcccaaacgg    1020

cgcagccgct ccaacatctc aggctggtgg agctccgatg
acaacttgga cggcgaggcc    1080

ggcgccttcc gcagcagtgg cccagcctct gggctgatga
cactaggccg ccaggcagaa    1140

cgcagccagc cacgctactt catgcacgcc tacaacacca
tcagtgggca catgctcaaa    1200

accaccaaga acaacactac tgagctgact gccccaccac
ccccgcccgc acccccagcc    1260

acctgcccca gccttggggt gggcactgac accaactacg
tcaaacgggg ctcctggtcc    1320

actctgaccc tcagccacgc ccacgaggtc tgccagaaga
cctcagccac cttggataag    1380

agcctgctca agtccaaatc ctgccaccag ggtctagcct
accattacct gcaggtgccc    1440

ggcggcggcg gcgagtggag caccacgctg ctgtccccac
gcgagacgga tgccgcggcc    1500

gagggcccta tcccgtgccg gcgcatgcgc agcggcagct
acatcaaggc catgggcgac    1560

gaggacagcg acgagtccgg cggcagcccc aagccctcac
ccaagaccgc ggcgcggcgc    1620

cagagctatc tgagggccac gcagcagtcg ctgggagagc
agagcaaccc ccgcaggagt    1680

ctggaccgcc tggattcagt ggacatgctg ctgccctcca
agtgtccgag ctgggaagag    1740

gactacaccc ccgtcagcga cagcctcaac gactccagct
gcatcagcca gattttgga    1800

caggcctccc tgatccccca gttgtttggc catgagcagc
aggtacggga ggcagagctg    1860

agtgaccagt atgaggcggc ctgcgagtca gcctgcagtg
aagcggagtc cacagcggca   1920

gagacgcttg acttgccact gcccagctac ttccgctccc
gcagccacag ctacctgcgt   1980

gccatccagg caggctgctc gcaggaggag gacagtgtct
ccctgcagtc cctctcccca   2040

ccgcccagta ccggcagcct cagcaatagt cgcacgcttc
cgagttcatc atgcctagtg   2100

gcgtataaga agaccccgcc accggtccct ccacgcacca
cttcaaagcc gttcatctca   2160

gtcacagtcc agagcagtac tgagtctgcc caggacacct
acctggacag ccaggaccac   2220

aagagcgagg tgactagcca gtcgggcctg agcaactcgt
cggacagcct ggacagcagt   2280

acccgaccgc ccagcgtgac acggggtgga gtcgccccag
cccctgaggc cccagagcca   2340

cccccaaaac atgcagctct gaaaagtgaa caagggacgc
tgaccagctc tgagtcccac   2400

cccgaggccg cccccaaaag gaaactgtca tcgataggaa
tacaagagag gactagaagg   2460

aacggttccc acctctcgga ggacaacgga cccaaagcga
tcgatgtgat ggcaccctcc   2520

tcagaaagca gcgtcccctc tcacagtatg tcctcccgac
gggacacaga ctcggatacc   2580

caggatgcca atgactcaag ctgtaagtca tctgagagga
gcctcccgga ctgtacccct   2640

caccccaact ccatcagcat cgatgccggt ccccggcagg
cccccaagat tgcccagatc   2700

aagcgcaacc tctcctatgg agacaacagc gaccctgccc
tagaggcgtc ctcgctgccc   2760

ccacccgacc cctggctcga gacctcctcc agctccccag

cagagccggc acagccaggg   2820

gcctgccgcc gagacggcta ctggttccta aagctactgc
aggcagaaac agagcggctg   2880

gaaggctggt gctgccagat ggacaaggag accaaagaga
acaacctctc tgaagaagtc   2940

ttaggaaaag tcctcagtgc tgtgggcagt gcccagctac
tgatgtccca gaaattccag   3000

cagttccggg gcctctgtga gcaaaacttg aaccctgatg
ccaacccacg ccccacagcc   3060

caggacctgg cagggttctg ggacctgcta cagctgtcca
tcgaggatat cagcatgaag   3120

ttcgatgaac tctaccacct caaggccaac agctggcagc
tggtggagac ccccgagaag   3180

aggaaggaag agaagaaacc accccctccg gtcccaaaga
agccagccaa atccaagccg   3240

gcagtgagcc gcgacaaggc ctcagacgcc agcgacaagc
agcgccagga ggcccgcaag   3300

agactcctgg cggccaagcg ggcagcttct gtgcggcaga
actcagccac cgagagcgca   3360

gacagcatcg agatttatgt cccggaggcc cagaccaggc
tctgagacca tgcaggagga   3420

aagaaacgat tttaaatcat taaaaacaca aaaactaagt
gcgaacggaa cagagttttc   3480

tcaacctttg ctatggttat tctgtctaga gaccctgagc
caactttcaa attgacgcat   3540

acaagggctc acaatttggc ttttttgggt ccctcccagc
tttaggttat gaagatttta   3600

ctcacaaaaa aaatcaacaa aaatcacgaa actagaaaac
ttttttttc ctcttgctgg   3660

ccgtggtgga ctagatagat ggacgtcggc aactcccggc
ccagcctcca tactgcggtc   3720

tttttactcg ttctatctga tgagaactca cactagcttg
tttacaagat gacgacagtc   3780

caagggcagc cttgggcacc tgccatgtcc ctcctttccc
cagctatccc cgctctgacc   3840

ttgattttca ttcttatgtt tttctctttt cccttcagag
ctcacacagt ggtcaccatt   3900

gtggcaagcg gctttctggg tctcagccct ctctgcggtt
gagggcccag aggacagaga   3960

gatggacatg cgtcccctcc ctccccccgc caagtgctca
cacacaacct cacgcgcaca   4020

cacacacacg cagatggagg cgcctcactg ggaggtgccc
cgccagccct gggcagtgtc   4080

aggcaggact cactcaccgc tgagcagatg agggaagttt
tagtcttggc gggtggaaat   4140

gagacgaagc cacagttatc acactccaga ctcctgccct
tttattttct ccagcccctt   4200

cttccttcag caaaatctag gactcccgag tggcttccag
ggggccgtca gtcctcagcc   4260

gcgcctgtgt ccggtgcccg aggggcgggc ggcggtgtct
gtatgtatgt gtacatatgc   4320

acatagacct tagagtgtat agttaacaaa cgcccatctg
ctcacccatg cccacccagc   4380

gccgccgccg ctggctctcg gggcacctgg caggaggcgg
gtgtgtgaat agcatatatt   4440

tttacatgta ctatatctag gtgtgtgtac aagtgtgtgt
aaaaatatat accttgtgtg   4500

taagcagccc tttttttttt tggtctccac ccccctcccc
ccgccccgca ctcctaaggg   4560

cccatctgcc cagcctctga gttttctgtt ctattttttt
tttaacccca attatccttc   4620

tctctctcct gcccccgcat cccactccca gggtgtcacg
agccctgagc tgcaatggcc   4680

cgggcctgca gggcggggta ggggagggca ggggctgagc
cccgaagcca gctcagtacc   4740

tgaggggctg ctctatgctg tgtatgcgcc tctctggcat
ccgagacatc ctcttggctg   4800

gcgcttgctg caggggggga cccccccccg tccccaggtg
aaccaagggt ctgctccggg   4860

gcccatttcc agcttggccg ccgtctgtga ccttgggcaa
gtcacttgac ctctgtgtgc   4920

ctcaacttcc tcctctgtaa aacggggaca gtccctgccc
ctccctacct cacaggcatg   4980

ttgtgagaat aaatgaggta acgtgtacca a
                    5011

<210>  32
<211>  1383
<212>  DNA
<213>  Homo sapiens

<400>  32
aaagtccaag aggacggaat gtggagacag tgttgtattt
ttgcggggag ttctaggccg     60

accgggagcg agagaacgct cgggggcgaa gcgcgccatt
gcggccctcc ccgccgcctg    120

cggtagtcca gtcccaagat ggcggccacc atgaagaagg
cggctgcaga agatgtcaat    180

gttactttcg aagatcaaca aaagataaac aaatttgcac
ggaatacaag tagaatcaca    240

gagctgaagg aagaaataga agtaaaaaag aaacaactcc
aaaacctaga agatgcttgt    300

gatgacatca tgcttgcaga tgatgattgc ttaatgatac
cttatcaaat tggtgatgtc    360

ttcattagcc attctcaaga agaaacgcaa gaaatgttag
aagaagcaaa gaaaaatttg    420

caagaagaaa ttgacgcctt agaatccaga gtggaatcaa
ttcagcgagt gttagcagat    480

ttgaaagttc agttgtatgc aaaattcggg agcaacataa
accttgaagc tgatgaaagt    540

taaacatttt ataatacttt ttttatttgt ttaataaact
tgaatattgt ttaaaatgat    600

aattttcctt cttcaaatga catggaaagc aaaactttct
tttttaaaaa ttttcattta    660

tttaatggaa acttgcccat tttcacatgt ctgcttattt
attttatatt tttaaaagaa    720

gacagtattc acctatgtat tttgcataac gattatatca
agtctagggg cttcatgtca    780

tgttattaaa atcagttaag caatctttta tgtttctata
ttatttagaa tatttgttgt    840

tgcaattttc acataagaaa atttaacagt tgtgtcatgt
tgtttctgtc tgattttaat    900

tgctgtctaa tgacggggaa agcacgatga aaagatgtac
aatcctgcat ccttgcttat    960

ttcacaacta aagctttgtc atagacttca aaatatatat
gtatatattt tatttaaata   1020

tatgttacat attatattta aacatacata tttaacattt
tttacatatc tatcaatatc   1080

agagatttgg gtaaaagaat gggtaatgtt taaacatgtg
gaggcatgtg gagctttata   1140

caaacagggc agaaccacag aagaacgttt tagaaaccaa
gagatgtgca gaaagaaatg   1200

tttagtgttt tttcgtttta aattttagat tttattttag
tgctttgtaa ttaattgggg   1260

tttatattga taaagatgtg gaagttaaac agctatgtat

gtaaaagtaa ggcttatttc   1320

ttaaataaag gatgcatttc ttcccaaaaa aaaaaaaaaa
aaaaaaaaaa aaaaaaaaaa   1380

aaa
                    1383

<210>  33
<211>  2722
<212>  DNA
<213>  Homo sapiens

<400>  33
ttggcgcgta aaagtggccg ggactttgca ggcagcggcg
gccgggggcg gagcgggatc     60

gagccctcgc cgaggcctgc cgccatgggc ccgcgccgcc
gccgccgcct gtcacccggg    120

ccgcgcgggc cgtgagcgtc atggccttgg ccggggcccc
tgcgggcggc ccatgcgcgc    180

cggcgctgga ggccctgctc ggggccggcg cgctgcggct
gctcgactcc tcgcagatcg    240

tcatcatctc cgccgcgcag gacgccagcg ccccgccggc
tcccaccggc cccgcggcgc    300

ccgccgccgg cccctgcgac cctgacctgc tgctcttcgc
cacaccgcag gcgccccggc    360

ccacacccag tgcgccgcgg cccgcgctcg gccgcccgcc
ggtgaagcgg aggctggacc    420

tggaaactga ccatcagtac ctggccgaga gcagtgggcc
agctcggggc agaggccgcc    480

atccaggaaa aggtgtgaaa tccccggggg agaagtcacg
ctatgagacc tcactgaatc    540

tgaccaccaa gcgcttcctg gagctgctga gccactcggc
tgacggtgtc gtcgacctga    600

actgggctgc cgaggtgctg aaggtgcaga agcggcgcat

ctatgacatc accaacgtcc    660

ttgagggcat ccagctcatt gccaagaagt ccaagaacca
catccagtgg ctgggcagcc    720

acaccacagt gggcgtcggc ggacggcttg aggggttgac
ccaggacctc cgacagctgc    780

aggagagcga gcagcagctg gaccacctga tgaatatctg
tactacgcag ctgcgcctgc    840

tctccgagga cactgacagc cagcgcctgg cctacgtgac
gtgtcaggac cttcgtagca    900

ttgcagaccc tgcagagcag atggttatgg tgatcaaagc
ccctcctgag acccagctcc    960

aagccgtgga ctcttcggag aactttcaga tctcccttaa
gagcaaacaa ggcccgatcg   1020

atgttttcct gtgccctgag gagaccgtag gtgggatcag
ccctgggaag accccatccc   1080

aggaggtcac ttctgaggag gagaacaggg ccactgactc
tgccaccata gtgtcaccac   1140

caccatcatc tcccccctca tccctcacca cagatcccag
ccagtctcta ctcagcctgg   1200

agcaagaacc gctgttgtcc cggatgggca gcctgcgggc
tcccgtggac gaggaccgcc   1260

tgtccccgct ggtggcggcc gactcgctcc tggagcatgt
gcgggaggac ttctccggcc   1320

tcctccctga ggagttcatc agcctttccc caccccacga
ggccctcgac taccacttcg   1380

gcctcgagga gggcgagggc atcagagacc tcttcgactg
tgactttggg gacctcaccc   1440

ccctggattt ctgacagggc ttggagggac cagggtttcc
agagatgctc accttgtctc   1500

tgcagccctg gagccccctg tccctggccg tcctcccagc
ctgtttggaa acatttaatt   1560

tatacccctc tcctctgtct ccagaagctt ctagctctgg
ggtctggcta ccgctaggag   1620

gctgagcaag ccaggaaggg aaggagtctg tgtggtgtgt
atgtgcatgc agcctacacc   1680

cacacgtgtg taccgggggt gaatgtgtgt gagcatgtgt
gtgtgcatgt accggggaat   1740

gaaggtgaac atacacctct gtgtgtgcac tgcagacacg
ccccagtgtg tccacatgtg   1800

tgtgcatgag tccatgtgtg cgcgtggggg ggctctaact
gcactttcgg cccttttgct   1860

ctgggggtcc cacaaggccc agggcagtgc ctgctcccag
aatctggtgc tctgaccagg   1920

ccaggtgggg aggctttggc tggctgggcg tgtaggacgg
tgagagcact tctgtcttaa   1980

aggttttttc tgattgaagc tttaatggag cgttatttat
ttatcgaggc ctctttggtg   2040

agcctgggga atcagcaaag gggaggaggg gtgtggggtt
gataccccaa ctccctctac   2100

ccttgagcaa gggcaggggt ccctgagctg ttcttctgcc
ccatactgaa ggaactgagg   2160

cctgggtgat ttatttattg ggaaagtgag ggagggagac
agactgactg acagccatgg   2220

gtggtcagat ggtggggtgg gccctctcca gggggccagt
tcagggcccc agctgccccc   2280

caggatggat atgagatggg agaggtgagt gggggacctt
cactgatgtg ggcaggaggg   2340

gtggtgaagg cctcccccag cccagaccct gtggtccctc
ctgcagtgtc tgaagcgcct   2400

gcctccccac tgctctgccc caccctccaa tctgcacttt
gatttgcttc ctaacagctc   2460

tgttccctcc tgctttggtt ttaataaata ttttgatgac
gtttgggccg ggttttggga   2520

ctctgttggg aacatttcgg ggcgggagag gccaaggttg
ctggggaaat gcccattctc   2580

cacttccctt ctccctgtcc gtgcccgatt tgatttgagc
ctcataactc gaagaaaggt   2640

cagcttcctc gctgttttgg tcctaactca aaagcagatc
cagtaaaggt tttgttgta   2700

aaaaaaaaaa aaaaaaaaaa aa
                    2722

<210>  34
<211>  3287
<212>  DNA
<213>  Homo sapiens

<400>  34
gacaggagga aacgcagcgc cagcagcatc tcatctaccc
tccttgacac ctccccgtgg     60

ctccagccag accctagagg tcagccttgc ggaccaacag
gaggactccc agctttccct    120

tttcaagagg tccccagaca ccggccaccc tcttccagcc
cctgcggcca gtgcaaggag    180

gcaccaatgc tctgaggctg tcgcgtggtg cagcgtcgag
catcctcgcc gaggtccttt    240

ctgctgcctg tcccgcctca ccccgctcca tcacaccagc
tggccctctt tgcttccttt    300

tcccagaatc gttaagcccc gactcccact agcacctcgt
accaacctcg ccccacccca    360

tcctcctgcc ttcccgcgct ccggtgtccc ccgctgccat
gagctccccc atcagcaaga    420

gccgctcgct tgccgccttc ctgcagcagc tgcgcagtcc
gaggcagccc ccgagactgg    480

tgacatctac ggcgtacacg tcccctcagc cgcgagaggt
gccagtctgc ccgctgacag    540

ctggtggcga gactcagaac gcggccgccc tgccgggccc
caccagctgg ccactgctgg    600

gcagcctgct gcagattctc tggaaagggg gtctcaagaa
acagcacgac accctggtgg    660

agtaccacaa gaagtatggc aagattttcc gcatgaagtt
gggttccttt gagtcggtgc    720

acctgggctc gccatgcctg ctggaagcgc tgtaccgcac
cgagagcgcg tacccgcagc    780

ggctggagat caaaccgtgg aaggcctatc gcgactaccg
caaagaaggc tacgggctgc    840

tgatcctgga aggggaagac tggcagcggg tccggagtgc
ctttcaaaag aaactaatga    900

aaccagggga agtgatgaag ctggacaaca aaatcaatga
ggtcttggcc gattttatgg    960

gcagaataga tgagctctgt gatgaaagag gccacgttga
agacttgtac agcgaactga   1020

acaaatggtc gtttgaaagt atctgcctcg tgttgtatga
gaagagattt gggcttctcc   1080

agaagaatgc aggggatgaa gctgtgaact tcatcatggc
catcaaaaca atgatgagca   1140

cgtttgggag gatgatggtc actccagtcg agctgcacaa
gagcctcaac accaaggtct   1200

ggcaggacca cactctggcc tgggacacca ttttcaaatc
agtcaaagct tgtatcgaca   1260

accggttaga gaagtattct cagcagccta gtgcagattt
cctttgtgac atttatcacc   1320

agaatcggct ttcaaagaaa gaattgtatg ctgctgtcac
agagctccag ctggctgcgg   1380

tggaaacgac agcaaacagt ctaatgtgga ttctctacaa

tttatcccgt aatccccaag   1440

tgcaacaaaa gcttcttaag gaaattcaaa gtgtattacc
tgagaatcag gtgccacggg   1500

cagaagattt gaggaatatg ccgtatttaa aagcctgtct
gaaagaatct atgaggctta   1560

cgccgagtgt accatttaca actcggactc ttgacaaggc
aacagttctg ggtgaatatg   1620

ctttacccaa aggaacagtg ctcatgctaa atacccaggt
gttgggatcc agtgaagaca   1680

attttgaaga ttcaagtcag tttagacctg aacgttggct
tcaggagaag gaaaaaatta   1740

atccttttgc gcatcttcca tttggcgttg gaaaaagaat
gtgcattggt cgccgattag   1800

cagagcttca actgcatttg gctctttgtt ggattgtccg
caaatacgac atccaggcca   1860

cagacaatga gcctgttgag atgctacact caggcaccct
ggtgcccagc cgggaactcc   1920

ccatcgcgtt ttgccagcga taatacgcct cagatggtgg
tatttgctaa catcatatcc   1980

aactcaggga agcggactga gtgctgggat ccaaggcatt
ctacagggtt cactgctggt   2040

ttacacttca cctgtgtcag caccatcttc aggtgcttag
aatggcctgg gagcctgttc   2100

tgtcttgcat cttccatgac atgaaaggga ggctggcact
tgtcagtcag gtagaggtta   2160

caaaccgttt caggccctgc ctaccacatt cactgtttga
atctttaatt cccaagaata   2220

agtttacatt tcacaatgaa tgacctacaa cagctaaatt
ttctggggct gggagtaata   2280

ctgacaatcc atttactgta gctctgctta atgtactact
taggaaaatg tccctgctta   2340

ataatgtaag ccaagctaaa tgatggttaa agttatcagg
cctcccatga aattgcgttc   2400

ttcctgcatt gaaataaaaa cattattggg aaactagaga
acacctctat ttttaaaagg   2460

actttaacga agtcaaacaa cttataagac tagtgattca
ctggggcatt attttgttag   2520

aggaccttaa aattgtttat tttttaaatg tgattccttt
atggcattag ggtaaagatg   2580

aagcaataat ttttaaattg tgtatgtgca tatgaagcac
agacatgcat gtgtgtgtgt   2640

gtctgtgtgt gtgtgtccgt gtatgtgtgt gtgggttcta
atggtaattt gcctcagtca   2700

tttttttaat atttgcagta cttgatttag gatctgtggt
gcagggcaat gtttcaaagt   2760

ttagtcacag cttaaaaaca ttcagtgtga ctttaatatt
ataaaatgat ttcccatgcc   2820

ataatttttc tgtctattaa atgggacaag tgtaaagcat
gcaaaagtta gagatctgtt   2880

atataacatt tgttttgtga tttgaactcc taggaaaaat
atgatttcat aaatgtaaaa   2940

tgcacagaaa tgcatgcaat acttataaga cttaaaaatt
gtgtttacag atggtttatt   3000

tgtgcatatt tttactactg cttttcctaa atgcatactg
tatataattc tgtgtatttg   3060

ataaatattt cttcctacat tatattttta gaatatttca
gaaatataca tttatgtctt   3120

tatattgtaa taaatatgta catatctagg tatatgcttt
ctctctgctg tgaaattatt   3180

tttagaatta taaattcacg tcttgtcaga tttcatctgt
ataccttcaa attctctgaa   3240

agtaaaaata aaagttttta aatattaaaa aaaaaaaaaa aaaaaaa
                    3287

<210>  35
<211>  4145
<212>  DNA
<213>  Homo sapiens

<400>  35
caaacaagtg cggccatttc accagcccag gctggcttct
gctgttgact ggctgtggca      60

cctcaagcag cccctttccc ctctagcctc agtttatcac
cgcaagagct accattcatc     120

tagcacaacc tgaccatcct cacactggtc agttccaacc
ttcccaggaa tcttctgtgg     180

ccatgttcac tccggtttta cagaacagag aacagaagct
cagagaagtg aagcaacttg     240

cccagctatg agagacagag ccaggatttg aaaccagatg
aggacgctga ggcccagaga     300

gggaaagcca cttgcctagg gacacacagc ggggagaggt
ggagcagggc ctctatttcg     360

agacccctga ctccacacct ggtgtttgtg ccaagacccc
aggctgcctc ccaggtcctc     420

tgggacagcc cctgccttct accaggacca tgggtagcaa
caagagcaag cccaaggatg     480

ccagccagcg gcgccgcagc ctggagcccg ccgagaacgt
gcacggcgct ggcgggggcg     540

ctttccccgc ctcgcagacc cccagcaagc cagcctcggc
cgacggccac cgcggcccca     600

gcgcggcctt cgcccccgcg gccgccgagc ccaagctgtt
cggaggcttc aactcctcgg     660

acaccgtcac ctccccgcag agggcgggcc cgctggccgg
tggagtgacc acctttgtgg     720

ccctctatga ctatgagtct aggacggaga cagacctgtc
cttcaagaaa ggcgagcggc    780

tccagattgt caacaacaca gagggagact ggtggctggc
ccactcgctc agcacaggac    840

agacaggcta catccccagc aactacgtgg cgccctccga
ctccatccag gctgaggagt    900

ggtattttgg caagatcacc agacgggagt cagagcggtt
actgctcaat gcagagaacc    960

cgagagggac cttcctcgtg cgagaaagtg agaccacgaa
aggtgcctac tgcctctcag   1020

tgtctgactt cgacaacgcc aagggcctca acgtgaagca
ctacaagatc cgcaagctgg   1080

acagcggcgg cttctacatc acctcccgca cccagttcaa
cagcctgcag cagctggtgg   1140

cctactactc caaacacgcc gatggcctgt gccaccgcct
caccaccgtg tgccccacgt   1200

ccaagccgca gactcagggc ctggccaagg atgcctggga
gatccctcgg gagtcgctgc   1260

ggctggaggt caagctgggc cagggctgct ttggcgaggt
gtggatgggg acctggaacg   1320

gtaccaccag ggtggccatc aaaaccctga agcctggcac
gatgtctcca gaggccttcc   1380

tgcaggaggc ccaggtcatg aagaagctga ggcatgagaa
gctggtgcag ttgtatgctg   1440

tggtttcaga ggagcccatt tacatcgtca cggagtacat
gagcaagggg agtttgctgg   1500

actttctcaa gggggagaca ggcaagtacc tgcggctgcc
tcagctggtg gacatggctg   1560

ctcagatcgc ctcaggcatg gcgtacgtgg agcggatgaa
ctacgtccac cgggaccttc   1620

gtgcagccaa catcctggtg ggagagaacc tggtgtgcaa

agtggccgac tttgggctgg   1680

ctcggctcat tgaagacaat gagtacacgg cgcggcaagg
tgccaaattc cccatcaagt   1740

ggacggctcc agaagctgcc ctctatggcc gcttcaccat
caagtcggac gtgtggtcct   1800

tcgggatcct gctgactgag ctcaccacaa agggacgggt
gccctaccct gggatggtga   1860

accgcgaggt gctggaccag gtggagcggg gctaccggat
gccctgcccg ccggagtgtc   1920

ccgagtccct gcacgacctc atgtgccagt gctggcggaa
ggagcctgag gagcggccca   1980

ccttcgagta cctgcaggcc ttcctggagg actacttcac
gtccaccgag ccccagtacc   2040

agcccgggga gaacctctag gcacaggcgg gcccagaccg
gcttctcggc ttggatcctg   2100

ggctgggtgg cccctgtctc ggggcttgcc ccactctgcc
tgcctgctgt tggtcctctc   2160

tctgtggggc tgaattgcca ggggcgaggc ccttcctctt
tggtggcatg gaaggggctt   2220

ctggacctag ggtggcctga gagggcggtg ggtatgcgag
accagcacgg tgactctgtc   2280

cagctcccgc tgtggccgca cgcctctccc tgcactccct
cctggagctc tgtgggtctc   2340

tggaagagga accaggagaa gggctggggc cggggctgag
ggtgcccttt tccagcctca   2400

gcctactccg ctcactgaac tccttcccca cttctgtgcc
acccccggtc tatgtcgaga   2460

gctggccaaa gagcctttcc aaagaggagc gatgggcccc
tggccccgcc tgcctgccac   2520

cctgcccctt gccatccatt ctggaaacac ctgtaggcag
aggctgccga gacagaccct   2580

ctgccgctgc ttccaggctg ggcagcacaa ggccttgcct
ggcctgatga tggtgggtgg 2640

gtgggatgag taccccctca aaccctgccc tccttagacc
tgagggaccc ttcgagatca 2700

tcacttcctt gcccccattt cacccatggg gagacagttg
agagcgggga tgtgacatgc 2760

ccaaggccac ggagcagttc agagtggagg cgggcttgga
acccggtgct ccctctgtca 2820

tcctcaggaa ccaacaattc gtcggaggca tcatggaaag
actgggacag cccaggaaac 2880

aaggggtctg aggatgcatt cgagatggca gattcccact
gccgctgccc gctcagccca 2940

gctgttggga acagcatgga ggcagatgtg gggctgagct
ggggaatcag ggtaaaaggt 3000

gcaggtgtgg agagagaggc ttcaatcggc ttgtgggtga
tgtttgacct tcagagccag 3060

ccggctatga aagggagcga gcccctcggc tctggaggca
atcaagcaga catagaagag 3120

ccaagagtcc aggaggccct ggtcctggcc tccttccccg
tactttgtcc cgtggcattt 3180

caattcctgg ccctgttctc ctccccaagt cggcaccctt
taactcatga ggagggaaaa 3240

gagtgcctaa gcgggggtga aagaggacgt gttacccact
gccatgcacc aggactggct 3300

gtgtaacctt gggtggcccc tgctgtctct ctgggctgca
gagtctgccc cacatgtggc 3360

catggcctct gcaactgctc agctctggtc caggccctgt
ggcaggacac acatggtgag 3420

cctagccctg ggacatcagg agactgggct ctggctctgt
tcggcctttg ggtgtgtggt 3480

ggattctccc tgggcctcag tgtgcccatc tgtaaagggg
cagctgacag tttgtggcat    3540

cttgccaagg gtccctgtgt gtgtgtatgt gtgtgcatgt
gtgcgtgtct ccatgtgcgt    3600

ccatatttaa catgtaaaaa tgtccccccc gctccgtccc
ccaaacatgt tgtacatttc    3660

accatggccc cctcatcata gcaataacat tcccactgcc
aggggttctt gagccagcca    3720

ggccctgcca gtggggaagg aggccaagca gtgcctgcct
atgaaatttc aacttttcct    3780

ttcatacgtc tttattaccc aagtcttctc ccgtccattc
cagtcaaatc tgggctcact    3840

caccccagcg agctctcaaa tccctctcca actgcctaag
gccctttgtg taaggtgtct    3900

taatactgtc cttttttttt ttttaacagt gttttgtaga
tttcagatga ctatgcagag    3960

gcctggggga cccctggctc tgggccgggc ctggggctcc
gaaattccaa ggcccagact    4020

tgcggggggt gggggggtat ccagaattgg ttgtaaatac
tttgcatatt gtctgattaa    4080

acacaaacag acctcagaaa aaaaaaaaaa aaaaaaaaaa
aaaaaaaaaa aaaaaaaaaa    4140

aaaaa
                4145


<210>  36
<211>  1368
<212>  DNA
<213>  Homo sapiens

<400>  36
ggagctgcga gccgcgaccg ccgggagcgc acctgccccg
cctccgccag gcggtccgcg      60

gggcatgcag cggaccgggg gcggggctcc gaggcccggg
cgcaaccacg ggctcccagg    120

cagcctccgc cagccggacc ccgtcgccct cctgatgctg
ctcgtggacg ctgatcagcc    180

ggagcccatg cgcagcgggg cgcgcgagct cgcgctcttc
ctgacccccg agcctggggc    240

cgaggcgaag gaggtggagg agaccatcga gggcatgctc
ctcaggctgg aagagttttg    300

cagcctggct gacctgatca ggagtgatac ttcacagatc
ctggaggaaa acatcccagt    360

ccttaaggcc aaactgacag aaatgcgtgg catctatgcc
aaagtggacc ggctagaggc    420

cttcgtcaag atggttggac accacgtcgc cttcctggaa
gcagacgtgc ttcaggctga    480

gcgggaccat ggggccttcc ctcaggccct gcggaggtgg
ctgggatccg cagggctccc    540

ctccttcagg aacgtggagt gcagtggcac aatcccagct
cgctgcaacc tccgcctccc    600

gggttcaagt gattctcctg cctccgcctc ccaagtagct
gggattacag aagtcacctg    660

caccggtgcc cgtgacgtac gagctgccca cactgtatag
gacggaggac tattttcctg    720

tggacgccgg ggaagcacag caccaccccc gcacctgccc
tcggcctttg tgagctttgt    780

ggtcttccca tcaggaacgc tggaaagtga cattgtgtac
acactgcagc ttgggggttt    840

tttctttgta ttgctgttta ttttatattt taaaaatatt
taaaaaaatg tcgagatggg    900

gtctcactat gttgtccaga ctgatctcaa actcctgggc
tcaagtgatc cacccacctt    960

ggccttccaa agtggtggga ttatgggcag gagcctccgt

gcccaggctg ctgccatttt   1020

caaatttcct ccctgcctca tgtgagacca cagggtttgg
agaagcagtt ggaacccacg   1080

tgtggtgatg cctcccacat cggcctgctt ggggttctac
aggggttgag ggaccaggcc   1140

tggccggggc tgatggacag tggggacttt ccttctctcc
atgatggctt tgcaggggct   1200

ccatggtcct tctctctgtg atgggttttt gcacggggtg
tgctctgcca ctgtggtggg   1260

tgggtggatg ctgcttctgt tgcctccaga cctcggtgcc
cacagccttg aggatccttc   1320

caataaaggt gtcaagagct caaaaaaaaa aaaaaaaaaa
aaaaaaaa                1368


<210>  37
<211>  2204
<212>  DNA
<213>  Homo sapiens

<400>  37
gagcggtgcg gaggctctgc tcggatcgag gtctgcagcg
cagcttcggg agcatgagtg     60

ctgcagtgac tgcagggaag ctggcacggg caccggccga
ccctgggaaa gccggggtcc    120

ccggagttgc agctcccgga gctccggcgg cggctccacc
ggcgaaagag atcccggagg    180

tcctagtgga cccacgcagc cggcggcgct atgtgcgggg
ccgcttttg ggcaagggcg    240

gctttgccaa gtgcttcgag atctcggacg cggacaccaa
ggaggtgttc gcgggcaaga    300

ttgtgcctaa gtctctgctg ctcaagccgc accagaggga
gaagatgtcc atggaaatat    360

ccattcaccg cagcctcgcc caccagcacg tcgtaggatt

ccacggcttt ttcgaggaca    420

acgacttcgt gttcgtggtg ttggagctct gccgccggag
gtctctcctg gagctgcaca    480

agaggaggaa agccctgact gagcctgagg cccgatacta
cctacggcaa attgtgcttg    540

gctgccagta cctgcaccga aaccgagtta ttcatcgaga
cctcaagctg ggcaaccttt    600

tcctgaatga agatctggag gtgaaaatag gggattttgg
actggcaacc aaagtcgaat    660

atgacgggga gaggaagaag accctgtgtg ggactcctaa
ttacatagct cccgaggtgc    720

tgagcaagaa agggcacagt ttcgaggtgg atgtgtggtc
cattgggtgt atcatgtata    780

ccttgttagt gggcaaacca ccttttgaga cttcttgcct
aaaagagacc tacctccgga    840

tcaagaagaa tgaatacagt attcccaagc acatcaaccc
cgtggccgcc tccctcatcc    900

agaagatgct tcagacagat cccactgccc gcccaaccat
taacgagctg cttaatgacg    960

agttctttac ttctggctat atccctgccc gtctccccat
cacctgcctg accattccac   1020

caaggttttc gattgctccc agcagcctgg accccagcaa
ccggaagccc ctcacagtcc   1080

tcaataaagg cttggagaac cccctgcctg agcgtccccg
ggaaaaagaa gaaccagtgg   1140

ttcgagagac aggtgaggtg gtcgactgcc acctcagtga
catgctgcag cagctgcaca   1200

gtgtcaatgc ctccaagccc tcggagcgtg ggctggtcag
gcaagaggag gctgaggatc   1260

ctgcctgcat ccccatcttc tgggtcagca agtgggtgga
ctattcggac aagtacggcc   1320

ttgggtatca gctctgtgat aacagcgtgg gggtgctctt
caatgactca acacgcctca   1380

tcctctacaa tgatggtgac agcctgcagt acatagagcg
tgacggcact gagtcctacc   1440

tcaccgtgag ttcccatccc aactccttga tgaagaagat
caccctcctt aaatatttcc   1500

gcaattacat gagcgagcac ttgctgaagg caggtgccaa
catcacgccg cgcgaaggtg   1560

atgagctcgc ccggctgccc tacctacgga cctggttccg
cacccgcagc gccatcatcc   1620

tgcacctcag caacggcagc gtgcagatca acttcttcca
ggatcacacc aagctcatct   1680

tgtgcccact gatggcagcc gtgacctaca tcgacgagaa
gcgggacttc cgcacatacc   1740

gcctgagtct cctggaggag tacggctgct gcaaggagct
ggccagccgg ctccgctacg   1800

cccgcactat ggtggacaag ctgctgagct cacgctcggc
cagcaaccgt ctcaaggcct   1860

cctaatagct gccctcccct ccggactggt gccctcctca
ctcccacctg catctggggc   1920

ccatactggt tggctcccgc ggtgccatgt ctgcagtgtg
ccccccagcc ccggtggctg   1980

ggcagagctg catcatcctt gcaggtgggg gttgctgtgt
aagttatttt tgtacatgtt   2040

cgggtgtggg ttctacagcc ttgtccccct ccccctcaac
cccaccatat gaattgtaca   2100

gaatatttct attgaattcg gaactgtcct ttccttggct
ttatgcacat taaacagatg   2160

tgaatattca aaaaaaaaaa aaaaaaaaaa aaaaaaaaaa aaaa
                    2204

<210>  38
<211>  4287
<212>  DNA
<213>  Homo sapiens

<400>  38
ctgcgcctgc gcgagggcta cgcgcgctcc ggccggggcg
cgggcgcgct ctcaggcggg     60

ctccggcggc agcgacgcga gcgcggcgat ggggagcggc
ggcgtggtcc actgtaggtg    120

tgccaagtgt ttctgttatc ctacaaagcg aagaataagg
aggaggcccc gaaacctgac    180

catcttgagt ctccccgaag atgtgctctt tcacatcctg
aaatggcttt ctgtagagga    240

catcctggcc gtccgagctg tacactccca gctgaaggac
ctggtggaca accacgccag    300

tgtgtgggca tgtgccagct tccaggagct gtggccgtct
ccagggaacc tgaagctctt    360

tgaaagggct gctgaaaagg ggaatttcga agctgctgtg
aagctgggca tagcctacct    420

ctacaatgaa ggcctgtctg tgtctgatga ggcccgcgca
gaagtgaatg gcctgaaggc    480

ctctcgcttc ttcagtctcg ctgagcggct gaatgtgggt
gccgcacctt tcatctggct    540

cttcatccgc cctccgtggt cggtgagcgg aagctgctgc
aaggccgtgg ttcacgagag    600

cctcagggca gagtgccagc tgcagaggac tcacaaagca
tccatattgc actgcttggg    660

cagagtgctg agtctgttcg aggatgagga gaagcagcag
caggcccatg acctgtttga    720

ggaggctgct catcagggat gtctgaccag ctcctacctc
ctctgggaaa gcgacaggag    780

gacagatgtg tcagatcctg ggcgatgcct ccacagcttc
cgaaaactca gggactacgc    840

tgccaaaggc tgctgggaag cgcagctgtc tttagccaaa
gcctgtgcaa atgcaaacca    900

gcttggactg gaggtgagag cttccagtga gatcgtctgc
cagctatttc aggcttccca    960

ggctgtcagt aaacaacaag tcttctccgt gcagaaggga
ctcaatgaca caatgaggta   1020

cattctgatc gactggctgg tggaagttgc caccatgaag
gacttcacaa gcctgtgcct   1080

gcacctgacc gtggagtgtg tggaccggta cctgcggagg
aggctggtgc cgcggtacag   1140

gctccagctg ctgggcatcg cctgcatggt catctgcacc
cggtttatca gtaaagagat   1200

cctgaccatc cgggaggccg tatggctcac ggacaacact
tacaagtacg aggacctggt   1260

gagaatgatg ggcgagatcg tctccgcctt ggaagggaag
attcgagtcc ccactgtggt   1320

ggattacaag gaggtcctgc tgacgctagt ccctgtggag
ctgagaaccc agcacctgtg   1380

cagcttcctc tgcgagctct ccctgctgca caccagcctg
tccgcctacg ccccagcccg   1440

cctggctgcc gcagccctgc tcctggccag actgacgcac
gggcagacac agccctggac   1500

cactcagctg tgggacctca ccggattctc ctatgaagac
ctcattccct gcgtcttgag   1560

cctccataag aagtgcttcc atgatgacgc ccccaaggac
tacaggcaag tctctctgac   1620

cgccgtgaag cagcggtttg aggacaagcg ctatggagaa
atcagccagg aagaggtgct   1680

gagctacagc cagttgtgtg ctgcattagg agtgacacaa

gacagccccg acccccccgac   1740

tttcctcagc acaggggaga tccacgcctt cctcagctct
ccctcggggc ggagaaccaa   1800

acggaagcgg gagaacagcc tccaggaaga cagaggcagc
ttcgttacca cccccactgc   1860

ggagctgtcc agccaggagg agacgctgct gggcagcttc
ctcgactgga gcctggactg   1920

ctgctctggc tatgaaggcg accaggagag tgagggcgag
aaggagggcg acgtgacagc   1980

tcccagcggc atcctcgatg tcaccgtggt ctacctgaac
ccagaacagc attgctgcca   2040

ggaatccagt gatgaggagg cttgtccaga ggacaaggga
ccccaggacc cacaggcact   2100

ggcgctggac acccagatcc ctgcaacccc tggacccaaa
cccctggtcc gcaccagccg   2160

ggagccaggg aaggacgtca cgacctcagg gtactcctcc
gtcagcaccg caagtcccac   2220

aagctccgtg gacggtggct tgggggccct gccccaacct
acctcagtgc tgtccctgga   2280

cagtgactcg cacacacagc cctgccacca tcaggccagg
aagtcatgtt tacagtgtcg   2340

tcccccaagt cccccggaga gcagtgttcc ccagcaacag
gtgaagcgga taaacctatg   2400

catacacagt gaggaggagg acatgaacct gggccttgtg
aggctgtaag tgtgtcagca   2460

catttgccgc agtggatgtg tactgagggg gctggaggcg
aagggtggga gcatagcata   2520

ggaacgctgc atagaccatg gaggcctttg cgcagagagc
agagaggatg acttgcggcc   2580

accaagtttc tgtctccgcg ggagtcccgt gcaagccatc
agaatgttga aatgagggtg   2640

aagagctcag atccctctct ttggaaagtt tagcctggaa
gcagttggcc acactgtgtg   2700

gagggcacct ctctgtccct tccgtgtctc actgtctctg
gaagcttcag cccatgtgtg   2760

tcctggtgtt cccagcccca ccagagcccc gtgccgggag
ctgacagctt tcacgcttaa   2820

ggcacgtgtg acctgggtag tcagacacca cttgagcccc
tgcccacatc tgctggtttg   2880

gggcttcagt ggggagctga cagctgtgag cacaccactg
tcccctcatc cacctcggcc   2940

tgcatggggc acccacttcc ttctgggtgg ggcttccatg
gtaagggggc ctgcgtccct   3000

gcacactgcg aggactgcct tggccacagg cccactccct
acgacacgtg actcgtttta   3060

gagctctgtc ccagaggcgt tcgtatgtga cccacagatg
gcgtcaatgt gaacacctct   3120

ctttgtgctg aatttctggg ccattctttt cctgtcttat
ttctaaattt ccttcttcca   3180

agatgaaaac aaaagaaaaa cttaaaacag aaggtattaa
aaaaacaaga gattcccacc   3240

attatttagg ttcacctgca aaacaaaaat cttactccag
cccctcaatg ccatcctgac   3300

acactttatg caaaaagaat tttcccagat aggctagcca
gaaaaaactt caagtcctct   3360

gtaacatctg aggtgaccaa gaggcagaag agcagagcag
tcgggggccg tgtcctggct   3420

gatcccaact gcagctctgc tgtggggccc cgtgggaggg
aggcagaccc ctgggctttc   3480

ctgctggcca cggagactct gctcctgcat ggaaagggag
cctgggagcc agcagcccac   3540

gcctggggag cctgcctggg gccatgtgac catggcctct
ccctgggaac gggctgacca   3600

caacacaccc tgctgccatc cacttctgtt tactctgcaa
atgtaagaaa gaaccacttg   3660

gccagaagtg tcccccagat gctttttttt tttttttttt
ggagacagtt ttgctcttgt   3720

ctccccggct ggagtgcagt ggcatgatct caactctcaa
ctcactgtaa cctccgcctc   3780

ccggatactc ctgcctcagc ctcctgggta gctgggatta
caagcaccca accacgccca   3840

gctaattttt gtattttcgg tagagacggg atttcaccat
gttggccagg ctagtctcga   3900

actcatgacc tcaagtgatc cgcccacttc ggtctcccaa
agtgctggga ttacaggcat   3960

gagccacggc gcctggcccc caaatgctct tgaaccggaa
acccagggat gggagatgct   4020

cactgagctg ctgcttttat gtgtgctggt gctatgtgtg
ttcatgtccg cggcagctgt   4080

ctttttgcta ctataaggga attctggcca ccctgggtgg
ggtgtggtcg gggtgagaac   4140

ccaagcgttg gaactgtaga cccgtcctgt cgactgtgtg
cccctgggca tgtgtgagcc   4200

tcagtttcct catctgtaag gggggcaatg atacctacct
cacaggggtg ttgtgaggat   4260

taaatgtgag gaggatagtg gcagatg
                4287


<210>  39
<211>  692
<212>  DNA
<213>  Homo sapiens

<400>  39

ggtcagaggc cacgcccccg gaagcggcgg tgcagaaccc
agggaccatg ggcgcctcca     60

ggctctatac cctggtgctg gtcctgcagc ctcagcgagt
tctcctgggc atgaaaaagc    120

gaggcttcgg ggccggccgg tggaatggct ttgggggcaa
agtgcaagaa ggagagacca    180

tcgaggatgg ggctaggagg gagctgcagg aggagagcgg
tctgacagtg gacgccctgc    240

acaaggtggg ccagatcgtg tttgagttcg tgggcgagcc
tgagctcatg gacgtgcatg    300

tcttctgcac agacagcatc caggggaccc ccgtggagag
cgacgaaatg cgcccatgct    360

ggttccagct ggatcagatc cccttcaagg acatgtggcc
cgacgacagc tactggtttc    420

cactcctgct tcagaagaag aaattccacg ggtacttcaa
gttccagggt caggacacca    480

tcctggacta cacactccgc gaggtggaca cggtctagcg
ggagcccagg gcagcccctg    540

ggcaggagac gtggctgctg aacagccgca aaccatcttc
acctgggggc attgagtggc    600

gcagagccgg gtttcatctg gaattaactg gatggaaggg
aaaataaagc tatctagcgg    660

tgaaaaaaaa aaaaaaaaaa aaaaaaaaaa aa
                    692

<210>  40
<211>  661
<212>  DNA
<213>  Homo sapiens

<400>  40
ccggcggtga caacggcaac atggccctga acggagctga
agtcgacgac ttctcctggg     60

agcccccgac tgaggcggag acgaaggtgc tgcaggcgcg
acgggagcgg caagatcgca    120

tctcccggct catgggcgac tatctgctgc gcggttaccg
catgctgggc gagacgtgtg    180

cggactgcgg gacgatcctc ctccaagaca aacagcggaa
aatctactgc gtggcttgtc    240

aggaactcga ctcagacgtg gataaagata atcccgctct
gaatgcccag gctgccctct    300

cccaagctcg ggagcaccag ctggcctcag cctcagagct
ccccctgggc tctcgacctg    360

cgccccagcc cccagtacct cgtccggagc actgtgaggg
agctgcagca ggactcaagg    420

cagcccaggg gccacctgct cctgctgtgc ctccaaatac
agatgtcatg gcctgcacac    480

agacagccct cttgcagaag ctgacctggg cctctgctga
actgggctcc agcacctccc    540

tggagactag catccagctg tgtggcctta tccgcgcatg
tgcggaggcc ctgcgcagcc    600

tgcagcagct acagcactaa gagaagcccc tgagaaaaac
cctctagaaa aacaaaaaaa    660

a
                 661


<210>  41
<211>  1288
<212>  DNA
<213>  Homo sapiens

<400>  41
gctaaaggcg gactggcagg gggcagggaa gctcaaagat
ctggggtgct gccaggaaaa     60

agcaaattct ggaagttaat ggttttgagt gatttttaaa
tccttgctgg cggagaggcc    120

cgcctctccc cggtatcagc gcttcctcat tctttgaatc
cgcggctccg cggtcttcgg    180

cgtcagacca gccggaggaa gcctgtttgc aatttaagcg
ggctgtgaac gcccagggcc    240

ggcgggggca gggccgaggc gggccatttt gaataaagag
gcgtgccttc caggcaggct    300

ctataagtga ccgccgcggc gagcgtgcgc gcgttgcagg
tcactgtagc gggacttctt    360

ttggttttct ttctctttgg ggcacctctg gactcactcc
ccagcatgaa ggcgctgagc    420

ccggtgcgcg gctgctacga ggcggtgtgc tgcctgtcgg
aacgcagtct ggccatcgcc    480

cggggccgag ggaagggccc ggcagctgag gagccgctga
gcttgctgga cgacatgaac    540

cactgctact cccgcctgcg ggaactggta cccggagtcc
cgagaggcac tcagcttagc    600

caggtggaaa tcctacagcg cgtcatcgac tacattctcg
acctgcaggt agtcctggcc    660

gagccagccc ctggaccccc tgatggcccc caccttccca
tccagacagc cgagctcact    720

ccggaacttg tcatctccaa cgacaaaagg agcttttgcc
actgactcgg ccgtgtcctg    780

acacctccag aacgcaggtg ctggcgcccg ttctgcctgg
gaccccggga acctctcctg    840

ccggaagccg gacggcaggg atgggcccca acttcgccct
gcccacttga cttcaccaaa    900

tcccttcctg gagactaaac ctggtgctca ggagcgaagg
actgtgaact tgtggcctga    960

agagccagag ctagctctgg ccaccagctg ggcgacgtca
ccctgctccc accccacccc   1020

caagttctaa ggtctcttca gagcgtggag gtgtggaagg

agtggctgct ctccaaacta    1080

tgccaaggcg gcggcagagc tggtcttctg gtctccttgg
agaaaggttc tgttgccctg    1140

atttatgaac tctataatag agtatatagg ttttgtacct
tttttacagg aaggtgactt    1200

tctgtaacaa tgcgatgtat attaaacttt ttataaaagt
taacattttg cataataaac    1260

gatttttaaa cacttgtgta tatgatga
                    1288

<210>  42
<211>  2116
<212>  DNA
<213>  Homo sapiens

<400>  42
acagtgagct tccttatttg aagcaggact caattcttgg
ttaaaagcta tggtatttga      60

gctagttaaa cacatatctc tctcccattc catagggaat
gagctgggct gtcctttctc    120

cccacgttca cctgcacttc gttagagagc agtgttcaca
tgccacacca caagatcccc    180

acaatgacat aactccattc agagactggc gtgactgggc
tgggtctccc cacccccctt    240

cagctcttgt atcactcaga atctggcagc cagttccgtc
ctgacagagt tcacagcata    300

tattggtgga ttcttgtcca tagtgcatct gctttaagaa
ttaacgaaag cagtgtcaag    360

acagtaagga ttcaaaccat ttgccaaaaa tgagtctaag
tgcatttact ctcttcctgg    420

cattgattgg tggtaccagt ggccagtact atgattatga
ttttccccta tcaatttatg    480

ggcaatcatc accaaactgt gcaccagaat gtaactgccc

tgaaagctac ccaagtgcca 540

tgtactgtga tgagctgaaa ttgaaaagtg taccaatggt
gcctcctgga atcaagtatc 600

tttaccttag gaataaccag attgaccata ttgatgaaaa
ggcctttgag aatgtaactg 660

atctgcagtg gctcattcta gatcacaacc ttctagaaaa
ctccaagata aaagggagag 720

ttttctctaa attgaaacaa ctgaagaagc tgcatataaa
ccacaacaac ctgacagagt 780

ctgtgggccc acttcccaaa tctctggagg atctgcagct
tactcataac aagatcacaa 840

agctgggctc ttttgaagga ttggtaaacc tgaccttcat
ccatctccag cacaatcggc 900

tgaaagagga tgctgtttca gctgctttta aaggtcttaa
atcactcgaa taccttgact 960

tgagcttcaa tcagatagcc agactgcctt ctggtctccc
tgtctctctt ctaactctct 1020

acttagacaa caataagatc agcaacatcc ctgatgagta
tttcaagcgt tttaatgcat 1080

tgcagtatct gcgtttatct cacaacgaac tggctgatag
tggaatacct ggaaattctt 1140

tcaatgtgtc atccctggtt gagctggatc tgtcctataa
caagcttaaa aacataccaa 1200

ctgtcaatga aaaccttgaa aactattacc tggaggtcaa
tcaacttgag aagtttgaca 1260

taaagagctt ctgcaagatc ctggggccat tatcctactc
caagatcaag catttgcgtt 1320

tggatggcaa tcgcatctca gaaaccagtc ttccaccgga
tatgtatgaa tgtctacgtg 1380

ttgctaacga agtcactctt aattaatatc tgtatcctgg
aacaatattt tatggttatg 1440

tttttctgtg tgtcagtttt catagtatcc atattttatt
actgtttatt acttccatga    1500

attttaaaat ctgagggaaa tgttttgtaa acatttattt
tttttaaaga aaagatgaaa    1560

ggcaggccta tttcatcaca agaacacaca catatacacg
aatagacatc aaactcaatg    1620

ctttatttgt aaatttagtg tttttttatt tctactgtca
aatgatgtgc aaaacctttt    1680

actggttgca tggaaatcag ccaagtttta taatccttaa
atcttaatgt tcctcaaagc    1740

ttggattaaa tacatatgga tgttactctc ttgcaccaaa
ttatcttgat acattcaaat    1800

ttgtctggtt aaaaaatagg tggtagatat tgaggccaag
aatattgcaa aatacatgaa    1860

gcttcatgca cttaaagaag tatttttaga ataagaattt
gcatacttac ctagtgaaac    1920

ttttctagaa ttatttttca ctctaagtca tgtatgtttc
tctttgatta tttgcatgtt    1980

atgtttaata agctactagc aaaataaaac atagcaaatg
gcatcactgt gtttgacttc    2040

ttgtgaaatt tctgtacttt gtatataaaa tacataaaac
aatagattag aaatcaaaag    2100

atatctctgg cctgca
                2116

<210>  43
<211>  5718
<212>  DNA
<213>  Homo sapiens

<400>  43
ctcctgaggc tgccagcagc cagcagtgac tgcccgccct
atctgggacc caggatcgct      60

ctgtgagcaa cttggagcca gagaggagat caacaaggag
gaggagagag ccggcccctc    120

agccctgctg cccagcagca gcctgtgctc gccctgccca
acgcagacag ccagacccag    180

ggcggcccct ctggcggctc tgctcctccc gaaggatgct
tggggagtga ggcgaagctg    240

ggccgctcct ctcccctaca gcagccccct tcctccatcc
ctctgttctc ctgagccttc    300

aggagcctgc accagtcctg cctgtccttc tactcagctg
ttacccactc tgggaccagc    360

agtctttctg ataactggga gagggcagta aggaggactt
cctggagggg gtgactgtcc    420

agagcctgga actgtgccca caccagaagc catcagcagc
aaggacacca tgcggcttcc    480

gggtgcgatg ccagctctgg ccctcaaagg cgagctgctg
ttgctgtctc tcctgttact    540

tctggaacca cagatctctc agggcctggt cgtcacaccc
ccggggccag agcttgtcct    600

caatgtctcc agcaccttcg ttctgacctg ctcgggttca
gctccggtgg tgtgggaacg    660

gatgtcccag gagcccccac aggaaatggc caaggcccag
gatggcacct tctccagcgt    720

gctcacactg accaacctca ctgggctaga cacgggagaa
tacttttgca cccacaatga    780

ctcccgtgga ctggagaccg atgagcggaa acggctctac
atctttgtgc cagatcccac    840

cgtgggcttc ctccctaatg atgccgagga actattcatc
tttctcacgg aaataactga    900

gatcaccatt ccatgccgag taacagaccc acagctggtg
gtgacactgc acgagaagaa    960

aggggacgtt gcactgcctg tcccctatga tcaccaacgt
ggcttttctg gtatctttga    1020

ggacagaagc tacatctgca aaaccaccat tggggacagg
gaggtggatt ctgatgccta    1080

ctatgtctac agactccagg tgtcatccat caacgtctct
gtgaacgcag tgcagactgt    1140

ggtccgccag ggtgagaaca tcaccctcat gtgcattgtg
atcgggaatg aggtggtcaa    1200

cttcgagtgg acataccccc gcaaagaaag tgggcggctg
gtggagccgg tgactgactt    1260

cctcttggat atgccttacc acatccgctc catcctgcac
atccccagtg ccgagttaga    1320

agactcgggg acctacacct gcaatgtgac ggagagtgtg
aatgaccatc aggatgaaaa    1380

ggccatcaac atcaccgtgg ttgagagcgg ctacgtgcgg
ctcctgggag aggtgggcac    1440

actacaattt gctgagctgc atcggagccg gacactgcag
gtagtgttcg aggcctaccc    1500

accgcccact gtcctgtggt tcaaagacaa ccgcaccctg
ggcgactcca gcgctggcga    1560

aatcgccctg tccacgcgca acgtgtcgga gacccggtat
gtgtcagagc tgacactggt    1620

tcgcgtgaag gtggcagagg ctggccacta caccatgcgg
gccttccatg aggatgctga    1680

ggtccagctc tccttccagc tacagatcaa tgtccctgtc
cgagtgctgg agctaagtga    1740

gagccaccct gacagtgggg aacagacagt ccgctgtcgt
ggccggggca tgccccagcc    1800

gaacatcatc tggtctgcct gcagagacct caaaaggtgt
ccacgtgagc tgccgcccac    1860

gctgctgggg aacagttccg aagaggagag ccagctggag

actaacgtga cgtactggga    1920

ggaggagcag gagtttgagg tggtgagcac actgcgtctg
cagcacgtgg atcggccact    1980

gtcggtgcgc tgcacgctgc gcaacgctgt gggccaggac
acgcaggagg tcatcgtggt    2040

gccacactcc ttgcccttta aggtggtggt gatctcagcc
atcctggccc tggtggtgct    2100

caccatcatc tcccttatca tcctcatcat gctttggcag
aagaagccac gttacgagat    2160

ccgatggaag gtgattgagt ctgtgagctc tgacggccat
gagtacatct acgtggaccc    2220

catgcagctg ccctatgact ccacgtggga gctgccgcgg
gaccagcttg tgctgggacg    2280

caccctcggc tctggggcct ttgggcaggt ggtggaggcc
acggctcatg gcctgagcca    2340

ttctcaggcc acgatgaaag tggccgtcaa gatgcttaaa
tccacagccc gcagcagtga    2400

gaagcaagcc cttatgtcgg agctgaagat catgagtcac
cttgggcccc acctgaacgt    2460

ggtcaacctg ttgggggcct gcaccaaagg aggacccatc
tatatcatca ctgagtactg    2520

ccgctacgga gacctggtgg actacctgca ccgcaacaaa
cacaccttcc tgcagcacca    2580

ctccgacaag cgccgcccgc ccagcgcgga gctctacagc
aatgctctgc ccgttgggct    2640

ccccctgccc agccatgtgt ccttgaccgg ggagagcgac
ggtggctaca tggacatgag    2700

caaggacgag tcggtggact atgtgcccat gctggacatg
aaaggagacg tcaaatatgc    2760

agacatcgag tcctccaact acatggcccc ttacgataac
tacgttccct ctgcccctga    2820

gaggacctgc cgagcaactt tgatcaacga gtctccagtg
ctaagctaca tggacctcgt   2880

gggcttcagc taccaggtgg ccaatggcat ggagtttctg
gcctccaaga actgcgtcca   2940

cagagacctg gcggctagga acgtgctcat ctgtgaaggc
aagctggtca agatctgtga   3000

ctttggcctg gctcgagaca tcatgcggga ctcgaattac
atctccaaag gcagcacctt   3060

tttgccttta aagtggatgg ctccggagag catcttcaac
agcctctaca ccaccctgag   3120

cgacgtgtgg tccttcggga tcctgctctg ggagatcttc
accttgggtg gcacccctta   3180

cccagagctg cccatgaacg agcagttcta caatgccatc
aaacggggtt accgcatggc   3240

ccagcctgcc catgcctccg acgagatcta tgagatcatg
cagaagtgct gggaagagaa   3300

gtttgagatt cggcccccct tctcccagct ggtgctgctt
ctcgagagac tgttgggcga   3360

aggttacaaa aagaagtacc agcaggtgga tgaggagttt
ctgaggagtg accacccagc   3420

catccttcgg tcccaggccc gcttgcctgg gttccatggc
ctccgatctc ccctggacac   3480

cagctccgtc ctctatactg ccgtgcagcc caatgagggt
gacaacgact atatcatccc   3540

cctgcctgac cccaaacccg aggttgctga cgagggccca
ctggagggtt cccccagcct   3600

agccagctcc accctgaatg aagtcaacac ctcctcaacc
atctcctgtg acagccccct   3660

ggagccccag gacgaaccag agccagagcc ccagcttgag
ctccaggtgg agccggagcc   3720

agagctggaa cagttgccgg attcggggtg ccctgcgcct
cgggcggaag cagaggatag 3780

cttcctgtag ggggctggcc cctaccctgc cctgcctgaa
gctcccccc tgccagcacc 3840

cagcatctcc tggcctggcc tgaccgggct tcctgtcagc
caggctgccc ttatcagctg 3900

tccccttctg gaagctttct gctcctgacg tgttgtgccc
caaaccctgg ggctggctta 3960

ggaggcaaga aaactgcagg ggccgtgacc agccctctgc
ctccagggag gccaactgac 4020

tctgagccag ggttccccca gggaactcag ttttcccata
tgtaagatgg gaaagttagg 4080

cttgatgacc cagaatctag gattctctcc ctggctgaca
ggtggggaga ccgaatccct 4140

ccctgggaag attcttggag ttactgaggt ggtaaattaa
ctttttttctg ttcagccagc 4200

tacccctcaa ggaatcatag ctctctcctc gcacttttat
ccacccagga gctagggaag 4260

agaccctagc ctccctggct gctggctgag ctagggccta
gccttgagca gtgttgcctc 4320

atccagaaga aagccagtct cctccctatg atgccagtcc
ctgcgttccc tggcccgagc 4380

tggtctgggg ccattaggca gcctaattaa tgctggaggc
tgagccaagt acaggacacc 4440

cccagcctgc agcccttgcc cagggcactt ggagcacacg
cagccatagc aagtgcctgt 4500

gtccctgtcc ttcaggccca tcagtcctgg ggctttttct
ttatcaccct cagtcttaat 4560

ccatccacca gagtctagaa ggccagacgg gccccgcatc
tgtgatgaga atgtaaatgt 4620

gccagtgtgg agtggccacg tgtgtgtgcc agtatatggc

cctggctctg cattggacct   4680

gctatgaggc tttggaggaa tccctcaccc tctctgggcc
tcagtttccc cttcaaaaaa   4740

tgaataagtc ggacttatta actctgagtg ccttgccagc
actaacattc tagagtattc   4800

caggtggttg cacatttgtc cagatgaagc aaggccatat
accctaaact tccatcctgg   4860

gggtcagctg ggctcctggg agattccaga tcacacatca
cactctgggg actcaggaac   4920

catgcccctt ccccaggccc ccagcaagtc tcaagaacac
agctgcacag gccttgactt   4980

agagtgacag ccggtgtcct ggaaagcccc cagcagctgc
cccagggaca tgggaagacc   5040

acgggacctc tttcactacc cacgatgacc tccgggggta
tcctgggcaa aagggacaaa   5100

gagggcaaat gagatcacct cctgcagccc accactccag
cacctgtgcc gaggtctgcg   5160

tcgaagacag aatggacagt gaggacagtt atgtcttgta
aaagacaaga agcttcagat   5220

gggtacccca agaaggatgt gagaggtggg cgctttggag
gtttgcccct cacccaccag   5280

ctgccccatc cctgaggcag cgctccatgg gggtatggtt
ttgtcactgc ccagacctag   5340

cagtgacatc tcattgtccc cagcccagtg ggcattggag
gtgccagggg agtcagggtt   5400

gtagccaaga cgcccccgca cggggagggt tgggaagggg
gtgcaggaag ctcaacccct   5460

ctgggcacca accctgcatt gcaggttggc accttacttc
cctgggatcc ccagagttgg   5520

tccaaggagg gagagtgggt tctcaatacg gtaccaaaga
tataatcacc taggtttaca   5580

aatattttta ggactcacgt taactcacat ttatacagca
gaaatgctat tttgtatgct   5640

gttaagtttt tctatctgtg tacttttttt taagggaaag
attttaatat taaacctggt   5700

gcttctcact cacaaaaa
                  5718



<210>  44
<211>  3178
<212>  DNA
<213>  Homo sapiens

<400>  44
gttgcctgtc tctaaacccc tccacattcc cgcggtcctt
cagactgccc ggagagcgcg     60

ctctgcctgc cgcctgcctg cctgccactg agggttccca
gcaccatgag ggcctggatc    120

ttctttctcc tttgcctggc cgggagggcc ttggcagccc
ctcagcaaga agccctgcct    180

gatgagacag aggtggtgga agaaactgtg gcagaggtga
ctgaggtatc tgtgggagct    240

aatcctgtcc aggtggaagt aggagaattt gatgatggtg
cagaggaaac cgaagaggag    300

gtggtggcgg aaaatccctg ccagaaccac cactgcaaac
acggcaaggt gtgcgagctg    360

gatgagaaca acacccccat gtgcgtgtgc caggacccca
ccagctgccc agcccccatt    420

ggcgagtttg agaaggtgtg cagcaatgac aacaagacct
tcgactcttc ctgccacttc    480

tttgccacaa agtgcaccct ggagggcacc aagaagggcc
acaagctcca cctggactac    540

atcgggcctt gcaaatacat ccccccttgc ctggactctg
agctgaccga attcccctg    600

cgcatgcggg actggctcaa gaacgtcctg gtcaccctgt
atgagaggga tgaggacaac    660

aaccttctga ctgagaagca gaagctgcgg gtgaagaaga
tccatgagaa tgagaagcgc    720

ctggaggcag gagaccaccc cgtggagctg ctggcccggg
acttcgagaa gaactataac    780

atgtacatct tccctgtaca ctggcagttc ggccagctgg
accagcaccc cattgacggg    840

tacctctccc acaccgagct ggctccactg cgtgctcccc
tcatccccat ggagcattgc    900

accacccgct ttttcgagac ctgtgacctg gacaatgaca
agtacatcgc cctggatgag    960

tgggccggct gcttcggcat caagcagaag gatatcgaca
aggatcttgt gatctaaatc   1020

cactccttcc acagtaccgg attctctctt taaccctccc
cttcgtgttt cccccaatgt   1080

ttaaaatgtt tggatggttt gttgttctgc ctggagacaa
ggtgctaaca tagatttaag   1140

tgaatacatt aacggtgcta aaaatgaaaa ttctaaccca
agacatgaca ttcttagctg   1200

taacttaact attaaggcct tttccacacg cattaatagt
cccatttttc tcttgccatt   1260

tgtagctttg cccattgtct tattggcaca tgggtggaca
cggatctgct gggctctgcc   1320

ttaaacacac attgcagctt caacttttct ctttagtgtt
ctgtttgaaa ctaatactta   1380

ccgagtcaga ctttgtgttc atttcatttc agggtcttgg
ctgcctgtgg gcttccccag   1440

gtggcctgga ggtgggcaaa gggaagtaac agacacacga
tgttgtcaag gatggttttg   1500

ggactagagg ctcagtggtg ggagagatcc ctgcagaacc
caccaaccag aacgtggttt   1560

gcctgaggct gtaactgaga gaaagattct ggggctgtgt
tatgaaaata tagacattct   1620

cacataagcc cagttcatca ccatttcctc ctttaccttt
cagtgcagtt tcttttcaca   1680

ttaggctgtt ggttcaaact tttgggagca cggactgtca
gttctctggg aagtggtcag   1740

cgcatcctgc agggcttctc ctcctctgtc ttttggagaa
ccagggctct tctcaggggc   1800

tctagggact gccaggctgt ttcagccagg aaggccaaaa
tcaagagtga gatgtagaaa   1860

gttgtaaaat agaaaaagtg gagttggtga atcggttgtt
ctttcctcac atttggatga   1920

ttgtcataag gtttttagca tgttcctcct tttcttcacc
ctcccctttt ttcttctatt   1980

aatcaagaga aacttcaaag ttaatgggat ggtcggatct
cacaggctga gaactcgttc   2040

acctccaagc atttcatgaa aaagctgctt cttattaatc
atacaaactc tcaccatgat   2100

gtgaagagtt tcacaaatcc ttcaaaataa aaagtaatga
cttagaaact gccttcctgg   2160

gtgatttgca tgtgtcttag tcttagtcac cttattatcc
tgacacaaaa acacatgagc   2220

atacatgtct acacatgact acacaaatgc aaacctttgc
aaacacatta tgcttttgca   2280

cacacacacc tgtacacaca caccggcatg tttatacaca
gggagtgtat ggttcctgta   2340

agcactaagt tagctgtttt catttaatga cctgtggttt
aaccctttg atcactacca   2400

ccattatcag caccagactg agcagctata tccttttatt

aatcatggtc attcattcat   2460

tcattcattc acaaaatatt tatgatgtat ttactctgca
ccaggtccca tgccaagcac   2520

tggggacaca gttatggcaa agtagacaaa gcatttgttc
atttggagct tagagtccag   2580

gaggaataca ttagataatg acacaatcaa atataaattg
caagatgtca caggtgtgat   2640

gaagggagag taggagagac catgagtatg tgtaacagga
ggacacagca ttattctagt   2700

gctgtactgt tccgtacggc agccactacc cacatgtaac
tttttaagat ttaaatttaa   2760

attagttaac attcaaaacg cagctcccca atcacactag
caacatttca agtgcttgag   2820

agccatgcat gattagtggt taccctattg aataggtcag
aagtagaatc ttttcatcat   2880

cacagaaagt tctattggac agtgctcttc tagatcatca
taagactaca gagcactttt   2940

caaagctcat gcatgttcat catgttagtg tcgtattttg
agctggggtt ttgagactcc   3000

ccttagagat agagaaacag acccaagaaa tgtgctcaat
tgcaatgggc cacataccta   3060

gatctccaga tgtcatttcc cctctcttat tttaagttat
gttaagatta ctaaaacaat   3120

aaaagctcct aaaaaatcaa aaaaaaaaaa aaaaaaaaaa
aaaaaaaaaa aaaaaaaa     3178


<210>  45
<211>  2305
<212>  DNA
<213>  Homo sapiens

<400>  45
gaatctacaa taagacaaat ttcaaatcaa gttgctccac

tatactgcat aagcagttta     60

gaatcttaag cagatgcaaa aagaataaag caaatgggag
gaaaaaaaag gccgataaag     120

tttctggcta caatacaaga gacatatcat taccatatga
tctaatgtgg gtgtcagccg     180

gattgtgttc attgagggaa accttatttt ttaactgtgc
tatggagtag aagcaggagg     240

ttttcaacct agtcacagag cagcacctac cccctcctcc
tttccacacc tgcaaactct     300

tttacttggg ctgaatattt agtgtaatta catctcagct
ttgagggctc ctgtggcaaa     360

ttcccggatt aaaaggttcc ctggttgtga aaatacatga
gataaatcat gaaggccact     420

atcatcctcc ttctgcttgc acaagtttcc tgggctggac
cgtttcaaca gagaggctta     480

tttgacttta tgctagaaga tgaggcttct gggataggcc
cagaagttcc tgatgaccgc     540

gacttcgagc cctccctagg cccagtgtgc cccttccgct
gtcaatgcca tcttcgagtg     600

gtccagtgtt ctgatttggg tctggacaaa gtgccaaagg
atcttccccc tgacacaact     660

ctgctagacc tgcaaaacaa caaaataacc gaaatcaaag
atggagactt taagaacctg     720

aagaaccttc acgcattgat tcttgtcaac aataaaatta
gcaaagttag tcctggagca     780

tttacacctt tggtgaagtt ggaacgactt tatctgtcca
agaatcagct gaaggaattg     840

ccagaaaaaa tgcccaaaac tcttcaggag ctgcgtgccc
atgagaatga gatcaccaaa     900

gtgcgaaaag ttactttcaa tggactgaac cagatgattg
tcatagaact gggcaccaat     960

ccgctgaaga gctcaggaat tgaaaatggg gctttccagg
gaatgaagaa gctctcctac   1020

atccgcattg ctgataccaa tatcaccagc attcctcaag
gtcttcctcc ttcccttacg   1080

gaattacatc ttgatggcaa caaaatcagc agagttgatg
cagctagcct gaaaggactg   1140

aataatttgg ctaagttggg attgagtttc aacagcatct
ctgctgttga caatggctct   1200

ctggccaaca cgcctcatct gagggagctt cacttggaca
acaacaagct taccagagta   1260

cctggtgggc tggcagagca taagtacatc caggttgtct
accttcataa caacaatatc   1320

tctgtagttg gatcaagtga cttctgccca cctggacaca
acaccaaaaa ggcttcttat   1380

tcgggtgtga gtcttttcag caacccggtc cagtactggg
agatacagcc atccaccttc   1440

agatgtgtct acgtgcgctc tgccattcaa ctcggaaact
ataagtaatt ctcaagaaag   1500

ccctcatttt tataacctgg caaaatcttg ttaatgtcat
tgctaaaaaa taaataaaag   1560

ctagatactg gaaacctaac tgcaatgtgg atgttttacc
cacatgactt attatgcata   1620

aagccaaatt tccagtttaa gtaattgcct acaataaaaa
gaaattttgc ctgccatttt   1680

cagaatcatc ttttgaagct ttctgttgat gttaactgag
ctactagaga tattcttatt   1740

tcactaaatg taaaatttgg agtaaatata tatgtcaata
tttagtaaag cttttctttt   1800

ttaatttcca ggaaaaaata aaaagagtat gagtcttctg
taattcattg agcagttagc   1860

tcatttgaga taaagtcaaa tgccaaacac tagctctgta
ttaatcccca tcattactgg   1920

taaagcctca tttgaatgtg tgaattcaat acaggctatg
taaaattttt actaatgtca   1980

ttattttgaa aaaataaatt taaaaataca ttcaaaatta
ctattgtata caagcttaat   2040

tgttaatatt ccctaaacac aattttatga agggagaaga
cattggtttg ttgacaataa   2100

cagtacatct tttcaagttc tcagctattt cttctacctc
tccctatctt acatttgagt   2160

atggtaactt atgtcatcta tgttgaatgt aagcttataa
agcacaaagc atacatttcc   2220

tgactggtct agagaactga tgtttcaatt tacccctctg
ctaaataaat attaaaacta   2280

tcatgtgaaa aaaaaaaaaa aaaaa
                2305


<210>  46
<211>  1212
<212>  DNA
<213>  Homo sapiens

<400>  46
ggagtccaga cccgacggcc ggcccagttc cacgcaccca
gcgagcccaa gcgccttctc     60

cgcaccaggg aagccccacc caccagaagc caagatgtcc
agcaagcggg ccaaagccaa    120

gaccaccaag aagcggccac agcgggccac atccaatgtc
ttcgcaatgt ttgaccagtc    180

ccagatccag gagtttaagg aggctttcaa catgattgac
cagaaccgtg atggcttcat    240

tgacaaggag gacctgcacg acatgctggc ctcgctgggg
aagaacccca cagacgaata    300

cctggagggc atgatgagcg aggccccggg gcccatcaac
ttcaccatgt tcctcaccat    360

gtttggggag aagctgaacg gcacggaccc cgaggatgtg
attcgcaacg cctttgcctg    420

cttcgacgag gaagcctcag gtttcatcca tgaggaccac
ctccgggagc tgctcaccac    480

catgggtgac cgcttcacag atgaggaagt ggacgagatg
taccgggagg cacccattga    540

taagaaaggc aacttcaact acgtggagtt cacccgcatc
ctcaaacatg gcgccaagga    600

taaagacgac taggccaccc cagccccctg acaccccagc
ccccgccagt cacccctccc    660

cgcacacacc cgtccatacc agctccctgc ccatgaccct
cgctcaggga tccccctttg    720

aggggttagg gtcccagttc ccagtggaag aaacaggcca
ggagaagtgc gtgccgagct    780

gaggcagatg ttcccacagt gaccccagag ccctgggcta
tagtctctga cccctccaag    840

gaaagaccac cttctgggga catgggctgg agggcaggac
ctagaggcac caagggaagg    900

ccccattccg gggctgttcc ccgaggagga agggaagggg
ctctgtgtgc cccccaggag    960

gaagaggccc tgagtcctgg gatcagacac cccttcacgt
gtatccccac acaaatgcaa   1020

gctcaccaag gtcccctctc agtccccttc cctacaccct
gaccggccac tgccgcacac   1080

ccacccagag cacgccaccc gccatgggag tgtgctcagg
agtcgcgggc agcgtggaca   1140

tctgtcccag agggggcaga atctccaata gaggactgag
cactgctaaa aaaaaaaaaa   1200

aaaaaaaaaa aa

<210>  47
<211>  2066
<212>  DNA
<213>  Homo sapiens

<400>  47
aggtctcgca ggccccgccc cctcgccgcg ggttcgctgt
tgggcggaga tattcgccgc      60

cggcgcttgc gcccggaagg tgtgccgcac cacacggggg
aggaaggaag gagctcccaa     120

ctcgccggcc tggccacggg atggccccca aattcccaga
ctctgtggag gagctccgcg     180

ccgccggcaa tgagagtttc cgcaacggcc agtacgccga
ggcctccgcg ctctacggcc     240

gcgcgctgcg ggtgctgcag gcgcaaggtt cttcagaccc
agaagaagaa agtgttctct     300

actccaaccg agcagcatgt cacttgaagg atggaaactg
cagagactgc atcaaagatt     360

gcacttcagc actggccttg gttcccttca gcattaagcc
cctgctgcgg cgagcatctg     420

cttatgaggc tctggagaag taccctatgg cctatgttga
ctataagact gtgctgcaga     480

ttgatgataa tgtgacgtca gccgtagaag gcatcaacag
aatgaccaga gctctcatgg     540

actcgcttgg gcctgagtgg cgcctgaagc tgccctcaat
cccccttggtg cctgtttcag     600

ctcagaagag gtggaattcc ttgccttcgg agaaccacaa
agagatggct aaaagcaaat     660

ccaaagaaac cacagctaca aagaacagag tgccttctgc
tggggatgtg gagaaagcca     720

gagttctgaa ggaagaaggc aatgagcttg taaagaaggg

aaaccataag aaagctattg    780

agaagtacag tgaaagcctc ttgtgtagta acctggaatc
tgccacgtac agcaacagag    840

cactctgcta tttggtcctg aagcagtaca cagaagcagt
gaaggactgc acagaagccc    900

tcaagctgga tggaaagaac gtgaaggcat tctacagacg
ggctcaagcc cacaaagcac    960

tcaaggacta taaatccagc tttgcagaca tcagcaacct
cctacagatt gagcctagga   1020

atggtcctgc acagaagttg cggcaggaag tgaagcagaa
cctacactaa aaacccaaca   1080

gggcaactgg aacccctgcc tgaccttacc cagagaagcc
atgggccacc tgctctgtgc   1140

ccgctcctga aacccagcat gccccaagtg agctctgaag
ccccctcctc aatcccttga   1200

tggcctccca ccctgtaaga ggctttgctt gttcaaatta
aactcagtgt agtcaaacac   1260

agacatggtt gttgcaccag aaaggtcccc actagagcta
agcgtgaagc tgaagctctg   1320

tccctattcc cccagcccag ctagctgatc acaccaacag
atcctcatca gcaaagcatt   1380

tggctttgtc ctgcccaagt gggctgcaga ctgagtgctg
cccttgtagc ttccccagac   1440

cccaactcac tgcagttcat ctgaacaacc tgagctcctg
ggccggggtg gaaggagggg   1500

gataaaccta aggccctgat ccaaagcagc ctgttgagct
ggttctccag ggctgcagtc   1560

tctccaggtg tacagctgct gtccctgccc tgtcctgtcc
ttgcacagtc tcctatgtct   1620

gagccccagt gccttctgtt cgggccctcc tttggtggga
aggcagagcc ctgacccttg   1680

aatggttgtc cttgactctg tgctgctgcc ttctgcagag
aggcacctaa gctgtttaaa   1740

gagcccagtg attgtggctg ctcctcctag aggtgggagg
gggcaagagg cctccttggt   1800

cagtgtccat gctttctggg cagggacttg gtttttgtt
ccaacagtgg ccttctccgg   1860

gcttcatagt tctttgtaat atgttgaagt taatttgaat
tgactgattt tgttgaactg   1920

tgtgtttaag ctgttgcatt aaaaagcttt cttctacatc
aatatctgct gtgctttcat   1980

ttatgccttt tcagctttgc acctggaact ctgtagtaat
aataaaagtt attgcttatt   2040

gggcattcaa aaaaaaaaaa aaaaaa
                2066

<210>  48
<211>  2630
<212>  DNA
<213>  Homo sapiens

<400>  48
ggttccagcg acagcagcac tggactcgtc cagagggcgg
cgggtgagcg gctggggccc     60

cgtggagcca ccatggaccc cgcaggggca gcagacccct
cagtgcctcc caatcctttg    120

actcacctga gcctgcagga cagatcagag atgcagctgc
agagcgaagc cgacaggcgg    180

agcctcccgg gcacttggac caggtcatcc ccagagcaca
ccaccattct gaggggaggc    240

gtgcgcaggt gcctgcagca acagtgtgaa cagactgtgc
ggatcctgca tgccaaggtg    300

gcccagaaat catacggaaa tgagaagcgg ttcttctgcc
ccccgccctg tgtctacctc    360

tcggggcctg gctggagggt gaagccaggg caggatcaag
ctcaccaggc gggggaaacg    420

gggcccacgg tctgcggtta catgggactg gacagcgcgt
ccggcagcgc cactgagacg    480

cagaagctga atttcgagca gcagccggac tccagggaat
tcggctgcgc caagaccctg    540

tacatctcag atgcagacaa gaggaagcac tttcggctgg
tgctgcggct ggtgctgcgc    600

gggggccggg agctgggtac cttccacagc cgccttatca
aggtcatctc gaagccctcg    660

cagaagaagc agtcgctgaa aaacaccgat ctgtgcatat
cctccggctc aaaggtctcc    720

ctcttcaacc gcctgcgctc tcagacggtc tccacacgct
acctctctgt ggaggatggg    780

gcctttgtgg ccagtgcacg acagtgggct gccttcacgc
tccacctggc tgatgggcac    840

tctgcccaag gagacttccc accgcgagag ggctacgttc
gctatggctc cctggtgcag    900

ctcgtctgca cggtcaccgg catcacacta cctcccatga
tcatccgtaa agtagcaaaa    960

cagtgtgcgc tccttgatgt ggatgagccc atctcccagc
tgcacaagtg tgcattccag   1020

tttccaggca gtcccccagg agggggtggc acctacttat
gccttgccac agagaaggtg   1080

gtgcaatttc aggcctctcc ctgccccaag gaggcgaaca
gggctctgct taacgacagc   1140

tcttgctgga ccatcatcgg caccgagtcg gtggaatttt
ccttcagcac cagcctggcg   1200

tgtaccctgg agccggtcac tccggtgcct ctcatcagca
ccctagagct gagcggcggg   1260

ggcgacgtgg ccacgctgga gctccacgga gagaacttcc
acgcggggct caaggtgtgg   1320

tttggggacg tggaggcaga aaccatgtac aggagcccgc
ggtccctggt gtgcgtggtg   1380

ccggacgtgg cggccttctg cagcgactgg cgctggctgc
gcgctcccat cacaatcccc   1440

atgagcctgg tgcgcgccga cgggctcttc taccctagtg
ccttctcctt cacctacacc   1500

ccggaataca gcgtgcggcc gggtcacccc ggcgtccccg
agcccgccac cgacgccgac   1560

gcgctcctgg agagcatcca tcaggagttc acgcgcacca
acttccacct cttcatccag   1620

acttaggcgc gcccggtagc cccggctgcc caccctggag
ggctgcgccc gcgccaggcg   1680

cggggacgtg tttctgggtt ctaggccctg cttccttgcc
cctttgctgc agaagggcag   1740

ctgaaggctc accctagaaa ccgggcctgg tgggtcttac
ccggctcact ccctcccttg   1800

tccttacaca tacaggaaga caagacctga gtggtgctgt
ctttgtgtcc gtcgtgtatg   1860

gctctccctg tcttcatttc ttctcactct gtctctaaac
ctctctctct ctcccttccc   1920

cctcagtact tagtctacag acctatgtgc gtgtccctat
ccttctgtcc ttttctctct   1980

tcagctctcc ctgcctctca cacacaattt tacatgcccc
gaggagccaa gtttgggaca   2040

tttaccctcc aggcatctgt gtcccctctt gaagagaaaa
cacacagctt cacacatcca   2100

ggcatagggg gcaagctctt ggggcatcag gaccctggag
caccaggtcc ttcctggaat   2160

attagatcca cctggagcac cgggtctctc taagtctcac

ctggggaatt cggtcccacc   2220

tggggcacca gttcccacct agagcactgt gtcctgccct
agagcacaaa gacctgctcc   2280

tcccgagact ctctctgact gcagccaggc atagtacctt
tgcctgtgtt tgctccctgg   2340

tccacagatt tggtggctgg gcaggtgcct ggacagtgat
gaggtcttgc cgccttaact   2400

gtccccccca gtcacttctc ccacaggccc agcaggacgc
agtcctgagg atcagggatt   2460

ctacagctgc attaaaatca atcctatcca aaaaaaaaaa
aaaaaaaaaa aaaaaaaaaa   2520

aaaaaaaaaa aaaaaaaaaa aaaaaaaaaa aaaaaaaaaa
aaaaaaaaaa aaaaaaaaaa   2580

aaaaaaaaaa aaaaaaaaaa aaaaaaaaaa aaaaaaaaaa
aaaaaaaaaa              2630


<210>  49
<211>  2775
<212>  DNA
<213>  Homo sapiens

<400>  49
actgcgagct gcggcgccgc acagcttcgt ggcgctctgg
gcacccctgt tcctgctgcg     60

ctccgccctg gccgacttca gcctggacaa cgaggtgcac
tcgagcttca tccaccggcg    120

cctccgcagc caggagcggc gggagatgca gcgcgagatc
ctctccattt tgggcttgcc    180

ccaccgcccg cgcccgcacc tccagggcaa gcacaactcg
gcacccatgt tcatgctgga    240

cctgtacaac gccatggcgg tggaggaggg cggcgggccc
ggcggccagg gcttctccta    300

cccctactgg atcatcgcgc ctgaaggctg cgccgcctac

tactgtgagg gggagtgtgc    360

cttccctctg aactcctaca tgaacgccac caaccacgcc
atcgtgcaga cgctggtcca    420

cttcatcaac ccggaaacgg tgcccaagcc ctgctgtgcg
cccacgcagc tcaatgccat    480

ctccgtcctc tacttcgatg acagctccaa cgtcatcctg
aagaaataca gaaacatggt    540

ggtccgggcc tgtggctgcc actagctcct ccgagaattc
agaccctttg gggccaagtt    600

tttctggatc ctccattgct cgccttggcc aggaaccagc
agaccaactg ccttttgtga    660

gaccttcccc tccctatccc caactttaaa ggtgtgagag
tattaggaaa catgagcagc    720

atatggcttt tgatcagttt ttcagtggca gcatccaatg
aacaagatcc tacaagctgt    780

gcaggcaaaa cctagcagga aaaaaaaaca acgcataaag
aaaaatggcc gggccaggtc    840

attggctggg aagtctcagc catgcacgga ctcgtttcca
gaggtaatta tgagcgccca    900

ccagccaggc cacccagccg tgggaggaag ggggcgtggc
aaggggtggg cacattggtg    960

tctgtgcgaa aggaaaattg acccggaagt tcctgtaata
aatgtcacaa taaaacgaat    1020

gaatgaaaat ggttaggacg ttacagatat attttcctaa
acaatttatc cccatttctc    1080

ggtttatcct gatgcgtaaa cagaagctgt gtcaagtgga
gggcggggag gtccctctcc    1140

attccctaca gttttcatcc tgaggcttgc agaggcccag
tgtttaccga ggtttgccca    1200

aatccaagat ctagtgggag gggaaagggc aaatgtctgc
tccgaggagg gcggtgtgtt    1260

gatctttgga ggaaaaatat gttctgttgt tcagctggat
ttgccgtggc agaaatgaaa    1320

ctaggtgtgt gaaatacccg cagacatttg ggattggctt
ttcacctcgc cccagtggta    1380

gtaaatccat gtgaaattgc agaggggaca aggacagcaa
gtaggatgga acttgcaact    1440

caaccctgtt gttaagaagc accaatgggc cgggcacagt
agctcccacc tgtaatccca    1500

gcacttcggg aggctgaggt gggcggatca tttgaggtca
ggagttcgag accagcctgg    1560

ccaacatggt gaaaccccat ctctactaaa aatacaaaaa
ttagccgggc atggtggcac    1620

gcacctgtaa tcccagctac tctggaggct gaggcaggag
aattgcttga acccgggagg    1680

tggaggttgc agtgagccaa gatcgtccca ctgcactcca
gcttgggtga caaaacaaga    1740

ctccatctca aaagaaaaaa aaaacagcac caatgaagcc
tagttctcca cgggagtggg    1800

gtgagcagga gcactgcaca tcgccccagt ggaccctctg
gtctttgtct gcagtggcat    1860

tccaaggctg ggccctggca agggcacccg tggctgtctc
ttcatttgca gaccctgatc    1920

agaagtctct gcaaacaaat ttgctccttg aattaagggg
gagatggcat aataggaggt    1980

ctgatgggtg caggatgtgc tggacttaca ttgcaaatag
aagccttgtt gagggtgaca    2040

tcctaaccaa gtgtcccgat ttggaggtgg catttctgac
atggctcttg gtgtaagcct    2100

gccttgcctt ggctggtgag tcccataaat agtatgcact
cagcctccgg ccacaaacac    2160

aaggcctcgg ggagggctag actgtctgca aaggttttct
gcatctgtaa agaaaacaag   2220

gtgatcgaaa actgtggcca tgtggaaccc ggtcttgtgg
gggactgtgt ctccatcttg   2280

actcagacag ttcctggaaa caccggggct ctgtttttat
tttctttgat gtttttcttc   2340

tttagtagct tgggctgcag cctccactct ctagtcactg
gggaggagta tttttgtta   2400

tgtttggttt catttgctgg cagagctggg gctttttgtg
tgatccctct tggtgtgagt   2460

tttctgaccc aaccagcctc tggttagcat catttgtaca
tttaaacctg taaatagttg   2520

ttacaaagca aagagattat ttatttccat ccaaagctct
tttgaacacc cccccccttt   2580

aatccctcgt tcaggacgat gagcttgctt tccttcaacc
tgtttgtttt cttatttaag   2640

actatttatt aatggttgga ccaatgtact cacggctgtt
gcgtcgagca gtccttagtg   2700

aaaattctgt ataaatagac aaaatgaaaa gggtttgacc
ttgcaataaa aggagacgtt   2760

tggttctggc tcttt
                2775

<210>  50
<211>  2453
<212>  DNA
<213>  Homo sapiens

<400>  50
agccgagctc aggatgtgct tcccagcttc actggttaat
ttgacctgaa cctatttaaa      60

gatcccttct gcccctgaag acctatccgc actcaaattc
taacatgaag aaatctactc     120

gaatgcatcc tttactttga atgagctcta ttcggttgca
tgttatatgt gatttccttc    180

ctcccaactg tttccactga gcgcacccac agtctcccct
agtcctcctc tgtgggtgtg    240

atttttgtga tttttacaaa caaaaccctt gaagttcttg
gcagatgtgt ttgtttctgt    300

ttgcatgtac tgcagatacc ccaggacaag cgggggattc
atttttcagc cattcagttg    360

tttcctcaat aatccgcagc aaagtgaaaa tattcttagc
actcagactg tacttagagt    420

gttttctcag tccagtctgt acagtctgta ggcagaaggc
ctcagaagaa agtcatggcc    480

actcagtgcc cactgtgggc tttgtaagtc ctggctctcc
cgtcaaggtt acccagaggt    540

aaaagcttcc tgggagtggg gccaggtgtg tttggcactc
cagatagaag gcaaaatgct    600

cagattcggg cctgtgcact tgtatgcaac ctgtcggtcg
atacctagca tttatttttc    660

cctgacaatg aacgaccttt ccctcaccca ccctaagctc
aaagagttta gcaaaattct    720

cttttaaata aacagaatgc cagtaagagg ttgaccccta
ccatggaact tctgggatgc    780

taaatacttc ctcatgaaca aaacaagttc cttattataa
gttccttata ctagcagctt    840

cacctaaaga attttctctc cagcaatatt gacttcactg
gggaaaagcc aagagtgtgt    900

ggtgagtgat ttgttctcac tcgacctggc taggactggc
taggagctgt tttttgtaca    960

tgagggaatt tgggctttcc tcagttatct gaatgtttta
cccaagtgcc ttcctgctat   1020

tgtagcaaag tagctcagct tccttgtcca cagggtgaaa

aaggactaat gcattttcca   1080

tcagttttct aactatgtta gcaaaaaggg cctcctggta
gctcaacctc ctgtacgcgt   1140

gtatgtgtgt aatacacaca caaataaacc cctctgtttt
tctaagacat cttagctgga   1200

tattatagga agcactttca taaacaactg taacaaatcg
caaaggaaag agaaacaaaa   1260

gcattagatt tgagacataa acaggcaaga gaaagtgtat
taggaactga cagctatcaa   1320

ggaagttttg tcagttacaa atgctaggag gaaattttgc
caggaaggat ggctcatgaa   1380

atatttccag tacgggaaga ggcaataaga tcctctaaga
gaatgagaaa gtaggggtgt   1440

ctaaatggta aagatgggtg tgttgcacgt gtgttagaag
gatctcagtt gagtgaaggt   1500

ttgcactgct acatctaagt taatgtaaat atgtagcact
ctgacaggtc taccgtgttg   1560

ctgaatgtag tatatttcca aagtttgcaa gtcttcctgt
attgtacaaa gatgctgctg   1620

cttgataata tgtatagcaa tccagattag tatgttatta
aattttattt tcttacctgt   1680

atttttatgc tttttacctg tcctcaaaat attacacccc
tgttggaatt agatttatat   1740

ttataaatgg tcagaaatct ttttaaatgt ctctttttac
acataggttg attttttttt   1800

tcttaagaga aatgatgtat tcttgaaaca tttgttactc
attccaggaa acaaaaaccc   1860

atataataaa acccccactc agagcctgtt agtcacctct
ctagaagatg gcatctcagg   1920

agaaggaatg gctttgtgga agaaggaatc acctttttct
tgctcaagaa ttatgctgac   1980

ttcagccctg agcctggatc tggtcactga gaatcatcaa
gtgtctagat cctcccccca   2040

aaataactaa tttagtaggt gattttgatt ttaaaaaatt
gacaccaaaa ccctgcctgc   2100

attgtaatgg aattcgaaaa gaattcatgt tcacagaact
caacgttcag gctaatattt   2160

acagaaggga ccaaatctaa atcctggtag gtaactcctg
tatgctttat ccaaaggaca   2220

cccacagttt tccagcatag atataaccaa ggatgaattg
attccttcaa agaactggga   2280

ggcacggata ttgcattttt tgtttacatc cagtagccaa
gacgcctcag tgagccagtc   2340

ttgggcagag gctgtcacat ttaggcagat tggaagttgg
tatgttctaa ttctcactct   2400

ggactacagt gaggctgaat ttatcatgtc aaaaaaaaaa
aaaaaaaaaa aaa          2453


<210>  51
<211>  5377
<212>  DNA
<213>  Homo sapiens

<400>  51
ggctccgttc catccttctg cacagggtat cgcctctctc
cgtttggtac atcccctcct     60

cccccacgcc cggactgggg tggtagacgc cgcctccgct
catcgcccct ccccatcggt    120

ttccgcgcga aaagccgggg cgcctgcgct gccgccgccg
cgtctgctga agcctccgag    180

atgccggcgc gtaccgcccc agcccgggtg cccacactgg
ccgtcccggc catctcgctg    240

cccgacgatg tccgcaggcg gctcaaagat ttggaaagag
acagcttaac agaaaaggaa    300

tgtgtgaagg agaaattgaa tctcttgcac gaatttctgc
aaacagaaat aaagaatcag    360

ttatgtgact tggaaaccaa attacgtaaa gaagaattat
ccgaggaggg ctacctggct    420

aaagtcaaat cccttttaaa taaagatttg tccttggaga
acggtgctca tgcttacaac    480

cgggaagtga atggacgtct agaaaacggg aaccaagcaa
gaagtgaagc ccgtagagtg    540

ggaatggcag atgccaacag cccccccaaa cccctttcca
aacctcgcac gcccaggagg    600

agcaagtccg atggagaggc taagcctgaa ccttcaccta
gccccaggat tacaaggaaa    660

agcaccaggc aaaccaccat cacatctcat tttgcaaagg
gccctgccaa acggaaacct    720

caggaagagt ctgaaagagc caaatcggat gagtccatca
aggaagaaga caaagaccag    780

gatgagaaga gacgtagagt tacatccaga gaacgagttg
ctagaccgct tcctgcagaa    840

gaacctgaaa gagcaaaatc aggaacgcgc actgaaaagg
aagaagaaag agatgaaaaa    900

gaagaaaaga gactccgaag tcaaaccaaa gaaccaacac
ccaaacagaa actgaaggag    960

gagccggaca gagaagccag ggcaggcgtg caggctgacg
aggacgaaga tggagacgag   1020

aaagatgaga agaagcacag aagtcaaccc aaagatctag
ctgccaaacg gaggcccgaa   1080

gaaaaagaac ctgaaaaagt aaatccacag atttctgatg
aaaaagacga ggatgaaaag   1140

gaggagaaga gacgcaaaac gacccccaaa gaaccaacgg
agaaaaaaat ggctcgcgcc   1200

aaaacagtca tgaactccaa gacccaccct cccaagtgca
ttcagtgcgg gcagtacctg    1260

gacgaccctg acctcaaata tgggcagcac ccaccagacg
cggtggatga gccacagatg    1320

ctgacaaatg agaagctgtc catctttgat gccaacgagt
ctggctttga gagttatgag    1380

gcgcttcccc agcacaaact gacctgcttc agtgtgtact
gtaagcacgg tcacctgtgt    1440

cccatcgaca ccggcctcat cgagaagaat atcgaactct
tcttttctgg ttcagcaaaa    1500

ccaatctatg atgatgaccc atctcttgaa ggtggtgtta
atggcaaaaa tcttggcccc    1560

ataaatgaat ggtggatcac tggctttgat ggaggtgaaa
aggccctcat cggcttcagc    1620

acctcatttg ccgaatacat tctgatggat cccagtcccg
agtatgcgcc catatttggg    1680

ctgatgcagg agaagatcta catcagcaag attgtggtgg
agttcctgca gagcaattcc    1740

gactcgacct atgaggacct gatcaacaag atcgagacca
cggttcctcc ttctggcctc    1800

aacttgaacc gcttcacaga ggactccctc ctgcgacacg
cgcagtttgt ggtggagcag    1860

gtggagagtt atgacgaggc cggggacagt gatgagcagc
ccatcttcct gacaccctgc    1920

atgcgggacc tgatcaagct ggctggggtc acgctgggac
agaggcgagc ccaggcgagg    1980

cggcagacca tcaggcattc taccagggag aaggacaggg
gacccacgaa agccaccacc    2040

accaagctgg tctaccagat cttcgatact ttcttcgcag
agcaaattga aaaggatgac    2100

agagaagaca aggagaacgc ctttaagcgc cggcgatgtg

gcgtctgtga ggtgtgtcag    2160

cagcctgagt gtgggaaatg taaagcctgc aaggacatgg
ttaaatttgg tggcagtgga    2220

cggagcaagc aggcttgcca agagcggagg tgtcccaata
tggccatgaa ggaggcagat    2280

gacgatgagg aagtcgatga taacatccca gagatgccgt
cacccaaaaa aatgcaccag    2340

gggaagaaga agaaacagaa caagaatcgc atctcttggg
tcggagaagc cgtcaagact    2400

gatgggaaga agagttacta taagaaggtg tgcattgatg
cggaaaccct ggaagtgggg    2460

gactgtgtct ctgttattcc agatgattcc tcaaaaccgc
tgtatctagc aagggtcacg    2520

gcgctgtggg aggacagcag caacgggcag atgtttcacg
cccactggtt ctgcgctggg    2580

acagacacag tcctcggggc cacgtcggac cctctggagc
tgttcttggt ggatgaatgt    2640

gaggacatgc agctttcata tatccacagc aaagtgaaag
tcatctacaa agccccctcc    2700

gaaaactggg ccatggaggg aggcatggat cccgagtccc
tgctggaggg ggacgacggg    2760

aagacctact tctaccagct gtggtatgat caagactacg
cgagattcga gtcccctcca    2820

aaaacccagc caacagagga caacaagttc aaattctgtg
tgagctgtgc ccgtctggct    2880

gagatgaggc aaaaagaaat ccccagggtc ctggagcagc
tcgaggacct ggatagccgg    2940

gtcctctact actcagccac caagaacggc atcctgtacc
gagttggtga tggtgtgtac    3000

ctgccccctg aggccttcac gttcaacatc aagctgtcca
gtcccgtgaa acgcccacgg    3060

aaggagcccg tggatgagga cctgtaccca gagcactacc
ggaaatactc cgactacatc   3120

aaaggcagca acctggatgc ccctgagccc taccgaattg
gccggatcaa agagatcttc   3180

tgtcccaaga agagcaacgg caggcccaat gagactgaca
tcaaaatccg ggtcaacaag   3240

ttctacaggc ctgagaacac ccacaagtcc actccagcga
gctaccacgc agacatcaac   3300

ctgctctact ggagcgacga ggaggccgtg gtggacttca
aggctgtgca gggccgctgc   3360

accgtggagt atggggagga cctgcccgag tgcgtccagg
tgtactccat gggcggcccc   3420

aaccgcttct acttcctcga ggcctataat gcaaagagca
aaagctttga agatcctccc   3480

aaccatgccc gtagccctgg aaacaaaggg aagggcaagg
gaaaagggaa gggcaagccc   3540

aagtcccaag cctgtgagcc gagcgagcca gagatagaga
tcaagctgcc caagctgcgg   3600

accctggatg tgttttctgg ctgcgggggg ttgtcggagg
gattccacca agcaggcatc   3660

tctgacacgc tgtgggccat cgagatgtgg gaccctgcgg
cccaggcgtt ccggctgaac   3720

aaccccggct ccacagtgtt cacagaggac tgcaacatcc
tgctgaagct ggtcatggct   3780

ggggagacca ccaactcccg cggccagcgg ctgccccaga
agggagacgt ggagatgctg   3840

tgcggcgggc cgccctgcca gggcttcagc ggcatgaacc
gcttcaattc gcgcacctac   3900

tccaagttca aaaactctct ggtggtttcc ttcctcagct
actgcgacta ctaccggccc   3960

cggttcttcc tcctggagaa tgtcaggaac tttgtctcct
tcaagcgctc catggtcctg   4020

aagctcaccc tccgctgcct ggtccgcatg ggctatcagt
gcaccttcgg cgtgctgcag   4080

gccggtcagt acggcgtggc ccagactagg aggcgggcca
tcatcctggc cgcggcccct   4140

ggagagaagc tccctctgtt cccggagcca ctgcacgtgt
ttgctccccg ggcctgccag   4200

ctgagcgtgg tggtggatga caagaagttt gtgagcaaca
taaccaggtt gagctcgggt   4260

cctttccgga ccatcacggt gcgagacacg atgtccgacc
tgccggaggt gcggaatgga   4320

gcctcggcac tggagatctc ctacaacggg gagcctcagt
cctggttcca gaggcagctc   4380

cggggcgcac agtaccagcc catcctcagg gaccacatct
gtaaggacat gagtgcattg   4440

gtggctgccc gcatgcggca catccccttg gccccagggt
cagactggcg cgatctgccc   4500

aacatcgagg tgcggctctc agacggcacc atggccagga
agctgcggta tacccaccat   4560

gacaggaaga acggccgcag cagctctggg gccctccgtg
gggtctgctc ctgcgtggaa   4620

gccggcaaag cctgcgaccc cgcagccagg cagttcaaca
ccctcatccc ctggtgcctg   4680

ccccacaccg ggaaccggca caaccactgg gctggcctct
atggaaggct cgagtgggac   4740

ggcttcttca gcacaaccgt caccaacccc gagcccatgg
gcaagcaggg ccgcgtgctc   4800

cacccagagc agcaccgtgt ggtgagcgtg cgggagtgtg
cccgctccca gggcttccct   4860

gacacctacc ggctcttcgg caacatcctg gacaagcacc

ggcaggtggg caatgccgtg    4920

ccaccgcccc tggccaaagc cattggcttg gagatcaagc
tttgtatgtt ggccaaagcc    4980

cgagagagtg cctcagctaa aataaaggag gaggaagctg
ctaaggacta gttctgccct    5040

cccgtcaccc ctgtttctgg caccaggaat ccccaacatg
cactgatgtt gtgtttttaa    5100

catgtcaatc tgtccgttca catgtgtggt acatggtgtt
tgtggccttg gctgacatga    5160

agctgttgtg tgaggttcgc ttatcaacta atgatttagt
gatcaaattg tgcagtactt    5220

tgtgcattct ggattttaaa agttttttat tatgcattat
atcaaatcta ccactgtatg    5280

agtggaaatt aagactttat gtagttttta tatgttgtaa
tatttcttca aataaatctc    5340

tcctataaac caaaaaaaaa aaaaaaaaaa aaaaaaa
                5377

<210>  52
<211>  1431
<212>  DNA
<213>  Homo sapiens

<400>  52
ccgtgaagtg ggcggagcga gcgatttgaa cgcgagcggc
gcggacttct gccaagcacc      60

ggctcatgtg aggctcgcgg cacagcgttc tctgggctcc
ccagaagcca gcctttcgct     120

cccggacccg gcagcccgag caggagccgt gggaccgggc
gccagcaccc tctgcggcgt     180

gtcatgggcc cgcgccgccg gagccgaaag cccgaggccc
cgaggaggcg cagcccgagc     240

ccgaccccga cccccggccc ctcccggcgg ggcccctcct

taggcgcttc ctcccatcaa 300

cacagtcggc ggagacaagg ttggctaaag gagatccgaa
agcttcagaa gagcacacac 360

ctcttgataa ggaagctgcc cttcagccgc ctggcaagag
aaatatgtgt taaattcact 420

cgtggtgtgg acttcaattg gcaagcccag gccctattgg
ccctacaaga ggcagcagaa 480

gcatttctag ttcatctctt tgaggacgcc tatctcctca
ccttacatgc aggccgagtt 540

actctcttcc caaaggatgt gcaactggcc cggaggatcc
ggggccttga ggagggactc 600

ggctgagctc ctgcacccag tgtttctgtc agtctttcct
gctcagccag gggggatgat 660

accggggact ctccagagcc atgactagat ccaatggatt
ctgcgatgct gtctggactt 720

tgctgtctct gaacagtatg tgtgtgttgc tttaaatatt
tttcttttt ttgagaagga 780

gaagactgca tgactttcct ctgtaacaga ggtaatatat
gagacaatca acaccgttcc 840

aaaggcctga aaataatttt cagataaaga gactccaagg
ttgactttag tttgtgagtt 900

actcatgtga ctatttgagg attttgaaaa catcagattt
gctgtggtat gggagaaaag 960

gctatgtact tattatttta gctctttctg taatatttac
atttttacc atatgtacat 1020

ttgtactttt attttacaca taagggaaaa aataagacca
ctttgagcag ttgcctggaa 1080

ggctgggcat ttccatcata tagacctctg cccttcagag
tagcctcacc attagtggca 1140

gcatcatgta actgagtgga ctgtgcttgt caacggatgt
gtagcttttc agaaacttaa 1200

ttggggatga atagaaaacc tgtaagcttt gatgttctgg
ttacttctag taaattcctg    1260

tcaaaatcaa ttcagaaatt ctaacttgga gaatttaaca
ttttactctt gtaaatcata    1320

gaagatgtat cataacagtt cagaatttta aagtacattt
tcgatgcttt tatgggtatt    1380

tttgtagttt ctttgtagag agataataaa aatcaaaata
tttaatgaaa a             1431


<210>  53
<211>  2372
<212>  DNA
<213>  Homo sapiens

<400>  53
cgaagctagg gcggggcccg cgggctgagg cagcggctgt
ggcggcgacg ctgggcgtga     60

ggtggcggcg gccgcgccct ggttgggtcc ccactgctct
cgggggcgcc atggacgagg    120

ccgtgggcga cctgaagcag gcgcttccct gtgtggccga
gtcgccaacg gtccacgtgg    180

aggtgcatca gcgcggcagc agcactgcaa agaaagaaga
cataaacctg agtgttagaa    240

agctactcaa cagacataat attgtgtttg gtgattacac
atggactgag tttgatgaac    300

cttttttgac cagaaatgtg cagtctgtgt ctattattga
cacagaatta aaggttaaag    360

actcacagcc catcgatttg agtgcatgca ctgttgcact
tcacattttc cagctgaatg    420

aagatggccc cagcagtgaa aatctggagg aagagacaga
aaacataatt gcagcaaatc    480

actgggttct acctgcagct gaattccatg ggctttggga
cagcttggta tacgatgtgg    540

aagtcaaatc ccatctcctc gattatgtga tgacaacttt
actgttttca gacaagaacg    600

tcaacagcaa cctcatcacc tggaaccggg tggtgctgct
ccacggtcct cctggcactg    660

gaaaaacatc cctgtgtaaa gcgttagccc agaaattgac
aattagactt tcaagcaggt    720

accgatatgg ccaattaatt gaaataaaca gccacagcct
cttttctaag tggttttcgg    780

aaagtggcaa gctggtaacc aagatgtttc agaagattca
ggatttgatt gatgataaag    840

acgccctggt gttcgtgctg attgatgagg tggagagtct
cacagccgcc cgaaatgcct    900

gcagggcggg caccgagcca tcagatgcca tccgcgtggt
caatgctgtc ttgacccaaa    960

ttgatcagat taaaaggcat tccaatgttg tgattctgac
cacttctaac atcaccgaga   1020

agatcgacgt ggccttcgtg gacagggctg acatcaagca
gtacattggg ccaccctctg   1080

cagcagccat cttcaaaatc tacctctctt gtttggaaga
actgatgaag tgtcagatca   1140

tataccctcg ccagcagctg ctgaccctcc gagagctaga
gatgattggc ttcattgaaa   1200

acaacgtgtc aaaattgagc cttcttttga atgacatttc
aaggaagagc gagggcctca   1260

gcggccgggt cctgagaaaa ctcccctttc tggctcatgc
gctgtatgtc caggccccca   1320

ccgtcaccat agaggggttc ctccaggccc tgtctctggc
agtggacaag cagtttgaag   1380

agagaaagaa gcttgcagct tacatctgat cctgggcttc
cccatctggt gcttttccca   1440

tggagaacac acaaccagta agtgaggttg ccccacacag
ccgtctccca gggaatccct   1500

tctgcaaacc aaacgttact tagactgcaa gctagaaagc
caccaaggcc aggctttgtt   1560

aaaagaagtg tattctattt atgttgtttt aaaatgcata
ctgagagaca aacatcttgt   1620

cattttcact gtttgtaaaa gataattcag attgtttgtc
tccttgtgaa gaaccatcga   1680

aacctgtttg ttcccagccc acccccagtg gatgggatgc
ataatgccag caagttttgt   1740

ttaacagcaa aaaaggaaga ttaatgcagg tgttatagaa
gccagaagag aaactgtgtc   1800

accctaaaga agcatataat catagcatta aaaatgcaca
cattactcca ggtggaaggt   1860

ggcaattgct ttctgatatc agctcgtttg atttagtgca
aaaatgtttt caagactatt   1920

taatggatgt aaaaaagcct atttctacat tataccaact
gagaaaaaaa tggtcggtaa   1980

agtgttcttt cataataaat aatcagacat ggtcccattt
gcaggaaaag tgcagactct   2040

gagtgttcca gggaaacaca tgctggacat cccttgtaac
ccggtatggg cgcccctgca   2100

ttgctgggat gtttctgccc acggttttgt ttgtgcaata
acgttatcac atttctaatg   2160

aggattcaca ttaatataat ataaaataaa taggtcagtt
actggtctct ttctccgaat   2220

gttatgtttt gcttttatct cacagtaaaa taaatataat
taatggtttg catgtgaaat   2280

tcacttttga aagaacatgt taccttacct tttgttttag
aagttttcaa gtattaaaat   2340

attttttaga aaaaaaaaaa aaaaaaaaaa aa

<210>  54
<211>  823
<212>  DNA
<213>  Homo sapiens

<400>  54
aaacgcgggc gggcgggccc gcagtcctgc agttgcagtc
gtgttctccg agttcctgtc     60

tctctgccaa cgccgcccgg atggcttccc aaaaccgcga
cccagccgcc actagcgtcg    120

ccgccgcccg taaaggagct gagccgagcg gggcgccgc
ccggggtccg gtgggcaaaa    180

ggctacagca ggagctgatg accctcatga tgtctggcga
taaagggatt tctgccttcc    240

ctgaatcaga caaccttttc aaatgggtag ggaccatcca
tggagcagct ggaacagtat    300

atgaagacct gaggtataag ctctcgctag agttccccag
tggctaccct tacaatgcgc    360

ccacagtgaa gttcctcacg ccctgctatc accccaacgt
ggacacccag ggtaacatat    420

gcctggacat cctgaaggaa aagtggtctg ccctgtatga
tgtcaggacc attctgctct    480

ccatccagag ccttctagga gaacccaaca ttgatagtcc
cttgaacaca catgctgccg    540

agctctggaa aaaccccaca gcttttaaga agtacctgca
agaaacctac tcaaagcagg    600

tcaccagcca ggagccctga cccaggctgc ccagcctgtc
cttgtgtcgt ctttttaatt    660

tttccttaga tggtctgtcc tttttgtgat ttctgtatag
gactctttat cttgagctgt    720

ggtatttttg ttttgttttt gtcttttaaa ttaagcctcg

gttgagccct tgtatattaa     780

ataaatgcat ttttgtcctt ttttagacaa aaaaaaaaaa aaa
                    823

<210>  55
<211>  4628
<212>  DNA
<213>  Homo sapiens

<400>  55
tcacttgcct gatatttcca gtgtcagagg gacacagcca
acgtggggtc ccttctaggc      60

tgacagccgc tctccagcca ctgccgcgag cccgtctgct
cccgccctgc ccgtgcactc     120

tccgcagccg ccctccgcca agccccagcg cccgctccca
tcgccgatga ccgcggggag     180

gaggatggag atgctctgtg ccggcagggt ccctgcgctg
ctgctctgcc tgggtttcca     240

tcttctacag gcagtcctca gtacaactgt gattccatca
tgtatcccag gagagtccag     300

tgataactgc acagctttag ttcagacaga agacaatcca
cgtgtggctc aagtgtcaat     360

aacaaagtgt agctctgaca tgaatggcta ttgtttgcat
ggacagtgca tctatctggt     420

ggacatgagt caaaactact gcaggtgtga agtgggttat
actggtgtcc gatgtgaaca     480

cttcttttta accgtccacc aacctttaag caaagaatat
gtggctttga ccgtgattct     540

tattattttg tttcttatca cagtcgtcgg ttccacatat
tatttctgca gatggtacag     600

aaatcgaaaa agtaaagaac caaagaagga atatgagaga
gttacctcag gggatccaga     660

gttgccgcaa gtctgaatgg cgccatcaaa cttatgggca

gggataacag tgtgcctggt 720

taatattaat attcccattt tattaataat atttatgttg
ggtcaagtgt taggtcaata 780

acactgtatt ttaatgtact tgaaaaatgt ttttattttt
gttttatttt tgacagacta 840

tttgctaatg tataatgtgc agaaaatatt taatatcaaa
agaaaattga tatttttata 900

caagtaattt cctgagctaa atgcttcatt gaaagcttca
aagtttatat gcctggtgca 960

cagtgcttag aagtaagcaa ttcccaggtc atagctcaag
aattgttagc aaatgacaga 1020

tttctgtaag cctatatata tagtcaaatc gatttagtaa
gtatgttttt tatgttcctc 1080

aaatcagtga taattggttt gactgtacca tggtttgata
tgtagttggc accatggtat 1140

catatattaa aacaataatg caattagaat ttgggagaag
caaatatagg tcctgtgtta 1200

aacactacac atttgaaaca agctaaccct ggggagtcta
tggtctcttc actcaggtct 1260

cagctataat tctgttatat gaggggcagt ggacagttcc
ctatgccaac tcacgactcc 1320

tacaggtact agtcactcat ctaccagatt ctgcctatgt
aaaatgaatt gaaaaacaat 1380

tttctgtaat cttttattta agtagtgggc atttcatagc
ttcacaatgt tccttttttg 1440

tatattacaa catttatgtg aggtaattat tgctcaacag
acaattagaa aaaagtccac 1500

acttgaagcc taaatttgtg ctttttaaga atatttttag
actatttctt tttatagggg 1560

ctttgctgaa ttctaacatt aaatcacagc ccaaaatttg
atggactaat tattatttta 1620

aaatatatga agacaataat tctacatgtt gtcttaagat
ggaaatacag ttatttcatc    1680

ttttattcaa ggaagtttta actttaatac agctcagtaa
atggcttctt ctagaatgta    1740

aagttatgta tttaaagttg tatcttgaca caggaaatgg
gaaaaaactt aaaaattaat    1800

atggtgtatt tttccaaatg aaaaatctca attgaaagct
tttaaaatgt agaaacttaa    1860

acacaccttc ctgtggaggc tgagatgaaa actagggctc
attttcctga catttgttta    1920

ttttttggaa gagacaaaga tttcttctgc actctgagcc
cataggtctc agagagttaa    1980

taggagtatt tttgggctat tgcataagga gccactgctg
ccaccacttt tggattttat    2040

gggaggctcc ttcatcgaat gctaaacctt tgagtagagt
ctccctggat cacataccag    2100

gtcagggagg atctgttctt cctctacgtt tatcctggca
tgtgctaggg taaacgaagg    2160

cataataagc catggctgac ctctggagca ccaggtgcca
ggacttgtct ccatgtgtat    2220

ccatgcatta tataccctgg tgcaatcaca cgactgtcat
ctaaagtcct ggccctggcc    2280

cttactatta ggaaaataaa cagacaaaaa caagtaaata
tatatggtca tatacatatt    2340

gtatatatat tcatatacaa acatgtatgt atacatgacc
ttaatggatc atagaattgc    2400

agtcatttgg tgctctgcta accatttata taaaacttaa
aaacaagaga aaagaaaaat    2460

caattagatc taaacagtta tttctgtttc ctatttaata
cagctgaagt caaaatatgt    2520

aagaacacat tttaaatact ctacttacag ttggccctct
gtggttagtt ccacatctgt   2580

ggattcaacc aaccaaggac ggaaaatgct taaaaaataa
tacaacaaca acaaaaaata   2640

cattataaca actatttact tttttttttt tctttttgag
atggagtctc gctctgttgc   2700

ccaggttgga gtgcagtggc acgatctcgg ctcactgcaa
cctcacctcc cgggttcaag   2760

agatcctcct gcctcagcct cctgagcagc tgggactaca
ggcgcatgcc accatgccca   2820

gctaattttt gtatttttag tagaggcggg gtttcaccat
gttggccagg atggtctcaa   2880

tctcctaacc ttgagatcca ccctccacag cctcccaaac
tgctgggatt acaggtgtga   2940

gccaccgcac gtagcattta cattaggtat tacaagtaat
gtaaagatga tttaagtata   3000

caggaggatg tgaataggtt atatgcaagc actatgccct
tttatataag tgacttgaac   3060

atctgtgccc gattttagta tgtgcagggg ggcgatctgg
gaatcagtcc cctgtggata   3120

ccaaggtaca actgtattta ttaacgctta ctagatgtga
ggagagtctg aatattttca   3180

gtgatcttgg ctgtttcaaa aaaatctatt gacttttcaa
taaatcagct gcaatccatt   3240

tatttcattt acaaaagatt tattgtaagc atctcaatct
tggtttgtca gtttatctta   3300

agcatgtcaa ttcataaaaa caagtcattt ttgtattttt
catctttaag aatgcttaaa   3360

aaagctaatc cctaaaatag ttagatcttt gtaaatgcat
attaaataat aaagtatgac   3420

ccacattact ttttatgggt gaaaataaga caaaaataat

agttttagtg aggatggtgc 3480

tgagtaaaca taaaaactga tttgctctca gctgatgtgt
cctgtacaca gtgggaagat 3540

tttagttcac acttagtcta actcccccat tttacagatt
tctcactata tatatttcta 3600

gaaggggcta tgcatattca atgtattgag aaccaaagca
accacaaatg cataaatgca 3660

taatttatgg tcttcaacca aggccacata ataacccagt
taacttactc tttaaccagg 3720

aatattaagt tctataacta gtactcaagg tttaacctta
aaattaagat ttccttaacc 3780

ttaaccttaa aattgatatt atattaaaca tacataatac
aatgtaactc cactgttctc 3840

ctgaatattt tttgctctaa tctctctgcc gaaagtcaaa
gtgatgggag aattggtata 3900

ctggtatgac tacgtcttaa gtcagatttt tatttatgag
tctttgagac taaattcaat 3960

caccaccagg tatcaaatca acttttatgc agcaaatata
tgattctagt gtctgacttt 4020

tgttaaattc agtaatgcag tttttaaaaa cctgtatctg
acccactttg taatttttgc 4080

tccaatatcc attctgtaga cttttgaaaa aaaagttttt
aatttgatgc ccaatatatt 4140

ctgaccgtta aaaaattctt gttcatatgg gagaaggggg
agtaatgact tgtacaaaca 4200

gtatttctgg tgtatatttt aatgttttta aaaagagtaa
tttcatttaa atatctgtta 4260

ttcaaatttg atgatgttaa atgtaatata atgtattttc
tttttatttt gcactctgta 4320

attgcacttt ttaagtttga agagccattt tggtaaacgg
tttttattaa agatgctatg 4380

gaacataaag ttgtattgca tgcaatttga agtaacttat
ttgactatga atgttatcgg    4440

attactgaat tgtatcaatt tgtttgtgtt caatatcagc
tttgataatt gtgtacctta    4500

agatattgaa ggagaaaata gataatttac aagatattat
taatttttat ttatttttct    4560

tgggaattga aaaaaattga aataaataaa aatgcattga
acatcttgca ttcaaaatct    4620

tcactgac
                    4628

<210>  56
<211>  5273
<212>  DNA
<213>  Homo sapiens

<400>  56
ggagtcgggt ttcagagcgc gggtgactcg ggcgcgggc
cgggagccgg gattctgccc      60

gccgccgccg ctgccgagcg ccgcctttgt tccctgcagg
aagggcgagc gcggcggcca     120

gcgctcagcg acccttcgtc ctccgctaag ctccaacgct
ctgctcgact agccgcgcgc     180

cttccggggc tccgcagacc cgcgagatgg caccaaggag
gaacaacggg cagtgctggt     240

gtctgctgat gctgctctcg gtctccacgc ccctccctgc
tgtcacccag acccgcggtg     300

cgacagagac tgcttcccag ggtcacctgg acctcacgca
gctcatcggt gtcccgctgc     360

cctcgtccgt atcctttgtc acaggctatg gtggcttccc
ggcctacagt ttcgggcctg     420

gtgccaatgt tggccgccca gccaggactc tcatcccatc
caccttcttc agggacttcg     480

ccatcagcgt cgtggtgaag cccagcagca cccgtggtgg
cgtgctcttc gccatcactg    540

acgccttcca gaaggtcatc tacctgggcc tgcggctctc
aggtgtggag gacggccacc    600

agcggatcat cctctactac acggagccag gctcccatgt
gtcccaagag gctgctgcct    660

tctcggtgcc tgtgatgacc cacaggtgga accgcttcgc
catgattgtc cagggtgagg    720

aagtgaccct cctcgtgaac tgtgaggagc acagccgcat
cccccttccag cggtcctccc    780

aggctttggc ttttgagtcc agcgctggaa tcttcatggg
caatgcagga gctacagggc    840

tcgagagatt cactggctcc ctccagcagc tcaccgtgca
ccccgacccc aggactcccg    900

aggagctgtg tgaccctgaa gagtcctcgg catctggaga
gaccagtggg ctgcaggagg    960

cagacggagt agctgagatc ttagaagccg tcacctacac
tcaagcctcg cccaaagaag   1020

caaaagttga acccataaac acacctccaa ctccatcctc
cccctttgaa gacatggaac   1080

tttctggtga acctgtaccc gaggggaccc tggaaaccac
caacatgagc atcatccagc   1140

acagcagccc caaacaaggg tctggtgaga tcctgaatga
cacactggag gggggttcatt   1200

ctgtggatgg tgaccccatt actgacagcg gctcaggggc
tggggccttc cttgacattg   1260

ctgaagaaaa gaatttagca gcaacagcag cggggctggc
cgaggtgccc atcagcactg   1320

ctggagaagc agaggccagc agtgtgccca ccgggggacc
aaccctctct atgtccacgg   1380

agaacccaga ggaaggggtc actccaggtc cagataatga
agagcgttta gcagcaacag   1440

cagcagggga ggccgaggca ctcgccagca tgcctgggga
agtggaggcc agtggtgtgg   1500

cccccgggga gctggacctc tccatgtccg cccagagcct
cggggaagag gccactgtgg   1560

gtccaagcag tgaagacagt ttaacaacag ctgcagctgc
aaccgaagtg tccctcagta   1620

cttttgagga tgaggaagcc agtggggtcc ccacagatgg
cctggctccc ctcacagcca   1680

ccatggcccc tgagcgggca gtcacttctg gtcctggtga
tgaagaagac ttggcagcag   1740

ccacaacaga ggagcccctc atcacagctg gggtgaaga
gtccggcagc cctcccctg   1800

atgggccacc gctgcccctg cccacagtgg ctcctgaaag
atggatcact ccagctcaaa   1860

gagaacatgt gggaatgaaa ggacaggctg ggcccaaagg
agaaaagggt gatgctgggg   1920

aggagcttcc tggccctcct gaaccttctg ggcctgttgg
acccacggca ggagcagaag   1980

cagagggctc tggcctaggc tggggctcgg acgtcggctc
tggctctggt gacctggtgg   2040

gcagtgagca gctgctgaga ggtcctccag gacccccagg
gccacctggc ttacctggga   2100

ttccaggaaa accaggaact gatgttttca tgggaccccc
tggatctcct ggagaggatg   2160

gacctgctgg tgaacctggg cccccggccc ctgagggaca
gcctggagtt gatggagcca   2220

ccggccttcc cgggatgaaa ggggagaagg gagcaagagg
gcctaatggc tcagttggtg   2280

aaaagggtga ccctggcaac agaggcttac ctggaccccc

ggggaaaaag ggacaagctg 2340

gccctcctgg ggtcatggga cccccagggc ctcctggacc
ccctgggccc ccaggccctg 2400

gatgcacaat gggacttgga ttcgaggata ccgaaggctc
tggaagcacc cagctattga 2460

atgaacccaa actctccaga ccaacggctg caattggtct
caaaggagag aaaggagacc 2520

ggggacccaa gggagaaagg gggatggatg gagccagtat
tgtgggaccc cctgggccga 2580

gagggccacc tgggcacatc aaggtcttgt ctaattcctt
gatcaatatc acccatggat 2640

tcatgaattt ctcggacatt cctgagctgg tggggcctcc
ggggccggac gggttgcctg 2700

ggctgccagg atttccaggt cctagaggac caaaaggtga
cactggttta cctggctttc 2760

caggactaaa aggagaacag ggcgagaagg gagagccggg
tgccatcctg acagaggaca 2820

ttcctctgga aaggctgatg ggaaaaaagg gtgaacctgg
aatgcatgga gccccaggac 2880

caatggggcc caaaggacca ccaggacata aaggagaatt
tggccttccc gggcgacctg 2940

gtcgcccagg actgaatggc ctcaagggta ccaaaggaga
tccaggggtc attatgcagg 3000

gcccacctgg cttacctggc cctccaggcc cccctgggcc
acctggagct gtgattaaca 3060

tcaaaggagc catttcccca atacccgtcc gaccacactg
caaaatgcca gttgatactg 3120

ctcatcctgg gagtccagag ctcatcactt ttcacggtgt
taaaggagag aaaggatcct 3180

ggggtcttcc tggctcaaag ggagaaaaag gcgaccaggg
agcccaggga ccaccaggtc 3240

ctccacttga tctagcttac ctgagacact ttctgaacaa
cttgaagggg gagaatggag   3300

acaaggggtt caaaggtgaa aaaggagaaa aaggagacat
taatggcagc ttccttatgt   3360

ctgggcctcc aggcctgccc ggaaatccag gcccggctgg
ccaaaaaggg gagacagtcg   3420

ttgggcccca aggaccccca ggtgctcctg gtctgcctgg
gccacctggc tttggaagac   3480

ctggtgatcc tgggccaccg gggcccccgg ggccaccagg
acctccagct atcctgggag   3540

cagctgtggc ccttccaggt ccccctggcc ctccaggaca
gccagggctt cccggatcca   3600

gaaacctggt cacagcattc agcaacatgg atgacatgct
gcagaaagcg catttggtta   3660

tagaaggaac attcatctac ctgagggaca gcactgagtt
tttcattcgt gttagagatg   3720

gctggaaaaa attacagctg ggagaactga tccccattcc
tgccgacagc cctccacccc   3780

ctgcgctttc cagcaaccca catcagcttc tgcctccacc
aaaccctatt tcaagtgcca   3840

attatgagaa gcctgctctg catttggctg ctctgaacat
gccattttct ggggacattc   3900

gagctgattt tcagtgcttc aagcaggcca gagctgcagg
actgttgtcc acctaccgag   3960

cattcttatc ttcccatttg caagatctgt ccaccattgt
gaggaaagca gagagataca   4020

gccttcccat agtgaacctc aagggccaag tactttttaa
taattgggac tcaatttttt   4080

ctggccacgg aggtcagttc aatatgcata ttccaatata
ctcctttgat ggtcgagaca   4140

taatgacaga tccttcttgg ccccagaaag tcatttggca
tggctccagc ccccatggcg    4200

tccgccttgt ggataactac tgtgaagcat ggcgaaccgc
ggacacagcg gtcacgggac    4260

ttgcctcccc gctgagcacg gggaagattc tggaccagaa
agcatacagc tgtgctaatc    4320

ggctaattgt cctatgtatc gaaaacagtt tcatgacaga
cgctaggaag taatggcctt    4380

ctgatgattc ttaaagagtt ttcaattttt tcttatgtga
agagttgaca ctgaaatcta    4440

aaatgtttaa ttgttgtaaa tattacagtt tttttttttt
actacatatt ctttacaaca    4500

gcaaccaaag aaaacatacc tcaatacact caaaactgaa
gacatagagg actcagatca    4560

aagacaaaat ctgatccata tattggtgct agattctgca
ggaaacccca gcagtgtgaa    4620

cgcatcccaa cataggttaa gagcaagttg aaaacaaagg
ccatggcatt ctgccactgc    4680

atccttcaga cagttatatc ctccttttaa accattgttg
ttgagtgtaa gatgtccttc    4740

atgttttctt ataaagtcag tgtttagaaa tgttaccctt
tctaagttat atacagatca    4800

aatgcttttt tctttcacgt acatccatca tttgcaactg
ctgttcgtac acagaaacag    4860

gactgctcaa atgatcctat ttgtattttc tgatgctatc
agactctaat gtttttttcc    4920

ctaaaatatt attgccatca tgctttagga attttatatt
tttacacaat catattttag    4980

tatggtgtct gtttatgtaa ctctgacttg ctggaaaagt
tgaaactcca aataatctga    5040

aactagaaaa gaaatagcac ataattacta ccttcccctt

ggcggctctc ctccccaacc 5100

cccaccccac aattttatga cttccatttg gcaattgttg
aattataact gcgactgaaa 5160

caaacaggtt catagagatg aattttctga gaaacatata
tctacatgtt gtataattgg 5220

attttttttc catgtaagtg aacataaaaa catcttttcc
gggtgctttc ttc 5273

<210> 57
<211> 8521
<212> DNA
<213> Homo sapiens

<400> 57
gtgtgcgcgt catggccgtc agcaccgcgt tcccgtcctc
ttccgcttgg ccccagaaag 60

tttcggttct gcccggcggt ggacccacga gcgcgtgcca
ccatggagtc tgaccactgc 120

tgagcagaca gccaccgagg gccgaaattc tgagccttcc
tctggaccca ggcaggagac 180

atacagacaa gaaaggcaaa ctcaccatgg cctccaccaa
tgcagagagc cagctccaga 240

gaatcatccg agacttgcaa gatgctgtga cagaactaag
caaagaattt caggaagcag 300

gggaacccat cacggatgac agcaccagct tgcataaatt
ttcttataaa cttgagtatc 360

tcctgcaatt tgatcagaaa gagaaggcca ccctcctggg
caacaagaag gactactggg 420

attacttctg tgcctgcctg gccaaggtga aaggagccaa
tgatgggatc cgctttgtca 480

agtctatctc agagctccga acatccttgg ggaaaggaag
agcatttatt cgctactcct 540

tggtgcacca gaggttggca gacaccttac agcagtgctt

catgaacacc aaagtgacca    600

gtgactggta ctatgcaaga agcccctttc tgcagccaaa
gctgagctcg gacattgtgg    660

gccaactcta tgagctgact gaggttcagt ttgacctggc
gtcgaggggc tttgacttgg    720

atgctgcctg gccaacattt gccaggagga cgctgaccac
tggctcttct gcttacctgt    780

ggaaaccccc tagccgcagc tccagcatga gcagcttggt
gagcagctac ctgcagactc    840

aagagatggt gtccaacttt gacctgaaca gcccctaaa
caacgaggca ttggagggct    900

ttgatgagat gcgactagag ctggaccagt tggaggtgcg
ggagaagcag ctacgggagc    960

gcatgcagca gctggacaga gagaaccagg agctgagggc
agctgtcagc cagcaagggg   1020

agcaactgca gacagagagg gagagggggc gcactgcagc
ggaggacaac gttcgcctca   1080

cttgcttggt agctgagctc cagaagcagt gggaggtcac
ccaggccacc cagaacactg   1140

tgaaggagct gcagacatgc ctgcagggcc tggagctagg
agcagcagag aaggaggagg   1200

actaccacac agccctgcgg cggctggagt ccatgctgca
gcccttggca caggagcttg   1260

aggccacacg ggactcactg gacaagaaaa accagcattt
agccagcttc ccaggctggc   1320

tagccatggc tcagcagaag gcagatacgg catcagacac
aaagggccgg caagaaccta   1380

ttcccagtga tgcggcccag gagatgcagg agctagggga
gaagcttcaa gccctagaaa   1440

gggagagaac caaggtcgag gaggtcaaca gacagcagag
tgcccaactg gaacagctgg   1500

tcaaggagct tcagctgaaa gaggatgccc gggccagcct
ggagcgcctg gtgaaggaga   1560

tggccccact ccaggaggag ttgtctggga agggacagga
ggcagaccag ctctggcgac   1620

ggctgcagga gttgctggcc cacacgagct cctgggagga
ggagctagca gagttgaggc   1680

gggagaaaaa acagcaacag gaggagaagg agctgctgga
gcaggaggtc aggtctctga   1740

cccggcagct gcagttcctg gagacccagc tggcacaggt
gagccaacat gtgagtgacc   1800

tggaggagca gaagaagcag ctcattcagg acaaagacca
cctcagccag caggtgggta   1860

tgctcgagcg gcttgctggg ccgcctggcc cagaactgcc
agtggcaggt gagaagaatg   1920

aggccctggt ccctgtgaac tccagtctgc aagaggcctg
ggggaagcca gaggaggagc   1980

agaggggcct gcaggaggca cagttagacg ataccaaggt
gcaagagggc agccaggagg   2040

aagagctccg gcaggccaac agggagctgg agaaggagct
acagaatgtg gtcgggcgta   2100

accagctcct ggagggcaag ctgcaagccc tgcaggccga
ttaccaggct ttgcagcagc   2160

gggaatcagc catccagggc tccttggcct ccctggaggc
cgagcaggcc agcatccggc   2220

acttgggtga ccagatggag gcgagcttgc tggctgtaag
gaaggccaag gaggccatga   2280

aagcccagat ggcagagaag gaggccattc tacagagcaa
ggagggcgag tgtcagcagc   2340

tgcgggagga ggtggagcag tgccagcaac tggcagaagc
ccggcacaga gagcttaggg   2400

ctctcgagag ccagtgccag cagcagaccc agctgattga
ggtcctcaca gcagagaaag 2460

gccaacaggg agttggccca cccactgaca atgaagcccg
tgagctggct gcccagctag 2520

ccctgtctca ggcgcagctg gaagtccatc agggggaggt
ccaacggctg caggctcagg 2580

tggtggacct ccaggccaag atgcgggcag ccctggatga
ccaggacaag gtgcagagcc 2640

agctaagcat ggctgaggcc gtcctgaggg agcacaaaac
ccttgtgcag cagctgaagg 2700

agcagaatga agcccttaac agagcccatg tccaggagct
gctgcaatgc tcggagcgtg 2760

aaggggcact gcaggaggag agggccgatg aggcccagca
gagggaggag gagctgcggg 2820

ccctgcagga ggagctgtcc caggccaaat gcagctccga
ggaagcacag ctggagcacg 2880

ctgagctgca agagcagctg caccgggcca acacagacac
agctgagctg ggcatccagg 2940

tttgcgcact gaccgtggaa aaggagcgag tggaggaggc
actggcctgt gctgtccagg 3000

agctccagga cgccaaagag gcagcctcaa gggagcgaga
gggcctggag cgccaagtag 3060

ctgggctgca gcaagagaag gagagcttgc aggagaagct
gaaggcggcc aaggcagcag 3120

ccggctcact gcctggcctg caggcccagc tcgcccaggc
agagcagcgg gcccagagcc 3180

tccaagaggc tgcacaccag gagctcaaca ccctcaagtt
ccagctgagt gctgaaatca 3240

tggactacca gagcagactt aagaatgctg gtgaagagtg
caagagcctc aggggccagc 3300

ttgaggagca aggccggcag ctgcaggctg ctgaggaagc

tgtggagaag ctgaaggcca    3360

cccaagcaga catgggagag aagctgagct gcactagcaa
ccatcttgca gagtgccagg    3420

cggccatgct gaggaaggac aaggaggggg ctgccctgcg
tgaagaccta gaaaggaccc    3480

agaaggaact cgaaaaagcc acaacaaaaa tccaagagta
ttacaacaaa ctctgccagg    3540

aggtgacaaa tcgtgagagg aatgaccaga agatgcttgc
tgacctggat gacctcaaca    3600

gaaccaagaa gtatctcgag gagcggctga tagagctgct
cagggacaag gatgctctct    3660

ggcagaagtc agatgccctg gaattccagc agaagctcag
tgctgaggag agatggctcg    3720

gagacacaga ggcaaaccac tgcctcgact gtaagcggga
gttcagctgg atggtgcggc    3780

ggcaccactg caggatatgt ggccgcatct tctgttacta
ctgctgcaac aactacgtcc    3840

tgagcaagca cggtggcaaa aaggagcgct gctgccgagc
ctgtttccag aagctcagtg    3900

aaggccctgg ctcccctgat agcagtggct caggcactag
ccagggagag cccagccctg    3960

cactgtcacc agcctcacct gggccccagg ccacaggagg
ccaaggagca aatacagact    4020

acaggccacc ggacgacgct gtgtttgata tcatcacaga
tgaggaattg tgccagatac    4080

aggagtccgg ctcctctttg cctgaaacac ccactgaaac
tgattctctc gacccaaatg    4140

cggctgaaca ggatactaca tcaacctcgc taacgcctga
ggacactgaa gacatgcccg    4200

tggggcagga ttcggaaatc tgcctgctga agtctggaga
actgatgatc aaagtacccc    4260

tcacagtgga tgagatcgcc agcttcgggg agggtagcag
ggagctgttt gtgaggtcca   4320

gcacctacag cctgatcccc atcactgtgg ccgaggcagg
cctcaccatc agctgggtct   4380

tctcctctga ccccaagagc atctccttca gtgtggtctt
ccaggaggcc gaggacacac   4440

cgctggatca gtgtaaggtc ctcattccca cgacccgatg
caactcccac aaggagaaca   4500

tccagggcca gctcaaggtt cgcacacccg gcatctacat
gctcatcttc gacaatacct   4560

tctcaaggtt tgtctctaaa aaggtatttt atcacttgac
ggttgatcgg cctgtgatct   4620

acgatggaag tgatttcctg tagcttcagc acctcagtaa
cttcacttca tccacaggaa   4680

acactgctct tcctcacctg tcacataaag cattttttta
aaaagtcagc tgctccaaaa   4740

tcatcaactc agcccctggg ctgcccctca gaggcggtgt
ctggggagga ctttgtgctc   4800

agcactctgc accggccact cttagccccc gaggcgttga
agggctcagg caatgtttcc   4860

attaagtaga gactcagctg ttgtcacacc caaagggatg
ctctgccaaa ggtttaaaca   4920

cccaggagac catcagcctc tcctgggagc acagttggct
acaggcctct tgtggagagt   4980

ttcacgggca ggggtgattc caacttctgc ctgtggagag
attttctgcc ctgccccacc   5040

agggccctgc atgttggaga ctgagctggg tgcactggcc
atgccctgtg aatcctcagg   5100

ctgtgacgcc ctcaggtact cctgggaaaa ggaggtacac
agccatcatg cgagtcggtg   5160

ccaggggacc ccccggagat cctgaccagc tcctccagtc
atgctcttgt ccctcactgc    5220

cccagtaagc tggaggctgc tccagaactc agcagtgttg
gaggggcctc taagctgcac    5280

tctctttctg gcccttttgt ctgggtgatt ctgtcctcaa
ataaagccct tcactcagcc    5340

agacctctcc acagctcaaa gcattgccct aagaatcaga
agtaaagata atccaagagc    5400

aaaacccact gtacttgggg cctgcaatgg ctgtgtgtac
actacatcta atgcccaaat    5460

gccagccagt gtggatgttg tgaccacaga gcaggattgt
gcattggctt tagagctact    5520

cctcagctga tggcccactt ttgtttatat aaataagagc
ttctgcccca cctgcagaca    5580

tgtttactaa tgatcatagc caggattaga accactttca
aacattgggg ccttcttaac    5640

aaaagtcttt gataacttaa gaaccaaagt aacagagtaa
acagaggcat gatggatccc    5700

tgggccccac tcccctcctg acaggttccc caacagccca
tttgcccact tcccactgct    5760

cagcccacac cagacctcca ggagacatcc ccccttgagg
cagagagatc ctgttcccta    5820

ttcccagaca agaattattt aatcttccct gttctctgtg
gtccttttct tccccaacaa    5880

cagatagctc accttggaca gctcttcgtc ccttgttcat
ggaaccagct gcctgcagtc    5940

aggccccagg ttcttccatg ggtgaacaga gcatctgaca
aaaggtccca gtttggccag    6000

gggtgaggga gagagcacca gacaggctat ccgagaatct
gagagctggg cccggcagtt    6060

cctccagcta cccttgtgac ctaagtccag tcacacattt

cccaaagttt ctctttgtca    6120

taaccctggt ctggctggtt ttgagggctt gagaatgggt
cagggactcc aggccaagtc    6180

caacagagac cccaaaccca ccacacacca gcagccacaa
cctcaccacc aacaaagagg    6240

acttttgtgg ggccacaagt aagaggtcat ttctggaatg
gactcagacc tttaaacagg    6300

agagttgagc acttccagtc agtttttaag caaggcatgg
ggaacaggga atagaacctt    6360

tcaaagaggt tgcccagaga aaagctgggc ctcttgcatt
cggcttcctt ggagcagcct    6420

cttctggcag aaagccatca ggtgctcaat catcttctcc
tggccaaggc tctgaccatg    6480

cttagtactg gaatagaggt ggccaggccc ccagcgactc
ttcttggcct gatgtttgtc    6540

ctcacaggca tgccacgtgg cctgagatga ttcagaacaa
atcatgctaa ctttgaatcc    6600

atccagccac ttgcaaatga taatcagaag tcagcttgtt
cactgttaga aagaaactaa    6660

caaaagagaa cccagagcaa tctagaatct ttgagtgctt
ggctttccaa ggatactgcg    6720

gagactctgg ccaagctgat gaccttctga agtgtcactg
gcaccatatg caacaagaac    6780

caccattcac tgagtagcta atgggtttgg ggcctgggac
attccatctg aggtccttcc    6840

tgaacatgtc actccacagc agaggaccgg ttgcagctta
cccagaacca ctcctccagg    6900

agagctggat gttttgcgtg caacaccttg agcactgact
gctattgttc aaaaaaagcc    6960

tttgctgcat tcggaggact gccccgtgcc ctgaggtgac
ttcctaacta tgtggtttca    7020

ttagcgaatt tattttttgt gctgggtgga catttgtatt
ttgttaggtt gctgtttaag   7080

ctcaagtttg ctgtgctctc tgcagctaca aaacatcttg
gcatatttaa gagtggcttt   7140

tataaatagc tttattctga tattaatcag attcccaact
ttactgagaa ttaaggactg   7200

gggtacttta aagaaatgca aatagcaatt gaagaaccac
tgctgcaggt ggtagccctg   7260

gctagactga attacactag aaatcagcca gaaggaagcg
tccttgggat cccagatcac   7320

tctttttttt tttttttaaa aggggcagcc ccttgatggc
tcatctctct gaataacagt   7380

tacgtcttca tatcgatacc agatgccttc ttcatcatgc
cactgaagcc actcaccacc   7440

ttcaagaaca tgccaacctc tgtcagattc acttacccac
aaacaaggag gcacgtttgg   7500

cacaaagtgt tgtcctccag gtccaagtgg actctacaga
gtgcttgacc tcaacacact   7560

ggattccagg tggactggac caagagcagg caaagacacg
ggaactgaaa aactccacag   7620

ggtttggaga atagaaatga aaagccacgt catataactc
aagaataaat ggtgttttgg   7680

aaattttaaa attatcatcg aaggtggtga aactatttca
ggcccaaatg aaaggaaatc   7740

gccagttggg gatgaaatca cagagcctgt gttttatgat
atggttggat gtccactgat   7800

gaaattttaa aggagtttca gttttaaaag tgcgcatgat
tctacatatg agaattcttt   7860

aggccaagaa actgtccttg gctcagaggt gttgggaatt
aaagcagaga gaagccattc   7920

gtgatgctta gaaccaagga tggtcatgta cacaaagacc
atcgagacgg ccattcttgt   7980

ttacaaaaca cttaccaaga aagcactttg taggggaact
ttagtaagtt cttctcattt   8040

cattatgttt cttccaagga aacaggagag actgaattaa
taattctctc tttcctctta   8100

agcactttta aaataataaa gtacatcttg aaatttgggg
aggcatctct gatttaaaaa   8160

aagaaaaagg ctgcttgatg tatgttatgc agagacactc
tgcctctggt ggctgcagag   8220

caatacccaa gcctcatttg gaaggctcaa catttggaat
tgcactttaa ttgattaatc   8280

ctcaattcat gtggccttac gggatggtgg gtctgggacc
ccaattcatt cttatctgcc   8340

aaagaattat ctagaagcac atcaaatacc agcaccccac
ctgctcaatg ggggtggaaa   8400

acttttgtat ccctaagcat attattttat agtgtctgcc
atgccatgtg gaaatacttt   8460

atttttaacc tcaggattta aataaagtaa acactatgac
atttaaaaaa aaaaaaaaaa   8520

a
                  8521

<210>  58
<211>  2396
<212>  DNA
<213>  Homo sapiens

<400>  58
agtgacgtca cgagatttgg agctcgcggg aaaacttgtc
tctgcgttgt ggggaggacg     60

cgcgctcgcg cgggattttc aagcgtaggc ccccgggaac
tcgagctgcc atgagcctct    120

gggtggacaa gtatcggccc tgctccttgg gacggctgga
ctatcacaag gagcaggcgg    180

cccagctgcg gaacctggtg cagtgtggtg actttcctca
tctgttagtg tacggaccat    240

caggtgctgg aaaaaagaca agaattatgt gtattctacg
tgaactttat ggtgttggag    300

tggaaaaatt gagaattgaa catcagacca tcacaactcc
atctaaaaaa aaaattgaaa    360

ttagcaccat tgcaagtaac taccaccttg aagttaatcc
tagtgatgct ggaaatagtg    420

accgagtagt cattcaggag atgttgaaaa cagtggcaca
atcacaacaa cttgaaacaa    480

actctcaaag ggattttaaa gtggtattat tgacagaagt
tgacaaactc accaaagatg    540

ctcagcatgc cttgcgaaga accatggaaa aatatatgtc
tacctgcaga ttgatcttgt    600

gctgcaattc tacatctaaa gtgatcccac ctattcgtag
taggtgcttg gcggttcgtg    660

tgcctgctcc cagcattgaa gatatttgcc acgtgttatc
tactgtgtgt aagaaggaag    720

gtctgaatct tccttcacaa ctggctcata gacttgcaga
gaagtcttgt agaaatctca    780

gaaaagccct gcttatgtgt gaagcctgca gagtgcaaca
atatcctttt actgcagatc    840

aagaaatccc tgagacagat tgggaggtgt atctgaggga
gactgcaaat gctattgtca    900

gtcagcaaac tccacaaagg ctccttgaag ttcgtggaag
gctgtatgag cttctaactc    960

attgtattcc tcctgagata ataatgaagg gccttctctc
agaactgtta cataattgtg   1020

atggacaact gaaaggggag gtggcacaaa tggcagctta

ctatgagcat cgtctacagc    1080

tgggtagcaa agccatttat cacttggaag cgtttgtggc
caaattcatg gcactttata    1140

agaagttcat ggaggatgga ttggaaggca tgatgttctg
acttctgtca gttattcttg    1200

caaagatttc tcagtatcag tatttacata cagcttatat
taaaagagct gtgggtaaat    1260

taactgaact taatcatgtc gtatttgcgt tttttggta
ataacttctc tgtgaactat    1320

taatcatcct ctgagttaaa taattgctcc tatactattg
aagtatgtag ttttgtacat    1380

aacttagaga ctttagagtc taagaaaatg atcttaattt
actttaagca ttggttattc    1440

aagtattcat tgttgatcct cctattctct tccgtctaat
ctctcacctg ctaaaggaga    1500

tttacacatt agaaagcaaa gattattttc atttatccag
atgaccattt tctgccacag    1560

gtaacatgat tgtttgacac accattatat ttaattctag
tttctctcaa tgaataattg    1620

tatttttgta ggaaatgtaa gatttcattc tgaaacataa
ttattggtat ggacaaaatt    1680

gcagatacca tttctgttga ggctgcagat ttccaacttt
tatttcagtg gttcagatta    1740

gtattaggtc ggtactaaga aataagcatg ttttcactaa
tttaagtact tgagactctt    1800

gaagaaaatt cagaatgaag ttctggagaa aggtatgtta
ctgtagtaat tactctttga    1860

acaggttttg tgttttgtca ttagctctgc cctttttaat
taaatatttt ggttcatgga    1920

ccaaagggtt tacttgacaa atttgtgtga cagactccga
acaattcctt tactacgaag    1980

tataatttat aaaataaaat atacccattt taagggtaca
gtttgatttt tgaccagtga   2040

aactatgatc ccaatcaagg tatagatgcc gtcaccccaa
aaagttccct ccatatccct   2100

ttgcagtcag ttcatcccta ccctggccca gatgatcact
gatcttgtca ttatagatga   2160

gttttgccag ttcaagaatt taatggaatc agatattgta
agcattcttg tgtaatactt   2220

cattctctct cattattgag attcatccat attgttgaat
gtttcactag ttaatgttta   2280

ttgttcaata tttttgtata tacttttaaa gcctattcac
ttgctgatgg atctttggtt   2340

tgtttccagg tttggttatt atgaataaag ttgctgtgaa
tacttacgtg taaaaa       2396


<210>  59
<211>  3810
<212>  DNA
<213>  Homo sapiens

<400>  59
gcggcggccg ccttcgggga agggggtggg tccggggagg
ggtttgccat cctcctctag     60

ttaaaagtaa gggggaaaag agtaaacgcg cgactccagc
gcgcggctac ctacgcttgg    120

tgcttgcttt ctccagccat cggagaccag agccgccccc
tctgctcgag aaaggggctc    180

agcggcggcg gaagcggagg gggaccaccg tggagagcgc
ggtcccagcc cggccactgc    240

ggatccctga aaccaaaaag ctcctgctgc ttctgtaccc
cgcctgtccc tcccagctgc    300

gcagggcccc ttcgtgggat catcagcccg aagacaggga
tggagaggcc tctgtgctcc    360

cacctctgca gctgcctggc tatgctggcc ctcctgtccc
ccctgagcct ggcacagtat    420

gacagctggc cccattaccc cgagtacttc cagcaaccgg
ctcctgagta tcaccagccc    480

caggcccccg ccaacgtggc caagattcag ctgcgcctgg
ctgggcagaa gaggaagcac    540

agcgagggcc gggtggaggt gtactatgat ggccagtggg
gcaccgtgtg cgatgacgac    600

ttctccatcc acgctgccca cgtcgtctgc cgggagctgg
gctacgtgga ggccaagtcc    660

tggactgcca gctcctccta cggcaaggga gaagggccca
tctggttaga caatctccac    720

tgtactggca acgaggcgac ccttgcagca tgcacctcca
atggctgggg cgtcactgac    780

tgcaagcaca cggaggatgt cggtgtggtg tgcagcgaca
aaaggattcc tgggttcaaa    840

tttgacaatt cgttgatcaa ccagatagag aacctgaata
tccaggtgga ggacattcgg    900

attcgagcca tcctctcaac ctaccgcaag cgcaccccag
tgatggaggg ctacgtggag    960

gtgaaggagg gcaagacctg gaagcagatc tgtgacaagc
actggacggc caagaattcc   1020

cgcgtggtct gcggcatgtt tggcttccct ggggagagga
catacaatac caaagtgtac   1080

aaaatgtttg cctcacggag gaagcagcgc tactggccat
tctccatgga ctgcaccggc   1140

acagaggccc acatctccag ctgcaagctg ggcccccagg
tgtcactgga ccccatgaag   1200

aatgtcacct gcgagaatgg gctaccggcc gtggtgagtt
gtgtgcctgg gcaggtcttc   1260

agccctgacg gaccctcaag attccggaaa gcgtacaagc
cagagcaacc cctggtgcga    1320

ctgagaggcg gtgcctacat cggggagggc cgcgtggagg
tgctcaaaaa tggagaatgg    1380

gggaccgtct gcgacgacaa gtgggacctg gtgtcggcca
gtgtggtctg cagagagctg    1440

ggctttggga gtgccaaaga ggcagtcact ggctcccgac
tggggcaagg gatcggaccc    1500

atccacctca acgagatcca gtgcacaggc aatgagaagt
ccattataga ctgcaagttc    1560

aatgccgagt ctcagggctg caaccacgag gaggatgctg
gtgtgagatg caacacccct    1620

gccatgggct tgcagaagaa gctgcgcctg aacggcggcc
gcaatcccta cgagggccga    1680

gtggaggtgc tggtggagag aaacgggtcc cttgtgtggg
ggatggtgtg tggccaaaac    1740

tggggcatcg tggaggccat ggtggtctgc cgccagctgg
gcctgggatt cgccagcaac    1800

gccttccagg agacctggta ttggcacgga gatgtcaaca
gcaacaaagt ggtcatgagt    1860

ggagtgaagt gctcgggaac ggagctgtcc ctggcgcact
gccgccacga cggggaggac    1920

gtggcctgcc cccagggcgg agtgcagtac ggggccggag
ttgcctgctc agaaaccgcc    1980

cctgacctgg tcctcaatgc ggagatggtg cagcagacca
cctacctgga ggaccggccc    2040

atgttcatgc tgcagtgtgc catggaggag aactgcctct
cggcctcagc cgcgcagacc    2100

gaccccacca cgggctaccg ccggctcctg cgcttctcct
cccagatcca caacaatggc    2160

cagtccgact tccggcccaa gaacggccgc cacgcgtgga

tctggcacga ctgtcacagg   2220

cactaccaca gcatggaggt gttcacccac tatgacctgc
tgaacctcaa tggcaccaag   2280

gtggcagagg gccacaaggc cagcttctgc ttggaggaca
cagaatgtga aggagacatc   2340

cagaagaatt acgagtgtgc caacttcggc gatcagggca
tcaccatggg ctgctgggac   2400

atgtaccgcc atgacatcga ctgccagtgg gttgacatca
ctgacgtgcc ccctggagac   2460

tacctgttcc aggttgttat taaccccaac ttcgaggttg
cagaatccga ttactccaac   2520

aacatcatga aatgcaggag ccgctatgac ggccaccgca
tctggatgta caactgccac   2580

ataggtggtt ccttcagcga agagacggaa aaaaagtttg
agcacttcag cgggctctta   2640

aacaaccagc tgtccccgca gtaaagaagc ctgcgtggtc
aactcctgtc ttcaggccac   2700

accacatctt ccatgggact tccccccaac aactgagtct
gaacgaatgc cacgtgccct   2760

cacccagccc ggcccccacc ctgtccagac ccctacagct
gtgtctaagc tcaggaggaa   2820

agggaccctc ccatcattca tggggggctg ctacctgacc
cttggggcct gagaaggcct   2880

tgcgggggtg gggtttgtcc acagagctgc tggagcagca
ccaagagcca gtcttgaccg   2940

ggatgaggcc cacagacagg ttgtcatcag cttgtcccat
tcaagccacc gagctcacca   3000

cagacacagt ggagccgcgc tcttctccag tgacacgtgg
acaaatgcgg gctcatcagc   3060

ccccccagag agggtcaggc cgaaccccat ttctcctcct
cttaggtcat tttcagcaaa   3120

cttgaatatc tagacctctc ttccaatgaa accctccagt
ctattatagt cacatagata   3180

atggtgccac gtgttttctg atttggtgag ctcagacttg
gtgcttccct atccacagcc   3240

cccacccctt gtttttcaag atactattat tatattttca
cagacttttg aagcacaaat   3300

ttattggcat ttaatattgg acatctgggc ccttggaagt
acaaatctaa ggaaaaacca   3360

acccactgtg taagtgactc atcttcctgt tgttccaatt
ctgtgggttt ttgattcaac   3420

ggtgctataa ccagggtcct gggtgacagg gcgctcactg
agcaccatgt gtcatcacag   3480

acacttacac atacttgaaa cttggaataa aagaaagatt
tatgaaacgt gtctgtgttt   3540

cctttgaccc acagcacctg ggccctgagc agcaggcttc
ctatgttcag tggccagaag   3600

cagagcttca ggtacattcg tggttttctc cggtggacat
gggtcctcag atcccctcca   3660

gcccagtgtg gccaccaggg cacctccttc aatagactcc
aaaaggggca gctcctacca   3720

tctgggagaa gcaatctaag gagatcacaa aaagtaacgg
aacaggagtc ataatctttc   3780

ttgaactcct gtggttttta ctgaaacttg
                3810


<210>  60
<211>  6725
<212>  DNA
<213>  Homo sapiens

<400>  60
tcggcgggaa gcggcgatcc tgccaccggg aggtgtggaa
gagccgggta gattctggct      60

acattggaga ttggttgctt tctaaaactg aaggagaagc
ccatgaagag atggtggatt     120

ctcactgagt tttgactagc ggaagaaaag agagagttca
agtggatggc cttgaggact     180

tgaaaagctg agatatgatg attttgaagt catttcacat
cgaagccatg atttaaatat     240

cggcgttaag atttcaacaa gaaaaactta agcttccttg
gattcccacg tcaaaggaaa     300

gtttcaagct ttcagaagga gttctcactc gaagataaag
aacagctcgc taaccacgaa     360

agaggaatcg atgctcagct tttagttgca cttcctaaag
ttgcagaatt aagacaaatc     420

tttgaaccaa agaagaaaga attcttagaa atgaaaagaa
aagaaagaat tgccaggcgc     480

ctggaaggga ttgaaaatga cactcagccc atcctcttgc
agagctgcac aggattggtg     540

actcaccgcc tgctggagga agacacccct cgatacatga
gagccagcga ccctgccagc     600

ccccacatcg gccgatcaaa tgaagaggag gaaacttctg
attcttctct agaaaagcaa     660

actcgatcca aatactgcac agaaacctcc ggtgtccacg
gtgactcacc ctatggttcg     720

ggtaccatgg acacccacag tctggagtcc aaagccgaaa
gaattgcaag gtacaaagca     780

gaaagaaggc gacagctggc agagaagtat gggctgactc
tggatcccga ggccgactcc     840

gagtatttat cccgctatac caagtccagg aaggagcctg
atgctgtcga gaagcgggga     900

ggaaaaagtg acaaacagga agagtcaagc agagatgcta
gttctctgta ccccgggacc     960

gagacgatgg ggctcaggac ctgtgccggt gaatccaagg
actatgccct ccatgtgggt    1020

gacggctctt ccgacccgga ggtgctgctg aacatagaaa
accaaagacg aggtcaagag    1080

ctgagtgcca cccggcaggc ccatgacctg tccccagcag
ccgagagttc ctcgaccttc    1140

tctttctctg ggcgagactc ctccttcact gaagtgccac
ggtcccccaa gcacgcccac    1200

agctcctccc tgcagcaggc agcctcccgg agcccctcct
ttggtgaccc acagctatcc    1260

cctgaggccc gacccaggtg cacttcacat tcagaaacgc
caactgtcga tgatgaagaa    1320

aaggtggatg aacgagccaa gctgagcgtc gccgccaaga
ggttgctttt cagggagatg    1380

gaaaaatctt ttgatgaaca aaatgttcca aagcgacgct
caagaaacac agctgtggag    1440

cagaggctac gccgtctgca ggacaggtcc ctcacccagc
ccatcaccac tgaagaggtg    1500

gtcatcgcag ccacattgca ggcctctgct caccaaaagg
ccttagccaa ggaccagaca    1560

aatgagggca aagagcttgc tgagcaagga gaacctgatt
cctccactct aagcttggcc    1620

gaaaagttgg ccttgtttaa caaattgtcc cagccagtct
caaaagcgat ttctacccgg    1680

aacagaatag acacgagaca gaggagaatg aacgctcgct
atcaaactca gccagtcaca    1740

ctgggagagg tggagcaggt gcagagtgga aagctcattc
ctttctcacc tgccgtgaac    1800

acatcagtgt ctaccgtagc atccacggtt gctccaatgt
atgccggaga tcttcgcaca    1860

aagccacctc ttgaccacaa tgcaagtgcc actgactata

agttttcttc ttcaatagaa 1920

aattcggact ctccagttag aagcattctg aaatcgcaag
cttggcagcc tttggtagag 1980

ggtagcgaga acaagggaat gttgagagaa tatggagaga
cagaaagcaa gagagctttg 2040

acaggtcgag acagtgggat ggagaagtat gggtcctttg
aggaagcaga agcatcctac 2100

cccatcctga accgagccag ggaaggagac agccataagg
aatctaaata tgctgttccc 2160

agaagaggaa gcctggaacg ggcgaaccct cccatcaccc
acctcgggga tgaaccgaag 2220

gaattttcca tggctaaaat gaatgcacaa ggaaacttgg
acttgaggga caggctgccc 2280

tttgaagaga aggtggaggt ggagaatgtt atgaaaagga
agttttcact aagagcggca 2340

gagttcgggg agcccacttc cgagcagacg ggacagctg
ctgggaaaac tattgctcaa 2400

accacagccc ccgtgtcctg gaagccccag gattcttcgg
aacagccaca ggagaagctc 2460

tgcaagaatc catgtgcgat gtttgctgct ggagagatca
aaacgccgac aggggagggc 2520

cttcttgact cacccagcaa aaccatgtct attaaagaaa
gattggcact gttgaagaaa 2580

agcggggagg aagattggag aaacagactc agcaggaggc
aggagggcgg caaggcgccg 2640

gccagcagcc tgcacaccca ggaagcaggg cggtccctca
tcaagaagcg ggtcacagaa 2700

agtcgagaga gccaaatgac gattgaggag aggaagcagc
tcatcactgt gagagaggag 2760

gcctggaaga cgagaggcag aggagcggcc aacgactcga
cccagttcac tgtggctggc 2820

aggatggtga agaaaggttt ggcgtcacct actgccataa
ccccagtagc ctcacccatt   2880

tgcggtaaaa caagaggcac cacacccgtt tccaaacccc
tggaagatat cgaagccaga   2940

ccagatatgc agttagaatc ggacctgaag ttggacaggc
tggaaacctt tctaagaagg   3000

ctgaataaca aagttggcgg gatgcacgaa acggtgctca
ctgtcaccgg caaatctgtg   3060

aaggaggtga tgaagccaga tgatgatgaa acctttgcca
aattttaccg cagcgtggat   3120

tataatatgc caagaagtcc tgtggagatg gatgaggact
tcgatgtcat tttcgatcct   3180

tatgcaccca aattgacgtc ttccgtggcc gagcacaagc
gggcagttag gcccaagcgc   3240

cgggttcagg cctccaaaaa ccccctgaaa atgctggcgg
caagagaaga tctccttcag   3300

gaatacactg agcagagatt aaacgttgcc ttcatggagt
caaagcggat gaaagtagaa   3360

aagatgtctt ccaactccaa cttctcagaa gtcaccctgg
cgggtttagc cagtaaagaa   3420

aacttcagca acgtcagcct gcggagcgtc aacctgacgg
aacagaactc taacaacagc   3480

gccgtgccct acaagaggct gatgctgttg cagattaaag
gaagaagaca tgtgcagacc   3540

aggctggtgg aacctcgagc ttcggcgctc aacagtgggg
actgcttcct cctgctctct   3600

ccccactgct gcttcctgtg ggtaggagag tttgcaaacg
tcatagaaaa ggcgaaggcc   3660

tcagaacttg caactttaat tcagacaaag agggaacttg
gttgtagagc tacttatatc   3720

caaaccattg aagaaggaat taatacacac actcatgcag
ccaaagactt ctggaagctt   3780

ctgggtggcc aaaccagtta ccaatctgct ggagacccaa
aagaagatga actctatgaa   3840

gcagccataa tagaaactaa ctgcatttac cgtctcatgg
atgacaaact tgttcctgat   3900

gacgactact gggggaaaat tccgaagtgc tcccttctgc
aacccaaaga ggtactggtg   3960

tttgattttg gtagtgaagt ttacgtatgg catgggaaag
aagtcacatt agcacaacga   4020

aaaatagcat ttcagctggc aaagcactta tggaatggaa
cctttgacta tgagaactgt   4080

gacatcaatc ccctggatcc tggagaatgc aatccgctta
tccccagaaa aggacagggg   4140

cggcccgact gggcgatatt tgggagactt actgaacaca
atgagacgat tttgttcaaa   4200

gagaagtttc tggattggac ggaactgaag agatcgaatg
agaagaaccc cggggaactt   4260

gcccagcaca aggaagaccc caggactgat gtcaaggcat
acgatgtgac acggatggtg   4320

tccatgcccc agacgacagc aggcaccatc ctggacggag
tgaacgtcgg ccgtggctat   4380

ggcctggtgg aaggacacga caggaggcag tttgagatca
ccagcgtttc cgtggatgtc   4440

tggcacatcc tggaattcga ctatagcagg ctccccaaac
aaagcatcgg gcagttccat   4500

gagggggatg cctatgtggt caagtggaag ttcatggtga
gcacggcagt gggaagtcgc   4560

cagaagggag agcactcggt gagggcagcc ggcaaagaga
agtgcgtcta cttcttctgg   4620

caaggccggc actccaccgt gagtgagaag ggcacgtcgg

cgctgatgac ggtggagctg    4680

gacgaggaaa gggggcccca ggtccaggtt ctccagggaa
aggagccccc ctgtttcctg    4740

cagtgtttcc agggggggat ggtggtgcac tcggggaggc
gggaagagga agaagaaaat    4800

gtgcaaagtg agtggcggct gtactgcgtg cgtggagagg
tgcccgtgga agggaatttg    4860

ctggaagtgg cctgtcactg tagcagcctg aggtccagaa
cttccatggt ggtgcttaac    4920

gtcaacaagg ccctcatcta cctgtggcac ggatgcaaag
cccaggccca cacgaaggag    4980

gtcggaagga ccgctgcgaa caagatcaag gaacaatgtc
ccctggaagc aggactgcat    5040

agtagcagca aagtcacaat acacgagtgt gatgaaggct
ccgagccact cggattctgg    5100

gatgccttag gaaggagaga caggaaagcc tacgattgca
tgcttcaaga tcctggaagt    5160

tttaacttcg cgccccgcct gttcatcctc agcagctcct
ctggggattt tgcagccaca    5220

gagtttgtgt accctgcccg agccccctct gtggtcagtt
ccatgccctt cctgcaggaa    5280

gatctgtaca gcgcgcccca gccagcactt ttccttgttg
acaatcacca cgaggtgtac    5340

ctctggcaag gctggtggcc catcgagaac aagatcactg
gttccgcccg catccgctgg    5400

gcctccgacc ggaagagtgc gatggagact gtgctccagt
actgcaaagg aaaaaatctc    5460

aagaaaccag cccccaagtc ttaccttatc cacgctggtc
tggagcccct gacattcacc    5520

aatatgtttc ccagctggga gcacagagag gacatcgctg
agatcacaga gatggacacg    5580

gaagtttcca atcagatcac cctcgtggaa gacgtcttag
ccaagctctg taaaaccatt   5640

tacccgctgg ccgacctcct ggccaggcca ctcccggagg
gggtcgatcc tctgaagctt   5700

gagatctatc tcaccgacga agacttcgag tttgcactag
acatgacgag ggatgaatac   5760

aacgccctgc ccgcctggaa gcaggtgaac ctgaagaaag
caaaaggcct gttctgagtg   5820

gggagacgcc agaggagcct cacggtcacg tccaacaaca
ccactgcacc agggaaatgg   5880

atatatattt ttggactggt gtttttcaca aagtattttt
caatcagagt tttcagaacc   5940

tgacattgtt aaagatactg cttgtcccgg agttgtgtat
tttgtaaatg ttcaagggaa   6000

ctgtttggaa acttctttcc accattcagg aggttatcag
aattaataaa agtatctgtt   6060

atgtgcactt aagccgcagc tgctatagat agcactgcct
tcttgttcca gctaggcaat   6120

gccttttttt tttttttttg aagcagttct ctttataaag
tgttattttg atagtttgtg   6180

gattctaaaa tatatatata tttatataaa caccatataa
gtcaaatatg tatttaacaa   6240

agcaatatgt attcattcac tttcaagatt tgttttggtg
tcaaaataac atgaaaaggt   6300

agatggagtt gcttctgttg aattagctct gccaccaata
tgtatcttca tacacgtttg   6360

gaaatgtttc ctgcagcatt aggtatgact tgttctgagt
actgcttccg gtgctaaaat   6420

gaacaaagaa tttgtactta atggcatgga ctctggagaa
tctatgcgaa tcaacctttc   6480

taccttaata tctccccaaa aatgtatagt gccttgtttt
tatgtacagt ttatatacag    6540

aaaagtttgc tctgcatttt tgatgatggt ttggaacatt
atctacaatt ttactctcaa    6600

atagtcaaaa taaaaacatc tcaatttcta ataccggttg
taaacagtac acatgtcatt    6660

ttgtgatata ggactcccaa ataaaagtat cagaataaac
acaacaatta actggtaaaa    6720

aaaaa
                6725

<210>  61
<211>  5580
<212>  DNA
<213>  Homo sapiens

<400>  61
cgcggagctt gtggcgccag aattcggagc gcggaagagc
cagagctgcg agcgcctgga      60

gctggatctc tctccggtcg cgcacgccga ggccagtagg
gagagaagat ggtggttctc     120

cgcagcagct tggagctgca caaccactcc gcggcctcgg
ccacgggctc cttggacctg     180

tccagtgact tcctcagtct ggagcacatc ggccggaggc
ggctccgctc ggccggcgcg     240

gcgcagaaga aacccgcggc gaccacagcc aaagcgggcg
atgggtcatc agttaaggaa     300

gttgaaacct accaccggac acgtgcttta agatctttga
gaaaagatgc acagaattct     360

tcagattcta gttttgagaa gaatgtggaa ataacggagc
aacttgctaa tggcaggcat     420

tttacaaggc agttggccag acagcaggct gataaaaaaa
aagaagagca cagagaagac     480

aaagtgattc cagttactcg gtcattgagg gctagaaaca
tcgttcaaag tacagaacac     540

ttacatgaag ataatggtga tgttgaagtg cgtcgaagtt
gtaggattag aagtcgttat     600

agtggtgtaa accagtccat gctgtttgac aaacttataa
ctaacactgc tgaagctgta     660

cttcaaaaaa tggatgacat gaagaagatg cgtagacagc
gaatgagaga acttgaagac     720

ttgggagtgt ttaatgaaac agaagaaagc aatcttaata
tgtacacaag aggaaaacag     780

aaagatattc aaagaactga tgaagaaaca actgataatc
aagaaggcag tgtggagtca     840

tctgaagagg gtgaagacca agaacatgaa gatgatggtg
aagatgaaga tgatgaagat     900

gatgatgatg atgacgatga tgatgatgat gatgatgatg
aagatgatga agatgaagaa     960

gatggagaag aagagaatca gaagcgatat tatcttagac
agagaaaagc tactgtttac    1020

tatcaggctc cattggaaaa acctcgtcac cagagaaagc
ccaacatatt ttatagtggc    1080

ccagcttctc ctgcaagacc aagataccga ttatcttccg
caggaccaag aagtccttac    1140

tgtaaacgaa tgaacaggcg aaggcatgca atccacagta
gtgactcgac ttcatcttcc    1200

tcctctgaag atgaacagca ctttgagagg cggaggaaaa
ggagtcgtaa tagggctatc    1260

aataggtgcc tcccactaaa ttttcggaaa gatgaattaa
aaggcattta taaagatcga    1320

atgaaaattg gagcaagcct tgccgatgtt gatccaatgc
aactagattc ttcagtacga    1380

tttgatagtg ttggtggcct gtctaatcat atagcagctc

taaaagagat ggtggtgttt 1440

ccattacttt atccagaagt ctttgaaaaa tttaaaattc
aaccccccaag aggttgtttg 1500

ttttatgggc cacctggaac tggaaagact ctggttgcca
gagcacttgc caatgagtgc 1560

agtcaagggg ataaaagagt agcatttttc atgaggaaag
gtgctgattg tctaagtaaa 1620

tgggtaggag aatctgaaag acagctacga ttgctgtttg
atcaggccta tcagatgcgc 1680

ccatcaatta ttttttttga cgaaattgat ggtctggctc
cagtacggtc aagcaggcaa 1740

gatcagattc acagttctat tgtttccacc ctgctagctc
ttatggatgg attggacagc 1800

agaggggaaa ttgtggtcat tggtgctacg aacaggctag
attctataga tcctgcttta 1860

cgaaggcctg gtcgctttga tagagaattc ctctttagcc
tgcctgataa agaggctcga 1920

aaagagattc taaagattca caccagggat tggaatccca
aaccactgga cacattttta 1980

gaagagctag cagaaaactg tgttggatac tgtggagcag
atattaaatc aatatgtgct 2040

gaagctgctt tatgtgcttt acgacgacgc tacccacaga
tctataccac tagtgagaaa 2100

ctgcagttgg atctctcttc aattaatatc tcagctaagg
atttcgaggt agctatgcaa 2160

aagatgatac cagcctccca aagagctgtg acatcacctg
ggcaggcact gtccaccgtt 2220

gtgaaaccac tcctgcaaaa cactgttgac aagattttag
aagccctgca gagagtattt 2280

ccacatgcag aattcagaac aaataaaaca ttagactcag
atatttcttg tcctctgcta 2340

gaaagtgact tggcttacag tgatgatgat gttccatcag
tttatgaaaa tggactttct   2400

cagaaatctt ctcataaggc aaaagacaat tttaattttc
ttcatttgaa tagaaatgct   2460

tgttaccaac ctatgtcttt tcgaccaaga atattgatag
taggagaacc aggatttggg   2520

caaggttctc acttggcacc agctgtcatt catgctttgg
aaaagtttac tgtatataca   2580

ttagacattc ctgttctttt tggagttagt actacatccc
ctgaagaaac atgtgcccag   2640

gtgattcgtg aagctaagag aacagcacca agtatagtgt
atgttcctca tatccacgtg   2700

tggtgggaaa tagttggacc gacacttaaa gccacattta
ccacattatt acagaatatt   2760

ccttcatttg ctccagtttt actacttgca acttctgaca
aaccccattc cgctttgcca   2820

gaagaggtgc aagaattgtt tatccgtgat tatggagaga
tttttaatgt ccagttaccg   2880

gataaagaag aacggacaaa attttttgaa gatttaattc
taaaacaagc tgctaagcct   2940

cctatatcaa aaaagaaagc agttttgcag gctttggagg
tactcccagt agcaccacca   3000

cctgagccaa gatcactgac agcagaagaa gtgaaacgac
tagaagaaca agaagaagat   3060

acatttagag aactgaggat tttcttaaga aatgttacac
ataggcttgc tattgacaag   3120

cgattccgag tgtttactaa gcctgttgac cctgatgagg
ttcctgatta tgtcactgta   3180

ataaagcaac caatggacct ttcatctgta atcagtaaaa
ttgatctaca caagtatctg   3240

actgtgaaag actatttgag agatattgat ctaatctgta
gtaatgcctt agaatacaat   3300

ccagatagag atcctggaga tcgtcttatt aggcatagag
cctgtgcttt aagagatact   3360

gcctatgcca taattaaaga agaacttgat gaagactttg
agcagctctg tgaagaaatt   3420

caggaatcta gaaagaaaag aggttgtagc tcctccaaat
atgccccgtc ttactaccat   3480

gtgatgccaa agcaaaattc cactcttgtt ggtgataaaa
gatcagaccc agagcagaat   3540

gaaaagctaa agacaccgag tactcctgtg gcttgcagca
ctcctgctca gttgaagagg   3600

aaaattcgca aaaagtcaaa ctggtactta ggcaccataa
aaaagcgaag gaagatttca   3660

caggcaaagg atgatagcca gaatgccata gatcacaaaa
ttgagagtga tacagaggaa   3720

actcaagaca caagtgtaga tcataatgag accggaaaca
caggagagtc ttcggtggaa   3780

gaaaatgaaa aacagcaaaa tgcctctgaa agcaaactgg
aattgagaaa taattcaaat   3840

acttgtaata tagagaatga gcttgaagac tctaggaaga
ctacagcatg tacagaattg   3900

agagacaaga ttgcttgtaa tggagatgct tctagctctc
agataataca tatttctgat   3960

gaaaatgaag gaaaagaaat gtgtgttctg cgaatgactc
gagctagacg ttcccaggta   4020

gaacagcagc agctcatcac tgttgaaaag gctttggcaa
ttctttctca gcctacaccc   4080

tcacttgttg tggatcatga gcgattaaaa aatcttttga
agactgttgt taaaaaaagt   4140

caaaactaca acatatttca gttggaaaat ttgtatgcag

taatcagcca atgtatttat 4200

cggcatcgca aggaccatga taaaacatca cttattcaga
aaatggagca agaggtagaa 4260

aacttcagtt gttccagatg atgatgtcat ggtatcgagt
attctttata ttcagttcct 4320

atttaagtca tttttgtcat gtccgcctaa ttgatgtagt
atgaaaccct gcatctttaa 4380

ggaaaagatt aaaatagtaa aataaaagta tttaaacttt
cctgatattt atgtacatat 4440

taagataaat gtcatgtgta agataactga taaatattgg
aactttgcta gaacaagacc 4500

ctgtagtaat agtaataata gttgaagttt ggccaactct
taataaagtt attttggtaa 4560

ctaatgtttt atggcactta agaataatta gcagcgttaa
attttgtttg tattaagcac 4620

ttttaatttt atccttccta aaaatagttt attgtatctg
acaagaaact tacttaacca 4680

ttgtgtcctt cccatctttt ttgtcatctt tgttttcttc
aaatgccctc ctcccatctg 4740

ccttgagatt ccctcgtctt cacttaaaag ccagagtgca
agtcatgatt tgcgggaggg 4800

ctcttgaacc acttctggct gcaccacaat tctgtacttg
agtatcacag tcattgtttt 4860

tgagacaaac atttttataa ttctaatttg ggttaataaa
gattttaaat atttcttggt 4920

ttacttttgt aattatatac acaacaaatg tattaataac
taccttgtta aacacctttt 4980

aatagcacaa ggtttttata tttgcaagct gttgatatct
ttctaaaact gtttaggtta 5040

tagtctattg atacttttta tatacaattt tataaatata
aatattataa ttttatatta 5100

atggtaccaa aaatacattt cttaaggtta aaagcatgca
cttccatgca tacttgcttt   5160

tggggagagt ggggagaaga cattctaata atcagtttgt
gaaatagctt ctgttggaaa   5220

ccttttgagg ggaataagga atggtcatct aaaatgagag
attctggatt ttaatgcagt   5280

tcaaagttga gctgtatttt tgttgttgat ttatctggat
ttttttttaaa gccttctaaa   5340

acccagtgaa ttcaatacct taattagtac atactatctt
atgtaatgca taaagcaatg   5400

ccagtcactg agaacattta aatatattta tattcctgga
gatacacatt ctcattttg   5460

ttggtttatt ataaattatt cttctagatg catcttttat
aactaggatt tcattttgtg   5520

tgtatagctt atgtaataaa ttttaaaggt gaaaactctc
ttaaaaaaaa aaaaaaaaaa   5580


<210>  62
<211>  1170
<212>  DNA
<213>  Homo sapiens

<400>  62
aagtttgaaa ctggtaactt cgggagttga gccacgagct
gttgtgcatc cagaggtgga     60

attggggccc ggcattccct cctcgtcccg ggctggccct
tgcccccacc ctgcaactcc    120

tggttgagat gggctcagcc aagagcgtcc cagtcacacc
agcgcggcct ccgccgcaca    180

acaagcatct ggctcgagtg gcggaccccc gttcacctag
tgctggcatc ctgcgcactc    240

ccatccaggt ggagagctct ccacagccag gcctaccagc
aggggagcaa ctggagggtc    300

ttaaacatgc ccaggactca gatccccgct ctcctactct
tggtattgca cggacaccta    360

tgaagaccag cagtggagac cccccaagcc cactggtgaa
acagctgagt gaagtatttg    420

aaactgaaga ctctaaatca aatcttcccc cagagcctgt
tctgccccca gaggcacctt    480

tatcttctga attggacttg cctctgggta cccagttatc
tgttgaggaa cagatgccac    540

cttggaacca gactgagttc ccctccaaac aggtgttttc
caaggaggaa gcaagacagc    600

ccacagaaac ccctgtggcc agccagagct ccgacaagcc
ctcaagggac cctgagactc    660

ccagatcttc aggttctatg cgcaatagat ggaaaccaaa
cagcagcaag gtactaggga    720

gatcccccct caccatcctg caggatgaca actcccctgg
caccctgaca ctacgacagg    780

gtaagcggcc ttcaccccta agtgaaaatg ttagtgaact
aaaggaagga gccattcttg    840

gaactggacg acttctgaaa actggaggac gagcatggga
gcaaggccag gaccatgaca    900

aggaaaatca gcactttccc ttggtggaga gctaggccct
gcatggcccc agcaatgcag    960

tcacccaggg cctggtgata tctgtgtcct ctcacccctt
ctttcccagg gatactgagg   1020

aatggcttgt tttcttagac tcctcctcag ctaccaaact
gggactcaca gctttattgg   1080

gctttctttg tgtcttgtgt gtttctttta tattaaagga
agtaatttta aatgttactt   1140

taaaaaggta tatgtaaacc ttgcaccgag
                1170

## Description

### FIELD OF THE INVENTION

The present invention relates to *in vitro* methods and compositions for diagnosing, preventing and/or treating an adenocarcinoma associated with a chromosomal aberration on chromosome 20q and/or the predisposition for developing such an adenocarcinoma by determining the expression levels of a set of particular marker genes, wherein an elevated expression level of said marker genes in target cells as compared to control cells is indicative of such an adenocarcinoma and by specifically modifying the gene expression of said marker genes in such way that the elevated expression level(s) identified in the target cells are down-regulated.

### BACKGROUND OF THE INVENTION

Most cancers are epithelial in origin and arise through a stepwise progression from normal cells, through dysplasia, into malignant cells that invade surrounding tissues and have metastatic potential. Colorectal cancer (CRC; also referred to as colon cancer or large bowel cancer) is one prominent type of cancer undergoing such tumor progression.

CRC includes cancerous growth in the colon, rectum and appendix. Colorectal cancer (CRC) is the most significant human cancer with an incidence of about 1.000.000 new cases worldwide every year. It is the third most common cancer and the fourth leading cause of cancer deaths in the world (the second leading cause in the Western world; reviewed, e.g., in Gryfe, R. et al. (1997) Curr. Probl. Cancer 21, 233-300; Petersen, G.M. et al. (1999) Cancer 86, 2540-2550).

CRC is curable if diagnosed at an early stage (i.e. stage I) of tumor development. The five-year survival rate for early stage CRC is > 90%. At this early stage, most patients have no phenotypic symptoms of the disease. Early detection can markedly improve chances of long-term survival, as the five-year survival rate for CRC at a late stage (i.e. stage IV) of tumor development is only < 5%. More than 95% of the cases of CRC are manifested as adenocarcinomas (Muto, T. et al. (1975) Cancer 36, 2251-2270; Fearon, E.R. and Vogelstein, B. (1990) Cell 61, 759-767).

Initially, CRC is characterized by the occurrence of a hyper-proliferative (dysplastic) epithelium in the colon, which first turns into inflammatory adenomatous polyps, then into adenomas, which are neoplasms (i.e. benign tumors) in the inner lining of the colon or rectum. Usually, only a small subset of the adenomas formed (i.e. about 5%) progress into malignant adenocarcinomas.

Genomic instability is another crucial step in progression from adenomas to adenocarcinomas and occurs in two ways in CRC (Lengauer, C. et al. (1997) Nature 386, 623-627). DNA mismatch repair deficiency leading to microsatellite instability, explains only about 15% of the cases of adenoma to carcinoma progression (Umar, A. et al. (2004) J. Natl. Cancer Inst. 96, 261-268; di Pietro, M. et al. (2005) Gastroenterology 129, 1047-1059). In the other 85%, genomic instability occurs at the chromosomal level (CIN), giving rise to aneuploidy.

While for a long time chromosomal aberrations have been regarded as random noise, secondary to cancer development, it is now well established that these DNA copy number changes occur in specific patterns and are associated with different clinical behavior (Hermsen, M. et al. (2002) Gastroenterology 123, 1109-1119; Rajagopalan, H. et al. (2003) Nat. Rev. Cancer 3, 695-701). Nevertheless, neither the cause of chromosomal instability in human cancer progression nor its biological consequences have been fully appreciated.

Chromosomal aberrations frequently reported in CRC are 7pq, 8q, 13q, and 20q gains and 4pq, 5q, 8p, 15q, 17p, and 18q losses (Douglas, E.J. et al. (2004) Cancer Res. 64, 4817-4825). Of these, especially 8q, 13q and 20q gains and 8p, 15q, 17p and 18q losses are associated with colorectal adenoma to carcinoma progression.

Gain of 20q is observed in more than 65% of CRCs (De Angelis, P.M. et al. (1999) Br. J. Cancer 80, 526-535). Gains of 20q are also common in other tumor types and have been associated with poor outcome in gastric cancer and CRC. Analysis of DNA copy number changes at gene level by multiplex ligation-dependent probe amplification (MLPA) showed that in CRC, besides 20q13, also 20q11 is frequently amplified (Postma, C. et al. (2005) J. Pathol. 205, 514-521).

A recent study demonstrated that a subset of seven genes (i.e. *C20orf24*/*RIP5, AURKA*/*STK6, RNPC1, THIL, ADRM1, C20orf20*, and *TCFL5*) mapping at 20q were over-expressed in adenocarcinomas as compared to adenomas as a consequence of a copy number gain of 20q (Carvalho, B. et al. (2009) Gut 58, 79-89). It was thus suggested that these genes may function as molecular markers for diagnosing adenocarcinoma associated with this chromosomal aberration or a predisposition to develop the same. However, no evidence has been provided that any of these genes are functionally involved in tumor etiology and/or development, particularly in the progression from adenomas to adenocarcinomas.

Accumulated cancer-associated chromosomal instability typically leads to changes in gene expression, and affects the activity of carcinogenic pathways. Biological processes that are frequently altered during carcinogenesis modulate the behavior of tumor cells and tissues. Tumor cells exhibit increased proliferation and lack of apoptosis, overcome senescence and may gain invasive and metastatic potential (reviewed, e.g., in Hanahan D., and Weinberg, R.A. (2000) Cell 100, 57-70) Expanding tumors become hypoxic and induce angiogenesis, while sustained angiogenesis results in poor vasculature and contributes to activation of stroma by serum exposure (Dvorak H.F. (1986) N. Engl. J. Med. 315, 1650-1659). However, with regard to CRC it is currently still unknown which of these pathways are most relevant to adenoma to adenocarcinoma progression.

Finally, no reliable and unique target sites (e.g., biomarker genes) for molecular intervention have been identified for preventing and/or treating adenocarcinoma.

The identification of such molecular targets would be of utmost clinical importance, particularly if they would allow early stage diagnosis and/or the treatment of adenocarcinoma while avoiding unnecessary surgical intervention. Ideally, the prevention or treatment of adenocarcinoma should be enabled at a stage where the presence of malignant cells is not yet detectable by *in situ* techniques or microscopic analysis of biopsy or resection material.

Thus, it is a continuing need for *in vitro* methods and pharmaceutical compositions for the reliable diagnosis as well as the efficient prevention and/or treatment of an adenocarcinoma associated with a chromosomal aberration on chromosome 20q, particularly at an early stage of adenoma to adenocarcinoma progression.

### OBJECT AND SUMMARY OF THE INVENTION

It is an objective of the present invention to provide novel approaches for diagnosing, preventing and/or treating an adenocarcinoma associated with a chromosomal aberration on chromosome 20q and/or the predisposition for developing such an adenocarcinoma by determining the expression levels of a set of particular marker genes, wherein an elevated expression level of said marker genes in target cells as compared to control cells is indicative of such an adenocarcinoma and by specifically modifying the gene expression of said marker genes in such way that the elevated expression level(s) identified in the target cells are down-regulated.

More specifically, it is an object of the present invention to provide *in vitro* methods and pharmaceutical compositions for diagnosing, preventing and/or treating the progression from an adenoma to an adenocarcinoma by determining and/or specifically modifying the gene expression of one or more of a particular selection of 45 marker genes whose elevated expression in target cells is indicative of an adenocarcinoma associated with a chromosomal aberration on chromosome 20q.

These objectives as well as others, which will become apparent from the ensuing description, are attained by the subject matter of the independent claims. Some of the preferred embodiments of the present invention are defined by the subject matter of the dependent claims.

In a first aspect, the present invention relates to an *in vitro* method for preventing and/or treating an adenocarcinoma associated with a chromosomal aberration on chromosome 20q in a subject, the method comprising:
(a) identifying in one or more target cells of the subject at least one marker gene whose expression level(s) is/are elevated as compared to one or more control cells, wherein an elevated expression level of any one of the at least one marker gene in the one or more target cells is indicative of an adenocarcinoma associated with a chromosomal aberration on chromosome 20q in the subject; and
(b) modifying in the one or more target cells the gene expression of the at least one marker gene in such way that the elevated expression level(s) identified in step (a) are down-regulated,
wherein the at least one marker gene is selected from the group consisting of *RNPC1* (GenBank acc. no. NM_017495.4), *TCFL5* (GenBank acc. no. NM_006602.2), *C20orf24*/*RIP5* (GenBank acc. no. NM_018840.2), *AURKAlSTK6* (GenBank acc. no. NM_003600.2), *C20orf20* (GenBank acc. no. NM_018270.4), *ADRM1* (GenBank acc. no. NM_007002.2), *THIL* (GenBank acc. no. NM_198976.1), *TGIF2* (GenBank acc. no. NM_021809.5), *DPM1* (GenBank acc. no. NM_003859.1), *TOP1* (GenBank acc. no. NM_003286.2), *C20orf11* (GenBank acc. no. NM_017896.2), *YWHAB* (GenBank acc. no. NM_003404.3), *ZNF217* (GenBank acc. no. NM_006526.2), *BCL2L1* (GenBank acc. no. NM_001191.2), *DATF1*/*DIDO1* (GenBank acc. no. NM_022105.3), *CSE1L* (GenBank acc. no. NM_001316.2), *C20orf126*/*PDRG1* (GenBank acc. no. NM_030815.2), *TPX2* (GenBank acc. no. NM_012112.4), *C20orf59* (GenBank acc. no. NM_022082.3), *UBE2C* (GenBank acc. no. NM_007019.2), *NCOA3* (GenBank acc. no. NM_006534.2), *C20orf14*/*PRPF6* (GenBank acc. no. NM_012469.3), *C20orf44*/*bFZb* (GenBank acc. no. NM_018244.3), *RNPC2* (GenBank acc. no. NM_004902.2), *HM13* (GenBank acc. no. NM_030789.2), *SLCO4A1* (GenBank acc. no. NM_016354.3), *VAPB* (GenBank acc. no. NM_004738.3), *KIAA1847* (GenBank acc. no. NM_032527.3), *CDC91L1* (GenBank acc. no. NM_080476.4), *PXMP4* (GenBank acc. no. NM_007238.4), *DLGAP4* (GenBank acc. no. NM_014902.3), *PFDN4* (GenBank acc. no. NM_002623.3), *E2F1* (GenBank acc. no. NM_005225.2), *CYP24A1* (GenBank acc. no. NM_000782.4), *SRC* (GenBank acc. no. NM_005417.3), *BCAS4* (GenBank acc. no. NM_017843.3), *PLK1* (GenBank acc. no. NM_005030.3), *CCNF* (GenBank acc. no. NM_001761.2), *NUDT1* (GenBank acc. no. NM_002452.3), *SSSCA1* (GenBank acc. no. NM_006396.1), *ID3* (GenBank acc. no. NM_002167.3), *LUM* (GenBank acc. no. NM_002345.3), *PDGFRB* (GenBank acc. no. NM_002609.3), *SPARC* (GenBank acc. no. NM_003118.2), and *DCN* (GenBank acc. no. NM_001920.3).

In a second aspect, the present invention relates to *an in vitro* method for diagnosing in a subject an adenocarcinoma associated with a chromosomal aberration on chromosome 20q, the method comprising:
(a) detecting in one or more target cells of the subject the expression level(s) of at least one marker gene; and
(b) comparing the expression level(s) obtained in step (a) to that/those obtained in one or more control cells,
wherein an elevated expression level of any one of the at least one marker gene in the one or more target cells as compared to the one or more control cells is indicative of an adenocarcinoma associated with a chromosomal aberration on chromosome 20q in the subject; and
wherein the at least one marker gene is selected from the group of marker genes as defined herein above.

In other preferred embodiments, the above methods are for the further use of diagnosing or of preventing and/or treating a predisposition for developing an adenocarcinoma, a progression of an adenoma to an adenocarcinoma or a predisposition for a progression of an adenoma to an adenocarcinoma, the adenocarcinoma being associated with a chromosomal aberration on chromosome 20q.

Preferably, the chromosomal aberration on chromosome 20q is a chromosomal gain, particularly preferably at position 20q11.22-20q11.23 and/or at position 20q13.31-20q13.33.

In specific embodiments of the above methods, step (a) comprises the determination of the expression level(s) of the at least one marker gene by any one or more of the methods selected from the group consisting of:
(i) detecting a mRNA encoded by the marker gene(s);
(ii) detecting a protein encoded by the marker gene(s); and
(iii) detecting a biological activity of a protein encoded by the marker gene(s).

In a further preferred embodiment of the method according to the second aspect of the present invention, step (b) comprises introducing into the one or more target cells:
(i) one or more nucleic acid molecules, each nucleic acid molecule encoding a sequence that is complementary to at least a part of the nucleic acid sequence of any one of the at least one marker gene to be down-regulated, wherein the one or more nucleic acid molecules are preferably selected from the group consisting of an anti-sense nucleic acid construct, a siRNA, a miRNA, and a ribozyme; and/or
(ii) one or more antibodies, each antibody directed against any polypeptide encoded by any one of the at least one marker gene to be down-regulated, or one or more dominant-negative polypeptide variants of any polypeptide encoded by any one of the at least one marker gene to be down-regulated.

In other preferred embodiments of the above methods, step (a) comprises the detection or identification of at least the marker genes *RNPC1* (GenBank acc. no. NM_017495.4) and *TCFL5* (GenBank acc. no. NM_006602.2). In specific embodiments, step (a) further comprises the detection or identification of any one or more of the marker genes *C20orf24*/*RIP5* (GenBank acc. no. NM_018840.2), *AURKA*/*STK6* (GenBank acc. no. NM_003600.2), *C20orf20* (GenBank acc. no. NM_018270.4), *ADRM1* (GenBank acc. no. NM_007002.2), and *THIL* (GenBank acc. no. NM_198976.1).

In alternative preferred embodiments of the above methods, step (a) comprises the detection or identification of at least the marker genes *AURKA*/*STK6* (GenBank acc. no. NM_003600.2) and *TPX2* (GenBank acc. no. NM_012112.4). In specific embodiments, step (a) further comprises the detection or identification of any one or more of the marker genes *BCL2L1* (GenBank acc. no. NM_001191.2), *C20orf59* (GenBank acc. no. NM_022082.3), and *RNPC2* (GenBank acc. no. NM_004902.2).

In further alternative preferred embodiments of the above methods, step (a) comprises the detection or identification of at least:
(i) any one or both of the marker genes *PLK1* (GenBank acc. no. NM_005030.3) and *CCNF* (GenBank acc. no. NM_001761.2); and/or
(ii) any one or both of the marker genes *ADRM1* (GenBank acc. no. NM_007002.2) and *NUDT1* (GenBank acc. no. NM_002452.3); and/or
(iii) any one or more of the marker genes *SSSCA1* (GenBank acc. no. NM_006396.1), *ID3* (GenBank acc. no. NM_002167.3), and *LUM* (GenBank acc. no. NM_002345.3); and/or
(iv) any one or more of the marker genes *PDGFRB* (GenBank acc. no. NM_002609.3), *SPARC* (GenBank acc. no. NM_003118.2), and *DCN* (GenBank acc. no. NM_001920.3); and/or
(v) any one or more of the marker genes *AURKA*/*STK6* (GenBank acc. no. NM_003600.2), *TPX2* (GenBank acc. no. NM_012112.4), and *C20orf24*/*RIP5* (GenBank acc. no. NM_018840.2).

In a third aspect, the present invention relates to a pharmaceutical composition for preventing and/or treating an adenocarcinoma associated with a chromosomal aberration on chromosome 20q, comprising one or more agents, as defined herein above, for down-regulating gene expression of at least one marker gene, wherein the at least one marker gene is selected from the group consisting of *RNPC1* (GenBank acc. no. NM_017495.4), *TCFL5* (GenBank acc. no. NM_006602.2), *C20orf24*/*RIP5* (GenBank acc. no. NM_018840.2), *AURKA*/*STK6* (GenBank acc. no. NM_003600.2), *C20orf20* (GenBank acc. no. NM_018270.4), *ADRM1* (GenBank acc. no. NM_007002.2), *THIL* (GenBank acc. no. NM_198976.1), *TGIF2* (GenBank acc. no. NM_021809.5), *DPM1* (GenBank acc. no. NM_003859.1), *TOP1* (GenBank acc. no. NM_003286.2), *C20orf11* (GenBank acc. no. NM_017896.2), *YWHAB* (GenBank acc. no. NM_003404.3), *ZNF217* (GenBank acc. no. NM_006526.2), *BCL2L1* (GenBank acc. no. NM_001191.2), *DATF1*/*DIDO1* (GenBank acc. no. NM_022105.3), *CSE1L* (GenBank acc. no. NM_001316.2), *C20orf126*/*PDRG1* (GenBank acc. no. NM_030815.2), *TPX2* (GenBank acc. no. NM_012112.4), *C20orf59* (GenBank acc. no. NM_022082.3), *UBE2C* (GenBank acc. no. NM_007019.2), *NCOA3* (GenBank acc. no. NM_006534.2), *C20orf14*/*PRPF6* (GenBank acc. no. NM_012469.3), *C20orf44*/*bFZb* (GenBank acc. no. NM_018244.3), *RNPC2* (GenBank acc. no. NM_004902.2), *HM13* (GenBank acc. no. NM_030789.2), *SLCO4A1* (GenBank acc. no. NM_016354.3), *VAPB* (GenBank acc. no. NM_004738.3), *KIAA1847* (GenBank acc. no. NM_032527.3), *CDC91L1* (GenBank acc. no. NM_080476.4), *PXMP4* (GenBank acc. no. NM_007238.4), *DLGAP4* (GenBank acc. no. NM_014902.3), *PFDN4* (GenBank acc. no. NM_002623.3), *E2F1* (GenBank acc. no. NM_005225.2), *CYP24A1* (GenBank acc. no. NM_000782.4), *SRC* (GenBank acc. no. NM_005417.3), *BCAS4* (GenBank acc. no. NM_017843.3), *PLK1* (GenBank acc. no. NM_005030.3), *CCNF* (GenBank acc. no. NM_001761.2), *NUDT1* (GenBank acc. no. NM_002452.3), *SSSCA1* (GenBank acc. no. NM_006396.1), *ID3* (GenBank acc. no. NM_002167.3), *LUM* (GenBank acc. no. NM_002345.3), *PDGFRB* (GenBank acc. no. NM_002609.3), *SPARC* (GenBank acc. no. NM_003118.2), and *DCN* (GenBank acc. no. NM_001920.3).

In another preferred embodiment, the pharmaceutical composition is for the further use of preventing and/or treating a predisposition for developing an adenocarcinoma, a progression of an adenoma to an adenocarcinoma or a predisposition for a progression of an adenoma to an adenocarcinoma, the adenocarcinoma being associated with a chromosomal aberration on chromosome 20q.

Preferably, the chromosomal aberration on chromosome 20q is a chromosomal gain, particularly preferably at position 20q11.22-20q11.23 and/or at position 20q13.31-20q13.33.

In a forth aspect, the present invention relates to the use of one or more agents, as defined herein above, for down-regulating gene expression of at least one marker gene for the manufacture of a medicament for the prevention and/or treatment of an adenocarcinoma associated with a chromosomal aberration on chromosome 20q.

Other embodiments of the present invention will become apparent from the detailed description hereinafter.

### DESCRIPTON OF THE FIGURES

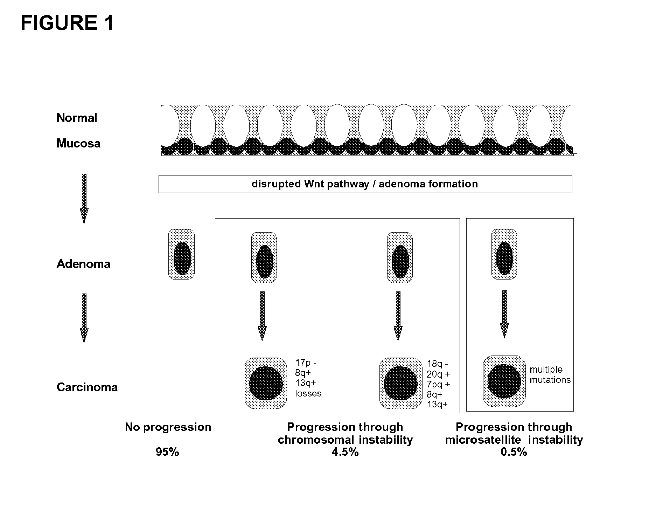

Fig. 1 schematically illustrates a model of colorectal cancer (CRC) progression from normal mucosa via an adenoma to an adenocarcinoma (adapted from Hermsen, M. et al. (2002) Gastroenterology 123, 1109-1119).

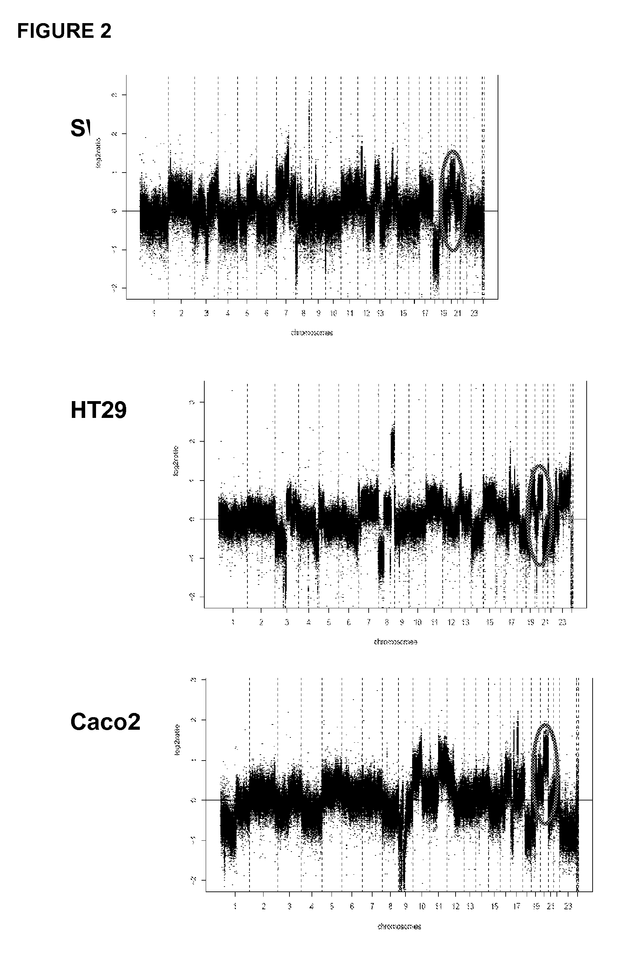

Fig. 2 shows a frequency plot of DNA copy number gains and losses as determined by BAC array comparative genomic hybridization in three CRC cell lines: SW480 (top panel), HT29 (medium panel), and Caco2 (bottom panel). Y-axis displays the fraction of tumors with either a gain (positive sign) or loss (negative sign) for all clones that are sorted by chromosome and base pair position. The respective chromosomal gains at 20q are encircled.

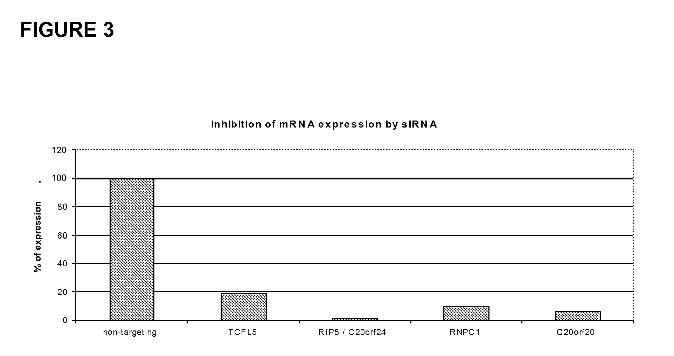

Fig. 3 shows the inhibition of expression of the four marker genes *TCFL5, C20orf24*/*RIP5, RNPC1*, and *C20orf20* in SW480 cells upon transfection with siRNAs. The respective target-specific siGENOME SMART-pool siRNAs (Dharmacon, Inc., Lafayette, CO, USA) were introduced into the CRC cells using DharmaFECT transfection reagents (also from Dharmacon, Inc.) according to the manufacturer's instructions. The siCONTROL Non-Targeting pool was used as a negative control, the *PLK1* siGENOME SMART-pool as a positive control. Expression levels were determined using quantitative RT-PCR. All four siRNAs employed reduced the expression level of the respective target gene by at least 80%.

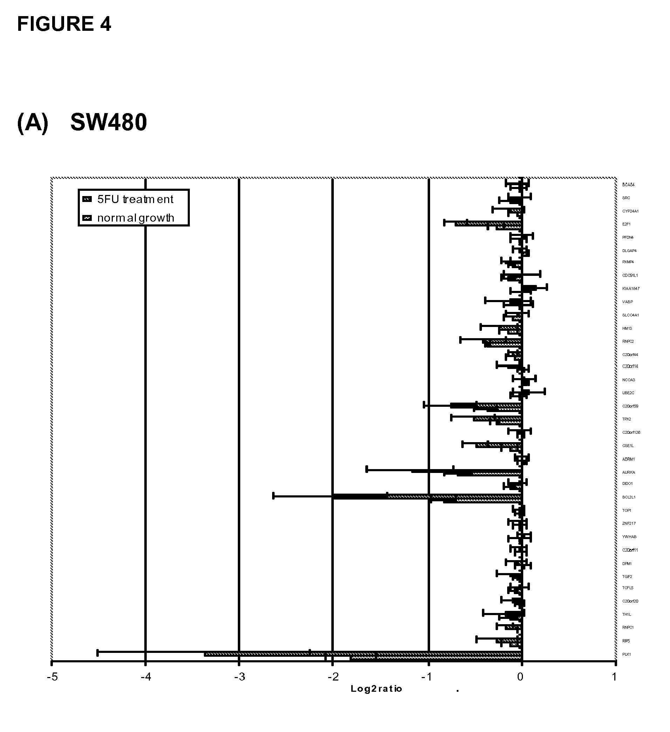

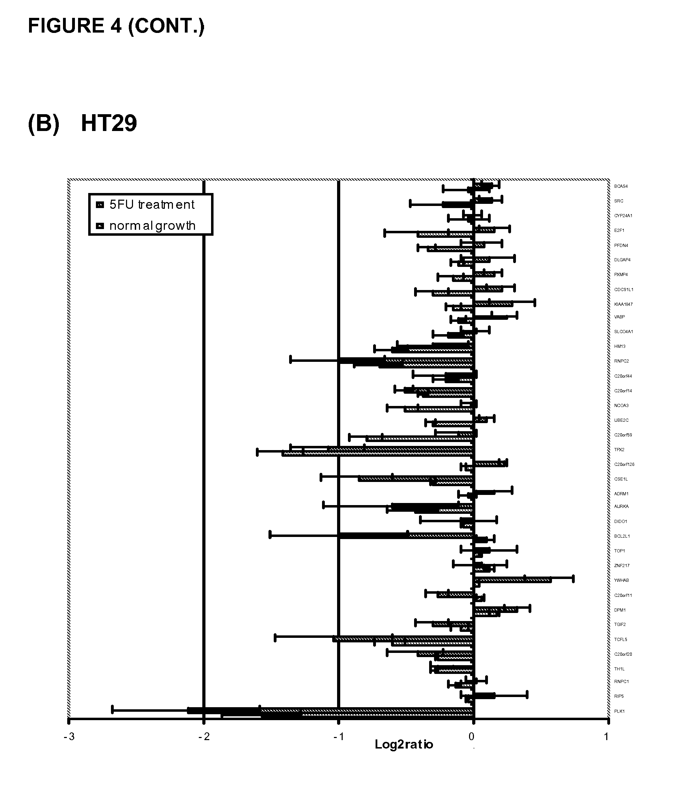

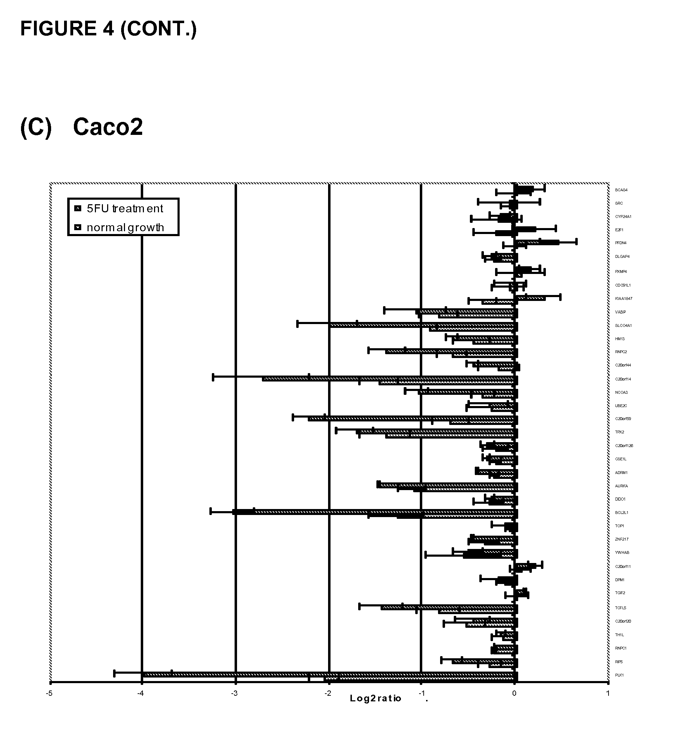

Fig. 4 depicts the effects of siRNA-mediated down-regulation of the expression of the 36 marker genes disclosed herein on cell numbers in SW480 (Fig. 4A), HT29 (Fig. 4B), and Caco2 (Fig. 4C) cells, respectively, as determined by the MTT (3-[4,5-dimethylthiazol-2-y]-2,5-diphenyltetrazolium bromide) cell viability assay (Plumb J.A. (2004) Methods Mol Med. 88, 165-169). Three days after transfection of the cells with the respective siRNAs the MTT-assay was performed by a two hours incubation of MTT followed by lysis of the formazan crystals by DMSO. Absorbance was measured with a spectrophotometer (TECAN) at 540 nm. The assay was performed under growth conditions (for determining cell proliferation) and upon 5 days treatment with 5-fluor-uracil (5FU) (for determining apoptosis). Five marker genes (i.e. *AURKA*/*STK6, TPX2, BCL2L1, C20orf59*/*RIP5*, and *RNPC2*) were shown to contribute to changes in cell numbers in all three CRC cell lines tested.

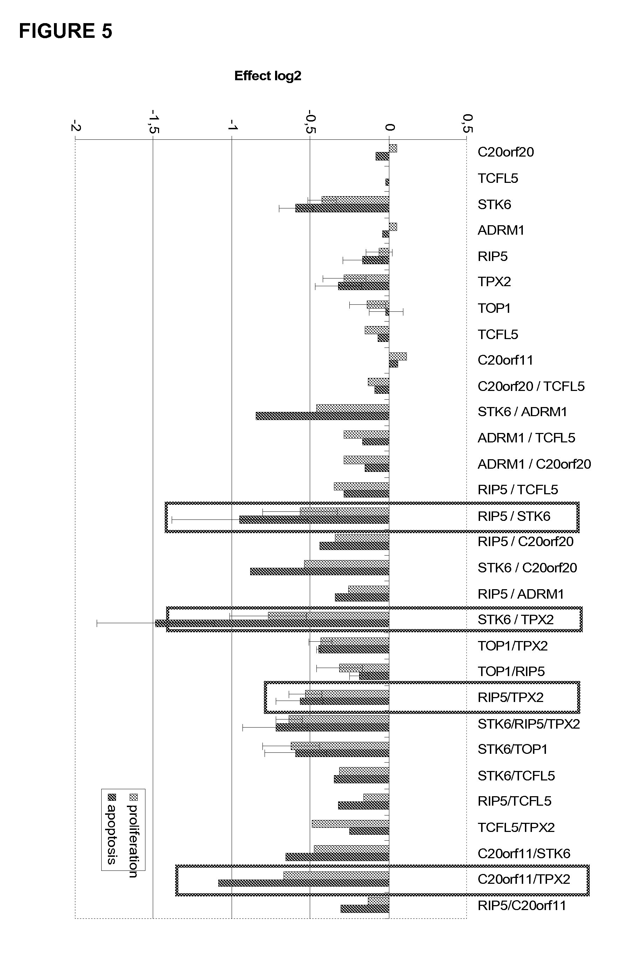

Fig. 5 depicts the effects of siRNA-mediated down-regulation of the expression of the 36 marker genes disclosed herein on cell numbers in SW480 cells, as determined by using the MTT viability assay described in Fig. 4 except that the cells were transfected with specific combinations of target gene-specific siRNAs. A particularly pronounced change of cell numbers under both growth conditions tested was observed for the following four combinations of markers genes: *C20orf24*/*RIP5* + *AURKA*/*STK6, AURKA*/*STK6* + *TPX2, C20orf24*/*RIP5* + *TPX2*, and *C20orf1* + *TPX2* (boxed).

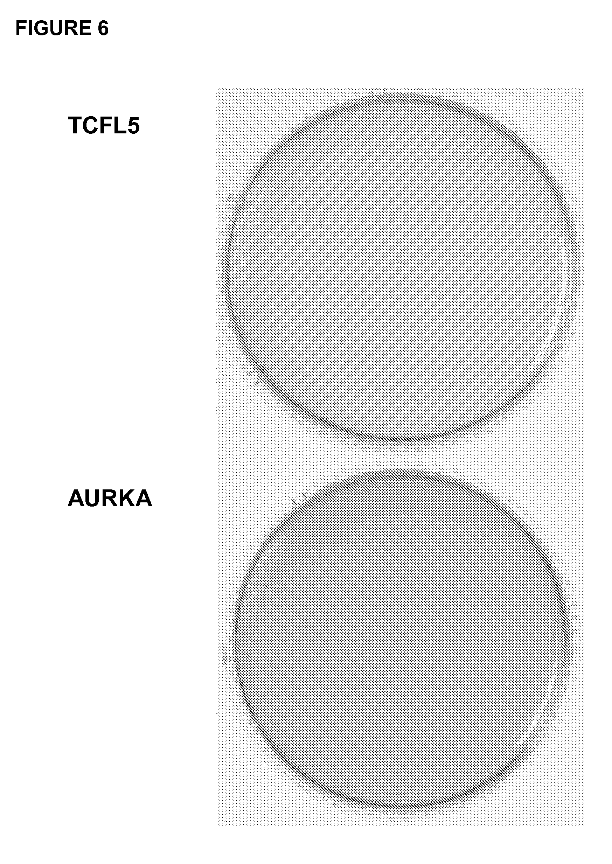

Fig. 6 depicts the effects of siRNA-mediated down-regulation of candidate gene expression (shown for *TCFL5* and *AURKA*) on anchorage-independent growth, as determined by using the soft agar colony formation assay. siRNA-transfected cells were allowed to grow in soft agarose (SeaPlaque Agarose purchased from Lonza Ltd., Basel, Switzerland) for 2-4 weeks. Shown is a photograph of two exemplary agar-plates. Digital images were taken of the soft agar dishes and analyzed using an appropriate software.

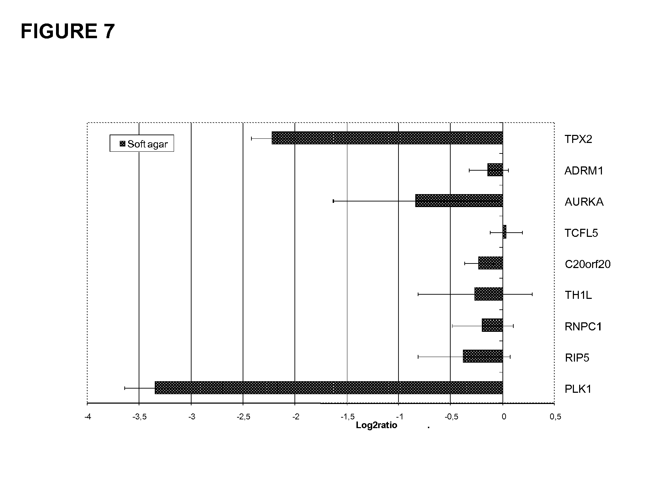

Fig. 7 depicts the results of a soft agar colony formation assay (anchorage-independent growth assay) in SW480 cells. The assay was performed as described in Fig.6. The *PLK1* siGENOME SMART-pool was used as a positive control. The results obtained show that the marker genes *AURKA*/*STK6* and *TPX2* have the most prominent effect on anchorage-independent growth

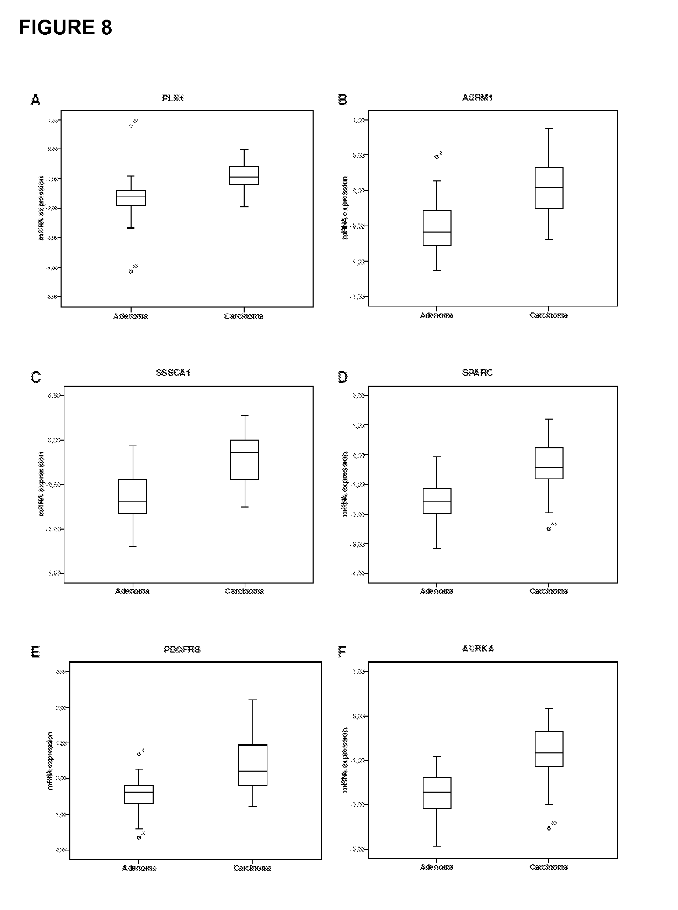

Fig. 8 depicts a comparison of mRNA expression levels for candidate biomarkers in colorectal adenomas and adenocarcinomas (CRC). The box-plots illustrate the mRNA expression levels (determined by oligonucleotide microarrays) of (a) *PLK1*, (b) *ADRM1*, (c) *SSSCA1*, (d) *SPARC*, (e) *PDGFRB*, and (f) *AURKA*/*STK6* in 37 colorectal adenomas and 31 CRC samples. The mRNA expression of the candidate biomarkers was significantly higher in CRC compared to adenomas (p-values < 1e-5).

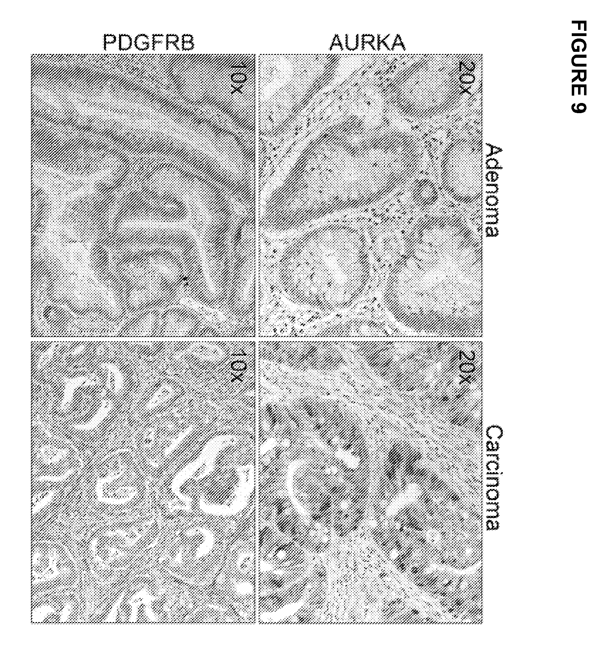

Fig. 9 depicts a comparison of protein expression levels for *AURKA*/*STK6* and *PDGFRB* in colorectal adenomas and adenocarcinomas (CRC) by means of immunohistochemistry. Both proteins are over-expressed in CRC. *AURKA*/*STK6* staining can be found in the epithelial cells, while *PDGFRB* expression is observed in the tumor stroma. A representative example of *AURKA*/*STK6* and *PDGFRB* staining is shown for both adenoma and CRC. Digital images were obtained using a 20x and 10x objective, respectively.

### DETAILED DESCRIPTION OF THE INVENTION

The present invention is based on the finding that targeting and specifically modifying the gene expression of a set of particular marker genes, whose elevated expression levels in tumor samples of an affected subject are indicative of an adenocarcinoma associated with a chromosomal aberration on chromosome 20q, enables a reliable diagnosis as well as an efficient prevention and/or treatment of such an adenoma or the predisposition for developing the same, even at an early disease state, that is, during the progression from an adenoma to an adenocarcinoma.

The present invention illustratively described in the following may suitably be practiced in the absence of any element or elements, limitation or limitations, not specifically disclosed herein.

The present invention will be described with respect to particular embodiments and with reference to certain drawings but the invention is not limited thereto but only by the claims. The drawings described are only schematic and are nonlimiting. In the drawings, the size of some of the elements may be exaggerated and not drawn on scale for illustrative purposes.

Where the term "comprising" is used in the present description and claims, it does not exclude other elements or steps. For the purposes of the present invention, the term "consisting of" is considered to be a preferred embodiment of the term "comprising of". If hereinafter a group is defined to comprise at least a certain number of embodiments, this is also to be understood to disclose a group, which preferably consists only of these embodiments.

Where an indefinite or definite article is used when referring to a singular noun e.g. "a" or "an", "the", this includes a plural of that noun unless something else is specifically stated.

The term "about" in the context of the present invention denotes an interval of accuracy that the person skilled in the art will understand to still ensure the technical effect of the feature in question. The term typically indicates deviation from the indicated numerical value of ± 10%, and preferably ± 5%.

Furthermore, the terms first, second, third, and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The following terms or definitions are provided solely to aid in the understanding of the invention. These definitions should not be construed to have a scope less than understood by a person of ordinary skill in the art.

The term "colorectal", as used herein, relates to the colon, the rectum and/or the appendix, i.e. the complete large intestine.

The term "cancer" (also referred to as "carcinoma"), as used herein, generally denotes any type of malignant neoplasm, that is, any morphological and/or physiological alterations (based on genetic re-programming) of target cells exhibiting or having a predisposition to develop characteristics of a carcinoma as compared to unaffected (healthy) wild-type control cells. Examples of such alterations may relate *inter alia* to cell size and shape (enlargement or reduction), cell proliferation (increase in cell number), cell differentiation (change in physiological state), apoptosis (programmed cell death) or cell survival. Hence, the term "colorectal cancer" refers to cancerous growths in the colon, rectum, and appendix.

The term "having a predisposition to develop cancer", as used herein, denotes any cellular phenotype being indicative for a pre-cancerous state, i.e. an intermediate state in the transformation of a normal cell into a tumor cell. In other words, the term denotes a state of risk of developing cancer.

The most common colorectal cancer (CRC) cell type is adenocarcinoma that accounts for about 95% of cases. Other types of CRC include *inter alia* lymphoma and squamous cell carcinoma.

The term "adenocarcinoma", as used herein, relates to a malignant neoplasm of epithelial cells of the colorectal mucosa. Typically, adenocarcinoma is a type of cancer that originates in glandular tissue. This tissue is part of a more general type of tissue known as epithelial tissue. Epithelial tissue includes skin, glands and a variety of other tissues lining/surrounding the cavities and organs of the body. Embryologically, the epithelium is derived from ectoderm, endoderm and mesoderm. In order to be classified as adenocarcinoma, the cells do not necessarily need to be part of a gland, as long as they have secretory properties. Hence, adenocarcinomas are also often referred to as "glandular cancer" or "glandular carcinoma". Highly differentiated adenocarcinomas tend to resemble the glandular tissue that they are derived from, while poorly differentiated may not.

The occurrence of a hyper-proliferative epithelium in the colon is the first step in cancer progression. This dysplastic epithelium turns into inflammatory adenomatous polyps, subsequently into adenomas, which are abnormal but benign neoplasms in the inner lining of the colon or rectum.

Thus, the term "adenoma", as used herein, thus relates to a benign epithelial neoplasm. Adenomas are usually well circumscribed and can be flat or polypoid. The neoplastic cells of benign adenomas do not infiltrate or invade adjacent tissue and rarely metastasize. Within the present invention, the term "adenoma" is understood as equivalent to "non-progressed adenoma". Malignant adenocarcinomas, however, invade other tissues and often metastasize given enough time to do so. Malignant cells are often characterized by progressive and uncontrolled growth. They can spread locally or through the blood stream and lymphatic system to other parts of the body. Particularly, hepatic metastases (i.e. metastases in the liver) are commonly found to be associated with adenocarcinomas. The occurrence of such metastases may be considered a late stage (or even a post-cancerous stage) of colorectal cancer.

The terms "progressed adenoma", as used herein, refer to an adenoma that harbors a focus of a cancer. This is also called a "malignant polyp". Colorectal adenomas are common in the elderly population, but only a small proportion of these pre-malignant tumors (estimated approximately 5%) progresses to malignant tumors. Such malignant tumors are herein referred to as (colorectal) "adenocarcinomas".

Adenocarcinomas may be classified according to the Dukes system (Dukes, C.E. (1932) J. Pathol. Bacteriol. 35, 323-325), which identifies the following stages: Dukes A - a tumour confined to the intestinal wall; Dukes B - a tumor invading through the intestinal wall; Dukes C - a tumor also involving the lymph node(s); and Dukes D - a tumor with distant metastasis.

Further definitions of term will be given in the following in the context of which the terms are used.

In a first aspect, the present invention relates to *an in vitro* method for preventing and/or treating an adenocarcinoma associated with a chromosomal aberration on chromosome 20q in a subject, the method comprising:
(a) identifying in one or more target cells of the subject at least one marker gene whose expression level(s) is/are elevated as compared to one or more control cells, wherein an elevated expression level of any one of the at least one marker gene in the one or more target cells is indicative of an adenocarcinoma associated with a chromosomal aberration on chromosome 20q in the subject; and
(b) modifying in the one or more target cells the gene expression of the at least one marker gene in such way that the elevated expression level(s) identified in step (a) are down-regulated,
wherein the at least one marker gene is selected from the group consisting of *RNPC1* (GenBank acc. no. NM_017495.4), *TCFL5* (GenBank acc. no. NM_006602.2), *C20orf24*/*RIP5* (GenBank acc. no. NM_018840.2), *AURKAlSTK6* (GenBank acc. no. NM_003600.2), *C20orf20* (GenBank acc. no. NM_018270.4), *ADRM1* (GenBank acc. no. NM_007002.2), *THIL* (GenBank acc. no. NM_198976.1), *TGIF2* (GenBank acc. no. NM_021809.5), *DPM1* (GenBank acc. no. NM_003859.1), *TOP1* (GenBank acc. no. NM_003286.2), *C20orf11* (GenBank acc. no. NM_017896.2), *YWHAB* (GenBank acc. no. NM_003404.3), *ZNF217* (GenBank acc. no. NM_006526.2), *BCL2L1* (GenBank acc. no. NM_001191.2), *DATF1*/*DIDO1* (GenBank acc. no. NM_022105.3), *CSE1L* (GenBank acc. no. NM_001316.2), *C20orf126*/*PDRG1* (GenBank acc. no. NM_030815.2), *TPX2* (GenBank acc. no. NM_012112.4), *C20orf59* (GenBank acc. no. NM_022082.3), *UBE2C* (GenBank acc. no. NM_007019.2), *NCOA3* (GenBank acc. no. NM_006534.2), *C20orf14*/*PRPF6* (GenBank acc. no. NM_012469.3), *C20orf44*/*bFZb* (GenBank acc. no. NM_018244.3), *RNPC2* (GenBank acc. no. NM_004902.2), *HM13* (GenBank acc. no. NM_030789.2), *SLCO4A1* (GenBank acc. no. NM_016354.3), *VAPB* (GenBank acc. no. NM_004738.3), *KIAA1847* (GenBank acc. no. NM_032527.3), *CDC91L1* (GenBank acc. no. NM_080476.4), *PXMP4* (GenBank acc. no. NM_007238.4), *DLGAP4* (GenBank acc. no. NM_014902.3), *PFDN4* (GenBank acc. no. NM_002623.3), *E2F1* (GenBank acc. no. NM_005225.2), *CYP24A1* (GenBank acc. no. NM_000782.4), *SRC* (GenBank acc. no. NM_005417.3), *BCAS4* (GenBank acc. no. NM_017843.3), *PLK1* (GenBank acc. no. NM_005030.3), *CCNF* (GenBank acc. no. NM_001761.2), *NUDT1* (GenBank acc. no. NM_002452.3), *SSSCA1* (GenBank acc. no. NM_006396.1), *ID3* (GenBank acc. no. NM_002167.3), *LUM* (GenBank acc. no. NM_002345.3), *PDGFRB* (GenBank acc. no. NM_002609.3), *SPARC* (GenBank acc. no. NM_003118.2), and *DCN* (GenBank acc. no. NM_001920.3).

In a second aspect, the present invention relates to *an in vitro* method for diagnosing in a subject an adenocarcinoma associated with a chromosomal aberration on chromosome 20q, the method comprising:
(a) detecting in one or more target cells of the subject the expression level(s) of at least one marker gene; and
(b) comparing the expression level(s) obtained in step (a) to that/those obtained in one or more control cells,
wherein an elevated expression level of any one of the at least one marker gene in the one or more target cells as compared to the one or more control cells is indicative of an adenocarcinoma associated with a chromosomal aberration on chromosome 20q in the subject; and
wherein the at least one marker gene is selected from the group of marker genes as defined herein above.

All 45 marker genes referred to above represent human sequences. All these marker genes are known in the art. Their respective nucleic acid sequences (encoding the corresponding mRNAs) are deposited in GenBank, the NIH genetic sequence database (Nucl. Acids Res. (2008) 36, D25-D30; http://www.ncbi.nlm.nih.gov/Genbank/; release 169.0), having the following accession numbers (herein also referred to as "acc. no."):

| | |
|---|---|
| *RNPC1* | (GenBank acc. no. NM_017495.4) (SEQ ID NO:1) |
| *TCFL5* | (GenBank acc. no. NM_006602.2) (SEQ ID NO:2) |
| *C20orf24*/*RIP5* | (GenBank acc. no. NM_018840.2) (SEQ ID NO:3) |
| *AURKA*/*STK* | (GenBank acc. no. NM_003600.2) (SEQ ID NO:4) |
| *C20orf20* | (GenBank acc. no. NM_018270.4) (SEQ ID NO:5) |
| *ADRM1* | (GenBank acc. no. NM_007002.2) (SEQ ID NO:6) |
| *TH1* | (GenBank acc. no. NM_198976.1) (SEQ ID NO:7) |
| *TGIF2* | (GenBank acc. no. NM_021809.5) (SEQ ID NO:8) |
| *DPM1* | (GenBank acc. no. NM_003859.1) (SEQ ID NO:9) |
| *TOP1* | (GenBank acc. no. NM_003286.2) (SEQ ID NO:10) |
| *C20orf11* | (GenBank acc. no. NM_017896.2) (SEQ ID NO:11) |
| *YWHAB* | (GenBank acc. no. NM_003404.3) (SEQ ID NO:12) |
| *ZNF217* | (GenBank acc. no. NM_006526.2) (SEQ ID NO:13) |
| *BCL2L1* | (GenBank acc. no. NM_001191.2) (SEQ ID NO:14) |
| *DATF1*/*DIDO1* | (GenBank acc. no. NM_022105.3) (SEQ ID NO:15) |
| *CSE1L* | (GenBank acc. no. NM_001316.2) (SEQ ID NO:16) |
| *C20orf126*/*PDRG* | (GenBank acc. no. NM_030815.2) (SEQ ID NO:17) |
| *TPX2* | (GenBank acc. no. NM_012112.4) (SEQ ID NO:18) |
| *C20orf59* | (GenBank acc. no. NM_022082.3) (SEQ ID NO:19) |
| *UBE2C* | (GenBank acc. no. NM_007019.2) (SEQ ID NO:20) |
| *NCOA3* | (GenBank acc. no. NM_006534.2) (SEQ ID NO:21) |
| *C20orf14*/*PRPF6* | (GenBank acc. no. NM_012469.3) (SEQ ID NO:22) |
| *C20orf44*/*bFZb* | (GenBank acc. no. NM_018244.3) (SEQ ID NO:23) |
| *RNPC2* | (GenBank acc. no. NM_004902.2) (SEQ ID NO:24) |
| *HM13* | (GenBank acc. no. NM_030789.2) (SEQ ID NO:25) |
| *SLC04A1* | (GenBank acc. no. NM_016354.3) (SEQ ID NO:26) |
| *VAPB* | (GenBank acc. no. NM_004738.3) (SEQ ID NO:27) |
| *KIAA1847* | (GenBank acc. no. NM_032527.3) (SEQ ID NO:28) |
| *CDC91L1* | (GenBank acc. no. NM_080476.4) (SEQ ID NO:29) |
| *PXMP4* | (GenBank acc. no. NM_007238.4) (SEQ ID NO:30) |
| *DLGAP4* | (GenBank acc. no. NM_014902.3) (SEQ ID NO:31) |
| *PFDN4* | (GenBank acc. no. NM_002623.3) (SEQ ID NO:32) |
| *E2F1* | (GenBank acc. no. NM_005225.2) (SEQ ID NO:33) |
| *CYP24A1* | (GenBank acc. no. NM_000782.4) (SEQ ID NO:34) |
| *SRC* | (GenBank acc. no. NM_005417.3) (SEQ ID NO:35) |
| *BCAS4* | (GenBank acc. no. NM_017843.3) (SEQ ID NO:36) |
| *PLK1* | (GenBank acc. no. NM_005030.3) (SEQ ID NO:37) |
| *CCNF* | (GenBank acc. no. NM_001761.2) (SEQ ID NO:38) |
| *NUDT1* | (GenBank acc. no. NM_002452.3) (SEQ ID NO:39) |
| *SSSCA1* | (GenBank acc. no. NM_006396.1) (SEQ ID NO:40) |
| *ID3* | GenBank acc. no. NM_002167.3) (SEQ ID NO:41) |
| *LUM* | (GenBank acc. no. NM_002345.3) (SEQ ID NO:42) |
| *PDGFRB* | (GenBank acc. no. NM_002609.3) (SEQ ID NO:43) |
| *SPARC* | (GenBank acc. no. NM_003118.2) (SEQ ID NO:44) |
| *DCN* | (GenBank acc. no. NM_001920.3) (SEQ ID NO:45) |

The genes or loci may also be designated by synonyms, which are known to the person skilled in the art and can be derived, for example, from the above mentioned database entries. These synonyms are also encompassed by the embodiments of the present invention.

The term "marker gene", as used herein, is a gene whose expression level is elevated in one or more target cells as compared to one or more control cells.

The target cells and/or control cells employed in the present invention are mammalian cells that may be of human or non-human origin. However, the invention is typically performed with human cells. The term "one or more cells", as used herein, is to be understood not only to include individual cells but also tissues, organs, and organisms. The term "target cell", as used herein, refers to a cell being at least supposed to exhibit or to have a predisposition to develop colorectal cancer, whereas the term "control cell" typically denotes a (healthy) wild-type cell not having characteristics of such a cancerous phenotype. However, in some applications, for example, when comparing cells exhibiting different cancerous or pre-cancerous states, the cells having the less severe disease characteristics are typically considered the "control cells".

Typically, the target and control cells used are derived from biological samples collected from the subjects to be treated. Furthermore, in order to corroborate the data obtained "comparative samples" may also be collected from subjects having a given known disease state.

The biological samples may include body tissues and fluids, such as blood, sputum, and urine. Furthermore, the biological sample may contain a cell extract derived from or a cell population including an epithelial cell, preferably a cancerous epithelial cell or an epithelial cell derived from tissue suspected to be cancerous. Even more preferably the biological sample comprises a cell population derived from a glandular tissue. Furthermore, the cell may be purified from the obtained body tissues and fluids if necessary, and then used as the biological sample.

According to the present invention, the expression level of the nucleic acid markers of the present invention is determined in subject-derived biological sample(s). The sample used for in the *in vitro* methods of the present invention should generally be collected in a clinically acceptable manner, preferably in a way that nucleic acids (in particular RNA) or proteins are preserved. The samples to be analyzed are typically of colorectal origin (e.g., biopsies or resections). Furthermore, colorectal adenocarcinoma cells may migrate into other tissues. Hence, blood and other types of sample can be used as well.

The respective terms "detecting" and "identifying", as used herein, may be interpreted in the sense of "selecting" at least one marker gene from the particular group of marker genes specified herein. The selection may vary, for example, depending on treatment modalities, including therapeutic intervention, diagnostic criteria such as disease stages, and disease monitoring and surveillance for the disease in the subject to be treated, from whom the sample to be analyzed is derived. Additionally, the term "identifying" also encompasses the determination of the extent of the difference between respective expression levels of the at least one marker genes in the one or more target cells and the one or more control cells, that is, "comparing" the results obtained.

Generally, the determination of the expression level of marker genes in a sample may be accomplished by any means known in the art. In preferred embodiments of the methods of the invention, the determination of the expression level(s) of the at least one marker gene is (are) accomplished by any one or more of the methods selected from the group consisting of:
(i) detecting a mRNA encoded by the marker gene(s);
(ii) detecting a protein encoded by the marker gene(s); and
(iii) detecting a biological activity of a protein encoded by the marker gene(s).

For example, expression levels of the marker genes may be assessed by separation of nucleic acid molecules (e.g. RNA or cDNA) obtained from the sample in agarose gels or polyacrylamide gels followed by hybridization with marker gene specific oligonucleotide probes. Alternatively, the difference in expression level may be determined by the labeling of nucleic acid obtained from the sample followed by separation on a sequencing gel. Nucleic acid samples are placed on the gel such that patient and control or standard nucleic acid are in adjacent lanes. Comparison of expression levels is accomplished visually or by means of a densitometer.

Methods for the detection of mRNA are known to the person skilled in the art or can be derived from standard textbooks, for example, Sambrook et al., Molecular Cloning: A Laboratory Manual, 2001, Cold Spring Harbor Laboratory Press. Typically, Northern blot analysis may be used for such a purpose. Preferably, mRNA may be detected in a microarray approach, e.g., sample nucleic acids derived from subjects to be tested are processed and labeled, preferably with a fluorescent label. Subsequently, such nucleic acid molecules are used in a hybridization approach with immobilized capture probes corresponding to one, more or all of the marker genes of the present invention. Suitable means for carrying out microarray analyses are known to the person skilled in the art. Typically, microarray based expression profiling may be carried out, for example, by the method as disclosed in Microarray Biochip Technology (Schena M., Eaton Publishing, 2000). A DNA array comprises immobilized high-density probes to detect a number of genes. The probes on the array are complementary to one or more parts of the sequence of a marker gene, or to the entire coding region of the marker gene. In the present invention, any type of polynucleotide can be used as probes for the DNA array. Typically, cDNAs, PCR products, and oligonucleotides are useful as probes. Thus, expression levels of a plurality of genes can be estimated at the same time by a single-round analysis.

A DNA array-based detection method generally comprises the following steps: (1) Isolating mRNA from a sample and optionally converting the mRNA to cDNA, and subsequently labeling this RNA or cDNA. Methods for isolating RNA, converting it into cDNA and for labeling nucleic acids are described in manuals for micro array technology. (2) Hybridizing the nucleic acids from step 1 with probes for the marker genes. The nucleic acids from a sample can be labeled with a dye, such as the fluorescent dyes Cy3 (red) or Cy5 (blue). Typically, a control sample is labeled with a different dye. (3) Detecting the hybridization of the nucleic acids from the sample with the probes and determining at least qualitatively, and more particularly quantitatively, the amounts of mRNA in the sample for the different marker genes investigated. The difference in the expression level between sample and control can be estimated based on a difference in the signal intensity. These can be measured and analyzed by appropriate software such as the software provided, for example, by Affymetrix.

There is no limitation on the number of probes corresponding to the marker genes used, which are spotted on a DNA array. Also, a marker gene can be represented by two or more probes, the probes hybridizing to different parts of a gene. Probes are designed for each selected marker gene. Such a probe is typically an oligonucleotide comprising 5-50 nucleotide residues. Longer DNAs can be synthesized by PCR or chemically. Methods for synthesizing such oligonucleotides and applying them on a substrate are well known in the field of micro-arrays. Genes other than the marker genes may be also spotted on the DNA array. For example, a probe for a gene whose expression level is not significantly altered may be spotted on the DNA array to normalize assay results or to compare assay results of multiple arrays or different assays.

The detection of proteins encoded by the marker gene or genes may be carried out via antibody detection techniques known in the art. For the analysis at the protein level, every marker gene described in the present invention can in principle be used, although some proteins may be less suitable, because of factors such as limited solubility, very high or small molecular weight or extreme isoelectric point. Determination of expression level of a marker gene at the protein level can be accomplished, for example, by the separation of proteins from a sample on a polyacrylamide gel, followed by identification of a specific marker gene-derived protein using appropriate antibodies in a Western blot analysis. Alternatively, proteins can be separated by two-dimensional gel electrophoresis systems. Two-dimensional gel electrophoresis is well known in the art and typically involves isoelectric focusing along a first dimension followed by SDS-PAGE electrophoresis along a second dimension. The analysis of two-dimensional (2D) SDS-PAGE gels can be performed by determining the intensity of protein spots on the gel, or can be performed using immune detection. In other embodiments, protein samples are analyzed by mass spectroscopy.

Alternatively, antibodies directed against the proteins encoded by any one of the marker genes of the present invention may be generated. Preferably, monoclonal antibodies are obtained. Subsequently, such specifically binding antibodies may be used to detect the proteins encoded by the marker genes. In a specific embodiment the antibodies may be stained with a dye or be label. Alternatively, antibodies binding proteins encoded by the marker genes may also be placed on a support and be immobilized. Proteins derived from samples or tissues to be analyzed may subsequently be mixed with the antibodies. A detection reaction may then be carried out, e.g. with a second specific antibody.

In addition, ligands to the proteins encoded by the marker genes of the present invention may be used for a detection of said proteins. Such ligands may preferably be label in order to allow the detection of a protein-ligand interaction.

The detection of a biological activity of a protein encoded by preferred marker genes of the present invention may be carried out by employing molecular or enyzmatic assays specific to the corresponding functions of the marker genes. These functions may be derived from the GenBank database entries mentioned in the context of the marker genes of the present invention or from corresponding literature, e.g. the citations mentioned herein below.

For instance, *TCFL5* is a transcription factor (Siep, M. et al. (2004) Nucleic Acids Res. 32, 6425-6436), *C20orf20* is a factor being involved in transcriptional regulation (Cai, Y. et al. (2003) J. Biol. Chem. 278, 42733-42736). *TH1L* product is involved in regulation of A-Raf kinase (Liu, W. et al. (2004) J. Biol. Chem. 279, 10167-10175). *ADRM1* encodes for a putative cell adhesion molecule that recently was shown to be component of the 26S proteosome (Jorgensen, J.P. et al. (2006) J. Mol. Biol. 360, 1043-1052). *RNPC1* product is predicted to bind to RNA, based on sequence motifs and *C20orf24*/*RIP5* interacts with Rab-5. *AURKA*/*STK6* has been well characterized and is involved in cell cycle regulation. It has been shown to be amplified in CRC (Bischoff, J.R. et al. (1998) EMBO J. 17, 3052-3065) and its over-expression induces centrosome amplification, aneuploidy and transformation *in vitro* (Zhou, H. et al. (1998) Nat. Genet. 20, 189-193). Moreover, inhibiting *AURKAlSTK6* by RNA interference lead to growth suppression of human pancreatic cancer cells (Hata, T. et al. (2005) Cancer Res. 65, 2899-2905). Knocking down *TCFL5* resulted in suppression of the number of multicellular HT29 tumor spheroids, supporting its role in cancer development (Dardousis, K. et al. (2007) Mol. Ther. 15, 94-102). *BCL2L1* represents an apoptotic switch in cancer development and therapy (Adams, J.M. and Cory, S. (2007) Oncogene 26, 1324-1337). *TPX2* is a microtubule-associated protein, which localizes the kinase to spindle tubules (Bayliss, R. et al. (2003) Mol. Cell 12, 851-862). *C20orf24* acts a vesicular nucleotide transporter and is a critical factor for ATP storage (Sawada, K. et al. (2008) Proc. Natl. Acad. Sci. USA 105, 5683-5686. Finally, RNPC2 encodes an RNA binding protein and possible splicing factor (Jung, D.J. et al. (2002) J. Biol. Chem. 277, 1229-11234). A person skilled in the art could envisage suitable and appropriate assays in order to test for the corresponding functions. For example, such assays may comprise kinase assays (e.g., for the detection of the biological function of *AURKA*/*STK6*) or transcription or transcription regulation assays (e.g., for the detection of the biological function of *TCFL5*) or RNA interaction assays (e.g., for the detection of the biological function of *RNPC1*).

As an alternative to experimental analysis, the respective measure of the expression level in the one or more control cells may be determined by a statistical method based on the results obtained by analyzing previously determined expression level(s) of the marker genes in samples from subjects whose disease state is known. Furthermore, the control level can be derived from a database of expression patterns from previously tested subjects or cells. Moreover, the expression level of the marker genes of the present invention in a biological sample to be tested may be compared to multiple control levels, whose control levels are determined from multiple reference samples. It is preferred to use a control level determined from a reference sample derived from a tissue type similar to that of the patient-derived biological sample. It is particularly preferred to use sample(s) derived from a subject/subjects whose disease state is non-cancerous or derived from a subject/subjects whose disease state is non-cancerous as defined herein above. In another embodiment of the present invention, the control level can be determined from a reference sample derived from a subject who has been diagnosed to suffer from adenoma.

In addition, the difference between the respective expression levels in the one or more target and control cells can be normalized to the expression level of further control nucleic acids, e.g. housekeeping genes whose expression levels are known not to differ depending on the cancerous or non-cancerous state of the cell. Exemplary control genes include *inter alia* β-actin, glycerinaldehyde 3-phosphate dehydrogenase, and ribosomal protein P1.

Within the context of the present invention, it is preferred to use the standard value of the expression levels of any of the marker genes disclosed herein in a population with a known disease state. The standard value may be obtained by any method known in the art. For example, a range of mean ± 2 SD (standard deviation) or mean ± 3 SD may be used as standard value.

The expression level of any one the maker genes disclosed herein in the one or more target genes is increased (elevated) compared to the respective expression level in the one or more control cells, and thus indicative of are indicative of an adenocarcinoma associated with a chromosomal aberration on chromosome 20q or variation of this indication as described herein below, i.e. a predisposition to adenocarcinoma associated with a chromosomal aberration on chromosome 20q, a progression of adenoma to adenocarcinoma associated with a chromosomal aberration on chromosome 20q or a predisposition for a progression of adenoma to adenocarcinoma associated with a chromosomal aberration on chromosome 20q.

The expression level may preferably be averaged over the expression level of both marker genes and/or normalized with an appropriate control as described herein above and herein below.

The term "elevated expression level" in the context of the present invention denotes an increase of the expression level. Expression levels are deemed to be "elevated" when the gene expression increases by, for example, 5%, 6%, 7%, 8%, 9%, 10%, 15%, 20%, 25%, 30%, 40%, 50%, or more than 50% from a control level, or at least 0.1 fold, at least 0.2 fold, at least 1 fold, at least 2 fold, at least 5 fold, or at least 10 fold or more in comparison to a control level.

In the context of the present invention, the term "diagnosing" is intended to encompass predictions and likelihood analysis. The method of the invention is intended to be used clinically in making decisions concerning treatment modalities, including therapeutic intervention, diagnostic criteria such as disease stages, and disease monitoring and surveillance for the disease. According to the present invention, an intermediate result for examining the condition of a subject may be provided. Such intermediate result may be combined with additional information to assist a doctor, nurse, or other practitioner to diagnose that a subject suffers from the disease. Alternatively, the present invention may be used to detect cancerous cells in a subject-derived tissue, and provide a doctor with useful information to diagnose that the subject suffers from the disease.

The term "at least one marker genes", as used herein, relates to any one, any subgroup of any two or more (i.e. any two, any three, any four, any five, any six, any seven, any eight, any nine, any ten, and so forth) or all of the 45 marker genes disclosed herein

In preferred embodiments of the above methods, step (a) comprises the detection or identification of at least the marker genes *RNPC1* (GenBank acc. no. NM_017495.4) and *TCFL5* (GenBank acc. no. NM_006602.2), that is, *RNPC1* and *TCFL5* in combination with any one or more of the remaining other 43 marker genes disclosed herein.

In specific preferred embodiments, step (a) comprises the detection or identification of at least the marker genes *RNPC1* and *TCFL5* in combination with any one or more of the marker genes *C20orf24*/*RIP5* (GenBank acc. no. NM_018840.2), *AURKA*/*STK6* (GenBank acc. no. NM_003600.2), *C20orf20* (GenBank acc. no. NM_018270.4), *ADRM1* (GenBank acc. no. NM_007002.2), and *THIL* (GenBank acc. no. NM_198976.1), that is, the combinations (*RNPC1, TCFL5*, and *C20orf24*/*RIP5*) or (*RNPC1, TCFL5*, and *AURKA*/*STK6*) or (*RNPC1, TCFL5*, and *C20orf20*) or (*RNPC1, TCFL5*, and *ADRM1*) or (*RNPC1, TCFL5*, and *TH1L*) or (*RNPC1, TCFL5*, *C20orf24*/ *RIP5,* and *AURKA*/*STK6*) or (*RNPC1, TCFL5, C20orf24*/*RIP5*, and *C20orf20*) or (*RNPC1, TCFL5, C20orf24*/*RIP5*, and *ADRM1*) or (*RNPC1, TCFL5, C20orf24*/*RIP5*, and *TH1L*) or (*RNPC1, TCFL5, AURKA*/*STK6*, and *C20orf20*) or (*RNPC1, TCFL5, AURKA*/*STK6*, and *ADRM1*) *or* (*RNPC1, TCFL5, AURKA*/*STK6*, and *TH1L*) or (*RNPC1, TCFL5, C20orf20*, and *ADRM1*) or (*RNPC1, TCFL5, C20orf20*, and *TH1L*) or (*RNPC1, TCFL5, C20orf24*/*RIP5, AURKA*/*STK6*, and *C20orf20*) or (*RNPC1, TCFL5, C20orf24*/*RIP5, AURKA*/*STK6*, and *ADRM1*) or (*RNPC1, TCFL5, C20orf24*/*RIP5, AURKA*/*STK6*, and *TH1L*) or (*RNPC1, TCFL5, C20orf24*/*RIP5, AURKA*/*STK6, C20orf20*, and *ADRM1*) or (*RNPC1, TCFL5, C20orf24*/*RIP5, AURKA*/*STK6, C20orf20*, and *TH1L*) or (*RNPC1, TCFL5, C20orf24*/*RIP5, AURKA*/*STK6, C20orf20, ADRM1*, and *TH1L*). Each of these subgroups may be further combined with any one or more of the respective remaining marker molecules disclosed herein.

In alternative preferred embodiments of the above methods, step (a) comprises the detection or identification of at least the marker genes *AURKA*/*STK6* (GenBank acc. no. NM_003600.2) and *TPX2* (GenBank acc. no. NM_012112.4), that is, *AURKA*/*STK6* and *TPX2* in combination with any one or more of the remaining other 43 marker genes disclosed herein.

In specific preferred embodiments, step (a) comprises the detection or identification of at least the marker genes *AURKA*/*STK6* and *TPX2* in combination with any one or more
of the marker genes *BCL2L1* (GenBank acc. no. NM_001191.2), *C20orf59* (GenBank acc. no. NM_022082.3), and *RNPC2* (GenBank acc. no. NM_004902.2), that is, the combinations (*AURKA*/*STK6, TPX2*, and *BCL2L1*) or (*AURKA*/*STK6, TPX2*, and *C20orf59*) or (*AURKA*/*STK6, TPX2*, and *RNPC2*) or (*AURKA*/*STK6, TPX2, BCL2L1*, and *C20orf59*) or (*AURKA*/*STK6, TPX2, BCL2L1*, and *RNPC2*) or (*AURKA*/*STK6, TPX2, BCL2L1, C20orf59*, and *RNPC2*). Each of these subgroups may be further combined with any one or more of the respective remaining marker molecules disclosed herein.

In another specific embodiment, step (a) comprises the detection or identification of at least the marker genes *C20orf24*/*RIP5* and *AURKA*/*STK6,* that is, *C20orf24*/*RIP5* and *AURKA*/*STK6* in combination with any one or more of the remaining other 43 marker genes disclosed herein, preferably with at least any one or more of the marker genes *RNPC1, TCFL5, C20orf20, ADRM1, THIL, TPX2, BCL2L1, C20orf59*, and *RNPC2.*

In yet another specific embodiment, step (a) comprises the detection or identification of at least the marker genes *C20orf24*/*RIP5* and *TPX2,* that is, *C20orf24*/*RIP5* and *TPX2* in combination with any one or more of the remaining other 43 marker genes disclosed herein., preferably with at least any one or more of the marker genes *RNPC1, TCFL5, AURKA*/*STK6, C20orf20, ADRM1, TH1L, BCL2L1, C20orf59*, and *RNPC2* disclosed herein.

In yet another specific embodiment, step (a) comprises the detection or identification of at least the marker genes *C20orf11* and *TPX2,* that is, *C20orf11* and *TPX2* in combination with any one or more of the other 43 marker genes disclosed herein., preferably with at least any one or more of the marker genes *RNPC1, TCFL5, C20orf24*/*RIP5, AURKA*/*STK6, C20orf20*, *ADRM1, THIL, BCL2L1, C20orf59*, and *RNPC2.*

In further alternative preferred embodiments of the above methods, step
(a) comprises the detection or identification of at least:
   (i) any one or both of the marker genes *PLK1* (GenBank acc. no. NM_005030.3) and *CCNF* (GenBank acc. no. NM_001761.2); and/or
   (ii) any one or both of the marker genes *ADRM1* (GenBank acc. no. NM_007002.2) *a*nd *NUDT1* (GenBank acc. no. NM_002452.3); and/or
   (iii) any one or more of the marker genes *SSSCA1* (GenBank acc. no. NM_006396.1), *ID3* (GenBank acc. no. NM_002167.3), and *LUM* (GenBank acc. no. NM_002345.3); and/or
   (iv) any one or more of the marker genes *PDGFRB* (GenBank acc. no. NM_002609.3), *SPARC* (GenBank acc. no. NM_003118.2), and *DCN* (GenBank acc. no. NM_001920.3); and/or
   (v) any one or more of the marker genes *AURKA*/*STK6* (GenBank acc. no. NM_003600.2), *TPX2* (GenBank acc. no. NM_012112.4), and *C20orf24*/*RIP5* (GenBank acc. no. NM_018840.2).
Any biological processes are controlled by the coordinate expression of sets of genes that are involved in the regulation of particular pathways. Several pathway analysis programs have been developed, such as Gene Set Enrichment Analysis (GSEA; Mootha, V.K. et al. (2003) Nat. Genet. 34, 267-273; Subramanian, A. et al. (2005) Proc. Natl. Acad. Sci. USA 102, 15545-15550). GSEA makes use of the fact that changes in biological characteristics require coordinate variation in expression of groups of genes (i.e. gene-sets) that regulate biological activity. While absolute changes in expression levels of the majority of individual genes are often modest to small and do not reach statistical significance, GSEA allows to estimate group-wise variation in expression of pre-defined gene-sets, indicative for pathway activity levels.

Analyses of carcinogenic pathways whose activities are increased during the malignant transformation of colorectal adenomas into adenocarcinomas revealed that (i) any one of the marker genes *PLK1* and *CCNF* is indicative for the aspect of tumor progression; (ii) any one of the marker genes *ADRM1* and *NUDT1* is indicative for tumor differentiation; (iii) any one of the marker genes *SSSCA1, ID3*, and *LUM* is indicative for tumor stroma activation; (iv) any one of the marker genes *PDGFRB, SPARC*, and *DCN* is indicative for tumor cell invasion; and (v) any one of the marker genes *AURKA*/*STK6, TPX2*, and *C20orf24*/*RIP5* is indicative for chromosomal instability.

In specific preferred embodiments, step (a) comprises the detection or identification of at least: (i) the marker genes *PLK1* and *CCNF* (i.e. the combination of both); and/or (ii) the marker genes *ADRM1* and *NUDT1* (i.e. the combination of both); and/or (iii) the marker genes *SSSCA1, ID3*, and *LUM* (i.e. the combination of all three); and/or (iv) the marker genes *PDGFRB, SPARC*, and *DCN* (i.e. the combination of all three); and/or (v) the marker genes *AURKA*/*STK6, TPX2*, and *C20orf24*/*RIP5* (i.e. the combination of all three), that is, the combinations [(*PLK1* and *CCNF*)] or [(*ADRM1* and NUDT1)] or [(SSSCA1, *ID3,* and *LUM*)] or [(*PDGFRB, SPARC*, and *DCN*)] or [(*AURKA*/*STK6, TPX2*, and *C20orf24*/*RIP5*)] or [(*PLK1* and *CCNF*) and (*ADRM1* and NUDT1)] or [(*PLK1* and *CCNF*) and (SSSCA1, *ID3,* and *LUM*)] or [(*PLK1* and *CCNF*) and (*PDGFRB, SPARC,* and *DCN*)] or [(*PLK1* and *CCNF*) and (*AURKA*/*STK6, TPX2,* and *C20orf24*/*RIP5*)] or [(*ADRM1* and NUDT1) and (SSSCA1, *ID3,* and *LUM*)] or [(*ADRM1* and NUDT1) and (*PDGFRB, SPARC,* and *DCN*)] or [(*ADRM1* and NUDT1) and (*AURKA*/*STK6, TPX2*, and *C20orf24*/*RIP5*)] or [(SSSCA1, *ID3*, and *LUM*) and (*PDGFRB, SPARC*, and *DCN*)] or [(SSSCA1, *ID3*, and *LUM*) and (*AURKA*/*STK6, TPX2*, and *C20orf24*/*RIP5*)] or [(*PDGFRB, SPARC*, and *DCN*) and (*AURKA*/*STK6, TPX2*, and *C20orf24*/*RIP5*)] or [(*PLK1* and *CCNF*) and (*ADRM1* and NUDT1) and (SSSCA1, *ID3,* and *LUM*)] or [(*PLK1* and *CCNF*) and (*ADRM1* and NUDT1) and *(PDGFRB, SPARC,* and *DCN)]* or [(*PLK1* and *CCNF*) and (*ADRM1* and NUDT1) and (*AURKA*/*STK6, TPX2*, and *C20orf24*/*RIP5*)] or [(*PLK1* and *CCNF*) and (SSSCA1, *ID3,* and *LUM*) and *(PDGFRB, SPARC,* and *DCN*)] or [(*PLK1* and *CCNF*) and (SSSCA1, *ID3,* and *LUM*) and (*AURKA*/*STK6, TPX2,* and *C20orf24*/*RIP5*)] or [(*PLK1* and *CCNF)* and (*PDGFRB, SPARC,* and *DCN*) and (*AURKA*/*STK6, TPX2*, and *C20orf24*/*RIP5*)] or [(*ADRM1* and NUDT1) and (SSSCA1, *ID3,* and *LUM*) and (*PDGFRB, SPARC,* and *DCN*)] or [(*ADRM1* and NUDT1) and (SSSCA1, *ID3,* and *LUM*) and (*AURKA*/*STK6, TPX2,* and *C20orf24*/*RIP5*)] or [(*ADRM1* and NUDT1) and (*PDGFRB, SPARC,* and *DCN*) and (*AURKA*/*STK6, TPX2,* and *C20orf24*/*RIP5*)] or [(SSSCA1, *ID3,* and *LUM*) and *(PDGFRB, SPARC,* and *DCN*) and (*AURKA*/*STK6, TPX2,* and *C20orf24*/*RIP5*)] or [(*PLK1* and *CCNF*) and (*ADRM1* and NUDT1) and (SSSCA1, *ID3,* and *LUM*) and (*PDGFRB, SPARC,* and *DCN*)] or [(*PLK1* and *CCNF*) and (*ADRM1* and NUDT1) and (SSSCA1, *ID3,* and *LUM*) and (*AURKA*/*STK6, TPX2,* and *C20orf24*/*RIP5*)] or [(*PLK1* and *CCNF*) and (*ADRM1* and NUDT1) and (*PDGFRB, SPARC,* and *DCN*) and (*AURKA*/*STK6, TPX2,* and *C20orf24*/*RIP5*)] or [(*PLK1* and *CCNF*) and (SSSCA1, *ID3,* and *LUM*) and (*PDGFRB, SPARC*, and *DCN*) and (*AURKA*/*STK6, TPX2,* and *C20orf24*/*RIP5*)] or [(*ADRM1* and NUDT1) and (SSSCA1, *ID3,* and *LUM*) and *(PDGFRB, SPARC*, and *DCN*) and (*AURKA*/*STK6, TPX2,* and *C20orf24*/*RIP5*)] or [(*PLK1* and *CCNF*) and (*ADRM1* and NUDT1) and (SSSCA1, *ID3,* and *LUM*) and (*PDGFRB, SPARC,* and *DCN*) and (*AURKA*/*STK6, TPX2,* and *C20orf24*/*RIP5*)]*.* Each of these subgroups may be further combined with any one or more of the respective remaining marker molecules disclosed herein.

In case, a subgroup is to be employed, e.g. one of the above mentioned, the expression level may also be seen as the expression level of the entire subgroup of marker genes, i.e. an averaged expression level, optionally normalized to a suitable control.

The term "adenocarcinoma associated with a chromosomal aberration on chromosome 20q" relates to a link or relationship between the presence of adenocarcinoma or any disease state(s) thereof, as defined herein above, and a chromosomal rearrangement on chromosome 20q. Thus, the presence of the disease is linked to a chromosomal aberration on chromosome 20, in particular in the region 20q, and preferably a chromosomal gain on 20q11.22 - 20q11.23 and/or at position 20q13.31 - 20q13.33.

The present invention refers, in preferred embodiments, to the diagnosis as well as prevention and/or treatment of specific adenocarcinoma-associated disease states, i.e. disease states that are (closely) related but not identical to adenocarcinoma.

The term "adenocarcinoma-associated disease states", as used herein, particularly relates to a predisposition for developing an adenocarcinoma associated with a chromosomal aberration on chromosome 20q, a progression of an adenoma to an adenocarcinoma associated with a chromosomal aberration on chromosome 20q or a predisposition for a progression of an adenoma to an adenocarcinoma associated with a chromosomal aberration on chromosome 20q.

A "predisposition for developing an adenocarcinoma associated with a chromosomal aberration on chromosome 20q" in the context of the present invention is a state of risk of developing adenocarcinoma associated with a chromosomal aberration on chromosome 20q. Preferably, a predisposition for developing such an adenocarcinoma may be present in cases in which the marker gene expression level, as defined herein, is below a reference expression level derived from tissues or samples of a subject which evidently suffers from adenocarcinoma associated with a chromosomal aberration on chromosome 20q. The term "below" in this context relates to an expression level of a marker gene which is reduced by about 40 % to 80% in comparison to such a cancerous control level, more preferably to a reduction of about 50% The reduction may be calculated over the averaged expression level or the entire group of marker genes. Alternatively, a reduction of 40 % to 80% or preferably of 50% of only one marker gene or a subgroup of the marker genes, e.g. those subgroups mentioned herein, may also be considered as indicative for a predisposition for developing an adenocarcinoma associated with a chromosomal aberration on chromosome 20q.

The term "progression of an adenoma to an adenocarcinoma associated with a chromosomal aberration on chromosome 20q", as used herein, relates to a state in which the expression level of one or several or all marker genes disclosed herein are elevated in one or more target cells compared to one or more control cells. Preferably, the term relates to cases in which the marker gene expression level, as defined herein above, is elevated by a value of between 3% to 50%, preferably by a value of 25%, as compared to an adenoma control sample. The increase may be calculated over the averaged expression level or the entire group of marker genes. Alternatively, an increase of 3% to 50%, preferably of 25%, of only one marker gene or a subgroup of the marker genes, e.g., those subgroups mentioned herein, may also be considered as indicative for a progression of an adenoma to an adenocarcinoma associated with a chromosomal aberration on chromosome 20q.

The term "predisposition for a progression of an adenoma to an adenocarcinoma associated with a chromosomal aberration on chromosome 20q", as used herein, relates to a similar state as the progression of adenoma to adenocarcinoma associated with a chromosomal aberration on chromosome 20q. However, in said condition the marker gene expression level, as defined herein, is elevated by a value of between 1% and 15%, preferably by a value of 10% in comparison to an adenoma control sample. The increase may be calculated over the averaged expression level or the entire group of marker genes. Alternatively, an increase of 1% to 15%, preferably by a value of 10% of only one marker gene or a subgroup of the marker genes, e.g., those subgroups mentioned herein, may also be considered as indicative for a predisposition for a progression of an adenoma to an adenocarcinoma associated with a chromosomal aberration on chromosome 20q.

In a preferred embodiment of the present invention, the chromosomal aberration on chromosome 20q is an aberration, preferably a chromosomal gain, at chromosomal position 20q11.22 - 20q11.23 and/or at position 20q13.31 - 20q13.33. These locations are known to the person skilled in the art and can be derived from any genetic map of chromosome 20. A "chromosomal gain" in the context of this embodiment denotes a duplication of (one or more) chromosomal regions of a size between about 0.3 kb and several Mb, e.g., between 0.3 kb and 50 Mb, or any sub-range thereof, e.g., 0.3 kb - 40 Mb, 0.3 kb - 30 Mb, 0.3 kb - 20 Mb, 0.3 kb - 15 Mb, 0.3 kb - 10Mb, 0.3 kb - 5 Mb, 0.3 kb - 2 Mb or 0.3kb - 1 Mb.

A composition of matter (i.e. a kit) for diagnosing adenocarcinoma associated with a chromosomal aberration on chromosome 20q typically comprises means for detecting the expression of at least one of the 45 marker genes disclosed herein. Usually, such kits contain one or more agents allowing the specific detection of marker gene expression. The nature of the agents is determined by the method of detection for which the kit is intended. Where detection at the DNA/RNA method is intended, the agents may be marker gene-specific primers or probes (i.e. oligonucleotides), which may be optionally labeled according to methods known in the art (e.g., with a fluorescent label, a luminescent label, an enzyme label etc.). Where detection is at the protein level, the agents may be antibodies or compounds containing an antigen-binding fragment of an antibody. However protein expression can also be detected using other compounds that specifically interact with the marker of interest, such as specific substrates (in case of enzymes) or ligands (in case of receptors). Typically, such a kit further comprises detection reagents such as buffer solutions, labels or washing liquids etc. Furthermore, the kit may comprise an amount of a known nucleic acid molecule, which can be used as a calibration standard. Additionally, the kit may also comprise an instruction leaflet.

The term "modifying the gene expression", as used herein, denotes any manipulation of any one or more of the marker genes disclosed herein (whose respective expression levels are elevated in the one or more target cells) resulting in a decreased expression level (i.e. a repression or down-regulation) of said marker gene(s), that is, the production of a lower amount of corresponding mRNA and/or protein as compared to the expression level in the unmodified one or more target cells. In other words, the modification of gene expression of the at least one marker gene occurs in an anti-cyclical pattern to the regulation of said molecule in the one or more target cells in order to interfere with the "excess activity" of an up-regulated molecule in the one or more target cells

In preferred embodiments of the method for preventing and/or treating an adenocarcinoma, step (b) comprises introducing into the one or more target cells:
(i) one or more nucleic acid molecules, each nucleic acid molecule encoding a sequence that is complementary to at least a part of the nucleic acid sequence of any one of the at least one marker gene to be down-regulated; and/or
(ii) one or more antibodies, each antibody directed against any polypeptide encoded by any one of the at least one marker gene to be down-regulated, or one or more dominant-negative polypeptide variants of any polypeptide encoded by any one of the at least one marker gene to be down-regulated.

In particularly preferred embodiments, the one or more nucleic acid molecules to be introduced into the one or more target cells are selected from the group consisting of an anti-sense nucleic acid construct, a siRNA, a miRNA, and a ribozyme.

The one or more nucleic acid molecules to be introduced into the one or more cells each encode a sequence that is complementary (herein also referred to as "anti-sense") to at least a part of the nucleic acid sequence (herein also referred to as "sense") of any one of the at least one marker gene to be down-regulated. Such anti-sense nucleic acid molecules act by binding to the nucleotide sequence of a marker gene or to a mRNA corresponding thereto, thereby inhibiting the transcription or translation of the gene, promoting the degradation of the mRNAs, and/or inhibiting the expression of the protein encoded by the gene. Thus, as a result, an anti-sense nucleic acid molecule inhibits the marker gene-derived protein function in the cancerous cell.

The term "introducing", as used herein, refers to any manipulation allowing the transfer of one or more nucleic acid molecules into the one or more cells. Examples of such techniques include inter alia transfection or transduction techniques all of them well established in the art (cf., for example, Sambrook, J. et al. (1989) Molecular, Cloning: A Laboratory Manual, 2nd ed., Cold Spring Harbor Laboratory Press, Cold Spring Harbor, NY; Ausubel, F.M. et al. (2001) Current Protocols in Molecular Biology, Wiley & Sons, Hoboken, NJ).

The term "complementary sequence", as used herein, is to be understood that the "complementary" nucleic acid molecule (herein also referred to as an "anti-sense nucleic acid molecule") introduced into the one or more target cells is capable of forming base pairs, preferably Watson-Crick base pairs, with the up-regulated endogenous "sense" nucleic acid molecule encoding a marker gene disclosed herein. The anti-sense nucleic acid molecule introduced into the cells may encode a sequence that is complementary to the entire nucleic sequence of the target gene (i.e. over the full length of the marker gene) or, preferably, to only a part (i.e. a segment or particular region) of the marker gene.

Two nucleic acid molecules (i.e. the "sense" and the "anti-sense" molecule) may be perfectly complementary, that is, they do not contain any base mismatches and/or additional or missing nucleotides. In other embodiments, the two molecules comprise one or more base mismatches or differ in their total numbers of nucleotides (due to additions or deletions). In further embodiments, the "complementary" anti-sense nucleic acid molecule comprises at least ten or at least 12, preferably at least 15 or at least 18 contiguous nucleotides showing perfect complementarity with a sequence comprised in the up-regulated "sense" nucleic acid molecule.

The "complementary" anti-sense nucleic acid molecule introduced into the one or more target cells may be a naturally occurring DNA- or RNA molecule or a synthetic nucleic acid molecule comprising in its sequence one or more modified nucleotides which may be of the same type or of one or more different types.

The "complementary" nucleic acid molecule (i.e. the nucleic acid molecule encoding a nucleic acid sequence that is complementary to the microRNA sequence encoded by nucleic acid molecule to be down-regulated) may be a naturally occurring DNA- or RNA molecule or a synthetic nucleic acid molecule comprising in its sequence one or more modified nucleotides which may be of the same type or of one or more different types.

For example, it may be possible that such a nucleic acid molecule comprises at least one ribonucleotide backbone unit and at least one deoxyribonucleotide backbone unit. Furthermore, the nucleic acid molecule may contain one or more modifications of the RNA backbone into 2'-O-methyl group or 2'-O-methoxyethyl group (also referred to as "2'-O-methylation"), which prevented nuclease degradation in the culture media and, importantly, also prevented endonucleolytic cleavage by the RNA-induced silencing complex nuclease, leading to irreversible inhibition of the miRNA. Another possible modification - which is functionally equivalent to 2'-O-methylation - involves locked nucleic acids (LNAs) representing nucleic acid analogs containing one or more LNA nucleotide monomers with a bicyclic furanose unit locked in an RNA-mimicking sugar conformation (cf., e.g., Orom, U.A. et al. (2006) Gene 372, 137-141).

The term "anti-sense construct", as used herein, refers to "classical" antisense-technology, that is, nucleotides that typically have more than about 25, more than 50 or more than 100 nucleotides in length that specifically hybridize to a target sequence. The anti-sense nucleic acid constructs of the present invention may have a homology (i.e. degree of complementarity) of at least 70% or higher, preferably of at least 80% or higher, more preferably of at least 90% or higher, even more preferably of at least 95% or higher over a span of at least 15 continuous nucleotides of any of the marker genes of the present invention or the complementary sequence thereof. Algorithms known in the art can be used to determine such homology.

The term "siRNA" refers to a particular type of exogenuous (i.e. produced recombinantly or artificially synthesized) anti-sense-molecules, namely small inhibitory RNA duplexes that induce the RNA interference (RNAi) pathway. These molecules can vary in length. A mature siRNA is usually 20, 21, 22, 23, 24, or 25 nucleotides in length, although other numbers of nucleotides may be present as well, for example 18, 19, 26 or 27 nucleotides. Some, but not all, siRNA have unpaired overhanging bases on the 5' or 3' end of the sense strand and/or the antisense strand. The term "siRNA" includes duplexes of two separate strands, as well as single strands that can form hairpin structures comprising a duplex region. Methods for designing suitable siRNAs directed to a given target nucleic acid are established in the art (cf., for example, Elbashir S.M. et al. (2001) Genes Dev. 15, 188-200)

The term "microRNA" (or "miRNA"), as used herein, is given its ordinary meaning in the art (reviewed, e.g. in Bartel, D.P. (2004) Cell 23, 281-292; He, L. and Hannon, G.J. (2004) Nat. Rev. Genet. 5, 522-531). Accordingly, the term "microRNA" denotes an endogenously produced RNA molecule derived from a genomic locus (in the target cell) that is processed from transcripts that can form local RNA precursor miRNA structures. The mature miRNA is usually 20, 21, 22, 23, 24, or 25 nucleotides in length, although other numbers of nucleotides may be present as well, for example 18, 19, 26 or 27 nucleotides.

The miRNA encoding sequence has the potential to pair with flanking genomic sequences, placing the mature miRNA within an imperfect RNA duplex (herein also referred to as stem-loop or hairpin structure or as pre-miRNA), which serves as an intermediate for miRNA processing from a longer precursor transcript. This processing typically occurs through the consecutive action of two specific endonucleases termed Drosha and Dicer, respectively. Drosha generates from the primary transcript (herein also denoted "pri-miRNA") a miRNA precursor (herein also denoted "pre-miRNA") that typically folds into a hairpin or stem-loop structure. From this miRNA precursor a miRNA duplex is excised by means of Dicer that comprises the mature miRNA at one arm of the hairpin or stem-loop structure and a similar-sized segment (commonly referred to miRNA*) at the other arm. The miRNA is then guided to its target mRNA to exert its function, whereas the miRNA* is degraded. In addition, miRNAs are typically derived from a segment of the genome that is distinct from predicted protein-coding regions.

The term "miRNA precursor" (or "precursor miRNA" or "pre-miRNA") as used herein, refers to the portion of a miRNA primary transcript from which the mature miRNA is processed. Typically, the pre-miRNA folds into a stable hairpin (i.e. a duplex) or a stem-loop structure. The hairpin structures typically range from 50 to 80 nucleotides in length, preferably from 60 to 70 nucleotides (counting the miRNA residues, those pairing to the miRNA, and any intervening segment(s) but excluding more distal sequences).

In the context of the present invention, any nucleic acid molecule coding for a microRNA (miRNA) may be employed. Thus, the term "anti-sense nucleic acid" does not only refer to mature miRNAs but also to the respective precursor miRNAs and primary miRNA transcripts as defined above. Furthermore, the present invention is not restricted to RNA molecules but also includes corresponding DNA molecules encoding a microRNA, e.g. DNA molecules generated by reverse transcribing a miRNA sequence. A nucleic acid molecule encoding a microRNA sequence according to the invention typically encodes a single miRNA sequence (i.e. an individual miRNA). However, it is also possible that such nucleic acid molecule encodes two or more miRNA sequences (i.e. two or more miRNAs), for example a transcriptional unit comprising two or more miRNA sequences under the control of common regulatory sequences such as a promoter or a transcriptional terminator.

The term "ribozyme" (i.e. a ribonucleic acid enzyme), as used herein, denotes an RNA molecule that catalyzes a chemical reaction. Many natural ribozymes catalyze either the hydrolysis of one of the phosphodiester bonds in their own backbone structure, or the hydrolysis of bonds in other. The term includes naturally occurring ribozymes (e.g., hairpin, hammerhead, VS, HDV, leadzymes, and the like) or, preferably, artificially designed (target-specific) ribozymes. Methods of generating such recombinant molecules are well known in the art (cf., e.g., Johnston, W. et al. (2001) Science 292, 1319-1325).

The nucleic acid molecules to be introduced into the one or more target cells in order to modify the expression of the at least one marker gene may be operably linked to a regulatory sequence in order to allow expression of the nucleotide sequence.

A nucleic acid molecule is referred to as "capable of expressing a nucleic acid molecule" or capable "to allow expression of a nucleotide sequence" if it comprises sequence elements which contain information regarding to transcriptional and/or translational regulation, and such sequences are "operably linked" to the nucleotide sequence encoding the polypeptide. An operable linkage is a linkage in which the regulatory sequence elements and the sequence to be expressed (and/or the sequences to be expressed among each other) are connected in a way that enables gene expression.

The precise nature of the regulatory regions necessary for gene expression may vary among species, but in general these regions comprise a promoter which, in prokaryotes, contains both the promoter *per se*, i.e. DNA elements directing the initiation of transcription, as well as DNA elements which, when transcribed into RNA, will signal the initiation of translation. Such promoter regions normally include 5' non-coding sequences involved in initiation of transcription and translation, such as the -35/-10 boxes and the Shine-Dalgarno element in prokaryotes or the TATA box, CAAT sequences, and 5'-capping elements in eukaryotes. These regions can also include enhancer or repressor elements as well as translated signal and leader sequences for targeting the native polypeptide to a specific compartment of a host cell.

In addition, the 3' non-coding sequences may contain regulatory elements involved in transcriptional termination, polyadenylation or the like. If, however, these termination sequences are not satisfactory functional in a particular host cell, then they may be substituted with signals functional in that cell.

Therefore, a nucleic acid molecule of the invention to be introduced into the one or more cells provided may include a regulatory sequence, preferably a promoter sequence, and optionally also a transcriptional termination sequence.

The promoters may allow for either a constitutive or an inducible gene expression. Suitable promoters include *inter alia* the *E. coli lacUV5* and *tet* (tetracycline-responsive) promoters, the T7 promoter as well as the SV40 promoter or the CMV promoter.

The nucleic acid molecules of the invention may also be comprised in a vector or other cloning vehicles, such as plasmids, phagemids, phages, cosmids or artificial chromosomes. In a preferred embodiment, the nucleic acid molecule is comprised in a vector, particularly in an expression vector. Such an expression vector can include, aside from the regulatory sequences described above and a nucleic acid sequence encoding a genetic construct as defined in the invention, replication and control sequences derived from a species compatible with the host that is used for expression as well as selection markers conferring a selectable phenotype on transfected cells. Large numbers of suitable vectors are known in the art, and are commercially available.

Additionally and/or alternatively to the introduction of one or more nucleic acid molecules into the one or more target cells, step (b) may comprise introducing into the one or more target cells an antibody directed against or a dominant-negative polypeptide variant of any one of the at least one marker gene to be down-regulated. Methods for generating such antibodies or protein variants are established in the art.

In a third aspect, the present invention relates to a pharmaceutical composition for preventing and/or treating an adenocarcinoma associated with a chromosomal aberration on chromosome 20q, comprising one or more agents, as defined herein above, for down-regulating gene expression of at least one marker gene, wherein the at least one marker gene is selected from the group consisting of *RNPC1* (GenBank acc. no. NM_017495.4), *TCFL5* (GenBank acc. no. NM_006602.2), *C20orf24*/*RIP5* (GenBank acc. no. NM_018840.2), *AURKA*/*STK6* (GenBank acc. no. NM_003600.2), *C20orf20* (GenBank acc. no. NM_018270.4), *ADRM1* (GenBank acc. no. NM_007002.2), *TH1L* (GenBank acc. no. NM_198976.1), *TGIF2* (GenBank acc. no. NM_021809.5), *DPM1* (GenBank acc. no. NM_003859.1), *TOP1* (GenBank acc. no. NM_003286.2), *C20orf11* (GenBank acc. no. NM_017896.2), *YWHAB* (GenBank acc. no. NM_003404.3), *ZNF217* (GenBank acc. no. NM_006526.2), *BCL2L1* (GenBank acc. no. NM_001191.2), *DATF1*/*DIDO1* (GenBank acc. no. NM_022105.3), *CSE1L* (GenBank acc. no. NM_001316.2), *C20orf126*/*PDRG1* (GenBank acc. no. NM_030815.2), *TPX2* (GenBank acc. no. NM_012112.4), *C20orf59* (GenBank acc. no. NM_022082.3), *UBE2C* (GenBank acc. no. NM_007019.2), *NCOA3* (GenBank acc. no. NM_006534.2), *C20orf14*/*PRPF6* (GenBank acc. no. NM_012469.3), *C20orf44*/*bFZb* (GenBank acc. no. NM_018244.3), *RNPC2* (GenBank acc. no. NM_004902.2), *HM13* (GenBank acc. no. NM_030789.2), *SLCO4A1* (GenBank acc. no. NM_016354.3), *VAPB* (GenBank acc. no. NM_004738.3), *KIAA1847* (GenBank acc. no. NM_032527.3), *CDC91L1* (GenBank acc. no. NM_080476.4), *PXMP4* (GenBank acc. no. NM_007238.4), *DLGAP4* (GenBank acc. no. NM_014902.3), *PFDN4* (GenBank acc. no. NM_002623.3), *E2F1* (GenBank acc. no. NM_005225.2), *CYP24A1* (GenBank acc. no. NM_000782.4), *SRC* (GenBank acc. no. NM_005417.3), *BCAS4* (GenBank acc. no. NM_017843.3), *PLK1* (GenBank acc. no. NM_005030.3), *CCNF* (GenBank acc. no. NM_001761.2), *NUDT1* (GenBank acc. no. NM_002452.3), *SSSCA1* (GenBank acc. no. NM_006396.1), *ID3* (GenBank acc. no. NM_002167.3), *LUM* (GenBank acc. no. NM_002345.3), *PDGFRB* (GenBank acc. no. NM_002609.3), *SPARC* (GenBank acc. no. NM_003118.2), and *DCN* (GenBank acc. no. NM_001920.3).

In another preferred embodiment, the pharmaceutical composition is for the further use of preventing and/or treating a predisposition for developing an adenocarcinoma, a progression of an adenoma to an adenocarcinoma or a predisposition for a progression of an adenoma to an adenocarcinoma, the adenocarcinoma being associated with a chromosomal aberration on chromosome 20q.

Preferably, the chromosomal aberration on chromosome 20q is a chromosomal gain, particularly preferably at position 20q11.22-20q11.23 and/or at position 20q13.31-20q13.33.

In a forth aspect, the present invention relates to the use of one or more agents, as defined herein above, for down-regulating gene expression of at least one marker gene for the manufacture of a medicament for the prevention and/or treatment of an adenocarcinoma associated with a chromosomal aberration on chromosome 20q.

In the context of the present invention, suitable pharmaceutical compositions include those suitable for oral, rectal, nasal, topical (including buccal and sub-lingual), peritoneal and parenteral (including intramuscular, subcutaneous and intravenous) administration, or for administration by inhalation or insufflation. Administration may be local or systemic. Preferably, administration is accomplished via the oral, rectal or intravenous routes. The formulations may be packaged in discrete dosage units.

Pharmaceutical compositions according to the present invention include any pharmaceutical dosage forms established in the art, such as *inter alia* capsules, microcapsules, cachets, pills, tablets, powders, pellets, multi-particulate formulations (e.g., beads, granules or crystals), aerosols, sprays, foams, solutions, dispersions, tinctures, syrups, elixirs, suspensions, water-in-oil emulsions such as ointments, and oil-in water emulsions such as creams, lotions, and balms.

The one or more agents described above can be formulated into pharmaceutical compositions using pharmacologically acceptable ingredients as well as established methods of preparation (Gennaro, A.L. and Gennaro, A.R. (2000) Remington: The Science and Practice of Pharmacy, 20th Ed., Lippincott Williams & Wilkins, Philadelphia, PA; Crowder, T.M. et al. (2003) A Guide to Pharmaceutical Particulate Science. Interpharm/CRC, Boca Raton, FL; Niazi, S.K. (2004) Handbook of Pharmaceutical Manufacturing Formulations, CRC Press, Boca Raton, FL).

In order to prepare the pharmaceutical compositions, pharmaceutically inert inorganic or organic excipients (i.e. carriers) can be used. To prepare e.g. pills, tablets, capsules or granules, for example, lactose, talc, stearic acid and its salts, fats, waxes, solid or liquid polyols, natural and hardened oils may be used. Suitable excipients for the production of solutions, suspensions, emulsions, aerosol mixtures or powders for reconstitution into solutions or aerosol mixtures prior to use include water, alcohols, glycerol, polyols, and suitable mixtures thereof as well as vegetable oils. The pharmaceutical composition may also contain additives, such as, for example, fillers, binders, wetting agents, glidants, stabilizers, preservatives, emulsifiers, and furthermore solvents or solubilizers or agents for achieving a depot effect. The latter is to be understood that the nucleic acid molecules may be incorporated into slow or sustained release or targeted delivery systems, such as liposomes, nanoparticles, and microcapsules.

To target most tissues within the body, clinically feasible noninvasive strategies are required for directing such pharmaceutical compositions, as defined herein, into cells. In the past years, several approaches have achieved impressive therapeutic benefit following intravenous injection into mice and primates using reasonable doses of anti-sense nucleic acid molecules, particularly of siRNAs or miRNAs, without apparent limiting toxicities.

One approach involves covalently coupling the passenger strand (siRNA*/miRNA* strand) of the miRNA to cholesterol or derivatives/conjugates thereof to facilitate uptake through ubiquitously expressed cell-surface LDL receptors (Soutschek, J. et al. (2004) Nature 432, 173-178). Alternatively, unconjugated, PBS-formulated locked-nucleic-acid-modified oligonucleotides (LNA-antimiR) may be used for systemic delivery (Elmen, J. et al. (2008) Nature 452, 896-899). Another strategy for delivering miRNAs involves encapsulating the miRNAs into specialized liposomes formed using polyethylene glycol to reduce uptake by scavenger cells and enhance time spent in the circulation. These specialized nucleic acid particles (stable nucleic acid-lipid particles or SNALPs) delivered miRNAs effectively to the liver (and not to other organs (cf., e.g., Zimmermann, T.S. et al. (2006) Nature 441, 111-114). Recently, a new class of lipid-like delivery molecules, termed lipidoids (synthesis scheme based upon the conjugate addition of alkylacrylates or alkyl-acrylamides to primary or secondary amines) has been described as delivery agents for RNAi therapeutics (Akinc, A. et al. (2008) Nat. Biotechnol. 26, 561-569).

A further cell-specific targeting strategy involves the mixing of siRNAs/miRNAs with a fusion protein composed of a targeting antibody fragment linked to protamine, the basic protein that nucleates DNA in sperm and binds miRNAs by charge (Song, E. et al. (2005) Nat. Biotechnol. 23, 709-717). Multiple modifications or variations of the above basic delivery approaches have recently been developed. These techniques are known in the art and reviewed, e.g., in de Fougerolles, A. et al. (2007) Nat. Rev. Drug Discov. 6, 443-453; Kim, D.H. and Rossi, J.J. (2007) Nat. Genet. 8, 173-184).

The invention is further described by the figures and the following examples, which are solely for the purpose of illustrating specific embodiments of this invention, and are not to be construed as limiting the scope of the invention in any way.

### EXAMPLES

### Example 1 Identification of putative biomarkers for CRC

### 1.1 Materials and methods

### Tumor samples

41 formalin-fixed and paraffin-embedded progressed adenomas (with a focus of adenocarcinoma present, also referred as malignant polyps) collected from the tissue archive of the department of pathology at the VU University Medical Center (VUmc), Amsterdam, the Netherlands, and 73 prospectively collected snap-frozen colorectal tumor samples (37 non-progressed adenomas and 36 adenocarcinomas) were investigated. All samples were used in compliance with the institution's ethical regulations.

The 41 progressed adenomas corresponded to 19 females and 18 males (three patients presented more than one lesion). Mean age was 67 (range 45-86). From these, adenoma and adenocarcinoma components were analyzed separately adding to a total of 82 archival samples (41 x 2).

The 73 frozen specimens corresponded to 31 females and 34 males (six patients had multiple tumors). Mean age was 69 (range 47-89). All histological sections were evaluated by a pathologist. Array CGH was performed on both sets of samples while expression microarrays were performed on the frozen samples only.

### DNA and RNA isolation

DNA from paraffin was obtained as described previously (Weiss, M.M. et al. (1999) Mol. Pathol. 52, 243-251). RNA and DNA from snap-frozen tissues were isolated using TRIzol (Invitrogen, Breda, NL) following the supplier's instructions with some modifications, described on http://www.english.vumc.nl/afdelingen/microarrays. Isolated RNA was subjected to purification using RNeasy Mini Kit (Qiagen, Venlo, NL). RNA and DNA concentrations and purities were measured on a Nanodrop ND-1000 spectrophotometer (Isogen, IJsselstein, NL) and integrity was evaluated on a 1% agarose ethidium bromide-stained gel.

### Array CGH

A BAC/PAC array platform was used as described elsewhere (Carvalho, B. et al. (2006) Cell. Oncol. 28, 283-294). Arrays were scanned (Agilent DNA Microarray scanner G2505B- Agilent Technologies, Palo Alto, USA) and Imagene 5.6 software (Biodiscovery Ltd, Marina del Rey, California) was used for automatic feature extraction with default settings. Local background was subtracted from the signal median intensities of both test and reference DNA. The median of the triplicate spots was calculated for each BAC clone and log₂ ratios (tumor/normal) were normalized by subtraction of the mode value of BAC clones on chromosomes 1-22 (UCSC July2003 freeze of the Human Golden Path - NCBI Build 34). Clones with standard deviation of the intensity of the three spots greater than 0.2 and with more than 20% missing values were excluded.

### Expression microarrays

The Human Release 2.0 oligonucleotide library, containing 60-mer oligonucleotides representing 28830 unique genes, designed by Compugen (San Jose, CA, USA) was obtained from Sigma-Genosys (Zwijndrecht, NL). Printing of slides was done as described elsewhere (Muris, J.J. et al. (2007) Br. J. Haematol. 136 38-47). Tumor RNA (30µg) was hybridized against Universal Human reference (Stratagene, Amsterdam, NL). cDNA labeling and hybridization procedures are described elsewhere (Muris, J.J. et al.., *supra*)*.* Scanning of arrays and feature extraction were performed as described above. Overall quality of experiments was judged on MA-plots of intensities of raw data. Normalization was done with TIGR Midas
(http://www.tm4.org/midas.html), using "Lowess" correction (Quackenbush, J. (2002) Nat. Genet. 32, Suppl. 496-501) or with "Median" normalization and implemented in the maNorm function (Marray R bioconductor package), with identical results. Inter-array normalization was also performed. Low intensity values were replaced by the intensity value of 50. Genes with more than 20% missing values were excluded.

Array CGH and expression microarray data sets are available at Gene Expression Omnibus (GEO) http://www.ncbi.nlm.nih.gov/geo/ (Edgar, R. et al. (2002) Nucleic Acids Res. 30, 207-210); accession number GSE8067.

### Microarray data analysis

Below, the steps of data analysis are discussed for array CGH data, expression data and integrative analysis. To account for multiple testing, either a False Discovery Rate (FDR) correction was applied to the p-values, or a very stringent p-value cut-off was used.

### Array CGH data

To segment DNA copy number alterations, a smoothing algorithm - "aCGH-Smooth" was applied (Jong, K. et al. (2004) Bioinformatics 20, 3636-3637). Smoothed log₂ ratios of -0.15 and 0.15 were used as thresholds to define gains and losses (99% confidence intervals) obtained for 15 normal-to-normal hybridizations. Only gains and losses covering at least three consecutive BAC clones were included. Amplifications were called when log₂ ratios exceeded 1.0. DNA copy number data were stored in the ArrayCGHbase (Menten, B. (2005) BMC Bioinformatics 6, 124; http://arraydb.vumc.nl/ arrayCGHbase). Median absolute deviation (MAD) was determined for each case as a quality control. Cases with MAD ≥ 0.2 were excluded. Array CGH profiles were visualized in ArrayCGHbase. Supervised analysis, comparing two groups, was done using CGHMultiArray (van de Wiel, M.A. et al. (2005) Bioinformatics 21, 3193-3194). For analysis of paired samples (adenoma and adenocarcinoma components within progressed adenomas) an adapted version of CGHMultiArray was used, based on the Wilcoxon sign-rank test corrected for ties. Reported p-values are adjusted for multiple testing (FDR), unless stated otherwise.

For defining the most frequent smallest regions of overlap (SRO) for gains on 20q, throughout all cases, STAC (Significance Testing for Aberrant Copy-number) was used (Diskin, S.J. et al. (2006) Genome Res. 16, 1149-1158).

### Microarray expression data

As all hybridizations were performed against a common reference, all comparisons were relative between colorectal adenomas and adenocarcinomas.

Supervised analysis for comparing carcinomas and adenomas was done using the Wilcoxon signed rank test, and a modified version of this test- total Thas score (http://www.cvstat.ugent.be/index.php?page=techrep/techrep.htm) that is powerful when the distributions of the expression levels of both groups do not differ over the whole range of expression levels. This occurs when not all cases in the adenocarcinomas and adenomas groups have differentially expressed genes, but differences rather appear in subpopulations. Genes were considered as differentially expressed when having a Wilcoxon test p-value of < 1e-5 and a Thas p-value of < 0.05, corresponding to a FDR of < 0.05.

To disclose genes which expression is influenced by 20q gain, tumors with and without a 20q gain were compared. Gene expression was regressed on copy number count using a linear model.

To evaluate the discriminatory power of candidate genes for classifying adenomas *versus* adenocarcinomas, a stepwise linear discriminant analysis with leave one out cross validation was performed on mRNA expression data (SPSS 15.0 for Windows, SPSS Inc, Chicago, IL, USA).

### Integration of copy number and expression data

ACE-it (Array CGH Expression integration tool) was applied to test whether gene dosage affects RNA expression (van Wieringen, W.N. et al. (2006) Bioinformatics 22, 1919-1920). Only genes on chromosome 20 are presented. We used a cut-off value of 0.15 for gains and losses, a default group value of 9 and a FDR ≤ 0.10.

### Quantitative RT-PCR

RNA (1 µg) was treated with DNase I and reverse transcribed to cDNA using oligo(dT)₂₀ Primer with Superscript II reverse transcriptase (Invitrogen, Breda, NL).

qRT-PCR was performed in duplicate on 15 adenomas and 15 adenocarcinomas for six candidate genes. A master mix was prepared with 12.5 µl of SYBR Green PCR master mix (Applied Biosystems, Nieuwerkerk a/d IJssel, NL), 0.5 µM of each primer in 22.5 µl. cDNA (25ng in 2.5µl) was added to the mix. Reactions were performed in a 7300 Real-time PCR System (Applied Biosystems, Nieuwerkerk a/d IJssel, NL). Amplification conditions comprised a denaturation step at 95°C for 10' and 50 cycles at 95°C for 15" and annealing temperature for 1' (Supplementary Table 1). Relative expression levels were determined following the 2ΔΔCt method (Livak, K.J. and Schmittgen, T.D. (2001) Methods 25, 402-408) using β2*M* (beta-2-microglobulin gene) as a reference. This gene was previously demonstrated not to differ in expression between adenomas and adenocarcinoma (Dydensborg, A.B. et al. (2006) Am. J. Physiol. Gastrointest. Liver Physiol. 290, G1067-G1074).

### Immunohistochemistry on tissue microarrays (TMAs)

A tissue microarray (TMA) was constructed with 57 tumors (32 adenomas and 25 adenocarcinomas) of which array CGH and/or expression microarray data were available. Of each tumor three cores from different locations within the tumor were included in the array. A 4 µm section of the array was used for immunohistochemistry. After deparaffination in xylene, and rehydration through graded alcohol to water, endogenous peroxidase was blocked with hydrogen peroxide (0.3% H₂O₂/methanol) for 25 min. Antigen retrieval was done by autoclaving in citrate buffer (10 mM; pH 6.0). Primary Aurora A monoclonal antibody NCL-L-AK2 from Novocastra Laboratories was incubated overnight at 4°C in a dilution of 1:50. The secondary antibody - K4006, mouse, from Envision kit (DAKO) was incubated for 30 min at room temperature. Counterstaining was done with Mayer's hematoxylin. Incubation without primary antibody was used as negative control. Colorectal cancer cell line Caco-2, which has a 20q gain and is known to express Aurora A, was used as positive control. Caco-2 cells were fixed and paraffin embedded, sections of which were taken along in the same run of immunohistochemistry as the tissue microarray was processed. Caco-2 produced strong nuclear, mostly along with cytoplasmic, staining in > 75% of tumor cells and this pattern was taken as reference for intense staining.

Next, the spectrum of staining in the respective cores on the TMA was surveyed in terms of intensity and positive nuclei. Only staining in tumor cells (i.e. either adenoma or adenocarcinoma cells) was considered. Cores of the TMA typically contained 4 to 17 crypts with in every crypt > 100 cells which all were evaluated. Basically, three staining patterns were seen; no staining at all, strong staining comparable to that observed in Caco-2 cells, and an intermediate pattern that showed positive staining, but clearly less intense than in Caco-2 cells. The intensity of staining was taken as most important parameter. In pattern 2, typically 50% to >75% of nuclei showed intense staining, while in pattern 1 typically 25% to >75% of nuclei showed weak staining. For score 0, no more than a scattered weakly positive cell was tolerated. Based on evaluation of up to three cores by two independent observers, a score ranging from 0 to 2 was assigned per tumor, with score 0 corresponding to no signal, score 2 corresponding to the strong signal that was observed in the positive control Caco-2 and score 1 for an intermediate intensity staining. In case of disagreement between observers, a third observer was consulted and the majority score was noted.

Cochran-Armitage test analysis was performed to compare protein expression with lesion type (adenoma, carcinoma). Jonckheere-Terpstra test was performed to compare protein expression with log₂ratios (expression data). Both tests make explicit use of the ordinality of the protein levels of expression. Differences were considered significant when p< 0.05.

### 1.2 Delimiting gained regions on 20q

41 progressed adenomas, which were previously studied by classical CGH, were analyzed by array CGH. The adenoma and adenocarcinoma components of these samples were tested separately. Gain of 20q occurred in more than 60% of the cases. The pattern of copy number changes did not differ between adenoma and adenocarcinoma components (as determined by CGHMultiArray), although sometimes showed lower amplitudes in the adenoma component.

Next, the DNA copy number status of 37 non-progressed adenomas and 36 adenocarcinomas was analyzed. From these 73 tumors, 67 (34 adenomas and 33 adenocarcinomas) showed high quality genomic profiles with MAD values < 0.2, giving an 8% drop-out. In these 67 tumors, chromosome 20 gain occurred in less than 15% of the adenomas but in more than 60% of the carcinomas (p < 0.00001, as determined by CGHMultiArray), mostly affecting either all of chromosome 20 or the q-arm only, similar to the progressed adenomas.

To determine the most relevant regions within 20q harboring putative oncogenes with a role in colorectal adenoma to adenocarcinoma progression, STAC (Diskin, S.J. et al., *supra*) was applied to the combined setof paraffin-embedded malignant polyps (n = 41) and frozen carcinomas (n = 33). This revealed 3 relevant regions of aberrant copy gains on 20q, one spanning 4 Mb (32-36Mb), one spanning 3 Mb (56-59Mb), and the third one spanning 2Mb (61-64Mb). These three regions (smallest regions of overlap - SROs) contained 80, 35, and 94 known genes, respectively.

### 1.3 Identification of differentially expressed genes

Microarray expression analysis on the 37 non-progressed adenomas and 36 adenocarcinomas of which snap-frozen material was available were performed. High quality expression array data were obtained from 68 cases (37 adenomas and 31 adenocarcinomas, 7% drop-out).

Supervised data analysis for identifying putative oncogenes on 20q, was done in two different ways; we compared carcinomas to adenomas, and we compared tumors with 20q gain to tumors without 20q gain. The first approach revealed genome-wide 122 up-regulated genes and 219 down-regulated genes (a total of 341 differentially expressed genes), in carcinomas when compared to adenomas (Wilcoxon test p-value < 1e-5 (FDR < 0.05) and Thas p-value < 0.05). Of these 122 up-regulated genes, 14 map at chromosome 20q (Table 1). For the second approach, only tumors (adenomas and adenocarcinomas) that had both array CGH data and expression data available (n = 64) were included. As a preselection, genes differentially expressed (both up and down) between carcinomas and adenomas were used that are involved in progression, using a less stringent cut-off (Thas p-value < 0.05). Thereby, 127 genes were identified genome-wide out of 931 differentially expressed genes (regression analysis; FDR ≤0.1), whose expression levels are influenced by the occurrence of 20q gain. Of these 127 genes, 21 are mapped at 20q (Table 2).

Nine genes common to these two approaches emerged, namely *TPX2, C20orf24*/*RIP5, AURKA*/*STK6, RNPC1, THIL, ADRM1, C20orf20, TCFL5* and *C20orf11.*

**Table 1. Genes significantly up-regulated in adenocarcinomas, when compared to adenomas, mapping at 20q (Wilcoxon ranking p-value < 1e-5 (i.e. FDR < 0.05) and Thas p-value < 0.05), ordered by chromosomal position (location in bp according to Freeze July 2003; NCBI Build 34) with HUGO gene symbols and GenBank accession numbers.**

| **Gene symbol** | **GenBank Accession #** | **Location** **(bp position)** | ***Wilcoxon*** **p-value** | **Thas** **p-value** |
|---|---|---|---|---|
| *C20orf1(TPX2)* | NM_012112.4 | 31103374 | 2E-06 | 8E-05 |
| *MYRL2* | NM_006097.3 | 35859501 | 5E-06 | 4E-05 |
| *C20orf24 (RIP5)* | NM_018840.2 | 35923014 | 2E-07 | 2E-05 |
| *TOMM34* | NM_006809.4 | 44265329 | 8E-08 | 0 |
| *RBPSUHL* | NM_014276.2 | 44626010 | 2E-07 | 6E-06 |
| *BCAS4* | NM_017843.3 | 50138063 | 2E-06 | 6E-05 |
| *AURKA (STK6)* | NM_003600.2 | 55641283 | 4E-10 | 0 |
| *FLJ37465 (BMP7)* | NM_001719.2 | 56477906 | 1 E-09 | 0 |
| *RNPC1* | NM_017495.4 | 56660843 | 8E-07 | 7E-05 |
| *TH1L* | NM_198976.1 | 58253070 | 1 E-06 | 1 E-05 |
| *ADRM1* | NM_007002.2 | 61566389 | 9E-07 | 8E-05 |
| *C20orf20* | NM_018270.4 | 62156238 | 9E-09 | 0 |
| *TCFL5* | NM_006602.2 | 62211152 | 2E-09 | 0 |
| *C20orf11* | NM_017896.2 | 62299593 | 4E-07 | 0 |

**Table 2. Genes significantly up-regulated in adenocarcinomas, when compared to adenomas, mapping at 20q, which expression is related to the 20q gain (FDR ≤ 0.10), ordered by chromosomal position (Location in bp according to Freeze July 2003; NCBI Build 34) with HUGO gene symbols and GenBank accession ID.**

| **Gene symbol** | **GenBank accession #** | **Location (bp position)** | **FDR** |
|---|---|---|---|
| *HM13* | NM_030789.2 | 30874805 | 0.03 |
| *C20orf1 (TPX2)* | NM_012112.4 | 31103374 | 0.03 |
| *CDC91L1* | NM_080476.4 | 33922394 | 0.02 |
| *C20orf44* | NM_018244.3 | 34608051 | 0.07 |
| *DLGAP4* | NM_014902.3 | 35761669 | 0.05 |
| *TGIF2* | NM_021809.5 | 35897616 | 0.003 |
| *C20orf24 (RIP5)* | NM_018840.2 | 35923014 | 0.0006 |
| *YWHAB* | NM_003404.3 | 44210177 | 0.0002 |
| *UBE2C* | NM_007019.2 | 45128792 | 0.01 |
| *DPM1* | NM_003859.1 | 50248672 | 0.000001 |
| *NFATC2* | NM_012340.3 | 50769018 | 0.003 |
| *AURKA (STK6)* | NM_003600.2 | 55641283 | 0.02 |
| *RNPC1* | NM_017495.4 | 56660843 | 0.04 |
| *TH1L* | NM_198976.1 | 58253070 | 0.007 |
| *ADRM1* | NM_007002.2 | 61566389 | 0.05 |
| *SLCO4A1* | NM_016354.3 | 62015102 | 0.08 |
| *C20orf20* | NM_018270.4 | 62156238 | 0.04 |
| *TCFL5* | NM_006602.2 | 62211152 | 0.03 |
| *C20orf11* | NM_017896.2 | 62299593 | 0.0009 |
| *C20orf59* | NM_022082.3 | 62323360 | 0.007 |
| *PRPF6* | NM_012469.3 | 63364789 | 0.03 |

The nucleic acid sequences (encoding the corresponding mRNAs) of the genes *MYRL2, TOMM34, RBPSUHL, FLJ37465*/*BMB7* (all included in Table 1), and *NFATC2* (included in Table 2) represent known human sequences that have been deposited in GenBank, the NIH genetic sequence database (Nucl. Acids Res. (2008) 36, D25-D30; http://www.ncbi.nlm.nih.gov/Genbank/; release 169.0), under the accession numbers indicated. The nucleic acid sequences are also given as SEQ ID NO:46 (*MYRL2*), SEQ ID NO:47 (*TOMM34*), SEQ ID NO:48 (*RBPSUHL*), SEQ ID NO:49 (*FLJ37465*/ *BMB7*), and SEQ ID NO:50 (*NFATC2*), respectively.

### 1.4 Integration of array CGH and expression data

BAC array CGH data were related to oligonucleotide expression array data, independently of adenoma or adenocarcinoma status, using a dedicated integration tool called ACEit (van Wieringen, W.N. et al., *supra*). A list of 151 genes located at chromosome 20 was obtained, for which gene dosage affected expression levels (FDR ≤ 0.1), 120 of which are on the q-arm. Combining this information with the results of the two supervised approaches for expression data analysis (adenocarcinoma *versus* adenoma and 20q gain *versus* no-20q gain), seven genes were shared, namely *C20orf24*/ *RIP5, AURKA*/*STK6, RNPC1, TH1L, ADMR1, C20orf20,* and *TCFL5.*

Of these seven candidate genes, 6 map within the SROs determined by STAC analysis. The seventh gene (*AURKA*/*STK6*) maps approximately 400kb proximal to SRO2 at 55.6Mb (20q13.31). *C20orf24*/*RIP5* maps within SRO1 at 35.9Mb (20q11.23), *RNPC1* and *TH1L* map within SRO2 at position 56.7 and 58.3Mb, respectively (20q13.32), and genes *ADMR1, C20orf20* and *TCFL5* map within SRO3 the first at 61.6 and the other two at 62.2Mb (20q13.33).

Stepwise linear discriminant analysis with leave one out cross validation showed that mRNA expression levels of two out of the seven candidate genes, i.e. *RNPC1* and *TCFL5*, allowed to correctly classify 88.2% of the cases (60/68) as adenomas or carcinomas (Table 3).

**Table 3. Results of stepwise linear discriminant analysis with leave one out cross validation of the seven candidate genes. From 68 tumors in total, 60 were correctly classified (88.2%), using expression levels of RNPC1 and TCFL5 only.**

| | | | **Predicted Group Membership** | | |
|---|---|---|---|---|---|
| | **Lesion** | | **Predicted Group Membership Adenoma** | **Carcinoma** | **Total** |
| **Original** | **Count** | **Adenoma** | 35 | 2 | 37 |
| | | **Carcinoma** | 6 | 25 | 31 |
| | **%** | **Adenoma** | 94.6 | 5.4 | 100.0 |
| | | **Carcinoma** | 19.4 | 80.6 | 100.0 |

### 1.5 Confirmation of differential expression by qRT-PCR

qRT-PCR was performed on a sub-sample (n = 30) of frozen tumors (15 adenomas and 15 adenocarcinomas) to confirm the expression levels of six of the seven genes identified. Adenocarcinomas showed higher expression of all 6 genes compared to adenomas and tumors with 20q gain (4 adenomas and 8 adenocarcinomas) showed higher expression compared to tumors without 20q gain (11 adenomas and 7 adenocarcinomas). Table 4 shows the fold changes observed between either adenocarcinomas *versus* adenomas or tumors with 20q gain *versus* tumors without 20q gain, by microarrays and by qRT-PCR.

**Table 4. Expression fold-changes and range of expression levels (log₂ ratio) determined by expression microarray and by qRT-PCR, comparing either adenocarcinomas versus adenomas (Ca/Ad) or tumors with 20q gain versus tumors without 20q gain (20q gain/non 20q gain); nd, not determined.**

| **Gene** | **Comparison** | **Array fold change** | **qRT-PCR fold change** | **Microarray Expression range ^{a}** | **qRT-PCR Expression range ^{a}** |
|---|---|---|---|---|---|
| *C20orf24* | Ca/Ad | 1.54 | 1.78 | [-0.45,1.60]/[-0.71,0.71] | [1.84,6.08]/[-0.26,4.81] |
| | 20q gain/non gain | 1.68 | 3.99 | [-0.17,1.60]/[-0.71,0.37] | [-0.26,6.08]/[1.85,4.95] |
| *AURKA* | Ca/Ad | 1.91 | 3.39 | [-2.01,0.17]/[-2.26,-1.11] | [-1.78,6.06]/[-0.64,3.72] |
| | 20q gain/non gain | 1.55 | 4.53 | [-2.11,0.17]/[-2.26,-0.48] | [1.03,6.06]/[-1.78,3.99] |
| *RNPC1* | Ca/Ad | 1.74 | nd | [-1.61,1.22]/[-1.80,-0.41] | nd |
| | 20q gain/non gain | 1.58 | nd | -1.71-1.22/-1.80- -0.01 | nd |
| *TH1L* | Ca/Ad | 1.52 | 4.98 | [-0.77,1.39]/[-1.06,-0.15] | [-1.97,6.27]/[-3.57,3.72] |
| | 20q gain/non gain | 1.59 | 6.4 | [-0.59,1.39]/[-1. 06,0.10] | [-3.57,6.27]/[-3.57,3.72] |
| *ADRM1* | Ca/Ad | 1.45 | 1.46 | [-0.62, 0.79]/[-1.14,0.02] | [-0.30,5.58]/[-1.29,5.34] |
| | 20q gain/non gain | 1.38 | 2.58 | [-0.69,0.78]/[-1.14,0.36] | [-1.29,5.58]/[-0.30,5.34] |
| *C20orf20* | Ca/Ad | 1.36 | 3.08 | [-0.94,0.49]/[-1.31,-0.59] | [-1.32,2.07]/[-2.79,0.14] |
| | 20q gain/non gain | 1.34 | 3.57 | [-0.89,0.49]/[-1.31,-0.36] | [-1.16,2.06]/[-2.79,0.35] |
| TCFL5 | Ca/Ad | 2.2 | 3.54 | [-2.14,0.83]/[-2.73,-1.17] | [2.07,6.94]/[-1.28,4.21] |
| | 20q gain/non gain | 2.02 | 3.54 | [-2.31,0.83]/[-2.73,-0.93] | [-1.28,6.94]/[1.99,4.41] |

| | | | | | |
|---|---|---|---|---|---|
| ^{a} Log₂ ratio. | | | | | |

*In situ* confirmation of AURKA/STK6 expression by immunohistochemistry on TMAs yielded higher expression of AURKA/STK6 in adenocarcinomas as compared to adenomas (p = 0.01) (Table 5) as well as a significant positive correlation with the mRNA expression levels (p = 0.01). Validation of other genes was hampered by the absence of adequate antibodies.

**Table 5. AURKA/STK6 protein expression in colorectal adenocarcinomas versus adenomas, determined by immunohistochemistry on TMAs.**

| | | **AURKA/STK6 staining** | | | | |
|---|---|---|---|---|---|---|
| | | **Negative** | **Weak** | **Strong** | **Total** | **p value^{a}** |
| **Lesion** | **Adenoma** | 12 | 12 | 1 | 25 | |
| | **Carcinoma** | 4 | 9 | 6 | 19 | |
| **Total** | | 16 | 21 | 7 | 44 | 0.01 |

| | | | | | | |
|---|---|---|---|---|---|---|
| ^{a} Cochran-Armitage test | | | | | | |

### 1.6 Evaluation of results

One of the most frequent chromosomal aberrations observed in CRC is a gain of the long arm of chromosome 20. In order to try to identify these putative oncogenes, a series of colorectal tumors, both adenomas and carcinomas, was analyzed at the DNA and RNA levels.

In this study, it was confirmed that chromosome 20 is the most frequently altered chromosome in the progressed adenomas and adenocarcinomas (in more than 60% of cases). In non-progressed adenomas, gains of 20q were detected in less than 20%, supporting a role of 20q gain in colorectal adenoma to adenocarcinoma progression consistent with earlier observations (Hermsen, M. et al., *supra*). Narrowing down the gained region by array CGH across all tumors analyzed yielded three smallest regions of overlap: SRO1 at 20q11.22-q11.23 (32-36 Mb), SRO2 at 20q13.32-q13.33 (56-59 Mb), and SRO3 at 20q13.33 (61-64 Mb).

Looking at the same expression data from a different angle, i.e. comparing the expression of tumors with and without 20q gain, it was aimed to find genes with a dosage effect on expression. Genome-wide, expression of 127 out of 931 genes was related to 20q gain, 21 of which are located at chromosome 20q itself. Although chromosome 20 has a high gene density, and copy number gains of the long arm are very frequent, certainly not all genes mapping at the gained regions are recurrently over-expressed. Two hundred and nine genes are mapped to the SROs defined here, but only 21 genes are recurrently up-regulated in association with 20q gain.

Nine genes overlapped between the 14 adenoma *versus* adenocarcinoma genes and the 21 genes associated with either or not 20q gain, namely *TPX2, C20orf24*/*RIP5, AURKA*/*STK6, RNPC1, TH1L, ADRM1, C20orf20, TCFL5* and *C20orf11.*

In the third approach, integration of DNA copy number changes and gene expression in the present study demonstrated that throughout the genome 507 genes showed a statistically significant association between DNA copy number and mRNA expression status, both for amplified/up-regulated and deleted/down-regulated genes, 120 of these being located on chromosome 20q. From these 120 genes, 17 overlapped with the 20q gain associated list, and 11 overlapped with the adenoma and adenocarcinoma *versus* adenocarcinoma list. Overlapping these three approaches (expression in adenomas *versus* adenocarcinomas, expression *versus* 20q gain, and genome wide expression *versus* whole genome copy-number changes) showed that seven genes are consistently significant, namely *C20orf24*/*RIP5, AURKA*/*STK6, RNPC1, TH1L, ADRM1, C20orf20* and *TCFL5.*

In addition to the already stringent data analysis, a permutation analysis was performed, comparing the differential expression of the seven 20q genes with the expression of over 50.000 random subsets out of genes 7946 in silent DNA regions (2q, 3, 5,10p, 11, 16, 21, 22). For each random subset, the Wilcoxon scores of the seven most differentially expressed (adenoma *versus* adenocarcinoma) genes were selected. The seven genes on 20q showed a significantly higher expression in adenocarcinomas *versus* adenomas compared to the best performing combination from the permutation test (p = 0.001), underlining that the copy number based discovery of putative oncogenes did not yield random differentially expressed genes. The fact these over-expressed putative oncogenes on 20q actually resulted in biologically active components, i.e. proteins, in the tumor cells was demonstrated by immunohistochemistry on TMA for AURKA/STK6. For the other candidates, antibodies did not perform adequately in the tissue samples or were not available at all.

The function of these genes include a function as transcription factors, like *TCFL5* (Siep, M. et al. (2004) Nucleic Acids Res. 32, 6425-6436), or factors being involved in transcriptional regulation, like *C20orf20* (Cai, Y. et al. (2003) J. Biol. Chem. 278, 42733-42736). *TH1L* product is involved in regulation of A-Raf kinase (Liu, W. et al. (2004) J. Biol. Chem. 279, 10167-10175). *ADRM1* encodes for a putative cell adhesion molecule that recently was shown to be component of the 26S proteosome (Jorgensen, J.P. et al. (2006) J. Mol. Biol. 360, 1043-1052). *RNPC1* product is predicted to bind to RNA, based on sequence motifs, and the *C20orf24*/*RIP5* product interacts with Rab-5. *AURKA*/*STK6* has been well characterized and is involved in cell cycle regulation. It has been shown to be amplified in CRC (Bischoff, J.R. et al. (1998) EMBO J. 17, 3052-3065) and its over-expression induces centrosome amplification, aneuploidy and transformation *in vitro* (Zhou, H. et al. (1998) Nat. Genet. 20, 189-193). Moreover, inhibiting *AURKA* by RNA interference lead to growth suppression of human pancreatic cancer cells (Hata, T. et al. (2005) Cancer Res. 65, 2899-2905). Knocking down *TCFL5* resulted in suppression of the number of multicellular HT29 tumor spheroids, supporting its role in cancer development (Dardousis, K. et al. (2007) Mol. Ther. 15, 94-102).

In summary, the above provided experimental results demonstrated the involvement of three SROs in the 20q amplicon in CRC and showed strong DNA copy number/mRNA expression associations for seven genes in these areas.

In addition, significant differences between colorectal adenomas and adenocarcinomas were shown at the DNA, mRNA and, for a one of the genes, at the protein level, supporting an important role as oncogenes in colorectal adenoma to adenocarcinoma progression. Furthermore, it was demonstrated that the expression levels of the marker genes of the present invention, in particular the expression levels of *RNPC1* and *TCFL5* allowed discriminating adenomas from adenocarcinomas with high accuracy.

### Example 2: Loss-of-function analysis of putative biomarkers for CRC

### 2.1 Materials and methods

### Cell lines

The following colorectal cancer (CRC) cell lines were used: SW480, Caco2 and HT29 (obtained from ATTC, LGC Standards, Teddington, United Kingdom).

### siRNA transfection

CRC cell lines were transfected with target-specific siGENOME SMART-pool siRNAs (Dharmacon, Inc., Lafayette, CO, USA) were introduced into the CRC cells using DharmaFECT transfection reagents (also from Dharmacon, Inc.) according to the manufacturer's instructions. The siCONTROL Non-Targeting pool was used as a negative control, the *PLK1* siGENOME SMART-pool as a positive control. Gene expression levels were determined using quantitative real-time PCR.

### MTT cell viability assay

The effects of siRNA-mediated down-regulation of the expression of the 36 marker genes disclosed herein on cell numbers cell viability) were determined by the MTT (3-[4,5-dimethylthiazol-2-y]-2,5-diphenyltetrazolium bromide) cell viability assay (Plumb J.A. (2004) Methods Mol Med. 88, 165-169). Three days after transfection of the cells with the respective siRNAs the MTT-assay was performed by a two hours incubation of MTT followed by lysis of the formazan crystals by DMSO. Absorbance was measured with a spectrophotometer (TECAN) at 540 nm. The assay was performed under growth conditions (determining cell proliferation) and upon 5 days treatment with 5-fluor-uracil (5FU) (determining apoptosis).

### Invasion assay

The effect of siRNA-mediated downregulation of candidate gene expression on invasion rates was measured by the transwell system. HTS FluoroBlok Cell Culture Inserts (Falcon) were coated with fibronectin (MP Biomedicals) and extracellular matrix (ECM) (Sigma-Aldrich). Transfected cells were allowed to invade for 48 hours. Cell invasion was measured by fluorescent labelling of invaded as well as non-invaded cells by Calcein AM (Molecular Probes) (Bijman, M.N. et al. (2008) Biochem. Pharmacol. 76, 707-716). Invasion rates were determined as a ratio of invaded/non-invaded cells.

### Anchorage-independent growth assay

The effects of siRNA-mediated down-regulation of candidate gene expression (shown for *TCFL5* and *AURKA*) on anchorage-independent growth, as determined by using the soft agar colony formation assay. siRNA-transfected cells were allowed to grow in soft agarose (SeaPlaque Agarose purchased from Lonza Ltd., Basel, Switzerland) for 2-4 weeks. Digital images were taken of the soft agar dishes and analyzed using appropriate software.

### Chromosomal instability assay

The effect of siRNA-mediated downregulation of candidate gene expression on chromosomal instability was measured by flow and image cytometry. For flow cytometry cells were fixed in paraformaldehyde and DNA stained with DAPI. DNA contents were analyzed with a Partec PAS II mercury lamp based flow cytometer (Partec Instruments) (Bergers, E. et al. (1996) J. Clin. Pathol. 49, 931-937). For image cytometry cells were subjected to pronase treatment after fixation. Cytospin were prepared and stained with Schiff-reagent (Feulgen method). DNA content of the stained nuclei was analyzed according to protocol (Rijken, A.M. et al. (1999) Anal. Quant. Cytol. Histol. 21, 303-310).

### 2.2 Marker genes on 20q amplicon have a functional role in CRC progression

The study aimed to identify the genes on the 20q amplicon that functionally contribute to colorectal adenoma-to-carcinoma progression For this purpose, the loss-of-function phenotype of candidate genes was investigated on cancer-related processes in *in vitro* assays using CRC cell lines with 20q gain (HT29, Caco2, and SW480).

Fig. 2 shows a frequency plot of DNA copy number gains and losses as determined by BAC array comparative genomic hybridization in three CRC cell lines: SW480 (top panel), HT29 (medium panel), and Caco2 (bottom panel). Y-axis displays the fraction of tumors with either a gain (positive sign) or loss (negative sign) for all clones that are sorted by chromosome and base pair position. The respective chromosomal gains at 20q are encircled

A list of 26 candidate genes was generated based on the combined analysis of array-CGH and RNA-expression data derived from a large panel of colorectal adenomas and carcinomas. This list was supplemented with ten candidate genes from literature.

The following 36 marker genes were analyzed: *RNPC1, TCFL5, C20orf24*/*RIP5, AURKA*/*STK6, C20orf20, ADRM1, TH1L, TGIF2, DPM1, TOP1, C20orf11, YWHAB, ZNF217, BCL2L1, DATF1*/*DIDO1, CSEI L, C20orf126*/*PDRG1, TPX2, C20orf59, UBE2C, NCOA3, C20orf14*/*PRPF6, C20orf44*/*bFZb, RNPC2, HM13, SLCO4A1, VAPB, KIAA1847, CDC91L1, PXMP4, DLGAP4, PFDN4, E2F1, CYP24A1, SRC*, and *BCAS4.*

Expression of candidate genes was down-modulated by transfection with commercially available siRNA pools. Fig. 3 shows the inhibition of expression of four exemplary marker genes (i.e. *TCFL5, C20orf24*/*RIP5, RNPC1*, and *C20orf20*) in SW480 cells upon transfection with siRNAs. The respective target-specific siGENOME SMART-pool siRNAs were introduced into the CRC cells using DharmaFECT transfection reagents according to the manufacturer's instructions. The siCONTROL Non-Targeting pool was used as a negative control, the *PLK1* siGENOME SMART-pool as a positive control. Expression levels were determined using quantitative real-time PCR. All four siRNAs employed reduced the expression level of the respective target gene by at least 80%.

The effects of siRNA-mediated down-regulation on 'cell proliferation' were measured by the MTT cell viability assay, both under normal growth conditions (72 hours of culture) and upon induction of 'cellular stress' (five day treatment with 5FU).

In addition, the effects of candidate genes are currently being investigated on progression-associated processes like anchorage-independent growth (colony formation in soft agar) and cell invasion (trans-well invasion assay).

Fig. 4 depicts the effects of siRNA-mediated down-regulation of the expression of the 36 marker genes disclosed herein on cell numbers in SW480 (Fig. 4A), HT29 (Fig. 4B), and Caco2 (Fig. 4C) cells, respectively, as determined by the MTT (3-[4,5-dimethylthiazol-2-y]-2,5-diphenyltetrazolium bromide) cell viability assay (Plumb J.A. (2004) Methods Mol Med. 88, 165-169). Three days after transfection of the cells with the respective siRNAs the MTT-assay was performed by a two hours incubation of MTT followed by lysis of the formazan crystals by DMSO. Absorbance was measured with a spectrophotometer (TECAN) at 540 nm. The assay was performed under growth conditions (determining cell proliferation) and upon 5 days treatment with 5-fluor-uracil (5FU) (determining apoptosis). Five marker genes (i.e. *AURKA*/*STK6, TPX2, BCL2L1, C20orf59*/*RIP5,* and *RNPC2*) were shown to contribute to changes in cell numbers in all three CRC cell lines tested.

In another experiment, the cells were transfected with specific combinations of target gene-specific siRNAs (cf. Fig. 5). A particularly pronounced change of cell numbers under both growth conditions tested was observed for the following four combinations of markers genes that are boxed: *C20orf24*/*RIP5* + *AURKA*/*STK6, AURKA*/*STK6* + *TPX2, C20orf24*/*RIP5* + *TPX2,* and *C20orf11* + *TPX2.*

Five candidate genes, i.e. *BCL2L1, AURKA*/*STK6, TPX2, C20orf59* and *RNPC2,* were found to significantly decrease cell viability in multiple CRC cell lines. These data indicate that multiple genes on the chromosome 20q amplicon affect CRC cell proliferation / survival, and may contribute to 20q gain driven progression of colorectal adenomas to carcinomas. Interestingly, *AURKA* which interacts with *TPX2* has already been reported to induce aneuploidy and cellular transformation.

In addition, the effects of candidate genes are currently being investigated on progression-associated processes like anchorage-independent growth (colony formation in soft agar), cell invasion (transwell invasion assay), and chromosomal instability (flow- and image-cytometry).

Fig. 6 depicts the effects of siRNA-mediated down-regulation of candidate gene expression (shown for *TCFL5* and *AURKA*/*STK6*) on anchorage-independent growth, as determined by using the soft agar colony formation assay. siRNA-transfected cells were allowed to grow in soft agarose (SeaPlaque Agarose purchased from Lonza Ltd., Basel, Switzerland) for 2-4 weeks. Shown is a photograph of two exemplary agar-plates. Digital images were taken of the soft agar dishes and analyzed using appropriate software. Fig. 7 depicts the results of a soft agar colony formation assay (anchorage-independent growth assay) in SW480 cells. The assay was performed as described in Fig.6. The *PLK1* siGENOME SMART-pool was used as a positive control. The results obtained show that the marker genes *AURKAlSTK6* and *TPX2* have the most prominent effect on anchorage-independent growth.

### Example 3: Identification of candidate genes for carcinogenic pathways

### 3.1 Materials and methods

### Microarray mRNA expression data of colorectal tumor samples

Genome-wide mRNA expression was determined by microarray analysis of a group of 37 colorectal adenomas and 31 adenocarcinomas, as described by Carvalho, B. et al. (2009), *supra.* The entire microarray expression dataset can be accessed using the Gene Expression Omnibus (GEO; http://www.ncbi.nlm.nih.gov/geo/) under accession number GSE8067. Genes with missing values in more than 20% of tumor samples were excluded from further analysis. Remaining missing values were imputed using k-nearest neighbor averaging, as implemented in the R-package 'impute' (Troyanskaya, O. et al. (2001) Bioinformatics 17, 520-525).

### Selection of gene-sets representing carcinogenic pathways

The pathway analysis tool GSEA makes use of pre-defined gene-sets. The present study aimed to examine which carcinogenic pathways might be relevant to adenoma-to-carcinoma progression, and therefore restricted GSEA analysis to carefully selected cancer-related gene-sets. The biological processes searched for comprised: proliferation, cell cycle, apoptosis, tumor differentiation, tumor cell invasion, metastasis, chromosomal instability, and micro-environmental changes such as hypoxia, immune response, angiogenesis, tumor-associated macrophages, and tumor stroma activation.

Firstly, Gene Ontology (GO) terms were used to obtain gene-sets from the NCBI Entrez Gene database. The following keywords yielded GO-derived gene-sets: proliferation, apoptosis, cell cycle, differentiation, hypoxia, angiogenesis and immune response. Secondly, gene-sets were collected whose coordinated expression in a carcinogenic process was based on experimental evidence, by searching the NCBI PubMed database. Cancer-related experiment-derived gene-sets included: proliferation (Whitfield, M.L. et al. (2006) Nat. Rev. Cancer 6, 99-106), tumor differentiation (Rhodes, D.R. et al. (2004) Proc. Natl. Acad. Sci. USA 101, 9309-9314), invasion (Jechlinger, M. et al. (2003) Oncogene 22, 7155-7169), metastasis (Li, D.Q. et al. (2006) Eur. J. Cancer 42, 3274-3286), chromosomal instability (Carter, S.L. et al. (2006) Nat. Genet. 38, 1043-1048), hypoxia (Greijer A.E. et al. (2005) J. Pathol. 206, 291-304), angiogenesis (Hu, J. et al. (2005) Oncogene 24, 1212-1219), tumor-associated macrophages (Biswas, S.K. et al. (2006) Blood 107, 2112-2122), and stroma activation (Chang, H.Y. et al. (2004) PLoS Biol. 2, e7). Together, seven GO-derived gene-sets and nine experiment-derived gene-sets were selected for carcinogenic pathway analysis by GSEA. The total number of genes within each gene-set, and the number of genes covered by the microarray expression data are listed in Table 6.

### GSEA pathway analysis of gene expression

Pathway analysis of gene expression was performed using the pathway analysis tool Gene Set Enrichment Analysis (GSEA; v2.0,
http://www.broad.mit.edu/gsea/) (Mootha, V.K. (2003), *supra*; Subramanian, A. et al. (2005), *supra*). GSEA performs a competitive analysis of pre-defined gene-sets, which is suited for examination of relatively heterogeneous biological samples (Goeman, J.J., and Buhlmann, P. (2007) Bioinformatics 23, 980-987).

In brief, GSEA first ranks all genes analyzed by expression arrays according to their differential expression between two categories of samples, in this case colorectal adenomas and carcinomas. Next, for each pre-defined gene-set to be analyzed, GSEA calculates a pathway enrichment score that estimates whether genes from these gene-sets are enriched among the highest- (or lowest-) ranked genes, or whether they are distributed randomly. Default settings were used except for the maximum size of gene-sets, which was set to 1500 to include all pre-defined gene-sets for analysis. Thresholds for significance were determined by permutation analysis (1000 permutations). False Discovery Rate (FDR) q-values <0.05 were considered significant.

### Identification of candidate biomarkers

Candidate biomarkers that may be indicative for carcinogenic pathway activity were identified by examination of expression differences at the single-gene level between colorectal adenomas and adenocarcinomas. For individual genes within carcinogenic pathways that contribute to the enrichment score, p-values were calculated using the student's t-test with unequal variance. P-values of < 1e-5 (uncorrected for multiple comparisons) were considered significant.

### Validation of candidate biomarkers by immunohistochemistry

Immunohistochemical staining of AURKA and PDGFRB were performed on 4 µm thick paraffin sections of a series of 10 colorectal adenomas and 12 adenocarcinomas. Sections were deparaffinized in xylene and rehydrated through a series of graded alcohol to water. Endogenous peroxidase was blocked with hydrogen peroxide (0.3 % H₂O₂ in methanol) for 30 min. Antigen retrieval was performed by autoclave heating in 10 mM citrate buffer (pH 6.0). Antibodies directed against AURKA (mouse monoclonal NCL-L-AK2, Novocastra Laboratories, Newcastle upon Tyne, UK) or PDGFRB (rabbit polyclonal 28E1, Cell Signaling Technology, Danvers, USA) were incubated overnight at 4°C (1:50 dilution).

AURKA staining was visualized using the Dako EnVision Kit-K4006 (Dako Cytomation, Copenhagen, Denmark), and PDGFRB staining using the PowerVision+ Kit (ImmunoLogic, Duiven, NL) according to the manufacturer's recommendations. Sections were counterstained with Mayer's hematoxylin. Incubation without primary antibody was used as negative control.

### 3.2 GSEA pathway analysis of colorectal adenomas and CRCs

The present study aimed to compare the activity of carcinogenic pathways in colorectal adenomas to adenocarcinomas. Genome-wide microarray expression profiles from 37 colorectal adenomas and 31 adenocarcinomas were subjected to GSEA pathway analysis, using a total of 16 pre-defined cancer-related gene-sets that represent different aspects of carcinogenesis. Six cancer-related gene-sets exhibited increased expression in CRCs compared to adenomas (FDR < 0.05), comprising proliferation, differentiation, angiogenesis, stroma activation, invasion and chromosomal instability (Table 6).

### 3.3 Identification of candidate biomarkers for malignant transformation

Having correlated increased expression of six carcinogenic pathways to colorectal adenoma-to-carcinoma progression, it was aimed to identify individual genes within these cancer-related gene-sets that may function as biomarkers indicative for malignant transformation. Using the expression data of 37 colorectal adenomas and 31 CRCs, individual genes from these six carcinogenic pathways were ranked according to their difference in mRNA expression between adenomas and CRCs. P-values < 1e-5 were considered significant at the genome-wide level. This procedure yielded candidate biomarkers for five out of six carcinogenic pathways: *PLK1* and *CCNF* (proliferation); *ADRM1* and *NUDT1* (differentiation); *SSSCA1, ID3, LUM, FYCO1, RFC3, LOAL2, SVIL, KIAA0367* and *NUDT1* (stroma activation); *SPARC, DCN* and *PDGFRB* (invasion); and *AURKA*/*STK6, C20orf24*/*RIP5, TPX2* (chromosomal instability). Only for the angiogenesis pathway did none of the individual genes reached the threshold for genome-wide significance. Table 7 lists all genes within the carcinogenic pathways that exhibited p-values < 1e-4.

The magnitude of differential expression between colorectal adenomas and adenocarcinomas for *PLK1, ADRM1, SSSCA1, SPARC, PDGFRB* and *AURKA*/*STK6* at the mRNA level is further illustrated by box plots (Figure 8).

**Table 6. GSEA comparison of carcinogenic pathway activity in colorectal adenomas and CRCs. Pathway analysis using GSEA was performed on a genome-wide microarray expression profile of 37 colorectal adenomas and 31 adenocarcinomas. Listed are the carcinogenic gene-sets analyzed, the number of genes covered by the microarray relative to the gene-set size¹ and the FDR q-value obtained by GSEA analysis. FDR q-value <0.05 (in bold) were considered significant.**

| **Carcinogenic pathway, gene ontology (GO)- or experiment size** **(Exp)-derived gene-set** | **Genes analyzed/ gene-set ¹** | **GSEA** **FDR q-value** |
|---|---|---|
| Proliferation (GO) | 639 / 680 | 0.25 |
| Proliferation (Exp) | 41 / 45 | **0.04** |
| Apoptosis (GO) | 549 / 584 | 0.51 |
| Cell cycle (GO) | 669 / 722 | 0.05 |
| Differentiation (GO) | 611 / 667 | 0.25 |
| Differentiation (Exp) | 66 / 69 | **0.04** |
| Hypoxia (GO) | 18 / 21 | 0.13 |
| Hypoxia (Exp) | 80 / 85 | 0.22 |
| Angiogenesis (GO) | 95 / 100 | **0.04** |
| Angiogenesis (Exp) | 55 / 61 | 0.85 |
| Immune response (GO) | 468 / 565 | 0.30 |
| Tumor-associated macrophages (Exp) | 56 / 59 | 0.06 |
| Stroma activation (Exp) | 258 / 276 | **0.03** |
| Invasion (Exp) | 93 / 97 | **0.03** |
| Metastasis (Exp) | 56 / 59 | 0.28 |
| Chromosomal instability (Exp) | 66 / 71 | **< 0.01** |

| | | |
|---|---|---|
| ¹ Some genes could not be analyzed, either due to technical problems (missing values in more than 20% of the samples) or because they were not represented on the microarray. | | |

**Table 7. Candidate biomarkers for carcinogenic pathway activity in colorectal adenoma-to-carcinoma progression. Within the six differentially expressed carcinogenic pathways, genes that contribute to the enrichment score were ranked based on the difference in mRNA expression between adenomas and CRCs (p-values). Genes with a p-value < 1e-5 were considered significant at the genome-wide level and selected as candidate biomarkers (in bold). Listed are the genes that exhibit p-values of < 3e-5.**

| **Carcinogenic pathway, gene ontology (GO)- or experiment** **(Exp)-derived gene-set** | **Gene symbol** | **p-value** **(student's t-test with unequal variance)** |
|---|---|---|
| Proliferation (Exp) | ***PLK1*** | **5.93 e-06** |
| Proliferation | ***CCNF*** | **7.46 e-06** |
| Proliferation | *DNMT1* | 1.81 e-05 |
| | | |
| Differentiation (Exp) | ***ADRM1*** | 8.42 e-07 |
| Differentiation | ***NUDT1*** | 9.57 e-06 |
| Differentiation | *CENPA* | 1.05 e-05 |
| Differentiation | *TRIP13* | 1.32 e-05 |
| Differentiation | *UBE2C* | 2.55 e-05 |
| | | |
| Angiogenesis (GO) | *EREG* | 1.28 e-05 |
| Angiogenesis | *COL15A1* | 1.75 e-05 |
| | | |
| Stroma activation (Exp) | ***SSSCA1*** | 9.25 e-09 |
| Stroma activation | ***ID3*** | 1.09 e-08 |
| Stroma activation | ***LUM*** | 5.69 e-08 |
| Stroma activation | ***FYCO1*** | 2.63 e-07 |
| Stroma activation | ***RFC3*** | 6.27 e-07 |
| Stroma activation | ***LOXL2*** | 6.80 e-07 |
| Stroma activation | ***SVIL*** | 1.50 e-06 |
| Stroma activation | ***KIAA0367*** | 6.37 e-06 |
| Stroma activation | ***NUDT1*** | 9.57 e-06 |
| | | |
| Invasion (Exp) | ***SPARC*** | 7.82 e-08 |
| Invasion | ***DCN*** | 8.04 e-07 |
| Invasion | ***PDGFRB*** | 3.96 e-06 |
| | | |
| Chromosomal instability (Exp) | ***AURKA*** | 3.58 e-09 |
| Chromosomal instability | ***C20orf24*** | 1.15 e-07 |
| Chromosomal instability | ***TPX2*** | 2.60 e-06 |
| Chromosomal instability | *TRIP13* | 1.32 e-05 |
| Chromosomal instability | *ATAD2* | 1.80 e-05 |
| Chromosomal instability | *CDCA3* | 1.88 e-05 |
| Chromosomal instability | *UBE2C* | 2.55 e-05 |

The nucleic acid sequences (encoding the corresponding mRNAs) of the genes in the above Table 7 that are not shown in bold represent known human sequences that have been deposited in GenBank, the NIH genetic sequence database (Nucl. Acids Res. (2008) 36, D25-D30; http://www.ncbi.nlm.nih.gov/Genbank/; release 169.0), under the following accession numbers:

| | |
|---|---|
| *DNMT1* | (GenBank acc. no. NM_001379.2) (SEQ ID NO:51) |
| *CENPA* | (GenBank acc. no. NM_001809.3) (SEQ ID NO:52) |
| *TRIP13* | (GenBank acc. no. NM_004237.2) (SEQ ID NO:53) |
| *UBE2C* | (GenBank acc. no. NM_007019.2) (SEQ ID NO:54) |
| *EREG* | (GenBank acc. no. NM_001432.2) (SEQ ID NO:55) |
| *COL15A1* | (GenBank acc. no. NM_001855.3) (SEQ ID NO:56) |
| *FYCO1* | (GenBank acc. no. NM_024513.2) (SEQ ID NO:57) |
| *RFC3* | (GenBank acc. no. NM_002915.3) (SEQ ID NO:58) |
| *LOXL2* | (GenBank acc. no. NM_002318.2) (SEQ ID NO:59) |
| *SVIL* | (GenBank acc. no. NM_003174.3) (SEQ ID NO:60) |
| *ATAD2* | (GenBank acc. no. NM_014109.3) (SEQ ID NO:61) |
| *CDCA3* | (GenBank acc. no. NM_031299.4) (SEQ ID NO:62) |

### 3.4 Validation of AURKA/STK6 and PDGFRB candidate biomarkers

To validate mRNA expression data at the protein level, immunohistochemical stainings were performed for *AURKA*/*STK6* (chromosomal instability pathway) and *PDGFRB* (invasion pathway) at a series of 10 colorectal adenomas and 12 adenocarcinomas.

For AURKA/STK6 little staining was observed in adenomas, while epithelial cells in CRCs clearly stained positive. Also for PDGFRB, staining was nearly absent in the adenomas while protein expression was observed in CRCs, in particular in the non-epithelial stromal compartment. Representative images for AURKA/STK6 and PDGFRB staining are provided in Figure 9.

### 3.5 Evaluation of data and discussion

Colorectal adenoma formation and further progression into carcinomas is caused by accumulation of (epi)genetic alterations. As such, one might expect colorectal carcinogenesis to be a gradual process in which, sooner or later, malignant progression is an inevitable event. However, biologically colorectal adenomas form a distinct, intermediate stage in CRC development from normal colon epithelium. It is estimated that only 5% of adenomas ever progress into adenocarcinomas, indicating that carcinoma formation requires significantly different biological and molecular alterations than those involved in adenoma formation. To identify candidate biomarkers for malignant transformation, the present study specifically addressed which carcinogenic pathways are altered during colorectal adenoma-to-carcinoma progression.

The present study results in the identification of six carcinogenic pathways whose activity is increased in CRCs compared to adenomas (Table 6), and a list of candidate biomarkers whose increased mRNA expression levels are associated with malignant transformation (Table 7). For two of these genes, i.e. *AURKA*/*STK6* and *PDGFRB,* differential expression was validated at the protein level (Figure 9).

The GSEA carcinogenic pathway analysis performed in the present study was restricted to a limited number of 16 carefully selected cancer-related gene-sets for two reasons. First, although databases that contain numerous pre-defined gene-sets are available, such as the Molecular Signatures Database
(www.broad.mit.edu/gsea/msigdb), none of them contain a well-defined subset of gene-sets representing various biological aspects of carcinogenesis. Second, it is not recommended to perform GSEA using large groups of gene-sets that are not relevant to the research question addressed, as this will increase the multiple comparison problem and lead to unnecessary decrease of statistical power.

Therefore, gene-sets were selected that represent carcinogenic pathways using two strategies, one based on Gene Ontology terms (7 gene-sets) and one based on PubMed literature search of *in vitro* and *in vivo* experimental data (9 gene-sets). For four cancer-related processes (proliferation, differentiation, hypoxia and angiogenesis) gene-sets were obtained using both strategies, allowing a comparison of their value for GSEA carcinogenic pathway analysis.

The experiment-derived 'proliferation' and 'differentiation' gene-sets yielded a significant difference between adenomas and CRCs while their GO-derived equivalents did not. In contrast, the GO-derived 'angiogenesis' gene-set yielded a significant difference while the experiment-derived gene-set did not. No significant differences were observed at all for the experiment- and GO-derived 'hypoxia' pathways. These data illustrate that both strategies revealed useful gene-sets for GSEA carcinogenic pathway analysis. However, they also imply that optimal gene-sets may not be available yet for all (colorectal) carcinogenic pathways, raising the question of how valuable the 16 pre-selected cancer-related gene-sets are compared to a large group of non-selected gene-sets.

To put our findings into perspective, GSEA analysis was performed using the curated gene-sets (c2) of the Molecular Signatures Database (MSigDB v2.5) combined with the 16 carcinogenic pathways selected for this study. When results were ranked according to their GSEA normalized enrichment scores, the six pre-selected gene-sets that were differentially expressed between adenomas and CRCs (Table 6) all ranked among the top 100 out of 1439 curated gene-sets (data not shown). This data confirms that the pre-selected carcinogenic pathways are indeed enriched for gene-sets that were relevant to our research question.

The GSEA carcinogenic pathway analysis results indicated significantly different rates of proliferation, differentiation, angiogenesis, stroma activation, invasion and chromosomal instability between colorectal adenomas and CRCs (Table 6). These results fit current knowledge about malignant transformation. Adenomas do not exhibit excessive cellular proliferation, probably because proliferative stress drives them into senescence and protects them from cancer development (Collado, M. et al. (2007) Cell 130, 223-233). However, when senescence is overcome, proliferation rates increase while differentiation rates decrease (Nakamura, T. et al. (2007) J. Gastroenterol. 42, 705-710). Chromosomal instability increases the rate of genomic mutations, necessary to bypass the rate-limiting steps in carcinogenesis (Loeb, L.A. (2001) Cancer Res. 61, 3230-3239). Analysis of chromosome copy number changes by comparative genomic hybridization has demonstrated that CRCs exhibit much more chromosomal instability than adenomas (Ried, T. et al. (1996) Genes Chrom. Cancer 15, 234-245; Hermsen, M. et al. (2002), *supra*; Carvalho, B. et al. (2009), *supra*).

The present data further emphasizes the importance of chromosomal instability in colorectal tumor progression. Angiogenesis is induced by growing tumors in an attempt to meet their increasing demand for oxygen and nutrients. Microvessel density, a widely used surrogate marker for angiogenesis, has been shown to be increased in CRCs compared to colorectal adenomas (Kondo, Y. et al. (2000) Cancer 88, 1820-1827). In comparison to adenomas, CRCs also contain much more tumor stroma, which is often composed of reactive tissue that resembles wounds that do not heal. Interestingly, the amount of stroma differs widely among CRCs (Fijneman, R.J. et al. (2007) Cancer Lett. 258, 223-229) and a high stroma percentage has been correlated with poor prognosis in CRC patients (Mesker, W.E. et al. (2007) Cell. Oncol. 29, 387-398). Invasion by tumor cells of the *muscularis mucosae*, per definition, does not occur in adenomas. Hence, increased expression of the invasion gene-set by CRCs obviously fits the concept of adenoma-to-carcinoma progression.

For several cancer-related pathways no significant differences were revealed between adenomas and CRCs, i.e. for gene-sets representing apoptosis, cell cycle, hypoxia, immune response, tumor-associated macrophages and metastasis. One interpretation is that these biological processes are more relevant during formation of colorectal adenomas from normal colon epithelium, than during adenoma-to-carcinoma progression.

Alternatively, although these biological processes could play a role in malignant transformation, the selected gene-sets may not adequately represent the *in vivo* situation analyzed here. For instance, GO-derived gene-sets are composed of groups of genes known to be involved in similar biological processes, irrespective of whether they actually function in a coordinated manner or not. In contrast, experiment-derived gene-sets are composed of groups of genes that are coordinately expressed during certain biological processes, however, assumptions have been made about conservation of these gene-sets across species, across tumor-types, and about the validity of extrapolation from *in vitro* to *in vivo* settings. Nevertheless, although the present approach may underestimate the effects of some carcinogenic pathways in adenoma-to-carcinoma progression, the positively identified gene-sets yield valuable information for further investigation, such as identification of candidate biomarkers for malignant transformation.

Expression of individual genes within gene-sets that were positively identified by GSEA yielded a list of candidate biomarkers for various carcinogenic pathways that may be used for molecular characterization of tumor samples (Table 7). Some of these genes have been described to contribute to (colorectal) carcinogenesis.

From the 'proliferation' gene-set, *PLK1* (polo-like kinase 1) is thought to play a role in spindle formation (Goto, H. et al. (2006) Nat. Cell Biol. 8, 180-187) and is involved in stimulation of G₂ to M phase transition (Ree, A.H. et al. (2003) Oncogene 22, 8952-8955). Interference with *PLK1* expression decreases proliferation, induces apoptosis and affects spindle assembly *in vitro* (Spannkuch-Schmitt, B. et al. (2002) J. Natl. Caner Inst. 94, 1863-1877). Moreover, down-modulation of *PLK1* expression was found to inhibit growth of bladder cancer in mice (Nogawa, M. et al. (2005) J. Clin. Invest. 115, 978-985). The expression of *PLK1* and *CCNF* (cyclin F), which also contributes to the G₂ to M phase transition, have both been related to treatment efficacy (Lyng, H. et al. (2005) Int. J. Cancer 115, 935-942).

From the 'chromosomal instability' gene-set, *AURKA*/*STK6* (aurora kinase A) and *TPX2* (targeting protein for XKLP2) have been reported to interact with each other, and to play a role in centrosome maturation and spindle formation (De Luca, M. et al. (2006) Cell Cycle 5, 296-303) Aberrant expression of *TPX2* has been reported in breast, endometrial and lung cancer and in neuroblastoma. Furthermore, TPX2 over-expression at the protein level was found to be associated with poor prognosis in lung cancer (Ma, Y. et al. (2006) Clin. Cancer Res. 12, 1121-1127). *AURKA*/*STK6,* when over-expressed, induces centrosome amplification, aneuploidy, and cellular transformation *in vitro* (Zhou, H. et al. (1998) Nat. Genet. 20, 189-193). In nasopharyngeal tissues *AURKAlSTK6* over-expression was correlated with clinical stage and invasiveness, and inhibition with small molecules or RNA interference reduced cell invasion *in vitro* (Wan, X.B. et al. (2008) Carcinogenesis 29, 1930-1937).

From the 'invasion' gene-set, *SPARC* (secreted protein acidic and rich in cysteine, also known as osteonectin) is over-expressed in CRCs and induces PAR-1 pro-invasive activity Nguyen, Q.D. et al. (2005) Oncogene 24, 8240-8251). *PDGFRB* (platelet-derived growth factor receptor beta) is up-regulated within CRC tumor stroma, and blocking of PDGFRB signaling has been shown to inhibit colon tumor growth and metastasis (Kitadai, Y. et al. (2006) Am. J. Pathol. 169, 2054-2065).

Immunohistochemical analysis of a series of colorectal adenomas and adenocarcinomas was used to validate some of the candidate biomarkers at the protein level, i.e. *AURKA*/*STK6* and *PDGFRB* (Figure 9). For both proteins, little to no staining was observed in adenoma tissue, while abundant staining was present in carcinomas. For AURKA/ STK6, this staining was restricted to epithelial colon cancer cells, while PDGFRB staining was predominantly observed within tumor stroma. Therefore, both AURKA/ STK6 and PDGFRB may be used as diagnostic biomarkers indicative for 'chromosomal instability' and 'invasion', respectively. Moreover, because pharmaceutical inhibitors have been developed for both proteins and are currently being tested in clinical trials, staining for AURKA/STK6 and PDGFRB may also have therapeutic implications in selecting patients that may respond well to these anti-cancer drugs.

In summary, GSEA was applied as a tool for pathway analysis of gene expression using a restricted number of gene-sets representing cancer-related biological processes. Expression of six gene-sets was increased in CRCs compared to adenomas, and individual genes within these gene-sets that exhibited significant differential expression were identified. These genes can serve as (a panel of) biomarkers indicative for various aspects of carcinogenesis, which may be applied in a diagnostic setting to improve molecular characterization of tumor samples, an essential step towards personalized medicine.

The present invention illustratively described herein may suitably be practiced in the absence of any element or elements, limitation or limitations, not specifically disclosed herein. Thus, for example, the terms "comprising", "including", "containing", etc. shall be read expansively and without limitation.

Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by embodiments and optional features, modifications and variations of the inventions embodied therein may be resorted to by those skilled in the art, and that such modifications and variations are considered to be within the scope of this invention.

The invention has been described broadly and generically herein. Each of the narrower species and sub-generic groupings falling within the generic disclosure also form part of the invention. This includes the generic description of the invention with a proviso or negative limitation removing any subject matter from the genus, regardless of whether or not the excised material is specifically recited herein.

Other embodiments are within the following claims. In addition, where features or aspects of the invention are described in terms of Markush groups, those skilled in the art will recognize that the invention is also thereby described in terms of any individual member or subgroup of members of the Markush group.

## Claims

1. *In vitro* method for preventing and/or treating an adenocarcinoma associated with a chromosomal aberration on chromosome 20q in a subject, the method comprising:
(a) identifying in one or more target cells of the subject at least one marker gene whose expression level(s) is/are elevated as compared to one or more control cells, wherein an elevated expression level of any one of the at least one marker gene in the one or more target cells is indicative of an adenocarcinoma associated with a chromosomal aberration on chromosome 20q in the subject; and
(b) modifying in the one or more target cells the gene expression of the at least one marker gene in such way that the elevated expression level(s) identified in step (a) are down-regulated,
wherein the at least one marker gene is selected from the group consisting of *RNPC1* (GenBank acc. no. NM_017495.4; SEQ ID NO:1), *TCFL5* (GenBank acc. no. NM_006602.2; SEQ ID NO:2), *C20orf24*/*RIP5* (GenBank acc. no. NM_018840.2; SEQ ID NO:3), *AURKA*/*STK6* (GenBank acc. no. NM_003600.2; SEQ ID NO:4), *C20orf20* (GenBank acc. no. NM_018270.4; SEQ ID NO:5), *ADRM1* (GenBank acc. no. NM_007002.2; SEQ ID NO:6), *TH1L* (GenBank acc. no. NM_198976.1; SEQ ID NO:7), *TGIF2* (GenBank acc. no. NM_021809.5; SEQ ID NO:8), *DPM1* (GenBank acc. no. NM_003859.1; SEQ ID NO:9), *TOP1* (GenBank acc. no. NM_003286.2; SEQ ID NO:10), *C20orf11* (GenBank acc. no. NM_017896.2; SEQ ID NO:11), *YWHAB* (GenBank acc. no. NM_003404.3; SEQ ID NO:12), *ZNF217* (GenBank acc. no. NM_006526.2; SEQ ID NO: 13), *BCL2L1* (GenBank acc. no. NM_001191.2; SEQ ID NO:14), *DATFI*/*DIDO1* (GenBank acc. no. NM_022105.3; SEQ ID NO:15), *CSE1L* (GenBank acc. no. NM_001316.2; SEQ ID NO:16), *C20orf126*/*PDRG1* (GenBank acc. no. NM_030815.2; SEQ ID NO:17), *TPX2* (GenBank acc. no. NM_012112.4; SEQ ID NO:18), *C20orf59* (GenBank acc. no. NM_022082.3; SEQ ID NO:19), *UBE2C* (GenBank acc. no. NM_007019.2; SEQ ID NO:20), *NCOA3* (GenBank acc. no. NM_006534.2; SEQ ID NO:21), *C20orf14*/*PRPF6* (GenBank acc. no. NM_012469.3; SEQ ID NO:22), *C20orf44*/*bFZb* (GenBank acc. no. NM_018244.3; SEQ ID NO: 23), *RNPC2* (GenBank acc. no. NM_004902.2; SEQ ID NO: 24), *HM13* (GenBank acc. no. NM_030789.2; SEQ ID NO:25), *SLCO4A1* (GenBank acc. no. NM_016354.3; SEQ ID NO:26), *VAPB* (GenBank acc. no. NM_004738.3; SEQ ID NO:27), *KIAA1847* (GenBank acc. no. NM_032527.3; SEQ ID NO:28), *CDC91L1* (GenBank acc. no. NM_080476.4; SEQ ID NO:29), *PXMP4* (GenBank acc. no. NM_007238.4; SEQ ID NO:30), *DLGAP4* (GenBank acc. no. NM_014902.3; SEQ ID NO:31), *PFDN4* (GenBank acc. no. NM_002623.3; SEQ ID NO:32), *E2F1* (GenBank acc. no. NM_005225.2; SEQ ID NO:33), *CYP24A1* (GenBank acc. no. NM_000782.4; SEQ ID NO:34), *SRC* (GenBank acc. no. NM_005417.3; SEQ ID NO:35), *BCAS4* (GenBank acc. no. NM_017843.3; SEQ ID NO: 36), *PLK1* (GenBank acc. no. NM_005030.3; SEQ ID NO:37), *CCNF* (GenBank acc. no. NM_001761.2; SEQ ID NO:38), *NUDT1* (GenBank acc. no. NM_002452.3; SEQ ID NO:39), *SSSCA1* (GenBank acc. no. NM_006396.1; SEQ ID NO:40), *ID3* (GenBank acc. no. NM_002167.3; SEQ ID NO:41), *LUM* (GenBank acc. no. NM_002345.3; SEQ ID NO:42), *PDGFRB* (GenBank acc. no. NM_002609.3; SEQ ID NO:43), *SPARC* (GenBank acc. no. NM_003118.2; SEQ ID NO:44), and *DCN* (GenBank acc. no. NM_001920.3; SED ID NO:45).

2. *In vitro* method for diagnosing in a subject an adenocarcinoma associated with a chromosomal aberration on chromosome 20q, the method comprising:
(a) detecting in one or more target cells of the subject the expression level(s) of at least one marker gene; and
(b) comparing the expression level(s) obtained in step (a) to that/those obtained in one or more control cells,
wherein an elevated expression level of any one of the at least one marker gene in the one or more target cells as compared to the one or more control cells is indicative of an adenocarcinoma associated with a chromosomal aberration on chromosome 20q in the subject; and
wherein the at least one marker gene is selected from the group of marker genes as defined in claim 1.

3. The method of claim 1 or 2, for the further use of diagnosing or of preventing and/or treating a predisposition for developing an adenocarcinoma, a progression of an adenoma to an adenocarcinoma or a predisposition for a progression of an adenoma to an adenocarcinoma, the adenocarcinoma being associated with a chromosomal aberration on chromosome 20q.

4. The method of any of claims 1 to 3, wherein the chromosomal aberration on chromosome 20q is a chromosomal gain, preferably at position 20q11.22-20q11.23 and/or at position 20q13.31-20q13.33.

5. The method of any of claims 1 to 4, wherein step (a) comprises the determination of the expression level(s) of the at least one marker gene by any one or more of the methods selected from the group consisting of:
(i) detecting a mRNA encoded by the marker gene(s);
(ii) detecting a protein encoded by the marker gene(s); and
(iii) detecting a biological activity of a protein encoded by the marker gene(s).

6. The method of any of claims 2 to 5, wherein step (b) comprises introducing into the one or more target cells:
(i) one or more nucleic acid molecules, each nucleic acid molecule encoding a sequence that is complementary to at least a part of the nucleic acid sequence of any one of the at least one marker gene to be down-regulated, wherein the one or more nucleic acid molecules are preferably selected from the group consisting of an anti-sense nucleic acid construct, a siRNA, a miRNA, and a ribozyme; and/or
(ii) one or more antibodies, each antibody directed against any polypeptide encoded by any one of the at least one marker gene to be down-regulated, or one or more dominant-negative polypeptide variants of any polypeptide encoded by any one of the at least one marker gene to be down-regulated.

7. The method of any of claims 1 to 6, wherein step (a) comprises the detection or identification of at least the marker genes *RNPC1* (GenBank acc. no. NM_017495.4; SEQ ID NO:1) and *TCFL5* (GenBank acc. no. NM_006602.2; SEQ ID NO:2).

8. The method of claim 7, wherein step (a) further comprises the detection or identification of any one or more of the marker genes *C20orf24*/*RIP5* (GenBank acc. no. NM_018840.2; SEQ ID NO:3), *AURKA*/*STK6* (GenBank acc. no. NM_003600.2; SEQ ID NO:4), *C20orf20* (GenBank acc. no. NM_018270.4; SEQ ID NO:5), *ADRM1* (GenBank acc. no. NM_007002.2; SEQ ID NO:6), and *TH1L* (GenBank acc. no. NM_198976.1; SEQ ID NO:7).

9. The method of any of claims 1 to 8, wherein step (a) comprises the detection or identification of at least the marker genes *AURKA*/*STK6* (GenBank acc. no. NM_003600.2; SEQ ID NO:4) and *TPX2* (GenBank acc. no. NM_012112.4; SEQ ID NO:18).

10. The method of claim 9, wherein step (a) further comprises the detection or identification of any one or more of the marker genes *BCL2L1* (GenBank acc. no. NM_001191.2; SEQ ID NO:14), *C20orf59* (GenBank acc. no. NM_02208.32; SEQ ID NO:19), and *RNPC2* (GenBank acc. no. NM_004902.2; SEQ ID NO:24).

11. The method of any of claims 1 to 10, wherein step (a) comprises the detection or identification of at least:
(i) any one or both of the marker genes *PLK1* (GenBank acc. no. NM_005030.3; SEQ ID NO:37) and *CCNF* (GenBank acc. no. NM_001761.2; SEQ ID NO:38); and/or
(ii) any one or both of the marker genes *ADRM1* (GenBank acc. no. NM_007002.2; SEQ ID NO:6) and *NUDT1* (GenBank acc. no. NM_002452.3; SEQ ID NO:39); and/or
(iii) any one or more of the marker genes *SSSCA1* (GenBank acc. no. NM_006396.1; SEQ ID NO:40), *ID3* (GenBank acc. no. NM_002167.3; SEQ ID NO:41), and *LUM* (GenBank acc. no. NM_002345.3; SEQ ID NO:42); and/or
(iv) any one or more of the marker genes *PDGFRB* (GenBank acc. no. NM_002609.3; SEQ ID NO:43), *SPARC* (GenBank acc. no. NM_003118.2; SEQ ID NO:44), and *DCN* (GenBank acc. no. NM_001920.3; SEQ ID NO:45); and/or
(v) any one or more of the marker genes *AURKA*/*STK6* (GenBank acc. no. NM_003600.2; SEQ ID NO:4), *TPX2* (GenBank acc. no. NM_012112.4; SEQ ID NO:18), and *C20orf241RIP5* (GenBank acc. no. NM_018840.2; SEQ ID NO:3).

12. Pharmaceutical composition for preventing and/or treating an adenocarcinoma associated with a chromosomal aberration on chromosome 20q, comprising one or more agents, as defined in claim 6, for down-regulating gene expression of at least one marker gene, wherein the at least one marker gene is selected from the group consisting of *RNPC1* (GenBank acc. no. NM_017495.4; SEQ ID NO:1), *TCFL5* (GenBank acc. no. NM_006602.2; SEQ ID NO:2), *C20orf24*/*RIP5* (GenBank acc. no. NM_018840.2; SEQ ID NO:3), *AURKA*/*STK6* (GenBank acc. no. NM_003600.2; SEQ ID NO:4), *C20orf20* (GenBank acc. no. NM_018270.4; SEQ ID NO:5), *ADRM1* (GenBank acc. no. NM_007002.2; SEQ ID NO:6), *TH1L* (GenBank acc. no. NM_198976.1; SEQ ID NO:7), *TGIF2* (GenBank acc. no. NM_021809.5; SEQ ID NO:8), *DPM1* (GenBank acc. no. NM_003859.1; SEQ ID NO:9), *TOP1* (GenBank acc. no. NM_003286.2; SEQ ID NO:10), *C20orf11* (GenBank acc. no. NM_017896.2; SEQ ID NO:11), *YWHAB* (GenBank acc. no. NM_003404.3; SEQ ID NO:12), *ZNF217* (GenBank acc. no. NM_006526.2; SEQ ID NO: 13), *BCL2L1* (GenBank acc. no. NM_001191.2; SEQ ID NO:14), *DATF1*/*DIDO1* (GenBank acc. no. NM_022105.3; SEQ ID NO:15), *CSE1L* (GenBank acc. no. NM_001316.2; SEQ ID NO:16), *C20orf126*/*PDRG1* (GenBank acc. no. NM_030815.2; SEQ ID NO:17), *TPX2* (GenBank acc. no. NM_012112.4; SEQ ID NO:18), *C20orf59* (GenBank acc. no. NM_022082.3; SEQ ID NO:19), *UBE2C* (GenBank acc. no. NM_007019.2; SEQ ID NO:20), *NCOA3* (GenBank acc. no. NM_006534.2; SEQ ID NO:21), *C20orf14*/*PRPF6* (GenBank acc. no. NM_012469.3; SEQ ID NO:22), *C20orf44*/*bFZb* (GenBank acc. no. NM_018244.3; SEQ ID NO: 23), *RNPC2* (GenBank acc. no. NM_004902.2; SEQ ID NO: 24), *HM13* (GenBank acc. no. NM_030789.2; SEQ ID NO:25), *SLCO4A1* (GenBank acc. no. NM_016354.3; SEQ ID NO:26), *VAPB* (GenBank acc. no. NM_004738.3; SEQ ID NO:27), *KIAA1847* (GenBank acc. no. NM_032527.3; SEQ ID NO:28), *CDC91L1* (GenBank acc. no. NM_080476.4; SEQ ID NO:29), *PXMP4* (GenBank acc. no. NM_007238.4; SEQ ID NO:30), *DLGAP4* (GenBank acc. no. NM_014902.3; SEQ ID NO:31), *PFDN4* (GenBank acc. no. NM_002623.3; SEQ ID NO:32), *E2F1* (GenBank acc. no. NM_005225.2; SEQ ID NO:33), *CYP24A1* (GenBank acc. no. NM_000782.4; SEQ ID NO:34), *SRC* (GenBank acc. no. NM_005417.3; SEQ ID NO:35), *BCAS4* (GenBank acc. no. NM_017843.3; SEQ ID NO: 36), *PLK1* (GenBank acc. no. NM_005030.3; SEQ ID NO:37), *CCNF* (GenBank acc. no. NM_001761.2; SEQ ID NO:38), *NUDT1* (GenBank acc. no. NM_002452.3; SEQ ID NO:39), *SSSCA1* (GenBank acc. no. NM_006396.1; SEQ ID NO:40), *ID3* (GenBank acc. no. NM_002167.3; SEQ ID NO:41), *LUM* (GenBank acc. no. NM_002345.3; SEQ ID NO:42), *PDGFRB* (GenBank acc. no. NM_002609.3; SEQ ID NO:43), *SPARC* (GenBank acc. no. NM_003118.2; SEQ ID NO:44), and *DCN* (GenBank acc. no. NM_001920.3; SED ID NO:45).

13. The pharmaceutical composition of claim 12, for the further use of preventing and/or treating a predisposition for developing an adenocarcinoma, a progression of an adenoma to an adenocarcinoma or a predisposition for a progression of an adenoma to an adenocarcinoma, the adenocarcinoma being associated with a chromosomal aberration on chromosome 20q.

14. The pharmaceutical composition of claim 12 and 13, wherein the chromosomal aberration is a chromosomal gain, preferably at position 20q11.22-20q11.23 and/or at position 20q13.31-20q13.33.

15. Use of one or more agents, as defined in claim 6, for down-regulating gene expression of at least one marker gene for the manufacture of a medicament for the prevention and/or treatment of an adenocarcinoma associated with a chromosomal aberration on chromosome 20q.
